(19) Europäisches Patentamt / European Patent Office / Office européen des brevets

(11) **EP 4 614 153 A2**

(12) **EUROPEAN PATENT APPLICATION**

(43) Date of publication:
**10.09.2025 Bulletin 2025/37**

(21) Application number: **25190997.4**

(22) Date of filing: **07.03.2018**

(51) International Patent Classification (IPC):
*G01N 33/66* $^{(2006.01)}$

(52) Cooperative Patent Classification (CPC):
**G01N 33/66; G01N 33/57407; G01N 33/57488;**
G01N 2800/52

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.03.2017 GB 201703640**
**07.03.2017 GB 201703641**

(62) Document number(s) of the earlier application(s) in
accordance with Art. 76 EPC:
**18710027.6 / 3 593 139**

(71) Applicant: **ELYPTA AB**
**171 65 Solna (SE)**

(72) Inventors:
• **GATTO, Francesco**
**36100 Vicenza (IT)**
• **NIELSEN, Jens**
**41128 Gothenburg (SE)**

(74) Representative: **Dehns**
**10 Old Bailey**
**London EC4M 7NG (GB)**

Remarks:
This application was filed on 22-07-2025 as a
divisional application to the application mentioned
under INID code 62.

(54) **CANCER BIOMARKERS**

(57) The present invention relates to a method of screening for a cancer selected from the group consisting of prostate cancer and bladder cancer in a subject, said method comprising determining the chemical composition of one or more of the glycosaminoglycans (GAGs) chondroitin sulfate (CS) and heparan sulfate (HS) in a urine sample, wherein said urine sample has been obtained from said subject.

**Description**

The present invention relates to biomarkers for cancer and to methods of screening for cancer. Such methods involve determining the level and/or composition of certain biomarkers which are indicative of cancer in a subject.

The number of cancer cases is predicted to increase substantially in the near future. The rising cancer population determines an urgent need to improve the current diagnostics landscape for cancer. In particular, affordable and practical tools for cancer diagnostics are needed to assist healthcare professionals in the early detection of high risk cancer, which typically correlates with more favorable clinical outcomes, or to guide treatment of current cancer patients.

Circulating biomarkers are molecules that can be measured in accessible body fluids of individuals, e.g. blood or urine, and whose levels are useful to assist in the diagnosis and/or prognosis and/or prediction of response to treatment. An example of a widely used biomarker is the prostate-specific antigen (PSA) for prostate cancer, the carcinoembryonic antigen (CEA) for colorectal cancer and the carbohydrate antigen 125 for ovarian cancer. However, the clinical value of these biomarkers for diagnosing cancer is highly debated. For example, a standard PSA test to detect prostate adenocarcinoma in men over 50 years old at average risk, assuming a cut-off value equal to 4 ng/ml, has typical values for sensitivity and specificity equal to 21% (51% for high grade lesions with Gleason score greater or equal to 8) and 91%, respectively (Wolf et al., 2010).

What is needed in the art are new methods of screening for cancer (e.g. diagnosing cancer). The identification of novel biomarkers for cancer may potentially have clinical implications for a large number of patients and would be an important clinical advancement. Here, the inventors provide evidence for a blood and/or urine marker of cancer which can be used in a highly specific and sensitive assay to detect cancer. Advantageously therefore, such methods are non-invasive and performed on readily obtainable samples, as well as being highly accurate. The inventors have also observed that expression levels of certain genes are indicative of cancer.

The availability of such tests also has value for a number of medical decisions, for example to determine the risk of progression in newly diagnosed cancers; to guide treatment options in cancer patients with uncertain clinical risk; to monitor cancer before and after surgery or drug treatment; to rule out the relapse of the disease during a longer period of time after which a patient is typically declared cured; to assess the occurrence of cancer in a population at risk, such as genetically predisposed individuals or individuals presenting risk factors or individuals presenting symptoms; to ascertain whether a metastasis is due to a particular cancer; to predict recurrence or relapse in patients with early stage cancer; to distinguish lesions suspicious of cancer from non-malignant diseases; or to screen for cancer in the general population.

In this regard, the present inventors have identified that certain glycosaminoglycans (GAGs), i.e. chondroitin sulfate (CS), heparan sulfate (HS) and hyaluronic acid (HA), and chemical compositions of said GAGs, are found at differential levels in body fluid samples from cancer patients in comparison to control subjects. These differential levels of the GAGs CS or HS or HA, or differential chemical compositions of the GAGs CS or HS or HA (GAG profiles), can act as biomarkers for cancer and thus are useful in screening for cancer in subjects. The present inventors have thus determined that GAG profiles from accessible fluids are suitable to be used as biomarker/diagnostic marker of cancer.

Surprisingly and advantageously, the present inventors have found that changes in the level of the GAGs CS and HS and HA are observed in accessible body fluids of cancer patients and that these GAG profiles are suitable to be used as a biomarker of cancer. The present inventors have also shown that in addition to the overall (total) levels or concentration of CS and HS and HA, other changes in the chemical composition, for example the specific disaccharide sulfation patterns of CS and HS are also observed between cancer samples and normal samples and can be used very effectively to diagnose cancer.

Clearly the finding that cancer diagnosis can be carried out in an accessible body fluid sample, e.g. blood or urine, from a subject is extremely advantageous. Here, the inventors have observed a systemic alteration of GAG composition that was concomitant with cancer.

Advantageously the present inventors have also shown that the identified markers that are distinctive of occurrence of cancer and that are calculated based on measurements in accessible body fluids are accurate and robust predictors of the disease.

Thus, in one aspect the present invention provides a method of screening for a cancer selected from the group consisting of prostate cancer, thyroid cancer, colon cancer, rectum cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer, skin cancer, melanoma and a neuroendocrine tumour in a subject, said method comprising determining the level and/or chemical composition of one or more of the glycosaminoglycans (GAGs) chondroitin sulfate (CS), heparan sulfate (HS), and hyaluronic acid (HA) in a body fluid sample, wherein said sample has been obtained from said subject.

Unless otherwise clear from the context, subsequent references to "cancer" herein refer to one or more of the 20 types of cancer set forth in the paragraph above.

In one aspect the present invention provides a method of screening for a cancer selected from the group consisting of prostate cancer, thyroid cancer, colon cancer, rectum cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer and skin cancer in a subject, said method comprising determining the level and/or chemical composition of one or more of the glycosaminoglycans (GAGs) chondroitin sulfate (CS), heparan sulfate (HS), and hyaluronic acid (HA) in a body fluid sample, wherein said sample has been obtained from said subject.

In some embodiments, the present invention provides a method of screening for prostate cancer, lung cancer, head and neck cancer or bladder cancer in a subject.

In some embodiments, the present invention provides a method of screening for prostate cancer, melanoma (e.g. skin melanoma or uveal melanoma), skin cancer, colon cancer or rectal cancer (which may be collectively referred to as colorectal cancer), a neuroendocrine tumour (e.g. gastrointestinal neuroendocrine tumour), blood cancer (e.g. chronic lymphoid leukaemia or non-Hodgkins lymphoma), bladder cancer, breast cancer, ovarian cancer, uterine cancer (e.g. endometrial cancer or cervical cancer), brain cancer (e.g. diffuse glioma) and lung cancer.

In a particularly preferred embodiment, the present invention provides a method of screening for prostate cancer in a subject. Thus, in a preferred aspect, the present invention provides a method of screening for prostate cancer in a subject, said method comprising determining the level and/or chemical composition of one or more of the glycosaminoglycans (GAGs) chondroitin sulfate (CS), heparan sulfate (HS), and hyaluronic acid (HA) in a body fluid sample, wherein said sample has been obtained from said subject.

In some embodiments, the present invention provides a method of screening for a cancer selected from the group consisting of prostate cancer, thyroid cancer, rectum cancer, pancreatic cancer, bladder cancer, liver cancer, bile duct cancer, stomach cancer, oesophageal cancer, brain cancer, blood cancer, ovarian cancer and skin cancer in a subject. Thus, in some embodiments, breast cancer, colon cancer, head and neck cancer, lung cancer and/or uterine cancer are not screened for.

In some embodiments, the present invention provides a method of screening for a cancer selected from the group consisting of prostate cancer, thyroid cancer, rectum cancer, pancreatic cancer, bladder cancer, liver cancer, bile duct cancer, stomach cancer, oesophageal cancer, brain cancer, blood cancer, ovarian cancer, skin cancer, melanoma and a neuroendocrine tumor in a subject. Thus, in some embodiments, breast cancer, colon cancer, head and neck cancer, lung cancer and/or uterine cancer are not screened for.

In some embodiments the present invention provides a method of screening for a cancer selected from the group consisting of prostate cancer, thyroid cancer, colon cancer, rectum cancer, uterine cancer, pancreatic cancer, bladder cancer, liver cancer, bile duct cancer, stomach cancer, oesophageal cancer, head and neck cancer, blood cancer and ovarian cancer. Thus, in some embodiments, the cancer is not skin cancer, lung cancer, brain cancer or breast cancer.

In some embodiments, the present invention provides a method of screening for a cancer selected from the group consisting of prostate cancer, thyroid cancer, colon cancer, rectum cancer, uterine cancer, pancreatic cancer, bladder cancer, liver cancer, bile duct cancer, stomach cancer, oesophageal cancer, head and neck cancer, blood cancer, ovarian cancer, melanoma and a neuroendocrine tumor. Thus, in some embodiments, the cancer is not skin cancer, lung cancer, brain cancer or breast cancer.

In some embodiments the cancer is selected from the group consisting of prostate cancer, thyroid cancer, colon cancer, rectum cancer, lung cancer, uterine cancer, breast cancer, pancreatic cancer, bladder cancer, liver cancer, bile duct cancer, stomach cancer, oesophageal cancer, brain cancer, blood cancer, ovarian cancer and skin cancer. Thus, in some embodiments the cancer is not head and neck cancer.

In some embodiments, the cancer is selected from the group consisting of prostate cancer, thyroid cancer, colon cancer, rectum cancer, lung cancer, uterine cancer, breast cancer, pancreatic cancer, bladder cancer, liver cancer, bile duct cancer, stomach cancer, oesophageal cancer, brain cancer, blood cancer, ovarian cancer, skin cancer, melanoma and a neuroendocrine tumour. Thus, in some embodiments the cancer is not head and neck cancer.

In some embodiments the cancer is selected from the group consisting of thyroid cancer, rectum cancer, bladder cancer, bile duct cancer, oesophageal cancer, head and neck cancer, ovarian cancer and skin cancer. Thus, in some embodiments the cancer is not blood cancer, brain cancer, uterine cancer, pancreatic cancer, colon cancer, breast cancer, liver cancer, stomach cancer, lung cancer or prostate cancer.

In some embodiments the cancer is selected from the group consisting of thyroid cancer, rectum cancer, bladder cancer, bile duct cancer, oesophageal cancer, head and neck cancer, ovarian cancer, skin cancer, melanoma and a neuroendocrine tumour. Thus, in some embodiments the cancer is not blood cancer, brain cancer, uterine cancer, pancreatic cancer, colon cancer, breast cancer, liver cancer, stomach cancer, lung cancer or prostate cancer.

In some embodiments the cancer is selected from the group consisting of prostate cancer, thyroid cancer, colon cancer, rectum cancer, lung cancer, uterine cancer, breast cancer, pancreatic cancer, bladder cancer, liver cancer, bile duct cancer, oesophageal cancer, head and neck cancer, brain cancer, blood cancer, ovarian cancer and skin cancer. Thus, in some embodiments the cancer is not stomach cancer.

In some embodiments the cancer is selected from the group consisting of prostate cancer, thyroid cancer, colon cancer, rectum cancer, lung cancer, uterine cancer, breast cancer, pancreatic cancer, bladder cancer, liver cancer, bile duct cancer, oesophageal cancer, head and neck cancer, brain cancer, blood cancer, ovarian cancer, skin cancer, melanoma and a neuroendocrine tumour. Thus, in some embodiments the cancer is not stomach cancer.

In some embodiments the cancer is selected from the group consisting of prostate cancer, thyroid cancer, lung cancer, uterine cancer, breast cancer, bladder cancer, liver cancer, bile duct cancer, oesophageal cancer, head and neck cancer, brain cancer, blood cancer, ovarian cancer and skin cancer. Thus, in some embodiments the cancer is not colon cancer, rectum cancer, pancreatic cancer or stomach cancer.

In some embodiments the cancer is selected from the group consisting of prostate cancer, thyroid cancer, lung cancer, uterine cancer, breast cancer, bladder cancer, liver cancer, bile duct cancer, oesophageal cancer, head and neck cancer, brain cancer, blood cancer, ovarian cancer, skin cancer, melanoma and a neuroendocrine tumour. Thus, in some embodiments the cancer is not colon cancer, rectum cancer, pancreatic cancer or stomach cancer.

In some embodiments the cancer is selected from the group consisting of prostate cancer, thyroid cancer, colon cancer, rectum cancer, lung cancer, uterine cancer, pancreatic cancer, bladder cancer, liver cancer, bile duct cancer, stomach cancer, oesophageal cancer, head and neck cancer, brain cancer, blood cancer, ovarian cancer and skin cancer. Thus, in some embodiments the cancer is not breast cancer.

In some embodiments the cancer is selected from the group consisting of prostate cancer, thyroid cancer, colon cancer, rectum cancer, lung cancer, uterine cancer, pancreatic cancer, bladder cancer, liver cancer, bile duct cancer, stomach cancer, oesophageal cancer, head and neck cancer, brain cancer, blood cancer, ovarian cancer, skin cancer, melanoma and a neuroendocrine tumour. Thus, in some embodiments the cancer is not breast cancer.

In some embodiments the cancer is selected from the group consisting of prostate cancer, thyroid cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer and skin cancer. Thus, in some embodiments the cancer is not colon cancer or rectum cancer.

In some embodiments the cancer is selected from the group consisting of prostate cancer, thyroid cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer, skin cancer, melanoma and a neuroendocrine tumour. Thus, in some embodiments the cancer is not colon cancer or rectum cancer.

In some embodiments the cancer is selected from the group consisting of thyroid cancer, colon cancer, rectum cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer and skin cancer. Thus, in some embodiments the cancer is not prostate cancer.

In some embodiments the cancer is selected from the group consisting of thyroid cancer, colon cancer, rectum cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer, skin cancer, melanoma and a neuroendocrine tumour. Thus, in some embodiments the cancer is not prostate cancer.

In some embodiments the cancer is selected from the group consisting of prostate cancer, thyroid cancer, colon cancer, rectum cancer, lung cancer, uterine cancer, breast cancer, pancreatic cancer, liver cancer, bile duct cancer, stomach cancer, oesophageal cancer, head and neck cancer, brain cancer, blood cancer, ovarian cancer and skin cancer. Thus, in some embodiments the cancer is not bladder cancer.

In some embodiments the cancer is selected from the group consisting of prostate cancer, thyroid cancer, colon cancer, rectum cancer, lung cancer, uterine cancer, breast cancer, pancreatic cancer, liver cancer, bile duct cancer, stomach cancer, oesophageal cancer, head and neck cancer, brain cancer, blood cancer, ovarian cancer, skin cancer, melanoma and a neuroencrine tumour. Thus, in some embodiments the cancer is not bladder cancer.

In some embodiments the cancer is selected from the group consisting of prostate cancer, thyroid cancer, rectum cancer, lung cancer, uterine cancer, pancreatic cancer, bladder cancer, liver cancer, bile duct cancer, stomach cancer, oesophageal cancer, head and neck cancer, brain cancer, and skin cancer. Thus, in some embodiments the cancer is not blood cancer, ovarian cancer, breast cancer or colon cancer.

In some embodiments the cancer is selected from the group consisting of prostate cancer, thyroid cancer, rectum cancer, lung cancer, uterine cancer, pancreatic cancer, bladder cancer, liver cancer, bile duct cancer, stomach cancer, oesophageal cancer, head and neck cancer, brain cancer, skin cancer, melanoma and a neuroendocrine tumour. Thus, in some embodiments the cancer is not blood cancer, ovarian cancer, breast cancer or colon cancer.

In some embodiments cancer is selected from the group consisting of thyroid cancer, uterine cancer, bile duct cancer, oesophageal cancer, head and neck cancer, brain cancer, blood cancer and skin cancer. Thus, in some embodiments the cancer is not liver cancer, prostate cancer, pancreatic cancer, lung cancer, breast cancer, stomach cancer, bladder cancer, rectum cancer, ovarian cancer or colon cancer.

In some embodiments cancer is selected from the group consisting of thyroid cancer, uterine cancer, bile duct cancer, oesophageal cancer, head and neck cancer, brain cancer, blood cancer, skin cancer, melanoma and a neuroendocrine tumour. Thus, in some embodiments the cancer is not liver cancer, prostate cancer, pancreatic cancer, lung cancer, breast cancer, stomach cancer, bladder cancer, rectum cancer, ovarian cancer or colon cancer.

In preferred methods of the invention an altered level and/or composition of chondroitin sulfate (CS) and/or heparan sulfate (HS) and/or hyaluronic acid (HA) in said sample in comparison to a control level and/or composition is indicative of cancer (e.g. prostate cancer) in said subject.

In preferred methods of the invention both the level and the chemical composition are determined. In other preferred methods of the invention the chemical composition alone is determined, or, in other preferred methods, the level (total level or total concentration) of CS and/or HS and/or HA alone is determined.

As described above, methods of the present invention comprise determining the level and/or chemical composition of one or more of the glycosaminoglycans (GAGs) chondroitin sulfate (CS), heparan sulfate (HS), and hyaluronic acid (HA) in a body fluid sample. In some embodiments, the level and/or chemical composition of one of said GAGs is determined. In some embodiments, the level and/or chemical composition of chondroitin sulfate (CS) is determined. In some embodiments, the level and/or chemical composition of heparan sulfate (HS) is determined. In some embodiments, the level and/or chemical composition of hyaluronic acid (HA) is determined. In some embodiments, the level and/or chemical composition of two of said GAGs is determined. In some embodiments, the level and/or chemical composition of chondroitin sulfate (CS) and heparan sulfate (HS) is determined. In some embodiments, the level and/or chemical composition of chondroitin sulfate (CS) and hyaluronic acid (HA) is determined. In some embodiments, the level and/or chemical composition of hyaluronic acid (HA) and heparan sulfate (HS) is determined. In some embodiments, the level and/or chemical composition of all three of said GAGs is determined, i.e. the level and/or chemical composition of chondroitin sulfate (CS) and heparan sulfate (HS) and hyaluronic acid (HA) is determined.

Glycosaminoglycans (GAGs) are sugar containing molecules which are attached to proteins on serine residues, i.e. can form a parts of a proteoglycan. They are formed from linear or unbranched chains of monosaccharides (i.e. are polysaccharides) which can be sulphated. Heparan sulphate (HS), chondroitin sulphate (CS), keratan sulphate (KS), hyaluronic acid (HA) and heparin are the common types of GAG, of which HS and CS are examples of sulfated GAGs. The different types of GAG are distinguished by different repeating disaccharide units. However, all types have the same tetrasaccharide core attached to the serine residue of the protein.

Thus, for example, CS and HS are GAGs that share a common biosynthetic route in the linkage to the core protein, but thereafter they differ in their polymerisation in that the CS repeating disaccharide is made up of repeating N-acetylgalactosamine (GalNAc) and glucuronic acid residues (GlcA), whilst the repeating disaccharide in HS is typically made up of repeating N-acetylglucosamine (GlcNAc) and glucuronic acid (GlcA) residues. Each monosaccharide is attached by a specific enzyme allowing for multiple levels of regulation over GAG synthesis.

The "level" of HS or CS or HA as referred to herein generally refers to the total level or amount (e.g. concentration) of the HS or CS or HA present in the sample. The level of CS and/or HS and/or HA in a sample can be measured or determined by any appropriate method which would be well-known and described in the art. A preferred method involves electrophoresis, in particular capillary electrophoresis, e.g. capillary electrophoresis with fluorescence detection, e.g. laser-induced fluorescence detection. Other suitable methods are gel electrophoresis, e.g. agarose gel electrophoresis (e.g. FACE, fluorophore-assisted carbohydrate electrophoresis) or mass spectrometry or liquid chromatography, e.g. HPLC, optionally in combination with mass spectrometry (HPLC-MS). Conveniently these levels can be measured as a concentration, for example, as a number of microgram per ml ($\mu$g/ml). However, again, any appropriate measure of level may be used.

In the methods of the present invention the levels of HS or CS or HA are determined separately or individually. In other words the methods do not involve the measurement of total GAG levels in a sample or the total levels of all the GAGs present in combination, but involve the measurement of the levels of one or more of the individual GAGs HS or CS or HA.

In particular embodiments, the level (e.g. total level, or concentration) of CS and/or HS and/or HA can be determined in, for example blood or urine samples.

In some embodiments of the invention, an increased level (or concentration) of CS in a urine sample and/or an increased level (or concentration) of HS in a urine sample and/or an increased level (or concentration) of HA in a urine sample is indicative of cancer (e.g. prostate cancer) in said subject and can be used to screen, diagnose, etc., subjects as described elsewhere herein.

In some embodiments of the invention, an increased level (or concentration) of HS in a blood sample is indicative of cancer (e.g. prostate cancer) in said subject and can be used to screen, diagnose, etc., subjects as described elsewhere herein.

In particular embodiments, an increased level (or concentration) of CS in a urine sample is indicative of cancer (e.g. prostate cancer) in said subject.

In particular embodiments, a decreased level (or concentration) of CS in a blood sample is indicative of cancer (e.g. prostate cancer) in said subject.

In particular embodiments, an increased level (or concentration) of HS in a blood sample is indicative of cancer (e.g. prostate cancer) in said subject.

In particular embodiments, an increased level (or concentration) of HS in a urine sample is indicative of cancer (e.g. prostate cancer) in said subject.

In particular embodiments, an increased level (or concentration) of HA in a urine sample is indicative of cancer (e.g. prostate cancer) in said subject.

In particular embodiments, an increased level (or concentration) of HA in a blood sample is indicative of cancer (e.g. prostate cancer) in said subject.

The individual monosaccharide units making up the CS and HS can have different sulfation patterns in terms of the position of the sulfate molecules and the amount/number of sulfate molecules. For CS, sulfation may most commonly occur at one or more of position 2 of the GlcA and positions 4 and 6 of the GalNAc. For HS, sulfation may occur at one or more of position 2 of the GlcA after epimerization to IdoA (iduronic acid), positions 3 and 6 of the GlcNAc, and N-sulfation of the GlcNAc. Thus, each individual disaccharide in the GAG chain may have 0 (i.e. be unsulfated), 1, 2, 3 or 4 (only in HS) sulfation forms and this in turn gives rise to different overall chemical compositions of GAG chains in terms of sulfation levels and specific disaccharide sulfation patterns.

As described elsewhere herein, preferred embodiments of the invention involve the determination of the chemical composition of 1, 2 or 3 of CS, HS and HA. The term "chemical composition" as used herein can refer to both the levels of the GAGs as well as the disaccharide sulfation composition of the GAGs. In particular, this term includes a determination of one or more particular forms, e.g. sulfation forms, of the disaccharides making up the CS or HS GAGs. Put another way, the term "chemical composition" refers to the amount or level of one or more of the various sulfated and/or unsulfated forms of CS or HS disaccharides, as well as, for example, some other properties of the individual GAGs present, such as total HS or CS or HA GAG levels, or other properties related to GAG sulfation such as HS charge or CS charge as described further elsewhere herein. Such a chemical composition which is analysed or determined in the present invention can also be referred to herein as a GAG profile, GAG forms, GAG features or GAG properties. In this regard, in some embodiments up to 22 different GAG properties (including up to 20 independent properties) as described in more detail below can be measured in the methods of the invention and a collection or group (e.g. two or more) of these measurements taken from a particular sample can be referred to as a GAG profile. In some preferred embodiments of the invention up to 21 different GAG properties (including up to 19 independent properties) can be measured (as described in more detail below).

Thus, for example, the term "chemical composition" as used herein may refer to a determination or analysis of the sulfation patterns (e.g. one or more of the sulfation forms) of the disaccharides making up CS and/or HS.

For example, for CS, there are 8 main sulfated and unsulfated forms (sulfation patterns, disaccharide sulfation forms) which are: 0s CS (also referred to as unsulfated CS or CS O unit), 2s CS (also referred to as chondroitin-2-sulfate), 4s CS (also referred to as chondroitin-4-sulfate or CS A unit), 6s CS (also referred to as chondroitin-6-sulfate or CS C unit), 2s4s CS (also referred to as chondroitin-2-4-sulfate), 2s6s CS (also referred to as chondroitin-2-6-sulfate or CS D unit), 4s6s CS (also referred to as chondroitin-4-6-sulfate or CS E unit) and Tris CS (also referred to as chondroitin-2-4-6-sulfate or trisulfated CS).

Each of the above is a form of CS GAG (a CS GAG form or property) which may be measured in the methods of the present invention. One or more of these forms may be measured, for example up to 8, e.g. 1, 2, 3, 4, 5, 6, 7 or all 8 of these sulfation forms may be measured. In some embodiments, measurement of all 8 of these sulfation forms is preferred. Another GAG property for CS which may be measured in the methods of the present invention is the total concentration of CS (also referred to herein as CS tot or Tot CS or Total CS) or the total level of CS. This is typically measured as a concentration, e.g. in $\mu$g/ml, as described elsewhere herein. In embodiments of the invention where the total concentration of CS is measured as one of the GAG properties, then it is preferred that at least one other GAG property or CS property is measured, e.g. a property that is not based on the total level of the other individual GAGs present (e.g. not total HS or total HA). In some embodiments the total concentration of CS is not measured. In some embodiments, the measurement of one or more CS GAG properties is preferred.

"Charge CS" is another GAG form or property which may be measured in the present invention, e.g. as part of the GAG profile. "Charge CS" refers to the total fraction of sulfated disaccharides of CS, i.e. the fraction of sulfated disaccharides of CS present or measured in a sample out of the total CS disaccharides present or measured in a sample (i.e. sulfated CS disaccharides/sulfated + unsulfated CS disaccharides).

For example, in addition to the unsulfated form of CS (0s CS), where the sample is a blood sample 4s CS may also be measured as a main sulfation form (e.g. instead of measuring all of the sulfated forms of CS) in order to calculate the charge CS (4s CS/4s CS + 0s CS). Alternatively, for example, in addition to the unsulfated form of CS (0s CS), where the sample is a urine sample 4s CS and 6s CS may also be measured as the main sulfation forms (e.g. instead of measuring all of the sulfated forms of CS) in order to calculate the charge CS (4s CS + 6s CS/4s CS + 6s CS + 0s CS).

As the measurement of "charge CS" is dependent on the measurement of other properties, i.e. the measurement of levels of sulfated and unsulfated CS disaccharides, this property is not referred to herein as an independent GAG property or CS property. Thus, up to 9 independent CS properties can be measured in the methods of the present invention, which are the 8 sulfated and unsulfated forms listed above, together with the total CS. In some embodiments all 9 of these independent CS properties are measured.

In some embodiments it is preferred to measure up to 8 (e.g. 1, 2, 3, 4, 5, 6, 7 or 8) or all 8 of the CS sulfation forms (i.e. the sulfated and unsulfated forms), together with total CS and charge CS.

For example, for HS, there are 8 main sulfated and unsulfated forms (sulfation patterns, disaccharide sulfation forms) which are: 0s HS (also referred to as unsulfated HS), 2s HS (which is sulfated at the 2-position of GlcA), Ns HS (which is sulfated at the N-position of the GlcNAc), 6s HS (which is sulfated at the 6-position of the GlcNAc), 2s6s HS (which is sulfated at the 2-position of GlcA and the 6-position of the GlcNAc), Ns6s HS (which is sulfated at the 6-position and N-position of GlcNAc), Ns2s HS (which is sulfated at the 2-position of GlcA and the N-position of GlcNAc), Tris HS (which is sulfated at the 2-position of GlcA and 6-position and N-position of GlcNAc, also referred to as trisulfated HS). Note that sulfation in position 3 of the GlcNAc is also possible but rarely observed.

Each of the above is a form of HS GAG (an HS GAG form or property) which may be measured or determined in the methods of the present invention. However, due to its rarity, in preferred embodiments of the invention, the sulfation form with sulfation in position 3 of the GlcNAc is not measured. Thus, in the methods of the invention, one or more of these 9 (or preferably 8) forms may be measured, for example up to 9 (or preferably up to 8), e.g. 1, 2, 3, 4, 5, 6, 7, 8 or all 9 of these sulfation forms may be measured. In some embodiments, measurement of all 8 of these sulfation forms (excluding the sulfation form with sulfation in position 3 of the GlcNAc) is preferred. Another GAG property for HS which may be measured in the methods of the present invention is the total concentration of HS (also referred to herein as HS tot or Tot HS or Total HS) or the total level of HS. This is typically measured as a concentration, e.g. in $\mu$g/ml, as described elsewhere herein. In embodiments of the invention where the total concentration of HS is measured as one of the GAG properties, then it is preferred that at least one other GAG property or HS property is measured, e.g. a property that is not based on the total level of the other individual GAGs present (e.g. not total CS or total HA). In some embodiments the total concentration of HS is not measured. The measurement of one or more HS GAG properties is preferred in the methods of the invention, e.g. where the sample is blood or urine, in particular where the sample is a urine sample.

"Charge HS" is another GAG form or property which may be measured in the present invention, e.g. as part of the GAG profile. Charge HS refers to the total fraction of sulfated disaccharides of HS, i.e. the fraction of sulfated disaccharides of HS present or measured in a sample out of the total HS disaccharides present or measured in a sample (i.e. sulfated HS disaccharides/sulfated + unsulfated HS disaccharides).

By way of example, in addition to the unsulfated form of HS (0s HS), where the sample is a blood or urine sample Ns HS and/or 6s HS may also be measured as main sulfation forms (e.g. instead of measuring all of the sulfated forms of HS) in order to calculate the charge HS (e.g. Ns HS + 6s HS /Ns HS + 6s HS + 0s HS).

As the measurement of "charge HS" is dependent on the measurement of other properties, i.e. the measurement of sulfated and unsulfated HS disaccharides, this property is not referred to herein as an independent GAG property or HS property. Thus, up to 10 independent HS properties can be measured in the methods of the present invention, which are the 9 sulfated and unsulfated forms listed above (preferably excluding the sulfation form with sulfation in position 3 of the GlcNAc), together with the total HS. Thus, in some embodiments 9 independent HS properties are measured (the 8 main sulfated and unsulfated HS forms plus total HS).

In some embodiments it is preferred to measure up to 8 (e.g. 1, 2, 3, 4, 5, 6, 7 or 8) or all 8 of the HS main sulfation forms (i.e. the sulfated and unsulfated forms listed above excluding the sulfation form with sulfation in position 3 of the GlcNAc), together with total HS and charge HS.

In some embodiments 9 independent HS properties are measured (the 8 main sulfated and unsulfated HS forms plus total HS) and the 9 independent CS properties are measured, i.e. 18 independent GAG properties.

As described above, in some embodiments the level and/or chemical composition of hyaluronic acid (HA) may be determined in a body fluid sample. Hyaluronic acid (HA) is typically non-sulfated. Accordingly, when HA is measured in accordance with the invention, it is typically and preferably the level (total level or total concentration) of HA that is measured (also referred to herein as HA tot or Tot HA or Total HA). This is typically measured as a concentration, e.g. in $\mu$g/ml, as described elsewhere herein. In some embodiments of the invention where the total concentration of HA is measured as one of the GAG properties, then it is preferred that at least one other GAG property (e.g. CS and/or HS property) is measured, e.g. a property that is not based on the total level of the other individual GAGs present (e.g. not total CS or total HS). In some embodiments, HA is not measured. In some embodiments, HA is not measured in urine. In some embodiments, HA is not measured in blood.

In some embodiments 9 independent HS properties are measured (the 8 main sulfated and unsulfated HS forms plus total HS) and the 9 independent CS properties are measured and total HA are measured, i.e. 19 independent GAG properties.

These GAG properties or GAG forms, e.g. disaccharide sulfation forms (with the exception of total CS or total HS) may be measured as a fraction size or fraction or proportion or relative measurement, rather than as absolute levels or concentrations, for example are given a value of less than 1 or are normalised to 1 depending on the levels of all the sulfation forms (or all the main sulfation forms) measured in the sample. In other words, the level of each of the desired sulfation forms is measured independently and then normalised to 1. In other words, the level of each of the desired sulfation forms is measured independently and then its mass fraction or volume fraction or mole fraction is computed. These fractions may also be expressed as percentage. In other words, these fractions may also be normalised to 100. For example, in some embodiments, the fraction size of a given sulfated CS form or unsulfated CS form may be determined by measuring the level of the given sulfated CS form or unsulfated CS form and dividing this by the sum of the levels of all of the CS sulfation forms (or all of the main sulfation forms) and the unsulfated CS form measured (or present) in the sample. In some embodiments, the fraction size of a given sulfated HS form or unsulfated HS form may be determined by measuring the level of the given sulfated HS form or unsulfated HS form and dividing this by the sum of the levels of all of the HS sulfation forms (or main sulfation forms) and the unsulfated HS form measured (or present) in the sample. When calculating such fractions, it is preferred that at least the main sulfation forms of CS or HS are measured in order to be able to normalise the fraction of the particular individual sulfation form to 1. In some embodiments, preferably at least the unsulfated forms of HS or CS are measured as the main sulfation form. In addition to the unsulfated form of CS, where the sample is a blood sample 4s CS may also be measured as the main sulfation forms. In addition to the unsulfated form of CS, where the sample is a urine sample 4s CS and 6s CS may also be measured as the main sulfation forms. In addition to the unsulfated form of HS, where the sample is a blood or urine sample Ns HS and/or 6s HS may also be measured as the main sulfation forms.

By way of example, levels of 0s CS, 4sCS and 6sCS are individually measured in a sample and then divided by the sum of the three measurements in order to obtain the measurement of the fraction. Relative measurements are generally preferred because they are more easy to interpret, for example, a measurement of 0s CS of 0.6 indicates that 60% of the CS disaccharides are unsulfated. However, absolute levels can also be measured.

In some preferred embodiments of the invention, the disaccharide composition (for example the specific sulfation patterns (e.g. sulfation forms)) of one or more of the disaccharides making up CS and/or HS is measured or determined. In more preferred embodiments one or more sulfation properties or forms of CS and/or HS such as those outlined above (e.g. 0s CS, 2s CS, etc), are measured or determined. Appropriate methods of doing this would be well known to a skilled person in the art and any of these could be used. However, a convenient method to achieve such quantification of disaccharide composition or the appropriate properties or forms of CS or HS (and separation of the disaccharide forms) is to use electrophoresis, in particular capillary electrophoresis, and preferably capillary electrophoresis with fluorescence detection, e.g. capillary electrophoresis with laser-induced fluorescence detection (CE-LIF). An alternative method is liquid chromatography, preferably HPLC (high-performance liquid chromatography), for example SAX HPLC. Preferably mass spectrometry is also used (e.g. HPLC-MS), for example electrospray ionization mass spectrometry (ESI-MS). Alternatively, mass spectrometry can be used without chromatography, e.g. liquid chromatography. Particularly preferred methods are outlined in the Examples. One example of a particularly preferred method is capillary electophoresis with laser-induced fluorescence detection. Another example of a particularly preferred method is HPLC ESI-MS.

In some methods of the invention where the levels of one or more individual disaccharide forms are measured, the GAGs are subjected to a processing step, for example a step of fragmentation or cleavage or digestion, e.g. by chemical digestion or enzyme treatment, e.g. with chondroitinase ABC or chondroitinase B, in order to obtain the disaccharide units which are then analysed.

In some methods of the invention the GAGs in the sample are subjected to a step of extraction (e.g. using a protease such as proteinase K) and/or purification, e.g. using an anion-exchange resin.

In some methods of the invention the GAGs in the sample (e.g. various different GAG forms in the sample) are subjected to a step of separation and/or quantification, as described elsewhere herein.

Other methods which might be used are known in the art. However, examples are analytical techniques involving the use of antibodies to various GAG forms, e.g. techniques such as Western blot, ELISA or FACS, or methods involving agarose gel electrophoresis (e.g. fluorophore-assisted carbohydrate electrophoresis (FACE)) or polyacrylamide gel electrophoresis (PAGE).

Preferred methods of the invention provide a method of screening for cancer (e.g. prostate cancer) in a subject, said method comprising determining or measuring the amount or level in a body fluid sample of one or more of the various sulfated and/or unsulfated forms of CS or HS disaccharides (in other words determining the GAG sulfation patterns), the total fraction of sulfated disaccharides of HS or CS (i.e. charge HS or charge CS), or the total concentration of CS or HS or HA. For CS this may involve determining one or more (or all) of the forms of CS selected from the group consisting of: charge CS, CS tot, 0s CS, 2s CS, 6s CS, 4s CS, 2s6s CS, 2s4s CS, 4s6s CS and Tris CS. For HS, this may involve determining one or more (or all) of the forms of HS selected from the group consisting of: charge HS, HS tot, 0s HS, 2s HS, 6s HS, 2s6s HS, Ns HS, Ns2s HS, Ns6s HS and Tris HS. For HA, this may involve determining the total concentration of HA, i.e. HA tot.

Thus, in some embodiments up to 22 GAG properties selected from the group consisting of 0s CS, 2s CS, 4s CS, 6s CS, 2s4s CS, 2s6s CS, 4s6s CS, Tris CS, charge CS, Total CS, 0s HS, 2s HS, Ns HS, 6s HS, 2s6s HS, Ns6s HS, Ns2s HS, Tris HS, HS sulfated at position 3 of the GlcNAc, Total HS, charge HS, Total HA can be measured or determined in methods of the invention. As described elsewhere herein, preferably HS sulfated at position 3 of the GlcNAc is not measured or determined. Thus, in some embodiments up to 21 GAG properties selected from the group consisting of 0s CS, 2s CS, 4s CS, 6s CS, 2s4s CS, 2s6s CS, 4s6s CS, Tris CS, charge CS, Total CS, 0s HS, 2s HS, Ns HS, 6s HS, 2s6s HS, Ns6s HS, Ns2s HS, Tris HS, Total HS, charge HS, Total HA can be measured or determined in methods of the invention. In some embodiments, one or more of the following GAG properties may be measured or determined: the relative level of 4s CS with respect to 6s CS (e.g. the ratio 4s CS/6s CS or the inverse ratio 6s CS/4s CS), the relative level of 6s CS with respect to 0s CS (e.g. the ratio 6s CS/0s CS or the inverse ratio 0s CS/6s CS) or the relative level of 4s CS with respect to 0s CS (e.g. the ratio 4s CS/0s CS or the inverse ratio 0s CS/4s CS). Thus, in some embodiments up to 25 GAG properties or up to 24 GAG properties may be measured or determined.

In some embodiments, one or more (or all) of the following GAG properties may be measured or determined: 0s CS, 6s CS, the ratio 4s CS/6s CS, Charge CS, Ns HS, 4s CS, the ratio 6s CS/0s CS, the ratio 4s CS/0s CS, 0s HS, Charge HS.

In some embodiments, for blood samples, one or more of the following GAG properties may be measured or determined: 0s CS, 6s CS, the ratio 4s CS/6s CS, Charge CS, Ns HS.

In some embodiments, for urine samples, one or more (or all) of the following GAG properties may be measured or determined: 4s CS, the ratio 6s CS/0s CS, the ratio 4s CS/0s CS, 0s HS, Charge HS.

In some embodiments, for blood samples, an increase in the level of one or more (or both) of: 6s CS or Charge CS, for example in comparison to a control level, is indicative of cancer in said subject.

In some embodiments, for blood samples, a decrease in the level of one or more (or all) of: 0s CS, the ratio 4s CS/6s CS or Ns HS, for example in comparison to a control level, is indicative of cancer in said subject.

In some embodiments, for urine samples, an increase in the level of Charge HS, for example in comparison to a control level, is indicative of cancer in said subject.

In some embodiments, for urine samples, a decrease in the level of one or more (or all) of: 4s CS, the ratio 6s CS/0s CS, the ratio 4s CS/0s CS or 0s HS, for example in comparison to a control level, is indicative of cancer in said subject.

In some embodiments, for blood samples, one or more (or all) of the following GAG properties may be measured or determined: 6s CS, the ratio 6s CS/0s CS, the ratio 4s CS/6s CS, 0s CS.

In some embodiments, for urine samples, one or more (or both) of the following GAG properties may be measured or determined: Ns2s HS, 4s CS.

In some embodiments, e.g. for blood samples, one or more (or all) of the following GAG forms may be measured or determined: Charge CS, Total CS or Total HS. In some embodiments an increase in the level of one or more (or all) of: Charge CS, Total CS or Total HS, for example in comparison to a control level, is indicative of cancer in said subject.

In some embodiments, e.g. for blood samples, one or more (or all) of the following GAG forms may be measured or determined: 0s CS, 4s CS or 6s CS. In some embodiments, e.g. for blood samples, an alteration in the level of one or more (or all) of: 0s CS, 4s CS or 6s CS, for example in comparison to a control level, is indicative of cancer in said subject. In some embodiments, e.g. for blood samples, a decrease in the level of 0s CS, for example in comparison to a control level, is indicative of cancer in said subject. In some embodiments, e.g. for blood samples, an increase in the level of one (or both) of 4s CS or 6s CS, for example in comparison to a control level, is indicative of cancer in said subject.

In some embodiments, e.g. for blood samples, one or more (or all) of the following GAG properties may be measured or determined: Charge CS, Total CS, 6s CS, 4s CS, 2s HS, 0s Hs.

In some embodiments, e.g. for blood samples, the following GAG properties may be measured or determined: the ratio 6s CS/4s CS+6s CS (or the inverse ratio 4s CS+6s CS/6s CS) or the relative level of 2s HS with respect to 0s HS (e.g. the ratio 2s HS/0s HS or the inverse ratio 0s HS/2s HS).

In some embodiments, e.g. for blood samples, the following GAG property may be measured or determined: the relative level of 2s HS with respect to 0s HS (e.g. the ratio 2s HS/0s HS or the inverse ratio 0s HS/2s HS).

In some embodiments, e.g. for blood samples, 2s HS may be measured or determined.

In some embodiments, e.g. for blood samples, one or more (or all) of the following GAG properties may be measured or determined: Total CS, 6s CS, Charge CS, the relative level of 4s CS with respect to 6s CS (e.g. the ratio 4s CS/6s CS or the inverse ratio 6s CS/4s CS) or the relative level of 2s HS with respect to 0s HS (e.g. the ratio 2s HS/0s HS or the inverse ratio 0s HS/2s HS). In some embodiments, e.g. for blood samples, one or more (or all) of the following GAG properties may be measured or determined: Total CS, 6s CS, Charge CS or the relative level of 4s CS with respect to 6s CS (e.g. the ratio 4s CS/6s CS or the inverse ratio 6s CS/4s CS).

In some embodiments, for blood samples, one or more (or all) of the following GAG properties may be measured or determined: Charge CS, Total CS, Total HS, 0s CS, 2s CS, 6s CS, 4s CS, the relative level of 4s CS with respect to 6s CS (e.g. the ratio 4s CS/6s CS or the inverse ratio 6s CS/4s CS), the relative level of 6s CS with respect to 0s CS (e.g. the ratio 6s CS/0s CS or the inverse ratio 0s CS/6s CS) or the relative level of 4s CS with respect to 0s CS (e.g. the ratio 4s CS/0s CS or the inverse ratio 0s CS/4s CS).

In some embodiments, e.g. for blood samples, an alteration in the level of one or more (or all) of: Charge CS, Total CS, Total HS, 0s CS, 2s CS, 6s CS, 4s CS, the relative level of 4s CS with respect to 6s CS (e.g. the ratio 4s CS/6s CS or the inverse ratio 6s CS/4s CS), the relative level of 6s CS with respect to 0s CS (e.g. the ratio 6s CS/0s CS or the inverse ratio 0s CS/6s CS) or the relative level of 4s CS with respect to 0s CS (e.g. the ratio 4s CS/0s CS or the inverse ratio 0s CS/4s CS), for example in comparison to a control level, is indicative of cancer in said subject.

In some embodiments, e.g. for blood samples, an increase in the level of one or more (or all) of: Charge CS, Total CS, Total HS, 6s CS, 4s CS, the ratio 6s CS/0s CS or the ratio 4s CS/0s CS, for example in comparison to a control level, is indicative of cancer in said subject.

In some embodiments, e.g. for blood samples, a decrease in the level of one or more (or all) of: 0s CS, 2s CS or the ratio 4s CS/6s CS, for example in comparison to a control level, is indicative of cancer in said subject.

In some embodiments, one or more (or all) of the following GAG properties may be measured or determined: 6s CS, the relative level of 6s CS with respect to 0s CS (e.g. the ratio 6s CS/0s CS or the inverse ratio 0s CS/6s CS) or Total HS.

In some embodiments, e.g. for blood samples, one or more (or all) of the following GAG properties may be measured or determined: 6s CS, the relative level of 4s CS with respect to 6s CS (e.g. the ratio 4s CS/6s CS or the inverse ratio 6s CS/4s CS), the relative level of 6s CS with respect to 0s CS (e.g. the ratio 6s CS/0s CS or the inverse ratio 0s CS/6s CS) or Total HS.

In some embodiments, an alteration in the level of one or more (or all) of: 6s CS, the relative level of 6s CS with respect to 0s CS (e.g. the ratio 6s CS/0s CS or the inverse ratio 0s CS/6s CS) or Total HS, for example in comparison to a control level, is indicative of cancer in said subject.

In some embodiments, e.g. for blood samples, an alteration in the level of one or more (or all) of: 6s CS, the relative level of 4s CS with respect to 6s CS (e.g. the ratio 4s CS/6s CS or the inverse ratio 6s CS/4s CS), the relative level of 6s CS with respect to 0s CS (e.g. the ratio 6s CS/0s CS or the inverse ratio 0s CS/6s CS) or Total HS, for example in comparison to a control level, is indicative of cancer in said subject.

In some embodiments, CS Tot, 0s CS, 4s CS and/or 6s CS is not determined. In some embodiments, if the cancer being screened for is head and neck cancer, CS Tot, 0s CS, 4s CS and/or 6s CS is not determined.

In some embodiments, CS Tot, 0s CS, 4s CS, 6s CS and/or HS Tot is not determined. In some embodiments, CS Tot, 0s CS, 4s CS, 6s CS, HS Tot and/or HA Tot is not determined. In some embodiments, if the cancer being screened for is blood cancer, brain cancer, uterine cancer, pancreatic cancer, colon cancer, breast cancer, liver cancer, stomach cancer, lung cancer or prostate cancer, CS Tot, 0s CS, 4s CS, 6s CS and/or HS Tot is not determined. In some embodiments, if the cancer being screened for is blood cancer, brain cancer, uterine cancer, pancreatic cancer, colon cancer, breast cancer, liver cancer, stomach cancer, lung cancer or prostate cancer, CS Tot, 0s CS, 4s CS, 6s CS, HS Tot and/or HA Tot is not determined.

In some embodiments, 0s CS, 4s CS, 6s CS, CS Tot and/or HA Tot is not determined. In some embodiments, if the cancer being screened for is colon cancer, rectum cancer, stomach cancer or pancreatic cancer, 0s CS, 4s CS, 6s CS, CS Tot and/or HA Tot is not determined.

In some embodiments, CS Tot and/or HA Tot is not determined. In some embodiments, if the cancer being screened for is breast cancer, CS Tot and/or HA Tot is not determined.

In some embodiments, CS Tot and/or HS Tot is not determined. In some embodiments, if the cancer being screened for is colon cancer or rectum cancer, CS Tot and/or HS Tot is not determined.

In some embodiments, HS Tot, CS Tot and/or HA Tot is not determined. In some embodiments, if the cancer being screened for is bladder cancer, HS Tot, CS Tot and/or HA Tot is not determined. In some embodiments, if the cancer being screened for is blood cancer, ovarian cancer, breast cancer or colon cancer, HS Tot, CS Tot and/or HA Tot is not determined.

In some embodiments, CS Tot, 4s CS, 6s CS, HS Tot and/or HA Tot is not determined. In some embodiments, if the cancer is prostate cancer, CS Tot, 4s CS, 6s CS, HS Tot and/or HA Tot is not determined.

In some embodiments, CS Tot, 0s CS, 4s CS, 6s CS, HS Tot and/or HA Tot is not determined.

In some embodiments, for blood samples, 0s CS is determined.

As indicated above, a particularly preferred cancer in accordance with the present invention is prostate cancer.

In some embodiments, in methods of screening for prostate cancer, Ns HS and/or 0s HS is not determined.

In some embodiments, in methods of screening for prostate cancer, 0s Hs is not determined.

In some embodiments, in methods of screening for prostate cancer, 2s6s HS is not determined.

In some embodiments, in methods of screening for prostate cancer, an increased level in said sample of one or more (or all) of: 2s CS, 6s CS, 2s6s CS, 2s4s CS, 4s6s CS, Tris CS, Total HA, Tris HS, Ns2s HS, 6s HS, charge HS and Total HS, for example in comparison to a control level, is indicative of prostate cancer in said subject. In some embodiments, a decreased level in said sample of 0s HS, for example in comparison to a control level, is indicative of prostate cancer in said subject.

In some embodiments, in methods of screening for prostate cancer, an increased level in said sample of Ns2s HS, for example in comparison to a control level, is indicative of prostate cancer in said subject.

Particularly preferred GAG forms to be measured or determined in the methods of screening for prostate cancer of the invention are one or more (or all) of: 4s CS, 0s CS, Total HA and Ns2s HS.

Other preferred GAG forms to be measured in the methods of the invention (e.g. methods of screening for prostate cancer) are one or more (or all) of: the relative level of 4s CS with respect to 6s CS (e.g. the ratio 4s CS/6s CS or the inverse ratio 6s CS/4s CS), the relative level of 6s CS with respect to 0s CS (e.g. the ratio 6s CS/0s CS or the inverse ratio 0s CS/6s CS) or the relative level of 4s CS with respect to 0s CS (e.g. the ratio 4s CS/0s CS or the inverse ratio 0s CS/4s CS).

Other preferred GAG forms to be measured or determined in the methods of the invention (e.g. methods of screening for prostate cancer) are one or more (or all) of: 0s CS, 4s CS, Ns2s HS, the relative level of 4s CS with respect to 0s CS (e.g. the ratio 4s CS/0s CS or the inverse ratio 0s CS/4s CS). Other preferred GAG forms to be measured in the methods of the invention (e.g. methods of screening for prostate cancer) are one or more (or all) of: 2s6s CS, 2s4s CS, Tris CS, the relative level of 4s CS with respect to 6s CS (e.g. the ratio 4s CS/6s CS or the inverse ratio 6s CS/4s CS), the relative level of 4s CS with respect to 0s CS (e.g. the ratio 4s CS/0s CS or the inverse ratio 0s CS/4s CS), charge CS, HA tot, CS tot, 2s CS, 6s CS, 4s6s CS, the relative level of 6s CS with respect to 0s CS (e.g. the ratio 6s CS/0s CS or the inverse ratio 0s CS/6s CS), Tris HS, Ns6s HS, 0s HS, charge HS, HS tot.

In some embodiments, in methods of screening for prostate cancer, for blood samples, an increase in the level of one or more (or all) of: 2s CS, 6s CS, 4s CS, 2s6s CS, 2s4s CS, 4s6s CS, Tris CS, the ratio 6s CS/0s CS, the ratio 4s CS/0s CS, charge CS, Total HA, Tris HS, Ns2s HS, 6s HS, 2s HS, charge HS and total HS, for example in comparison to a control level, is indicative of prostate cancer in said subject.

In some embodiments, in methods of screening for prostate cancer, for blood samples, a decrease in the level of one or more (or all) of: 0s CS, the ratio 4s CS/6s CS, Total CS, Ns6s HS, Ns HS, 2s6s HS and 0s HS, for example in comparison to a control level, is indicative of prostate cancer in said subject.

For blood samples, in preferred embodiments of the invention the methods of screening for prostate cancer involve the determination of one or more (or all) of: 0s CS, 2s CS, 6s CS, 4s CS, 2s6s CS, 2s4s CS, 4s6s CS, Tris CS, charge CS, Total CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS) or the relative level of 4s CS with respect to 0s CS (e.g. the ratio 4s CS/0s CS or the inverse ratio 0s CS/4s CS).

For blood samples, in some embodiments of the methods of screening for prostate cancer an increase in one or more (or all) of the sulphated forms of CS or total CS is indicative of prostate cancer. In particular, an increase in one or more (or all) of: 2s CS, 6s CS, 4s CS, 2s6s CS, 2s4s CS, 4s6s CS, Tris CS, charge CS, the relative level of 6s CS with respect to 4s CS (e.g. the ratio 6s CS/4s CS), the relative level of 6s CS with respect to 0s CS (e.g. the ratio 6s CS/0s CS) or the relative level of 4s CS with respect to 0s CS (e.g. the ratio 4s CS/0s CS), is indicative of prostate cancer in said subject. For blood samples, in some embodiments of the methods of screening for prostate cancer a decrease in one or more (or both) of: 0s CS or Total CS is indicative of prostate cancer in said subject. Thus, in some embodiments of the methods of screening for prostate cancer an alteration in one or more of the sulphated forms of CS or total CS is indicative of prostate cancer.

For blood samples, in some embodiments of the methods of screening for prostate cancer a decrease in the relative level of 4s CS with respect to 6s CS (e.g. the ratio 4s CS/6s CS) is indicative of prostate cancer in said subject. In some embodiments of the methods of screening for prostate cancer, a decrease in the relative level of 0s CS with respect to 6s CS (e.g. the ratio 0s CS/6s CS) or the relative level of 0s CS with respect to 4s CS (e.g. the ratio 0s CS/4s CS), is also indicative of prostate cancer in said subject.

Thus, these markers can be used in the methods of the invention (e.g. in methods of screening for prostate cancer) individually, although they can also be used in combination, e.g. in the form of a multi-marker assay. The ratios are particularly preferred markers (e.g. the ratios 4s CS/6s CS or 6s CS/4s CS). In some embodiments (e.g. in methods of screening for prostate cancer), the relative level of 4s CS with respect to 0s CS (e.g. the ratios 4s CS/0s CS or 0s CS/4s CS) is a preferred marker.

For blood samples, in preferred embodiments of the methods of screening for prostate cancer of the invention the methods involve the determination of one or more (or all) of: Tris HS, Ns2s HS, 2s6s HS, 6s HS or Total HS. In particular in some embodiments of methods of screening for prostate cancer an increase in the level of one or more of: Tris HS, Ns2s HS, 6s HS or Total HS, for example in comparison to a control level, is indicative of prostate cancer in said subject. Alternatively, in some embodiments of methods of screening for prostate cancer a decrease in the level of 2s6s HS, for example in comparison to a control level, is indicative of prostate cancer in said subject. In some embodiments of the invention (e.g. of methods of screening for prostate cancer) the methods involve the determination of one or more (or all) of: Tris HS, Ns2s HS and Total HS. In some embodiments of methods of screening for prostate cancer an alteration in one or more of the sulphated forms of HS or total HS is indicative of prostate cancer. These markers can be used in the methods of the invention (e.g. in methods of screening for prostate cancer) individually, although they can also be used in combination, e.g. in the form of a multi-marker assay.

For blood samples, in some embodiments (e.g. in methods of screening for prostate cancer) HA tot (Total HA) may be measured. In some embodiments of methods of screening for prostate cancer an increase in the level of HA tot, for example in comparison to a control level, is indicative of prostate cancer in said subject.

In some embodiments, in methods of screening for prostate cancer, for urine samples, an increase in the level of one or more (or all) of: 0s CS, 2s CS, 6s CS, 2s6s CS, 2s4s CS, 4s6s CS, Tris CS, Total HA, Total CS, Tris HS, Ns6s HS, Ns2s HS, Ns HS, 2s6s HS, 6s HS, charge HS and Total HS, for example in comparison to a control level, is indicative of prostate cancer in said subject.

In some embodiments, in methods of screening for prostate cancer, for urine samples, a decrease in the level of one or more (or all) of: 4s CS, the ratio 4s CS/6s CS, the ratio 6s CS/0s CS, the ratio 4s CS/0s CS, charge CS, 2sHS and 0s HS, for example in comparison to a control level, is indicative of prostate cancer in said subject.

For urine samples, in preferred embodiments of methods of screening for prostate cancer the methods involve the determination of one or more (or all) of: 4s CS, 2s6s CS, 2s4s CS, Tris CS, Total CS, the relative level of 4s CS with respect to 6s CS (e.g. the ratio 4s CS/6s CS or the inverse ratio 6s CS/4s CS) or the relative level of 4s CS with respect to 0s CS (e.g. the ratio 4s CS/0s CS or the inverse ratio 0s CS/4s CS). In some preferred embodiments, the methods (e.g. methods of screening for prostate cancer) involve the determination of 4s CS.

For example, in some embodiments of methods of screening for prostate cancer, in urine samples, an increase in the level of one or more of the sulphated forms of CS or total CS, for example in comparison to a control level, is indicative of prostate cancer. In particular, in some embodiments of methods of screening for prostate cancer, for urine samples, an increase in the level of one or more (or all) of: 2s6s CS, 2s4s CS, Tris CS, Total CS, the relative level of 6s CS with respect to 4s CS (e.g. the ratio 6s CS/4s CS) or the relative level of 0s CS with respect to 4s CS (e.g. the ratio 0s CS/4s CS), for example in comparison to a control level, is indicative of prostate cancer in said subject. Alternatively, in some embodiments of methods of screening for prostate cancer a decrease in the level of one or more of the sulfated forms of CS in urine samples, for example in comparison to a control level, is indicative of prostate cancer in said subject. In particular, in some embodiments of methods of screening for prostate cancer a decrease in the level of one or more (or all) of: 4s CS, the relative level of 4s CS with respect to 6s CS (e.g. the ratio 4s CS/6s CS) or the relative level of 4s CS with respect to 0s CS (e.g. the ratio 4s CS/0s CS), for example in comparison to a control level, is indicative of prostate cancer in said subject. Thus, in some embodiments of methods of screening for prostate cancer an alteration in one or more of the sulphated forms of CS or total CS is indicative of prostate cancer.

These markers can be used in the methods of the invention individually, although they can also be used in combination, e.g. in the form of a multi-marker assay. The ratios are particularly preferred markers.

For urine samples, in preferred embodiments of methods of screening for prostate cancer of the invention the methods involve the determination of one or more (or all) of: charge HS, HS tot, 2s HS, Ns2s HS, Ns6s HS and Tris HS. In some embodiments of the invention (e.g. methods of screening for prostate cancer) the methods involve the determination of one or more of: Tris HS, Ns6s HS, Ns2s HS, and charge HS. In some preferred embodiments of the invention (e.g. methods of screening for prostate cancer) the methods involve the determination of Ns2s HS.

In some embodiments of methods of screening for prostate cancer, in urine samples, an increase in the level of one or more of the sulphated forms of HS or total HS, for example in comparison to a control level, is indicative of prostate cancer. In particular, in some embodiments of methods of screening for prostate cancer an increase in the level of one or more (or all) of: charge HS, HS tot, Ns2s HS, Ns6s HS and Tris HS, for example in comparison to a control level, is indicative of prostate cancer in said subject. Alternatively, in some embodiments of methods of screening for prostate cancer a decrease in the level of one or more of the sulfated forms of HS in urine samples, for example in comparison to a control level, is indicative of prostate cancer in said subject. In particular, in some embodiments of methods of screening for prostate cancer a decrease in the level of 2s HS, for example in comparison to a control level, is indicative of prostate cancer in said subject. Thus, in some embodiments of methods of screening for prostate cancer an alteration in one or more of the sulphated forms of HS or total HS is indicative of prostate cancer.

These markers can be used in the methods of the invention individually, although they can also be used in combination, e.g. in the form of a multi-marker assay.

For urine samples, in some embodiments (e.g. in methods of screening for prostate cancer) HA tot (Total HA) may be measured. In some embodiments of methods of screening for prostate cancer an increase in the level of HA tot, for example in comparison to a control level, is indicative of prostate cancer in said subject.

In some embodiments, the level of a single GAG form (GAG property) is determined. For example, in one embodiment of methods of screening for prostate cancer, the method comprises determining the level in a sample of one or more GAG features (GAG properties) that are identified in Table B herein as being significantly altered between prostate cancer and healthy samples, i.e. those features with a "% in ROPE" (Region of Practical Equivalence) of less than 5.00.

In other embodiments of the present invention, the level of more than one of the GAG forms (GAG properties) is determined (e.g. the level of two or more GAG forms, or three or more GAG forms, or four or more GAG forms, or five or more GAG forms is determined). By "more than one" is meant 2, 3, 4, 5, 6, 7, 8, 9, 10 etc.... 21, 22, 24 or 25 (including all integers between 2 and 21 or 2 and 22 or 2 and 24 or 2 and 25). In any list of markers or GAG properties provided herein, it is a preferred embodiment that all are measured. Also, a determination of the level of each and every possible combination of the GAG forms can be performed.

Thus, in some embodiments multi-marker methods are performed. Determining the level of multiple of the GAG forms (biomarker multiplexing) may improve screening (e.g. diagnostic) accuracy.

In a preferred embodiment, the level of two of the stated GAG forms is determined. In another preferred embodiment, the level of three of the stated GAG forms is determined. In yet another preferred embodiment, the level of four or five of the stated GAG forms is determined.

In the case of blood, particularly preferred markers to be determined for prostate cancer screening are one or both (preferably both) of: 4s CS and 0s CS. Thus, one or both of these GAG forms might be measured in the methods of the invention.

In the case of blood, the highest accuracy for prostate cancer was reached by determining the level of the following GAG forms (properties), ranked by accuracy: the ratio 4s CS to 0s CS (e.g. 4s CS/0s CS), 0s CS, 4s CS, charge CS, HS tot, 6s CS, the ratio 6s CS to 0s CS (e.g. 6s CS/0s CS), 4s6s CS. Thus, one or more, two or more, three or more, four or more, five or more, six or more, seven or more or preferably all of these GAG forms might be measured in the methods of the invention. In preferred embodiments the most accurate form is used first, i.e. the ratio 4s CS to 0s CS (e.g. 4s CS/0s CS). If other forms are added, then preferably they are added in the order shown in the list, i.e. 0s CS next, etc. In other embodiments the ratio 4s CS to 0s CS (e.g. 4s CS/0s CS) is not measured.

Other preferred GAG forms in blood for prostate cancer screening can be identified as having a % in ROPE value of less than 5.00 in Table B, e.g. less than 4.00, 3.00, 2.00 or 1.00 or even a value of 0.00.

In the case of urine, particularly preferred markers to be determined for prostate cancer screening are one or more, or all, of: Total HA, Ns2s HS and 4sCS. Thus, one or more, two or more, or preferably all of these GAG forms might be measured in the methods of the invention.

In the case of urine, the highest accuracy for prostate cancer was reached by determining the level of the following GAG forms (properties), ranked by accuracy: Ns2s HS, Tris HS, 4s CS, Ns6s HS, HA tot, the ratio 4s CS to 6s CS (e.g. 4s CS/6s CS). Thus, one or more, two or more, three or more, four or more, five or more or preferably all of these GAG forms might be measured in the methods of the invention. In preferred embodiments the most accurate form is used first, i.e. Ns2s HS. If other forms are added, then preferably they are added in the order shown in the list, i.e. Tris HS next, etc. In other embodiments Ns2s HS is not measured.

Other preferred GAG forms in urine for prostate cancer screening can be identified as having a % in ROPE value of less than 5.00 in Table B, e.g. less than 4.00, 3.00, 2.00 or 1.00 or even a value of 0.00.

In some embodiments of the invention the methods of screening for prostate cancer involve the determination of one or more (or all) of: 0s CS, Tris CS, Tot CS, Tris HS, 2s6s HS or 6s HS in blood samples.

In some embodiments of the invention the methods of screening for prostate cancer involve the determination of one or more (or all) of: 2s6s CS, 2s4s CS or the relative level of 4s CS with respect to 6s CS (e.g. the ratio 4s CS/6s CS or the inverse ratio 6s CS/4s CS) in urine samples.

In some embodiments (e.g. in methods of screening for prostate cancer), for example in urine samples, one or more (or all) of the GAG properties selected from the group consisting of 2s CS, 2s4s CS, 2s6s CS, 4s6s CS, Tris CS, charge CS, 0s HS, 2s HS, Ns HS, 6s HS, 2s6s HS, Ns6s HS, Ns2s HS, Tris HS, charge HS and Total HA can be measured or determined in methods of the invention. In some embodiments (e.g. in methods of screening for prostate cancer), for example in urine samples, one or more of the GAG properties selected from the group consisting of 2s CS, 2s4s CS, 2s6s CS, 4s6s CS, Tris CS, charge CS, 0s HS, 2s HS, Ns HS, 6s HS, 2s6s HS, Ns6s HS, Ns2s HS, Tris HS and charge HS can be measured or determined in methods of the invention.

In some embodiments (e.g. in methods of screening for prostate cancer), for example in urine samples, one or more (or all) of the GAG properties selected from the group consisting of 2s CS, 2s4s CS, 2s6s CS, 4s6s CS, Tris CS, charge CS, the relative level of 4s CS with respect to 6s CS, the relative level of 6s CS with respect to 0s CS, the relative level of 4s CS with respect to 0s CS, 0s HS, 2s HS, Ns HS, 6s HS, 2s6s HS, Ns6s HS, Ns2s HS, Tris HS, charge HS and Total HA can be measured or determined in methods of the invention. In some embodiments (e.g. in methods of screening for prostate cancer), for example in urine samples, one or more (or all) of the GAG properties selected from the group consisting of 2s CS, 2s4s CS, 2s6s CS, 4s6s CS, Tris CS, charge CS, the relative level of 4s CS with respect to 6s CS, the relative level of 6s CS with respect to 0s CS, the relative level of 4s CS with respect to 0s CS, 0s HS, 2s HS, Ns HS, 6s HS, 2s6s HS, Ns6s HS, Ns2s HS, Tris HS and charge HS can be measured or determined in methods of the invention.

In some embodiments (e.g. in methods of screening for prostate cancer), one or more (or all) GAG properties selected from the group consisting of 0s CS, 2s CS, 2s4s CS, 2s6s CS, 4s6s CS, Tris CS, charge CS, the relative level of 4s CS with respect to 6s CS, the relative level of 6s CS with respect to 0s CS, the relative level of 4s CS with respect to 0s CS, 0s HS, 2s HS, Ns HS, 6s HS, 2s6s HS, Ns6s HS, Ns2s HS, Tris HS and charge HS, can be measured or determined in methods of the invention.

In some embodiments (e.g. in methods of screening for prostate cancer), one or more (or all) GAG properties selected from the group consisting of 0s CS, 2s CS, 2s4s CS, 2s6s CS, 4s6s CS, Tris CS, charge CS, 0s HS, 2s HS, Ns HS, 6s HS, 2s6s HS, Ns6s HS, Ns2s HS, Tris HS and charge HS, can be measured or determined in methods of the invention.

In some embodiments (e.g. in methods of screening for prostate cancer), for example in urine samples, one or more (or all) GAG properties selected from the group consisting of 0s CS, 2s CS, 4s CS, 6s CS, 2s4s CS, 2s6s CS, 4s6s CS, Tris CS, charge CS, the relative level of 4s CS with respect to 6s CS, the relative level of 6s CS with respect to 0s CS, the relative level of 4s CS with respect to 0s CS, 0s HS, 2s HS, Ns HS, 6s HS, 2s6s HS, Ns6s HS, Ns2s HS, Tris HS and charge HS, can be measured or determined in methods of the invention.

In some embodiments (e.g. in methods of screening for prostate cancer), for example in urine samples, one or more (or all) GAG properties selected from the group consisting of 0s CS, 2s CS, 4s CS, 6s CS, 2s4s CS, 2s6s CS, 4s6s CS, Tris CS, charge CS, the relative level of 4s CS with respect to 6s CS, the relative level of 6s CS with respect to 0s CS, the relative level of 4s CS with respect to 0s CS, 0s HS, 2s HS, Ns HS, 6s HS, 2s6s HS, Ns6s HS, Ns2s HS, Tris HS, charge HS and HA Tot can be measured or determined in methods of the invention.

In some embodiments (e.g. in methods of screening for prostate cancer), one or more (or all) GAG properties selected from the group consisting of 0s CS, 2s CS, 2s4s CS, 2s6s CS, 4s6s CS, Tris CS, charge CS, 0s HS, 2s HS, Ns HS, 6s HS, 2s6s HS, Ns6s HS, Ns2s HS, Tris HS and charge HS can be measured or determined in methods of the invention.

In some embodiments (e.g. in methods of screening for prostate cancer), one or more (or all) GAG properties selected from the group consisting of 0s CS, 2s CS, 2s4s CS, 2s6s CS, 4s6s CS, Tris CS, charge CS, the relative level of 4s CS with respect to 6s CS, the relative level of 6s CS with respect to 0s CS, or the relative level of 4s CS with respect to 0s CS, 0s HS, 2s HS, Ns HS, 6s HS, 2s6s HS, Ns6s HS, Ns2s HS, Tris HS and charge HS can be measured or determined in methods of the invention.

In some embodiments (e.g. in methods of screening for prostate cancer), one or more (or all) GAG properties selected from the group consisting of 0s CS, 2s CS, 4s CS, 2s4s CS, 2s6s CS, 4s6s CS, Tris CS, charge CS, CS tot, 0s HS, 2s HS, Ns HS, 6s HS, 2s6s HS, Ns6s HS, Ns2s HS, Tris HS, charge HS and HS tot can be measured or determined in methods of the invention.

In some embodiments (e.g. in methods of screening for prostate cancer), one or more (or all) GAG properties selected from the group consisting of 0s CS, 2s CS, 4s CS, 2s4s CS, 2s6s CS, 4s6s CS, Tris CS, charge CS, CS tot, HS tot, the relative level of 4s CS with respect to 6s CS, the relative level of 6s CS with respect to 0s CS, or the relative level of 4s CS with respect to 0s CS, 0s HS, 2s HS, Ns HS, 6s HS, 2s6s HS, Ns6s HS, Ns2s HS, Tris HS and charge HS can be measured or determined in methods of the invention.

In some embodiments of methods of screening for prostate cancer, for example in urine samples, the methods involve the determination of one or more (or all) of: 2s6s CS, 2s4s CS, Tris CS, charge HS, 2s HS, Ns2s HS, Ns6s HS, Tris HS and HA tot.

In some embodiments of methods of screening for prostate cancer, for example in urine samples, the methods involve the determination of one or more of: 2s6s CS, 2s4s CS, Tris CS, charge HS, 2s HS, Ns2s HS, Ns6s HS and Tris HS.

In some embodiments of methods of screening for prostate cancer, for example in urine samples, the methods involve the determination of one or more (or all) of: 2s6s CS, 2s4s CS, Tris CS, the relative level of 4s CS with respect to 6s CS, the relative level of 4s CS with respect to 0s CS charge HS, 2s HS, Ns2s HS, Ns6s HS, Tris HS and HA tot.

In some embodiments of methods of screening for prostate cancer, for example in urine samples, the methods involve the determination of one or more (or all) of: 2s6s CS, 2s4s CS, Tris CS, the relative level of 4s CS with respect to 6s CS, the relative level of 4s CS with respect to 0s CS charge HS, 2s HS, Ns2s HS, Ns6s HS and Tris HS.

In some embodiments of methods of screening for prostate cancer, for example in urine samples, the methods involve the determination of one or more (or all) of: 4s CS, 2s6s CS, 2s4s CS, Tris CS, the relative level of 4s CS with respect to 6s CS (e.g. the ratio 4s CS/6s CS or the inverse ratio 6s CS/4s CS), the relative level of 4s CS with respect to 0s CS (e.g. the ratio 4s CS/0s CS or the inverse ratio 0s CS/4s CS), charge HS, 2s HS, Ns2s HS, Ns6s HS and Tris HS.

In some embodiments of methods of screening for prostate cancer, for example in urine samples, the methods involve the determination of one or more (or all) of: 4s CS, 2s6s CS, 2s4s CS, Tris CS, the relative level of 4s CS with respect to 6s CS (e.g. the ratio 4s CS/6s CS or the inverse ratio 6s CS/4s CS), the relative level of 4s CS with respect to 0s CS (e.g. the ratio 4s CS/0s CS or the inverse ratio 0s CS/4s CS), charge HS, 2s HS, Ns2s HS, Ns6s HS, Tris HS and HA tot.

In some embodiments of methods of screening for prostate cancer, for urine samples, an increase in the level of one or more (or all) of: charge HS, Ns2s HS, Ns6s HS, Tris HS, 2s6s CS, 2s4s CS, Tris CS, the ratio of 6s CS/4s CS, the ratio of 0s CS/4s CS, and HA tot, for example in comparison to a control level, is indicative of prostate cancer in said subject.

In some embodiments of methods of screening for prostate cancer, for urine samples, an increase in the level of one or more (or all) of: charge HS, Ns2s HS, Ns6s HS, Tris HS, 2s6s CS, 2s4s CS, Tris CS, the ratio of 6s CS/4s CS, the ratio of 0s CS/4s CS, CS tot, and HA tot, for example in comparison to a control level, is indicative of prostate cancer in said subject.

In some embodiments of methods of screening for prostate cancer, for urine samples, an increase in the level of one or more (or all) of: charge HS, Ns2s HS, Ns6s HS, Tris HS, 2s6s CS, 2s4s CS, Tris CS and HA tot, for example in comparison to a control level, is indicative of prostate cancer in said subject.

In some embodiments of methods of screening for prostate cancer, for urine samples, an increase in the level of one or more (or all) of: charge HS, Ns2s HS, Ns6s HS, Tris HS, 2s6s CS, 2s4s CS, Tris CS, CS tot, and HA tot, for example in comparison to a control level, is indicative of prostate cancer in said subject.

In some embodiments of methods of screening for prostate cancer, for urine samples, an increase in the level of one or more (or all) of: 2s6s CS, 2s4s CS, Tris CS, the relative level of 6s CS with respect to 4s CS (e.g. the ratio 6s CS/4s CS) or the relative level of 0s CS with respect to 4s CS (e.g. the ratio 0s CS/4s CS), charge HS, Ns2s HS, Ns6s HS and Tris HS, for example in comparison to a control level, is indicative of prostate cancer in said subject.

In some embodiments of methods of screening for prostate cancer, for urine samples, an increase in the level of one or more (or all) of: 2s6s CS, 2s4s CS, Tris CS, the relative level of 6s CS with respect to 4s CS (e.g. the ratio 6s CS/4s CS) or the relative level of 0s CS with respect to 4s CS (e.g. the ratio 0s CS/4s CS), charge HS, Ns2s HS, Ns6s HS, Tris HS and Total HA, for example in comparison to a control level, is indicative of prostate cancer in said subject.

In some embodiments of methods of screening for prostate cancer, for urine samples, an increase in the level of one or more (or all) of: 2s6s CS, 2s4s CS, Tris CS, the relative level of 6s CS with respect to 4s CS (e.g. the ratio 6s CS/4s CS), the relative level of 0s CS with respect to 4s CS (e.g. the ratio 0s CS/4s CS), charge HS, Ns2s HS, Ns6s HS and Tris HS, for example in comparison to a control level, is indicative of prostate cancer in said subject.

In some embodiments of methods of screening for prostate cancer, for urine samples, an increase in the level of one or more (or all) of: 2s6s CS, 2s4s CS, Tris CS, the relative level of 6s CS with respect to 4s CS (e.g. the ratio 6s CS/4s CS), the relative level of 0s CS with respect to 4s CS (e.g. the ratio 0s CS/4s CS), CS tot, HS tot, charge HS, Ns2s HS, Ns6s HS and Tris HS, for example in comparison to a control level, is indicative of prostate cancer in said subject.

In some embodiments of methods of screening for prostate cancer, for urine samples, an increase in the level of one or more (or all) of: 2s6s CS, 2s4s CS, Tris CS, charge HS, Ns2s HS, Ns6s HS and Tris HS, for example in comparison to a control level, is indicative of prostate cancer in said subject.

In some embodiments of methods of screening for prostate cancer, for urine samples, an increase in the level of one or more (or all) of: 2s6s CS, 2s4s CS, Tris CS, CS tot, HS tot, charge HS, Ns2s HS, Ns6s HS and Tris HS, for example in comparison to a control level, is indicative of prostate cancer in said subject.

In some embodiments of methods of screening for prostate cancer, for blood samples, an increase in one or more (or all) of: 2s CS, 2s6s CS, 2s4s CS, 4s6s CS, Tris CS, charge CS, the relative level of 6s CS with respect to 4s CS (e.g. the ratio 6s CS/4s CS), the relative level of 6s CS with respect to 0s CS (e.g. the ratio 6s CS/0s CS), the relative level of 4s CS with respect to 0s CS (e.g. the ratio 4s CS/0s CS), Tris HS, Ns2s HS or 6s HS, for example in comparison to a control level, is indicative of prostate cancer in said subject.

In some embodiments of methods of screening for prostate cancer, for blood samples, an increase in one or more (or all) of: 2s CS, 4s CS, 2s6s CS, 2s4s CS, 4s6s CS, Tris CS, charge CS, the relative level of 6s CS with respect to 4s CS (e.g. the ratio 6s CS/4s CS), the relative level of 6s CS with respect to 0s CS (e.g. the ratio 6s CS/0s CS), the relative level of 4s CS with respect to 0s CS (e.g. the ratio 4s CS/0s CS), Tris HS, Ns2s HS, 6s HS or HS tot, for example in comparison to a control level, is indicative of prostate cancer in said subject.

In some embodiments of methods of screening for prostate cancer, for blood samples, an increase in one or more (or all) of: 2s CS, 2s6s CS, 2s4s CS, 4s6s CS, Tris CS, charge CS, Tris HS, Ns2s HS or 6s HS, for example in comparison to a control level, is indicative of prostate cancer in said subject.

In some embodiments of methods of screening for prostate cancer, for blood samples, an increase in one or more (or all) of: 2s CS, 4s CS, 2s6s CS, 2s4s CS, 4s6s CS, Tris CS, charge CS, Tris HS, Ns2s HS, 6s HS or HS tot, for example in comparison to a control level, is indicative of prostate cancer in said subject.

In some embodiments of methods of screening for prostate cancer, for example in urine samples, the relative levels of CS and HS are not determined (e.g. the ratio of CS/HS or the inverse ratio of HS/CS are not determined).

In some embodiments (e.g. of methods of screening for prostate cancer), one or more GAG forms with multiple (e.g. 2 or 3) sulfation sites is measured or determined (e.g. in urine samples). Thus, in some embodiments, one or more (or all) of the GAG properties selected from the group consisting of 2s6s CS, 2s4s CS, 4s6s CS, Tris CS, Tris HS, Ns6s HS, Ns2s Hs and 2s6s HS are measured or determined. In some embodiments, one or more forms of CS with multiple (e.g. 2 or 3) sulfation sites are measured or determined (e.g. in urine samples).

In some embodiments of methods of screening for prostate cancer, for example in blood samples, the methods involve the determination of one or more (or all) of: 2s CS, 2s6s CS, 2s4s CS, 4s6s CS, Tris CS, charge CS, Tris HS, Ns2s HS, 2s6s HS or 6s HS.

In some embodiments of methods of screening for prostate cancer, for example in blood samples, the methods involve the determination of one or more (or all) of: 2s CS, 2s6s CS, 2s4s CS, 4s6s CS, Tris CS, charge CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS) or the relative level of 4s CS with respect to 0s CS (e.g. the ratio 4s CS/0s CS or the inverse ratio 0s CS/4s CS), Tris HS, Ns2s HS, 2s6s HS or 6s HS.

In some embodiments of methods of screening for prostate cancer, for blood samples, an increase in the level of one or more (or all) of: 2s CS, 2s6s CS, 2s4s CS, 4s6s CS, Tris CS, charge CS, the relative level of 6s CS with respect to 4s CS (e.g. the ratio 6s CS/4s CS), the relative level of 6s CS with respect to 0s CS (e.g. the ratio 6s CS/0s CS) or the relative level of 4s CS with respect to 0s CS (e.g. the ratio 4s CS/0s CS), Tris HS, Ns2s HS or 6s HS, for example in comparison to a control level, is indicative of prostate cancer in said subject.

In some embodiments of methods of screening for prostate cancer, for blood samples, an increase in the level of one or more (or all) of: 2s CS, 2s6s CS, 2s4s CS, 4s6s CS, Tris CS, charge CS, Tris HS, Ns2s HS or 6s HS, for example in comparison to a control level, is indicative of prostate cancer in said subject.

Optionally, in some embodiments (e.g. of methods of screening for prostate cancer), methods may further comprise measuring (or determining) one or more (or all) of the GAG properties selected from the group consisting of 0s CS, 6s CS, 4s CS, CS Tot, HS Tot and HA Tot (e.g. in addition to one or more of the other GAG properties or groups of GAG properties described or listed elsewhere herein).

In some embodiments (e.g. of methods of screening for prostate cancer), for example in urine samples, one or more (or all) of the GAG properties selected from the group consisting of 0s CS, 6s CS, 4s CS, CS Tot and HS Tot are not measured or determined. In some embodiments (e.g. of methods of screening for prostate cancer), for example in urine samples, one or more (or all) of the GAG properties selected from the group consisting of 0s CS, 6s CS, 4s CS, CS Tot, HS Tot and HA Tot are not measured or determined. In some embodiments (e.g. of methods of screening for prostate cancer), for example in urine samples, one or more (or all) of the GAG properties selected from the group consisting of CS Tot, HS Tot and HA Tot are not measured or determined. Thus, in some embodiments (e.g. of methods of screening for prostate cancer), 0s CS is not measured or determined. In some embodiments (e.g. of methods of screening for prostate cancer), 4s CS is not measured or determined. In some embodiments (e.g. of methods of screening for prostate cancer), 6s CS is not measured or determined. In some embodiments (e.g. of methods of screening for prostate cancer), CS tot is not measured or determined. In some embodiments (e.g. of methods of screening for prostate cancer), HS tot is not measured or determined. In some embodiments (e.g. of methods of screening for prostate cancer), HA tot is not measured or determined. In some embodiments (e.g. of methods of screening for prostate cancer), one or more (or all) of the GAG properties selected from the group consisting of 0s CS, 4s CS and 6s CS are not measured or determined. In some embodiments (e.g. of methods of screening for prostate cancer) 0s CS is not measured or determined. In some embodiments (e.g. of methods of screening for prostate cancer), one or more (or all) of the GAG properties selected from the group consisting of 4s CS, 6s CS, Tot CS, Tot HS and Tot HA are not measured or determined. In some embodiments (e.g. of methods of screening for prostate cancer) 6s CS is not measured or determined.

In some embodiments one or more (or all) of the GAG properties selected from the group consisting of 6s CS, CS Tot, the relative level of 4s CS to 6s CS, Ns HS, Ns6s HS, Charge HS and 4s CS is not measured or determined. In some embodiments one or more (or all) of the GAG properties selected from the group consisting of 6s CS, CS Tot, the relative level of 4s CS to 6s CS and Ns HS is not measured or determined, e.g. in plasma samples. In some embodiments one or more (or all) of the GAG properties selected from the group consisting of Ns6s HS, Charge HS and 4s CS is not measured or determined, e.g. in urine samples.

In some embodiments one or more (or all) of the GAG properties selected from the group consisting of 2s CS, 4s CS, 6s CS, 2s4s CS, 2s6s CS, 4s6s CS, the relative level of 4s CS with respect to 6s CS (e.g. the ratio 4s CS/6s CS or the inverse ratio 6s CS/4s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), charge CS, 0s HS, Ns HS, Ns2s HS, Total HS, charge HS, Total HA are not measured or determined in plasma samples.

In some embodiments one or more (or all) of the GAG properties selected from the group consisting of 0s CS, 4s CS, 6s CS, Tris CS, the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), charge CS, Total CS, 0s HS, 2s HS, Ns HS, 6s HS, Ns6s HS, Ns2s HS, Tris HS, Total HS, charge HS, Total HA are not measured or determined in urine samples.

Based on the observed alterations in the levels of various GAG forms in cancer patients (e.g. prostate cancer patients) versus healthy patients, if desired, scoring methods, scoring systems, markers or formulas can be designed which use such levels of various GAG forms in order to arrive at an indication, e.g. in the form of a value or score, which can then be used for diagnosis. Appropriate scoring systems and parameters (e.g. GAG forms) to be measured can readily be designed based for example on the data described herein, for example in Table B, for example, based on one or more of the individual GAG features or properties which show significant differences in particular samples (blood or urine) as indicated in Table B. In particular, GAG properties in Table B that are most different between prostate cancer samples and healthy samples (e.g. preferably one or more or all properties which have a % in ROPE value of 0.00 or close to 0.00, e.g. one or more or all properties which have a % in ROPE value equal to or less than 5.00 or 4.00 or 3.00 or 2.00 or 1.00) may be selected.

With reference to Table B, two examples of blood GAG properties that are most differential in prostate cancer vs. healthy subjects in blood samples are 4s CS and 0s CS. In some embodiments, a simple formula for use in the methods of the invention is based on the ratio of these two properties:

$$\text{Blood score} = \frac{[4s\ CS]}{[0s\ CS]}$$

Appropriate threshold or cut-off values (used to declare a sample positive or negative) for use with this formula can be designed by a person skilled in the art. For example, the inventor used a cut-off value of 0.2511, where scores above this cut-off classify the sample as prostate cancer with 98% accuracy and an AUC of 0.997.

In the Example section herein, scoring systems (formulas) have been designed using measurements of multiple GAG forms such that a high score (or elevated score) results in a positive diagnosis (i.e. the finding of the presence of prostate cancer), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table B.

Some preferred and exemplary scoring systems or methods (formulae) are provided herein.

Thus, a preferred scoring system for prostate cancer giving rise to a score when a blood sample from a subject is analysed is:

$$Blood\ score = \frac{[4s\ CS]}{\frac{4}{10}[0s\ CS]}$$

A preferred scoring system for prostate cancer when urine from a subject is analysed is:

$$Urine\ score = \frac{3[HA] + \frac{6}{10}[Ns2s\ HS]}{\frac{1}{3}[4s\ CS]}$$

A preferred scoring system for prostate cancer to be used when both blood and urine samples are analysed is:

$$Combined\ score = \frac{Blood\ score + Urine\ score}{2}$$

In the above scoring systems the terms in brackets represent the fraction of the particular GAG form concerned (as described elsewhere herein) and [HA] is the total concentration of HA (in $\mu$g/mL). In other embodiments, these specific formulae are not used.

As described in Example 2 herein, scores have also been designed that can differentiate between cancer subjects and healthy subjects and thus may be used for screening for cancer (e.g. diagnosis of cancer etc.) These GAG scores are:

$$Blood\ score = \frac{10[6s\ CS] + 20\left[\frac{6s}{0s}CS\right]}{\frac{4}{100}\left[\frac{4s}{6s}CS\right] + [0s\ CS]}$$

$$Urine\ score = 2\frac{[Ns2s\ HS]}{[4s\ CS]}$$

$$Combined\ score = \frac{(Blood\ score + Urine\ score)}{2}$$

In the above scoring systems the terms in brackets represent the fraction (mass fraction) of the particular GAG form concerned (as described elsewhere herein) In some embodiments, the above scores (formulae) of Example 2 are preferred. In other embodiments, these specific formulae are not used.

Appropriate threshold or cut-off values (used to declare a sample positive or negative) for use with these formulae can be designed by a person skilled in the art.

In one embodiment, the above blood score (or formula, the blood score of Example 2) is used and the cut-off score is 0.961, wherein a score above this cut-off score is indicative of cancer and a score below this cut-off score is indicative of not being cancer (e.g. indicative of a normal or healthy sample). In some embodiments, a score that is at least 5%, at least 10%, at least 20%, at least 30%, at least 40%, at least 50% or more higher than 0.961 is indicative of cancer. In some embodiments, a score that is at least 5%, at least 10%, at least 20%, at least 30%, at least 40%, at least 50% or more lower than 0.961 is indicative of there being no cancer (e.g. indicative of a normal or healthy subject).

In one embodiment, the above urine score (or formula, the urine score of Example 2) is used and the cut-off score is 0.942, wherein a score above this cut-off score is indicative of cancer and a score below this cut-off score is indicative of not being cancer (e.g. indicative of a normal or healthy sample). In some embodiments, a score that is at least 5%, at least 10%, at least 20%, at least 30%, at least 40%, at least 50% or more higher than 0.942 is indicative of cancer. In some embodiments, a score that is at least 5%, at least 10%, at least 20%, at least 30%, at least 40%, at least 50% or more lower than 0.942 is indicative of there being no cancer (e.g. indicative of a normal or healthy subject).

In one embodiment, the above combined score (or formula, the combined score of Example 2) is used and the cut-off score is 0.998, wherein a score above this cut-off score is indicative of cancer and a score below this cut-off score is indicative of not being cancer (e.g. indicative of a normal or healthy sample). In some embodiments, a score that is at least 5%, at least 10%, at least 20%, at least 30%, at least 40%, at least 50% or more higher than 0.998 is indicative of cancer. In some embodiments, a score that is at least 5%, at least 10%, at least 20%, at least 30%, at least 40%, at least 50% or more lower than 0.998 is indicative of there being no cancer (e.g. indicative of a normal or healthy subject).

As described in Example 2, the three scores were calculated for each sample and it was observed that cancer (case) samples have recurrently elevated scores with respect to control samples. The difference in GAG scores between cancer (case) and controls probed were able to discriminate subjects with very high accuracy in that ROC curves generated for each GAG score had an AUC equal to 0.961 for blood, 0.942 for urine, and 0.998 for combined.

Overall, these results indicate that scores can be designed from blood and/or urine measurements of specific GAG properties to detect cancer regardless of the cancer type.

As described in Example 15 herein, an alternative score (formula) has also been designed that can differentiate between cancer subjects and healthy subjects in blood samples and thus may be used for screening for cancer (e.g. diagnosis of cancer etc.) This GAG score, also referred to herein as Cancer GAG Score #2 (or Cancer Blood Score #2), is:

$$Cancer\ GAG\ score = \frac{10}{3}\text{Charge CS} + \text{Total CS}\left(\frac{[6s\ CS]}{[4s\ CS] + [6s\ CS]}\right) + \log_2\left(1 + \frac{[2s\ HS]}{[0s\ HS]}\right)$$

where terms in square brackets represent the fraction (mass fraction) of the disaccharide for the corresponding GAG, *Charge CS* is the weighted charge of CS and *Total CS* is the total concentration of CS (in µg/mL). In some embodiments, Cancer GAG Score #2 is preferred. As described in Example 15, the performance of this GAG score was evaluated using the receiver-operating-characteristic (ROC) curves, and the area under the curve (AUC) was found to be 0.986. We identified an optimal cut-off equal to 1.39 for this score. Thus, in one embodiment, the cut-off score is 1.39, wherein a score above this cut-off score is indicative of cancer and a score below this cut-off score is indicative of not being cancer (e.g. indicative of a normal or healthy sample). In some embodiments, a score that is at least 5%, at least 10%, at least 20%, at least 30%, at least 40%, at least 50% or more higher than 1.39 is indicative of cancer. In some embodiments, a score that is at least 5%, at least 10%, at least 20%, at least 30%, at least 40%, at least 50% or more lower than 1.39 is indicative of there being no cancer (e.g. indicative of a normal or healthy subject).

In the above scoring systems the terms in brackets represent the fraction (mass fraction) of the particular GAG form concerned (as described elsewhere herein) In other embodiments, these specific formulae are not used.

It should be noted that these preferred scoring systems are given as preferred examples, wherein a high score gives rise to a positive screening for, diagnosis of etc., the presence of cancer (e.g. prostate cancer). However, it is clear that minor changes to these formula and/or to the parameters (GAG properties, GAG forms) measured in a particular sample could be made without having a significant impact on the score or the diagnostic result.

Where the aim is to provide a scoring system in which a high score is indicative of the presence of cancer (e.g. prostate cancer) then conveniently said scoring system can be designed as a ratio or fraction, where the numerator is the sum of the values associated with one or more GAG properties (GAG forms) associated with cancer (e.g. prostate cancer) and the denominator is the sum of the values associated with one or more GAG properties (GAG forms) associated with the healthy state.

Of course, as discussed above, alternative scoring systems (formulae) could equally be designed where for example the numerator is the sum of the values associated with one or more GAG properties (GAG forms) associated with the healthy state and the denominator is the sum of the values associated with one or more GAG properties (GAG forms) associated with cancer (e.g. prostate cancer), and a low score is indicative of the presence of cancer (e.g. prostate cancer).

For instance, alternative scoring methods, scoring systems, markers or formulas can be used that comprises any appropriate combination of the GAG properties in order to arrive at an indication, e.g. in the form of a value or score, which can then be used for diagnosis of cancer (e.g. prostate cancer). For example, said methods etc., can be an algorithm that comprises any appropriate combination of the GAG properties as input, to e.g. perform pattern recognition of the samples, in order to arrive at an indication, e.g. in the form of a value or score, which can then be used for diagnosis of cancer (e.g. prostate cancer). Non-limiting examples of such algorithms include machine learning algorithms that implement classification (algorithmic classifiers), such as linear classifiers (e.g. Fisher's linear discriminant, logistic regression, naive Bayes classifier, perceptron); support vector machines (e.g. least squares support vector machines); quadratic classifiers; kernel estimation (e.g. k-nearest neighbor); boosting; decision trees (e.g. random forests); neural networks; learning vector quantization.

The use of such classifiers, e.g. machine learning classifiers, e.g. random forest classifiers, would be within the skill of a person skilled in the art. For example, such classifiers can conveniently be trained on GAG properties from a training set of samples and then tested in terms of accuracy on a test set of samples. The classifier generates a black-box model that is trained on the most important GAG properties and can thus be used to identify the most important GAG properties which can be used to arrive at an accurate diagnosis of cancer (e.g. prostate cancer).

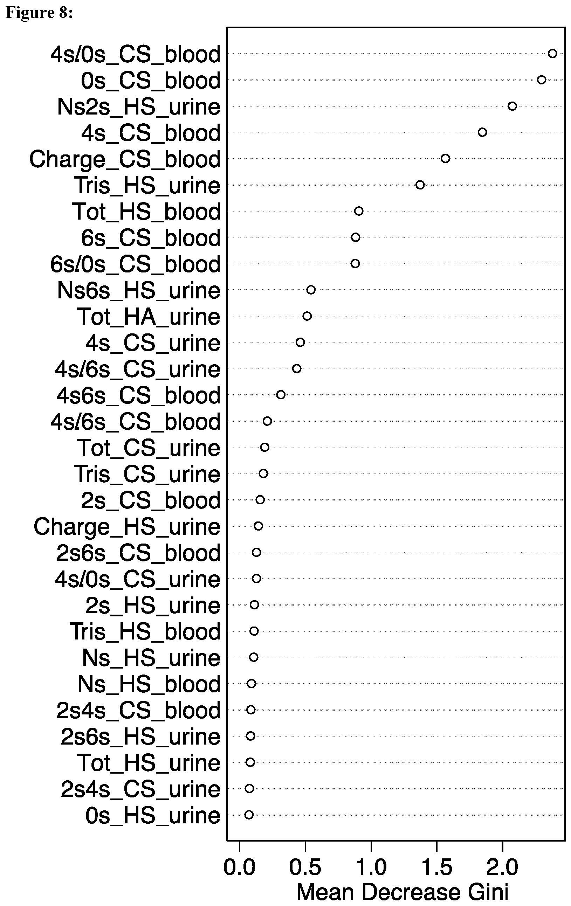

By using a random forest classifier the five most important GAG properties for an accurate prostate cancer diagnosis in blood were (in order): the ratio of 4s CS to 0s CS, 0s CS, 4s CS, charge CS and Total HS. The next nine most important GAG properties in blood were (in order): 6s CS, the ratio of 6s CS to 0s CS, 4s6s CS, the ratio of 4s CS to 6s CS, 2s CS, 2s6s CS, Tris HS, Ns HS and 2s4s CS (see Figure 8). Thus, one or more, two or more, three or more, etc., or all 5 or all 14 of these GAG forms might be measured in the methods of the invention. In preferred embodiments the most accurate form is used first, i.e. the ratio of 4s CS to 0s CS. If other forms are added, then preferably they are added in the order shown in the list, i.e. 0s CS next, etc. In some embodiments the top 4 most accurate forms are used.

By using a random forest classifier the five most important GAG properties for an accurate prostate cancer diagnosis in urine were (in order): Ns2s HS, Tris HS, Ns6s HS, Total HA and 4s CS. The next eleven most important GAG properties in urine were (in order): the ratio of 4s CS to 6s CS, Total CS, Tris CS, charge HS, the ratio of 4s CS to 0s CS, 2s HS, Ns HS, 2s6s HS, Total HS, 2s4s CS and 0s HS (see Figure 8). Thus, one or more, two or more, three or more, etc., or all 5 or all 16 of these GAG forms might be measured in the methods of the invention. In preferred embodiments the most accurate form is used first, i.e. Ns2s HS. If other forms are added, then preferably they are added in the order shown in the list, i.e. the ratio Tris HS next, etc. In some embodiments the top 2 most accurate forms are used.

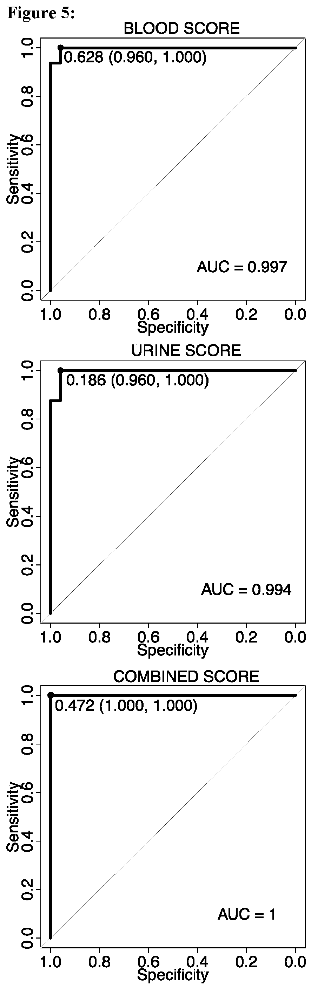

The performance of the three specific prostate cancer scores given above (blood, urine and combined scores) was evaluated using the receiver operating characteristic (ROC) curves and the area under the curve (AUC) was found to be 1 (i.e. a perfect classifier) in the case of the combined score, 0.997 for the blood score and 0.994 for the urine score. This is shown in Figure 5 and Table C. Taken together, these findings demonstrate that alterations in blood and urine GAG chemical composition occurring in prostate cancer can be summarised into scores. In turn, these scores accurately distinguished prostate cancer individuals from healthy individuals and can thus be used for screening, diagnosis, etc..

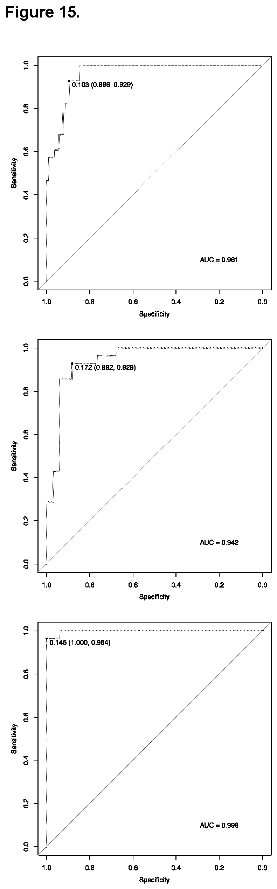

The performance of the three specific cancer scores (Example 2 cancer scores) given above (blood, urine and combined scores) was evaluated using the receiver operating characteristic (ROC) curves and the area under the curve (AUC) was found to be 0.961 for blood, 0.942 for urine, and 0.998 for combined. This is shown in Figure 15. Taken together, these findings demonstrate that alterations in blood and urine GAG chemical composition occurring in cancer can be summarised into scores. In turn, these scores accurately distinguished cancer individuals from healthy individuals and can thus be used for screening, diagnosis, etc..

In the methods of the invention, appropriate threshold or cut-off scores or values can be calculated by methods known in the art, for example from the ROC curve, for use in the methods of the invention. Such cut-off scores or values may be used to declare a sample positive or negative. Thus, for example, in Example 1 herein, for the three specific prostate cancer marker systems discussed above, an optimal threshold (cut-off) score was determined/selected which would maximise accuracy in the classification of prostate cancer subjects as opposed to healthy subjects, i.e. a sample whose marker score is below this threshold (cut-off) value has the maximum probability of not being prostate cancer, or, put another way, a sample whose marker score is above this cut-off value has the maximum probability of being prostate cancer. These cut-off scores were 0.63 (for the blood marker score), 0.19 (for the urine marker score) and 0.47 (for the combined marker score), see Figure 5. This way of determining threshold values could be used for any of the markers described herein. Such threshold (cut-off) scores can then conveniently be used to assess the appropriate samples in subjects and to arrive at a diagnosis. Using the cut-offs described above, prostate cancer subjects could be distinguished from healthy individuals with 97.4% accuracy using the blood or urine score, and 100% accuracy using the combined score. Using an appropriate cut-off or threshold value (used to declare a sample positive or negative), the scores discussed above show excellent results (AUC of 1 for the combined score, AUC of 0.997 for the blood score and AUC of 0.994 for the urine score), with a sensitivity of 100% for all three marker scores and specificities of 100% (for the combined marker score), 96% (for the blood marker score) and 96 % (for the urine marker score), see Table C. Thus, these results show that the above invention provides a simple and accessible test to allow accurate diagnosis of the presence of prostate cancer in an individual.

In one embodiment, the prostate cancer blood score (formula)

$$Blood\ score = \frac{[4s\ CS]}{\frac{4}{10}[0s\ CS]}$$

is used and the cut-off score is 0.63, wherein a score above this cut-off score is indicative of prostate cancer and a score below this cut-off score is indicative of not being prostate cancer (e.g. indicative of a normal or healthy sample). In some embodiments, a score that is at least 5%, at least 10%, at least 20%, at least 30%, at least 40%, at least 50% or more higher than 0.63 is indicative of prostate cancer. In some embodiments, a score that is at least 5%, at least 10%, at least 20%, at least 30%, at least 40%, at least 50% or more lower than 0.63 is indicative of there being no prostate cancer (e.g. indicative of a normal or healthy subject).

In one embodiment, the prostate cancer urine score (formula)

$$Urine\ score = \frac{3[HA] + \frac{6}{10}[Ns2s\ HS]}{\frac{1}{3}[4s\ CS]}$$

is used and the cut-off score is 0.19, wherein a score above this cut-off score is indicative of prostate cancer and a score below this cut-off score is indicative of not being prostate cancer (e.g. indicative of a normal or healthy sample). In some embodiments, a score that is at least 5%, at least 10%, at least 20%, at least 30%, at least 40%, at least 50% or more higher than 0.19 is indicative of prostate cancer. In some embodiments, a score that is at least 5%, at least 10%, at least 20%, at least 30%, at least 40%, at least 50% or more lower than 0.19 is indicative of there being no prostate cancer (e.g. indicative of a normal or healthy subject).

In one embodiment, the prostate cancer combined score (formula)

$$Combined\ score = \frac{Blood\ score + Urine\ score}{2}$$

is used and the cut-off score is 0.47, wherein a score above this cut-off score is indicative of prostate cancer and a score below this cut-off score is indicative of not being prostate cancer (e.g. indicative of a normal or healthy sample). In some embodiments, a score that is at least 5%, at least 10%, at least 20%, at least 30%, at least 40%, at least 50% or more higher than 0.47 is indicative of prostate cancer. In some embodiments, a score that is at least 5%, at least 10%, at least 20%, at least 30%, at least 40%, at least 50% or more lower than 0.47 is indicative of there being no prostate cancer (e.g. indicative of a normal or healthy subject).

As indicated above, a preferred cancer in accordance with the invention is melanoma (e.g. skin melanoma or uveal melanoma).

In some embodiments, in methods of screening for melanoma, particularly preferred GAG forms to be measured or determined (preferably in blood samples) in the methods of the invention are one or more (or all) of: 4s6s CS, 6s CS, 4s CS, Total CS, Total HA, 0s HS, Ns HS and 2s HS. In such embodiments, particularly preferred GAG forms to be measured or determined are one or more (or all) of: 6s CS, 4s CS and Total CS.

In some embodiments, in methods of screening for melanoma the methods involve the determination (preferably in blood samples) of one or more (or all) of: 6s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), Total CS, 2s HS, 0s HS and Total HS. In some such embodiments, an alteration in the level of one or more (or all) of said GAG forms, e.g. in comparison to a control level, is indicative of melanoma.

In some embodiments, in methods of screening for melanoma the methods involve the determination (preferably in blood samples) of one or more (or all) of: 6s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS) and Total CS.

In some embodiments, in methods of screening for melanoma the methods involve the determination (preferably in blood samples) of one or more (or all) of: 2s HS, 0s HS and Total HS.

In some embodiments, in methods of screening for melanoma an increase (e.g. in blood samples) in one or more (or all) of: 6s CS, 4s6s CS, the relative level of 6s CS with respect to 0s CS (e.g. the ratio 6s CS/0s CS) and Total CS, for example in comparison to a control level, is indicative of melanoma in said subject.

In some embodiments, in methods of screening for melanoma a decrease (e.g. in blood samples) in the relative level of 4s CS with respect to 6s CS (e.g. the ratio 4s CS/6s CS), for example in comparison to a control level, is indicative of melanoma in said subject.

In some embodiments, in methods of screening for melanoma an increase (e.g. in blood samples) in Total HS, for example in comparison to a control level, is indicative of melanoma in said subject.

In some embodiments, in methods of screening for melanoma cancer a decrease (e.g. in blood samples) in one or both of: 2s HS and 0s HS, for example in comparison to a control level, is indicative of melanoma in said subject.

Preferred GAG forms (or properties) that may be used in melanoma screening (e.g. in blood samples) are those GAG forms identified as having a % in ROPE value of less than 5.00 in Table M, e.g. less than 4.00. 3.00, 2.00, 1.00 or even a value of 0.00.

In some embodiments, in methods of screening for melanoma an increase (e.g. in blood samples) in one or more (or all) of the GAG properties that are indicated in Table M as having an increased value in melanoma in comparison to healthy may be indicative of melanoma. In some embodiments, in methods of screening for melanoma a decrease (e.g. in blood samples) in one or more (or all) of the GAG properties that are indicated in Table M as having a decreased value in melanoma in comparison to healthy may be indicative of melanoma.

In some embodiments, in methods of screening (e.g. in blood samples) for melanoma the methods involve the determination of one (or both) of: Total CS and 2s HS.

In Example 3 herein, scoring systems (formulae) have been designed using measurements of multiple GAG forms such that a high score (or elevated score) results in a positive diagnosis (i.e. the finding of the presence of melanoma), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table M. Scoring systems are discussed elsewhere herein and that discussion may be applied, *mutatis mutandis,* to methods of screening for (e.g. diagnosing) melanoma.

For melanoma, a preferred scoring system giving rise to a score when a blood sample from a subject is analysed is:

## Melanoma scoring formula #1

$$M\,GAG\,score = \frac{7}{10}\,[4s6s\,CS] + 5\left(\frac{[6s\,CS]}{[6s\,CS] + [4s\,CS]}\right) + \frac{1}{10}\,[Tot\,CS]\left(1 + \frac{1}{[Tot\,HA]}\right)$$
$$-\frac{[0s\,HS]}{[0s\,HS] + [Ns\,HS]} - \frac{7}{1000}\,([2s\,HS] + [0s\,HS])$$

For melanoma, another preferred scoring system giving rise to a score when a blood sample from a subject is analysed is:

## Melanoma scoring formula #2

$$M\,GAG\,score\,2 = \frac{4[6s\,CS] + 5[Tot\,CS]}{100} + 5\left(\frac{[6s\,CS]}{[4s\,CS] + [6s\,CS]}\right)$$

In the above scoring systems the terms in square brackets represent the fraction (mass fraction) of the particular GAG form concerned (disaccharide for the corresponding GAG) (as described elsewhere herein) and [*Tot* CS] is the total concentration of CS (in μg/ml) and [*Tot* HA] is the total concentration of HA (in μg/ml).

The performance of the Melanoma scoring formula #1 given above was evaluated using the receiver operating characteristic (ROC) curves and the area under the curve (AUC) was found to be 0.973. This is described in Example 3.

The performance of the Melanoma scoring formula #2 given above was evaluated using the receiver operating characteristic (ROC) curves and the area under the curve (AUC) was found to be 0.933. This is described in Example 3.

These findings demonstrate that alterations in GAG chemical composition (e.g. in blood samples) occurring in melanoma can be summarised into scores. In turn, these scores accurately distinguished melanoma individuals from healthy individuals and can thus be used for screening, diagnosis, etc.. The discussion elsewhere herein in connection with scoring systems, may be applied, *mutatis mutandis,* to embodiments of the invention in which melanoma is screened for (e.g. diagnosed).

As described elsewhere herein, in the methods of the invention, appropriate threshold or cut-off scores or values can be calculated by methods known in the art, for example from the ROC curve, for use in the methods of the invention. For the melanoma marker (scoring) systems discussed above, optimal thresholds (cut-off) scores were determined/selected which would maximise accuracy in the classification of melanoma subjects as opposed to healthy subjects, i.e. a sample whose marker score is below this threshold (cut-off) value has the maximum probability of not being melanoma, or, put another way, a sample whose marker score is above this cut-off value has the maximum probability of being melanoma.

The optimal cut-off score was 0.92 for Melanoma scoring formula #1. Thus, in one embodiment, Melanoma scoring formula #1 is used and the cut-off score is 0.92, wherein a score above this cut-off score is indicative of melanoma and a score below this cut-off score is indicative of not being melanoma (e.g. indicative of a normal or healthy sample). In some embodiments, a score that is at least 5%, at least 10%, at least 20%, at least 30%, at least 40%, at least 50% or more higher than 0.92 is indicative of melanoma. In some embodiments, a score that is at least 5%, at least 10%, at least 20%, at least 30%, at least 40%, at least 50% or more lower than 0.92 is indicative of there being no melanoma (e.g. indicative of a normal or healthy subject).

The optimal cut-off score was 1.19 for Melanoma scoring formula #2. Thus, in one embodiment, Melanoma scoring formula #2 is used and the cut-off score is 1.19, wherein a score above this cut-off score is indicative of melanoma and a score below this cut-off score is indicative of not being melanoma (e.g. indicative of a normal or healthy sample). In some embodiments, a score that is at least 5%, at least 10%, at least 20%, at least 30%, at least 40%, at least 50% or more higher than 1.19 is indicative of melanoma. In some embodiments, a score that is at least 5%, at least 10%, at least 20%, at least 30%, at least 40%, at least 50% or more lower than 1.19 is indicative of there being no melanoma (e.g. indicative of a normal or healthy subject).

As indicated above, preferred cancers in accordance with the invention include colon cancer and rectum cancer. These cancers may also be referred to collectively as colorectal cancer.

In some embodiments, in methods of screening for colorectal cancer (or colon cancer or rectum cancer), particularly preferred GAG forms to be measured or determined (preferably in blood samples) in the methods of the invention are one or more (or all) of: 6s CS, 4sCS, 2s6s CS, 2sCS, 6sHS, 2s HS and Total HS.

In some embodiments, in methods of screening for colorectal cancer the methods involve the determination (preferably in blood samples) of one or more (or all) of: 2s CS, 6s CS, 2s6s CS, 2s4s CS, 4s6s CS, Tris CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), Total CS, Ns6s HS, Ns2s HS, 2s6s HS, 6s HS, 2s HS, 0s HS, Charge HS and Total HS. In some such embodiments, an alteration in the level of one or more (or all) of said GAG forms, e.g. in comparison to a control level, is indicative of colorectal cancer.

In some embodiments, in methods of screening for colorectal cancer the methods involve the determination (preferably in blood samples) of one or more (or all) of: 2s CS, 6s CS, 2s6s CS, 2s4s CS, 4s6s CS, Tris CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS) and Total CS.

In some embodiments, in methods of screening for colorectal cancer the methods involve the determination (preferably in blood samples) of one or more (or all) of: Ns6s HS, Ns2s HS, 2s6s HS, 6s HS, 2s HS, 0s HS, Charge HS and Total HS.

In some embodiments, in methods of screening for colorectal cancer an increase (e.g. in blood samples) in one or more (or all) of: 2s CS, 6s CS, 2s6s CS, 2s4s CS, 4s6s CS, Tris CS, the relative level of 6s CS with respect to 0s CS (e.g. the ratio 6s CS/0s CS) and Total CS, for example in comparison to a control level, is indicative of colorectal cancer in said subject.

In some embodiments, in methods of screening for colorectal cancer a decrease (e.g. in blood samples) in the relative level of 4s CS with respect to 6s CS (e.g. the ratio 4s CS/6s CS), for example in comparison to a control level, is indicative of colorectal cancer in said subject.

In some embodiments, in methods of screening for colorectal cancer an increase (e.g. in blood samples) in one or more (or all) of: 6s HS, 2s HS, Charge HS and Total HS, for example in comparison to a control level, is indicative of colorectal cancer in said subject.

In some embodiments, in methods of screening for colorectal cancer a decrease (e.g. in blood samples) in one (or both) of: Ns6s HS, Ns2s HS, 2s6s HS and 0s HS, for example in comparison to a control level, is indicative of colorectal cancer in said subject.

Preferred GAG forms (or properties) that may be used in colorectal cancer screening (e.g. in blood samples) are those GAG forms identified as having a % in ROPE value of less than 5.00 in Table O, e.g. less than 4.00. 3.00, 2.00, 1.00 or even a value of 0.00.

In some embodiments, in methods of screening for colorectal cancer an increase (e.g. in blood samples) in one or more (or all) of the GAG properties that are indicated in Table O as having an increased value in colorectal cancer in comparison to healthy may be indicative of colorectal cancer. In some embodiments, in methods of screening for colorectal cancer a decrease (e.g. in blood samples) in one or more (or all) of the GAG properties that are indicated in Table O as having a decreased value in colorectal cancer in comparison to healthy may be indicative of colorectal cancer.

In some embodiments, in methods of screening (e.g. in blood samples) for colorectal cancer the methods involve the determination of one or more (or all) of: Tris CS, Total CS, Ns6s HS, 2s6s HS, 6s HS and 2s HS.

In Example 4 herein, a scoring system (formula) has been designed using measurements of multiple GAG forms such that a high score (or elevated score) results in a positive diagnosis (i.e. the finding of the presence of colorectal cancer), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table O. Scoring systems are discussed elsewhere herein and that discussion may be applied, *mutatis mutandis,* to methods of screening for (e.g. diagnosing) colorectal cancer.

For colorectal cancer, a preferred scoring system giving rise to a score when a blood sample from a subject is analysed is:

$$CRC\ GAG\ score = \frac{5}{2}\left(\frac{[6s\ CS]}{[4s\ CS]+[6s\ CS]}\right)+\frac{2}{15}\left(\frac{[2s6s\ CS]}{[2s6s\ CS]+[2s\ CS]}\right)+\frac{1}{300}[6s\ HS]$$

$$+\frac{1}{30}([2s\ HS]+Tot\ HS)$$

In the above scoring system the terms in square brackets represent the fraction (mass fraction) of the particular GAG form concerned (disaccharide for the corresponding GAG) (as described elsewhere herein) and *Tot HS* is the total concentration of HS (in μg/ml).

The performance of the colorectal cancer disease score given above was evaluated using the receiver operating characteristic (ROC) curves and the area under the curve (AUC) was found to be 0.998. This is described in Example 4. These findings demonstrate that alterations in GAG chemical composition (e.g. in blood samples) occurring in colorectal cancer can be summarised into scores. In turn, these scores accurately distinguished colorectal cancer individuals from healthy individuals and can thus be used for screening, diagnosis, etc.. The discussion elsewhere herein in connection with scoring systems, may be applied, *mutatis mutandis,* to embodiments of the invention in which colorectal cancer is screened for (e.g. diagnosed).

As described elsewhere herein, in the methods of the invention, appropriate threshold or cut-off scores or values can be calculated by methods known in the art, for example from the ROC curve, for use in the methods of the invention. For the colorectal cancer marker (scoring) system discussed above, an optimal threshold (cut-off) score was determined/-selected which would maximise accuracy in the classification of colorectal cancer subjects as opposed to healthy subjects, i.e. a sample whose marker score is below this threshold (cut-off) value has the maximum probability of not being colorectal cancer, or, put another way, a sample whose marker score is above this cut-off value has the maximum probability of being colorectal cancer. This optimal cut-off score was 0.74. Thus, in one embodiment, the cut-off score is 0.74, wherein a score above this cut-off score is indicative of colorectal cancer and a score below this cut-off score is indicative of not being colorectal cancer (e.g. indicative of a normal or healthy sample). In some embodiments, a score that is at least 5%, at least 10%, at least 20%, at least 30%, at least 40%, at least 50% or more higher than 0.74 is indicative of colorectal cancer. In some embodiments, a score that is at least 5%, at least 10%, at least 20%, at least 30%, at least 40%, at least 50% or more lower than 0.74 is indicative of there being no colorectal cancer (e.g. indicative of a normal or healthy subject).

As indicated above, a preferred cancer in accordance with the invention is neuroendocrine tumour (e.g. gastrointestinal neuroendocrine tumour, GNET).

In some embodiments, in methods of screening for neuroendocrine tumour (preferably GNET), particularly preferred GAG forms to be measured or determined (preferably in blood samples) in the methods of the invention are one or more (or all) of: 2s6s CS, Tris CS, Charge CS, 2s HS and 0s HS.

In some embodiments, in methods of screening for a neuroendocrine tumour (preferably GNET) the methods involve the determination (preferably in blood samples) of one or more (or all) of: 0s CS, 6s CS, 4s CS, 2s6s CS, 2s4s CS, 4s6s CS, Tris CS, the relative level of 6s CS with respect to 4s CS (e.g. the ratio of 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Ns6s HS, Ns HS, 6s HS, 2s HS, 0s HS, Charge HS and Total HS. In some such embodiments, an alteration in the level of one or more (or all) of said GAG forms, e.g. in comparison to a control level, is indicative of a neuroendocrine tumour (preferably GNET).

In some embodiments, in methods of screening for neuroendocrine tumour (preferably GNET) the methods involve the determination (preferably in blood samples) of one or more (or all) of: 0s CS, 6s CS, 4s CS, 2s6s CS, 2s4s CS, 4s6s CS, Tris CS, the relative level of 6s CS with respect to 4s CS (e.g. the ratio of 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS) and Charge CS.

In some embodiments, in methods of screening for neuroendocrine tumour (preferably GNET) the methods involve the determination (preferably in blood samples) of one or more (or all) of: Ns6s HS, Ns HS, 6s HS, 2s HS, 0s HS blood, Charge HS and Total HS.

In some embodiments, in methods of screening for neuroendocrine tumour (preferably GNET) an increase (e.g. in blood samples) in one or more (or all) of: 6s CS, 4s CS, 2s6s CS, 2s4s CS, 4s6s CS, Tris CS, the relative level of 6s CS with respect to 0s CS (e.g. the ratio 6s CS/0s CS), the relative level of 4s CS with respect to 0s CS (e.g. the ratio 4s CS/0s CS) and Charge CS, for example in comparison to a control level, is indicative of neuroendocrine tumour (preferably GNET) in said subject.

In some embodiments, in methods of screening for a neuroendocrine tumour (preferably GNET) a decrease (e.g. in blood samples) in 0s CS, the relative level of 4s CS with respect to 6s CS (e.g. the ratio 4s CS/6s CS), for example in comparison to a control level, is indicative of a neuroendocrine tumour (preferably GNET) in said subject.

In some embodiments, in methods of screening for neuroendocrine tumour (preferably GNET) an increase (e.g.

in blood samples) in one or more (or all) of: Ns HS, 6s HS, 2s HS, Charge HS and Total HS, for example in comparison to a control level, is indicative of a neuroendocrine tumour (preferably GNET) in said subject.

In some embodiments, in methods of screening for neuroendocrine tumour (preferably. GNET) a decrease (e.g. in blood samples) in one (or both) of: Ns6s HS and 0s HS, for example in comparison to a control level, is indicative of a neuroendocrine tumour (preferably GNET) in said subject.

Preferred GAG forms (or properties) that may be used in neuroendocrine tumour screening (preferably GNET) (e.g. in blood samples) are those GAG forms identified as having a % in ROPE value of less than 5.00 in Table Q, e.g. less than 4.00. 3.00, 2.00, 1.00 or even a value of 0.00.

In some embodiments, in methods of screening for neuroendocrine tumour screening (preferably GNET) an increase (e.g. in blood samples) in one or more (or all) of the GAG properties that are indicated in Table Q as having an increased value in GNET in comparison to healthy may be indicative of a neuroendocrine tumour (e.g. GNET). In some embodiments, in methods of screening for neuroendocrine tumour screening (preferably GNET) a decrease (e.g. in blood samples) in one or more (or all) of the GAG properties that are indicated in Table Q as having a decreased value in GNET in comparison to healthy may be indicative of a neuroendocrine tumour (e.g. GNET).

In some embodiments, in methods of screening (e.g. in blood samples) for neuroendocrine tumour screening (preferably GNET) the methods involve the determination of one or more (or all) of: 0s CS, Tris CS, Ns6s HS, 6s HS and 2s HS.

In Example 5 herein, a scoring system (formula) has been designed using measurements of multiple GAG forms such that a high score (or elevated score) results in a positive diagnosis (i.e. the finding of the presence of neuroendocrine tumour, particularly GNET), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table Q. Scoring systems are discussed elsewhere herein and that discussion may be applied, *mutatis mutandis,* to methods of screening for (e.g. diagnosing) a neuroendocrine tumour (preferably GNET).

For neuroendocrine tumour (preferably GNET), a preferred scoring system giving rise to a score when a blood sample from a subject is analysed is:

$$GNET\ GAG\ score = \frac{3}{4}[2s6s\ CS] - \frac{3}{20}[Tris\ CS] + 2\ Charge\ CS + \frac{1}{30}[2s\ HS] - \frac{1}{300}[0s\ HS]$$

In the above scoring system the terms in square brackets represent the fraction (mass fraction) of the particular GAG form concerned (disaccharide for the corresponding GAG) (as described elsewhere herein) and *Charge CS* is the weighted charge of CS.

The performance of the GNET disease score given above was evaluated using the receiver operating characteristic (ROC) curves and the area under the curve (AUC) was found to be 1 (perfect classifier). This is described in Example 5. These findings demonstrate that alterations in GAG chemical composition (e.g. in blood samples) occurring in neuroendocrine tumour subjects (particularly GNET subjects) can be summarised into scores. In turn, these scores accurately distinguished neuroendocrine tumour individuals (particularly GNET) from healthy individuals and can thus be used for screening, diagnosis, etc.. The discussion elsewhere herein in connection with scoring systems, may be applied, *mutatis mutandis,* to embodiments of the invention in which neuroendocrine tumour (e.g. GNET) is screened for (e.g. diagnosed).

As described elsewhere herein, in the methods of the invention, appropriate threshold or cut-off scores or values can be calculated by methods known in the art, for example from the ROC curve, for use in the methods of the invention. For the GNET marker (scoring) system discussed above, an optimal threshold (cut-off) score was determined/selected which would maximise accuracy in the classification of neuroendocrine tumour (particularly GNET) subjects as opposed to healthy subjects, i.e. a sample whose marker score is below this threshold (cut-off) value has the maximum probability of not being neuroendocrine tumour (particularly GNET), or, put another way, a sample whose marker score is above this cut-off value has the maximum probability of being neuroendocrine tumour (particularly GNET). This optimal cut-off score was 0.90. Thus, in one embodiment, the cut-off score is 0.90, wherein a score above this cut-off score is indicative of neuroendocrine tumour (preferably GNET) and a score below this cut-off score is indicative of not being neuroendocrine tumour (preferably GNET) (e.g. indicative of a normal or healthy sample). In some embodiments, a score that is at least 5%, at least 10%, at least 20%, at least 30%, at least 40%, at least 50% or more higher than 0.90 is indicative of a neuroendocrine tumour (preferably GNET). In some embodiments, a score that is at least 5%, at least 10%, at least 20%, at least 30%, at least 40%, at least 50% or more lower than 0.90 is indicative of there being no neuroendocrine tumour (preferably GNET) (e.g. indicative of a normal or healthy subject).

As indicated above, a preferred cancer in accordance with the invention is blood cancer (e.g. chronic lymphoid leukaemia, CLL).

In some embodiments, in methods of screening for blood cancer (preferably CLL), particularly preferred GAG forms to be measured or determined (preferably in blood samples) in the methods of the invention are one or more (or all) of: 6s CS, 2s6s CS, 4s6s CS, Charge CS, 6s HS and 0s HS. In some embodiments, particularly preferred GAG forms to be measured or determined are one or more (or all) of: 6s CS, 2s6s CS, 4s6s CS, 4s CS and 6s CS (and optionally the relative level of 6s CS with respect to 4s CS (e.g. the ratio 6s CS/4s CS or the inverse ratio 4s CS/6s CS)).

In some embodiments, in methods of screening for blood cancer (preferably CLL) the methods involve the determination (preferably in blood samples) of one or more (or all) of: 0s CS, 6s CS, 4s CS, 2s6s CS, 2s4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Total CS, Ns6s HS, Ns HS, 2s6s HS, 6s HS, 2s HS, 0s HS, Charge HS and Total HS. In some such embodiments, an alteration in the level of one or more (or all) of said GAG forms, e.g. in comparison to a control level, is indicative of blood cancer (preferably CLL).

In some embodiments, in methods of screening for blood cancer (preferably CLL) the methods involve the determination (preferably in blood samples) of one or more (or all) of: 0s CS, 6s CS, 4s CS, 2s6s CS, 2s4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS and Total CS.

In some embodiments, in methods of screening for blood cancer (preferably CLL) the methods involve the determination (preferably in blood samples) of one or more (or all) of: Ns6s HS, Ns HS, 2s6s HS, 6s HS, 2s HS, 0s HS, Charge HS and Total HS.

In some embodiments, in methods of screening for blood cancer (preferably CLL) an increase (e.g. in blood samples) in one or more (or all) of: 6s CS, 4s CS, 2s6s CS, 2s4s CS, 4s6s CS, the relative level of 6s CS with respect to 0s CS (e.g. the ratio 6s CS/0s CS), the relative level of 4s CS with respect to 0s CS (e.g. the ratio 4s CS/0s CS), Charge CS and Total CS, for example in comparison to a control level, is indicative of blood cancer (preferably CLL) in said subject.

In some embodiments, in methods of screening for blood cancer (preferably CLL) a decrease (e.g. in blood samples) in one (or both) of: 0s CS and the relative level of 4s CS with respect to 6s CS (e.g. the ratio 4s CS/6s CS), for example in comparison to a control level, is indicative of blood cancer (preferably CLL) in said subject.

In some embodiments, in methods of screening for blood cancer (preferably CLL) an increase (e.g. in blood samples) in one or more (or all) of: Ns HS, 6s HS, 2s HS, Charge HS and Total HS, for example in comparison to a control level, is indicative of blood cancer (preferably CLL) in said subject.

In some embodiments, in methods of screening for blood cancer (preferably CLL) cancer a decrease (e.g. in blood samples) in one or more (or all) of: Ns6s HS, 2s6s HS and 0s HS, for example in comparison to a control level, is indicative of blood cancer (preferably CLL) in said subject.

Preferred GAG forms (or properties) that may be used in blood cancer (preferably CLL) screening (e.g. in blood samples) are those GAG forms identified as having a % in ROPE value of less than 5.00 in Table S, e.g. less than 4.00. 3.00, 2.00, 1.00 or even a value of 0.00.

In some embodiments, in methods of screening for blood cancer (preferably CLL) an increase (e.g. in blood samples) in one or more (or all) of the GAG properties that are indicated in Table S as having an increased value in CLL in comparison to healthy may be indicative of blood cancer (preferably CLL). In some embodiments, in methods of screening for blood cancer (preferably CLL) a decrease (e.g. in blood samples) in one or more (or all) of the GAG properties that are indicated in Table S as having a decreased value in CLL in comparison to healthy may be indicative of blood cancer (preferably CLL).

In some embodiments, in methods of screening (e.g. in blood samples) for blood cancer (preferably CLL) the methods involve the determination of one (or both) of: 0s CS, Total CS, Ns6s HS, 2s6s HS, 6s HS and 2s HS.

In Example 6 herein, scoring systems (formulae) have been designed using measurements of multiple GAG forms such that a high score (or elevated score) results in a positive diagnosis (i.e. the finding of the presence of blood cancer, particularly CLL), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table S. Scoring systems are discussed elsewhere herein and that discussion may be applied, *mutatis mutandis,* to methods of screening for (e.g. diagnosing) blood cancer (preferably CLL).

For blood cancer (preferably CLL), a preferred scoring system giving rise to a score when a blood sample from a subject is analysed is:

<u>CLL scoring formula #1</u>

$$CLL\ GAG\ score = \frac{2}{15}[6s\ CS] + \frac{2}{3}[2s6s\ CS] - [4s6s\ CS] + \frac{1}{2}Charge\ CS - \frac{1}{150}[6s\ HS]$$
$$- \frac{1}{250}[0s\ HS] + \frac{2}{15}Tot\ HS$$

For blood cancer (preferably CLL), another preferred scoring system giving rise to a score when a blood sample from a subject is analysed is:

<u>CLL scoring formula #2</u>

$$CLL\ GAG\ score = \frac{1}{12}[6s\ CS] + \frac{4}{7}[2s6s\ CS] - [4s6s\ CS] - \frac{1}{800}\left(\frac{[4s\ CS]}{[6s\ CS]}\right)$$

In the above scoring systems the terms in square brackets represent the fraction (mass fraction) of the particular GAG form concerned (disaccharide for the corresponding GAG) (as described elsewhere herein) and [*Tot HS*] is the total concentration of HS (in µg/ml) and *Charge* CS is the weighted charge of CS.

The performance of the CLL scoring formula #1 given above was evaluated using the receiver operating characteristic (ROC) curves and the area under the curve (AUC) was found to be 0.974. This is described in Example 6.

The performance of the CLL scoring formula #2 given above was evaluated using the receiver operating characteristic (ROC) curves and the area under the curve (AUC) was found to be 0.974. This is described in Example 6.

These findings demonstrate that alterations in GAG chemical composition (e.g. in blood samples) occurring in blood cancer (preferably CLL) can be summarised into scores. In turn, these scores accurately distinguished blood cancer (particularly CLL) individuals from healthy individuals and can thus be used for screening, diagnosis, etc.. The discussion elsewhere herein in connection with scoring systems, may be applied, *mutatis mutandis,* to embodiments of the invention in which blood cancer (preferably CLL) is screened for (e.g. diagnosed).

As described elsewhere herein, in the methods of the invention, appropriate threshold or cut-off scores or values can be calculated by methods known in the art, for example from the ROC curve, for use in the methods of the invention. For the CLL marker (scoring) systems discussed above, optimal thresholds (cut-off) scores were determined/selected which would maximise accuracy in the classification of blood cancer (particularly CLL) subjects as opposed to healthy subjects, i.e. a sample whose marker score is below this threshold (cut-off) value has the maximum probability of not being blood cancer (particularly CLL), or, put another way, a sample whose marker score is above this cut-off value has the maximum probability of being blood cancer (particularly CLL).

The optimal cut-off score was 0.25 for CLL scoring formula #1. Thus, in one embodiment, CLL scoring formula #1 is used and the cut-off score is 0.25, wherein a score above this cut-off score is indicative of blood cancer (preferably CLL) and a score below this cut-off score is indicative of not being blood cancer (preferably CLL) (e.g. indicative of a normal or healthy sample). In some embodiments, a score that is at least 5%, at least 10%, at least 20%, at least 30%, at least 40%, at least 50% or more higher than 0.25 is indicative of blood cancer (preferably CLL). In some embodiments, a score that is at least 5%, at least 10%, at least 20%, at least 30%, at least 40%, at least 50% or more lower than 0.25 is indicative of there being no blood cancer (preferably CLL) (e.g. indicative of a normal or healthy subject).

The optimal cut-off score was 0.23 for CLL scoring formula #2. Thus, in one embodiment, CLL scoring formula #2 is used and the cut-off score is CLL, wherein a score above this cut-off score is indicative of blood cancer (preferably CLL) and a score below this cut-off score is indicative of not being blood cancer (preferably CLL) (e.g. indicative of a normal or healthy sample). In some embodiments, a score that is at least 5%, at least 10%, at least 20%, at least 30%, at least 40%, at least 50% or more higher than 0.23 is indicative of blood cancer (preferably CLL). In some embodiments, a score that is at least 5%, at least 10%, at least 20%, at least 30%, at least 40%, at least 50% or more lower than 0.23 is indicative of there being no blood cancer (preferably CLL) (e.g. indicative of a normal or healthy subject).

As indicated above, a preferred cancer in accordance with the invention is bladder cancer (BCa).

In some embodiments, in methods of screening for BCa particularly preferred GAG forms to be measured or determined (preferably in urine samples) in the methods of the invention are one or more (or all) of: Charge CS, Tris HS, Ns HS, 2s HS, Total HS.

In some embodiments, in methods of screening for BCa the methods involve the determination (preferably in urine samples) of one or more (or all) of: 0s CS, 2s CS, 6s CS, Tris CS, the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Tris HS, Ns2s HS, Ns HS, 2s HS, Total HS. In some such embodiments, an alteration in the level of one or more (or all) of said GAG forms, e.g. in comparison to a control level, is indicative of bladder cancer.

In some embodiments, in methods of screening for BCa the methods involve the determination (preferably in urine samples) of one or more (or all) of: 0s CS, 2s CS, 6s CS, Tris CS, the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS) and Charge CS.

In some embodiments, in methods of screening for BCa the methods involve the determination (preferably in urine samples) of one or more (or all) of: Tris HS, Ns2s HS, Ns HS, 2s HS and Total HS.

In some embodiments, in methods of screening for BCa an increase (e.g. in urine samples) in one or more (or all) of: 2s CS, 6s CS, the relative level of 6s CS with respect to 0s CS (e.g. the ratio 6s CS/0s CS), the relative level of 4s CS with respect to 0s CS (e.g. the ratio 4s CS/0s CS) and Charge CS, for example in comparison to a control level, is indicative of BCa in said subject.

In some embodiments, in methods of screening for BCa a decrease (e.g. in urine samples) in one (or both) of: 0s CS and Tris CS, for example in comparison to a control level, is indicative of BCa in said subject.

In some embodiments, in methods of screening for BCa an increase (e.g. in urine samples) in one or more (or all) of: Tris HS, Ns2s HS, 2s HS and Total HS, for example in comparison to a control level, is indicative of BCa in said subject.

In some embodiments, in methods of screening for BCa a decrease (e.g. in urine samples) in Ns HS, for example in comparison to a control level, is indicative of BCa in said subject.

Preferred GAG forms (or properties) that may be used in BCa screening (e.g. in urine samples) are those GAG forms identified as having a % in ROPE value of less than 5.00 in Table T, e.g. less than 4.00. 3.00, 2.00, 1.00 or even a value of 0.00.

In some embodiments, in methods of screening for BCa an increase (e.g. in urine samples) in one or more (or all) of the GAG properties that are indicated in Table T as having an increased value in BCa in comparison to healthy may be indicative of BCa. In some embodiments, in methods of screening for BCa a decrease (e.g. in urine samples) in one or more (or all) of the GAG properties that are indicated in Table T as having a decreased value in BCa in comparison to healthy may be indicative of BCa.

In some embodiments, in methods of screening (e.g. in urine samples) for BCa the methods involve the determination of 2s CS.

In Example 7 herein, a scoring system (formula) has been designed using measurements of multiple GAG forms such that a high score (or elevated score) results in a positive diagnosis (i.e. the finding of the presence of BCa), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table T. Scoring systems are discussed elsewhere herein and that discussion may be applied, *mutatis mutandis,* to methods of screening for (e.g. diagnosing) BCa.

For BCa, a preferred scoring system giving rise to a score when a urine sample from a subject is analysed is:

$$BCa\ GAG\ score = \frac{3}{4}Charge\ CS + \frac{1}{3}[Tris\ HS] - \frac{1}{50}[Ns\ HS] + \frac{1}{250}[2s\ HS] + \frac{3}{4}Tot\ HS$$

In the above scoring system the terms in square brackets represent the fraction (mass fraction) of the particular GAG form concerned (disaccharide for the corresponding GAG) (as described elsewhere herein), *Charge CS* is the weighted charge of CS and *Tot HS* is the total concentration of HS (in $\mu$g/ml).

The performance of the BCa disease score given above was evaluated using the receiver operating characteristic (ROC) curves and the area under the curve (AUC) was found to be 1.0. This is described in Example 7. These findings demonstrate that alterations in GAG chemical composition (e.g. in urine samples) occurring in BCa can be summarised into scores. In turn, these scores accurately distinguished BCa individuals from healthy individuals and can thus be used for screening, diagnosis, etc.. The discussion elsewhere herein in connection with scoring systems, may be applied, *mutatis mutandis,* to embodiments of the invention in which BCa is screened for (e.g. diagnosed).

As described elsewhere herein, in the methods of the invention, appropriate threshold or cut-off scores or values can be calculated by methods known in the art, for example from the ROC curve, for use in the methods of the invention. For the BCa marker (scoring) system discussed above, an optimal threshold (cut-off) score was determined/selected which would maximise accuracy in the classification of BCa subjects as opposed to healthy subjects, i.e. a sample whose marker score is below this threshold (cut-off) value has the maximum probability of not being BCa, or, put another way, a sample whose marker score is above this cut-off value has the maximum probability of being BCa. This optimal cut-off score was 0.92. Thus, in one embodiment, the cut-off score is 0.92, wherein a score above this cut-off score is indicative of BCa and a score below this cut-off score is indicative of not being BCa (e.g. indicative of a normal or healthy sample). In some embodiments, a score that is at least 5%, at least 10%, at least 20%, at least 30%, at least 40%, at least 50% or more higher than 0.92 is indicative of BCa. In some embodiments, a score that is at least 5%, at least 10%, at least 20%, at least 30%, at least 40%, at least 50% or more lower than 0.92 is indicative of there being no BCa (e.g. indicative of a normal or healthy subject).

As indicated above, a preferred cancer in accordance with the invention is breast cancer (BC).

In some embodiments, in methods of screening for BC particularly preferred GAG forms to be measured or determined (preferably in blood samples) in the methods of the invention are one or more (or all) of: 6s CS, 0s CS, Charge CS and Total CS. In some such embodiments the relative level of 6s CS with respect to 0s CS (e.g. the ratio 6s CS/0s CS or the inverse ratio 0s CS/6s CS) is measured or determined.

In some embodiments, in methods of screening for BC the methods involve the determination (preferably in blood samples) of one or more (or all) of: 0s CS, 6s CS, 4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Total CS, Ns2s HS, Ns HS, 6s HS, 0s HS and Total HS. In some such embodiments, an alteration in the level of one or more (or all) of said GAG forms, e.g. in comparison to a control level, is indicative of breast cancer.

In some embodiments, in methods of screening for BC the methods involve the determination (preferably in blood samples) of one or more (or all) of: 0s CS, 6s CS, 4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS and Total CS.

In some embodiments, in methods of screening for BC the methods involve the determination (preferably in blood samples) of one or more (or all) of: Ns2s HS, Ns HS, 6s HS, 0s HS and Total HS.

In some embodiments, in methods of screening for BC an increase (e.g. in blood samples) in one or more (or all) of: 6s CS, 4s CS, 4s6s CS, the relative level of 6s CS with respect to 0s CS (e.g. the ratio 6s CS/0s CS), the relative level of 4s CS with respect to 0s CS (e.g. the ratio 4s CS/0s CS), Charge CS and Total CS, for example in comparison to a control level, is indicative of BC in said subject.

In some embodiments, in methods of screening for BC a decrease (e.g. in blood samples) in one (or both) of: 0s CS and the relative level of 4s CS with respect to 6s CS (e.g. the ratio 4s CS/6s CS), for example in comparison to a control level, is indicative of BC in said subject.

In some embodiments, in methods of screening for BC an increase (e.g. in blood samples) in one or more (or all) of: Ns HS, 6s HS and Total HS, for example in comparison to a control level, is indicative of BC in said subject.

In some embodiments, in methods of screening for BC a decrease (e.g. in blood samples) in one (or both) of: Ns2s HS and 0s HS, for example in comparison to a control level, is indicative of BC in said subject.

Preferred GAG forms (or properties) that may be used in BC screening (e.g. in blood samples) are those GAG forms identified as having a % in ROPE value of less than 5.00 in Table V, e.g. less than 4.00. 3.00, 2.00, 1.00 or even a value of 0.00.

In some embodiments, in methods of screening for BC an increase (e.g. in blood samples) in one or more (or all) of the GAG properties that are indicated in Table V as having an increased value in BC in comparison to healthy may be indicative of BC. In some embodiments, in methods of screening for BC a decrease (e.g. in blood samples) in one or more (or all) of the GAG properties that are indicated in Table V as having a decreased value in BC in comparison to healthy may be indicative of BC.

In some embodiments, in methods of screening (e.g. in blood samples) for BC the methods involve the determination of one or more (or or all) of: 0s CS, Total CS and 6s HS.

In Example 8 herein, a scoring system (formula) has been designed using measurements of multiple GAG forms such that a high score (or elevated score) results in a positive diagnosis (i.e. the finding of the presence of BC), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table V. Scoring systems are discussed elsewhere herein and that discussion may be applied, *mutatis mutandis,* to methods of screening for (e.g. diagnosing) BC.

For BC, a preferred scoring system giving rise to a score when a blood sample from a subject is analysed is:

$$BC\ GAG\ score = 5\left(\frac{[6s\ CS]}{[0s\ CS]}\right) + \frac{5}{3}Charge\ CS + \frac{1}{50}Tot\ CS$$

In the above scoring system the terms in square brackets represent the fraction (mass fraction) of the particular GAG form concerned (disaccharide for the corresponding GAG) (as described elsewhere herein), *Charge* CS is the weighted charge of CS and *Tot* CS is the total concentration of CS (in μg/ml).

The performance of the BC disease score given above was evaluated using the receiver operating characteristic (ROC) curves and the area under the curve (AUC) was found to be 1 (perfect classifier). This is described in Example 8. These findings demonstrate that alterations in GAG chemical composition (e.g. in blood samples) occurring in BC can be summarised into scores. In turn, these scores accurately distinguished BC individuals from healthy individuals and can thus be used for screening, diagnosis, etc.. The discussion elsewhere herein in connection with scoring systems, may be applied, *mutatis mutandis,* to embodiments of the invention in which BC is screened for (e.g. diagnosed).

As described elsewhere herein, in the methods of the invention, appropriate threshold or cut-off scores or values can be calculated by methods known in the art, for example from the ROC curve, for use in the methods of the invention. For the BC marker (scoring) system discussed above, an optimal threshold (cut-off) score was determined/selected which would maximise accuracy in the classification of BC subjects as opposed to healthy subjects, i.e. a sample whose marker score is below this threshold (cut-off) value has the maximum probability of not being BC, or, put another way, a sample whose marker score is above this cut-off value has the maximum probability of being BC. This optimal cut-off score was 0.94. Thus, in one embodiment, the cut-off score is 0.94, wherein a score above this cut-off score is indicative of BC and a score below this cut-off score is indicative of not being BC (e.g. indicative of a normal or healthy sample). In some embodiments, a score that is at least 5%, at least 10%, at least 20%, at least 30%, at least 40%, at least 50% or more higher than 0.94 is indicative of BC. In some embodiments, a score that is at least 5%, at least 10%, at least 20%, at least 30%, at least 40%, at least 50% or more lower than 0.94 is indicative of there being no BC (e.g. indicative of a normal or healthy subject).

As indicated above, a preferred cancer in accordance with the invention is ovarian cancer (OV).

In some embodiments, in methods of screening for OV particularly preferred GAG forms to be measured or determined (preferably in blood samples) in the methods of the invention are one or more (or all) of: Charge CS, Total CS, Total HS and Total HA.

In some embodiments, in methods of screening for OV the methods involve the determination (preferably in blood samples) of one or more (or all) of: 0s CS, 6s CS, 4s CS, 2s6s CS, 2s4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Total CS, Total HS and Total HA. In some such embodiments, an alteration in the level of one or more (or all) of said GAG forms, e.g. in comparison to a control level, is indicative of ovarian cancer.

In some embodiments, in methods of screening for OV the methods involve the determination (preferably in blood samples) of one or more (or all) of: 0s CS, 6s CS, 4s CS, 2s6s CS, 2s4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS and Total CS.

In some embodiments, in methods of screening for OV the methods involve the determination (preferably in blood samples) of Total HS.

In some embodiments, in methods of screening for OV the methods involve the determination (preferably in blood samples) of Total HA.

In some embodiments, in methods of screening for OV an increase (e.g. in blood samples) in one or more (or all) of: 6s CS, 4s CS, 2s6s CS, 2s4s CS, 4s6s CS, the relative level of 6s CS with respect to 0s CS (e.g. the ratio 6s CS/0s CS), the relative level of 4s CS with respect to 0s CS (e.g. the ratio 4s CS/0s CS), Charge CS and Total CS, for example in comparison to a control level, is indicative of OV in said subject.

In some embodiments, in methods of screening for OV a decrease (e.g. in blood samples) in one (or both) of: 0s CS and the relative level of 4s CS with respect to 6s CS (e.g. the ratio 4s CS/6s CS), for example in comparison to a control level, is indicative of OV in said subject.

In some embodiments, in methods of screening for OV an increase (e.g. in blood samples) in Total HA, for example in comparison to a control level, is indicative of OV in said subject.

In some embodiments, in methods of screening for OV an increase (e.g. in blood samples) in Total HS, for example in comparison to a control level, is indicative of OV in said subject.

Preferred GAG forms (or properties) that may be used in OV screening (e.g. in blood samples) are those GAG forms identified as having a % in ROPE value of less than 5.00 in Table X, e.g. less than 4.00. 3.00, 2.00, 1.00 or even a value of 0.00.

In some embodiments, in methods of screening for OV an increase (e.g. in blood samples) in one or more (or all) of the GAG properties that are indicated in Table X as having an increased value in OV in comparison to healthy may be indicative of OV. In some embodiments, in methods of screening for OV a decrease (e.g. in blood samples) in one or more (or all) of the GAG properties that are indicated in Table X as having a decreased value in OV in comparison to healthy may be indicative of OV.

In some embodiments, in methods of screening (e.g. in blood samples) for OV the methods involve the determination of one (or both) of: 0s CS, and Total CS.

In Example 9 herein, a scoring system (formula) has been designed using measurements of multiple GAG forms such that a high score (or elevated score) results in a positive diagnosis (i.e. the finding of the presence of OV), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table X. Scoring systems are discussed elsewhere herein and that discussion may be applied, *mutatis mutandis,* to methods of screening for (e.g. diagnosing) OV.

For OV, a preferred scoring system giving rise to a score when a blood sample from a subject is analysed is:

$$OV\ GAG\ score = 2\ Charge\ CS + \frac{1}{50}(Tot\ CS - TotHS) + \frac{1}{2}Tot\ HA$$

In the above scoring system *Charge* CS is the weighted charge of CS, *Tot* CS is the total concentration of CS (in μg/ml), *Tot HS* is the total concentration of HS (in μg/ml) and *Tot HA* is the total concentration of HA (in μg/ml).

The performance of the OV disease score given above was evaluated using the receiver operating characteristic (ROC) curves and the area under the curve (AUC) was found to be 1 (perfect classifier). This is described in Example 9. These findings demonstrate that alterations in GAG chemical composition (e.g. in blood samples) occurring in OV can be summarised into scores. In turn, these scores accurately distinguished OV individuals from healthy individuals and can thus be used for screening, diagnosis, etc.. The discussion elsewhere herein in connection with scoring systems, may be applied, *mutatis mutandis,* to embodiments of the invention in which OV is screened for (e.g. diagnosed).

As described elsewhere herein, in the methods of the invention, appropriate threshold or cut-off scores or values can be calculated by methods known in the art, for example from the ROC curve, for use in the methods of the invention. For the OV marker (scoring) system discussed above, an optimal threshold (cut-off) score was determined/selected which would maximise accuracy in the classification of OV subjects as opposed to healthy subjects, i.e. a sample whose marker score is below this threshold (cut-off) value has the maximum probability of not being OV, or, put another way, a sample whose marker score is above this cut-off value has the maximum probability of being OV. This optimal cut-off score was 0.99. Thus, in one embodiment, the cut-off score is 0.99, wherein a score above this cut-off score is indicative of OV and a score below this cut-off score is indicative of not being OV (e.g. indicative of a normal or healthy sample). In some embodiments, a score that is at least 5%, at least 10%, at least 20%, at least 30%, at least 40%, at least 50% or more higher than 0.99 is indicative of OV. In some embodiments, a score that is at least 5%, at least 10%, at least 20%, at least 30%, at least 40%, at least 50% or more lower than 0.99 is indicative of there being no OV (e.g. indicative of a normal or healthy subject).

As indicated above, a preferred cancer in accordance with the invention is uterine cancer (e.g. endometrial cancer, EC).

In some embodiments, in methods of screening for uterine cancer (preferably EC) particularly preferred GAG forms to be measured or determined (preferably in blood samples) in the methods of the invention are one or more (or all) of: Charge CS, 6s CS, 2s4s CS, Total CS and Total HS.

In some embodiments, in methods of screening for uterine cancer (preferably EC) the methods involve the determination (preferably in blood samples) of one or more (or all) of: 0s CS, 6s CS, 4s CS, 2s4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Total CS, Total HS and Total HA. In some such embodiments, an alteration in the level of one or more (or all) of said GAG forms, e.g. in comparison to a control level, is indicative of uterine cancer (preferably EC).

In some embodiments, in methods of screening for uterine cancer (preferably EC) the methods involve the determination (preferably in blood samples) of one or more (or all) of: 0s CS, 6s CS, 4s CS, 2s4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS and Total CS.

In some embodiments, in methods of screening for uterine cancer (preferably EC) the methods involve the determination (preferably in blood samples) of Total HS.

In some embodiments, in methods of screening for uterine cancer (preferably EC) the methods involve the determination (preferably in blood samples) of Total HA.

In some embodiments, in methods of screening for uterine cancer (preferably EC) an increase (e.g. in blood samples) in one or more (or all) of: 6s CS, 4s CS, 2s4s CS, 4s6s CS, the relative level of 6s CS with respect to 0s CS (e.g. the ratio 6s CS/0s CS), the relative level of 4s CS with respect to 0s CS (e.g. the ratio 4s CS/0s CS), Charge CS and Total CS, for example in comparison to a control level, is indicative of uterine cancer (preferably EC) in said subject.

In some embodiments, in methods of screening for uterine cancer (preferably EC) a decrease (e.g. in blood samples) in one (or both) of: 0s CS and the relative level of 4s CS with respect to 6s CS (e.g. the ratio 4s CS/6s CS), for example in comparison to a control level, is indicative of uterine cancer (preferably EC) in said subject.

In some embodiments, in methods of screening for uterine cancer (preferably EC) an increase (e.g. in blood samples) in Total HA, for example in comparison to a control level, is indicative of uterine cancer (preferably EC) in said subject.

In some embodiments, in methods of screening for uterine cancer (preferably EC) an increase (e.g. in blood samples) in Total HS, for example in comparison to a control level, is indicative of uterine cancer (preferably EC) in said subject.

Preferred GAG forms (or properties) that may be used in uterine cancer (preferably EC) screening (e.g. in blood samples) are those GAG forms identified as having a % in ROPE value of less than 5.00 in Table Z, e.g. less than 4.00. 3.00, 2.00, 1.00 or even a value of 0.00.

In some embodiments, in methods of screening for uterine cancer (preferably EC) an increase (e.g. in blood samples) in one or more (or all) of the GAG properties that are indicated in Table Z as having an increased value in EC in comparison to healthy may be indicative of uterine cancer (preferably EC). In some embodiments, in methods of screening for uterine cancer (preferably EC) a decrease (e.g. in blood samples) in one or more (or all) of the GAG properties that are indicated in Table Z as having a decreased value in EC in comparison to healthy may be indicative of uterine cancer (preferably EC).

In some embodiments, in methods of screening (e.g. in blood samples) for uterine cancer (preferably EC) the methods involve the determination of one (or both) of: 0s CS and Total CS.

In Example 10 herein, a scoring system (formula) has been designed using measurements of multiple GAG forms such that a high score (or elevated score) results in a positive diagnosis (i.e. the finding of the presence of uterine cancer (preferably EC), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table Z. Scoring systems are discussed elsewhere herein and that discussion may be applied, *mutatis mutandis,* to methods of screening for (e.g. diagnosing) uterine cancer (preferably EC).

For uterine cancer (preferably EC), a preferred scoring system giving rise to a score when a blood sample from a subject is analysed is:

$$EC\ GAG\ score = \frac{8}{5}\ Charge\ CS + \frac{2}{15}[6s\ CS] - \frac{1}{500}[2s4s\ CS] + \frac{1}{65}Tot\ CS - \frac{1}{20}Tot\ HS$$

In the above scoring system the terms in square brackets represent the fraction (mass fraction) of the particular GAG form concerned (disaccharide for the corresponding GAG) (as described elsewhere herein), *Charge* CS is the weighted charge of CS, *Tot* CS is the total concentration of CS (in µg/ml) and *Tot HS* is the total concentration of HS (in µg/ml).

The performance of the EC disease score given above was evaluated using the receiver operating characteristic (ROC) curves and the area under the curve (AUC) was found to be 1 (perfect classifier). This is described in Example 10. These findings demonstrate that alterations in GAG chemical composition (e.g. in blood samples) occurring in uterine cancer (particularly EC) can be summarised into scores. In turn, these scores accurately distinguished uterine cancer (particularly EC) individuals from healthy individuals and can thus be used for screening, diagnosis, etc.. The discussion elsewhere herein in connection with scoring systems, may be applied, *mutatis mutandis,* to embodiments of the invention in which uterine cancer (preferably EC) is screened for (e.g. diagnosed).

As described elsewhere herein, in the methods of the invention, appropriate threshold or cut-off scores or values can be calculated by methods known in the art, for example from the ROC curve, for use in the methods of the invention. For the EC marker (scoring) system discussed above, an optimal threshold (cut-off) score was determined/selected which would maximise accuracy in the classification of uterine cancer (particularly EC) subjects as opposed to healthy subjects, i.e. a sample whose marker score is below this threshold (cut-off) value has the maximum probability of not being uterine cancer (particularly EC), or, put another way, a sample whose marker score is above this cut-off value has the maximum probability of being uterine cancer (particularly EC). This optimal cut-off score was 0.97. Thus, in one embodiment, the cut-off score is 0.97, wherein a score above this cut-off score is indicative of uterine cancer (preferably EC) and a score below this cut-off score is indicative of not being uterine cancer (preferably EC) (e.g. indicative of a normal or healthy sample). In some embodiments, a score that is at least 5%, at least 10%, at least 20%, at least 30%, at least 40%, at least 50% or more higher than 0.97 is indicative of uterine cancer (preferably EC). In some embodiments, a score that is at least 5%, at least 10%, at least 20%, at least 30%, at least 40%, at least 50% or more lower than 0.97 is indicative of there being no uterine cancer (preferably EC) (e.g. indicative of a normal or healthy subject).

As indicated above, a preferred cancer in accordance with the invention is uterine cancer (e.g. cervical cancer, also referred to herein as CST),

In some embodiments, in methods of screening for uterine cancer (preferably CST) particularly preferred GAG forms to be measured or determined (preferably in blood samples) in the methods of the invention are one or more (or all) of: Charge CS, 6s CS, Total CS, Ns2s HS and Total HS.

In some embodiments, in methods of screening for uterine cancer (preferably CST) the methods involve the determination (preferably in blood samples) of one or more (or all) of: 0s CS, 6s CS, 4s CS, 2s4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Total CS, Ns2s HS, 6s HS, Total HS and Total HA. In some such embodiments, an alteration in the level of one or more (or all) of said GAG forms, e.g. in comparison to a control level, is indicative of uterine cancer (preferably CST).

In some embodiments, in methods of screening for uterine cancer (preferably CST) the methods involve the determination (preferably in blood samples) of one or more (or all) of: 0s CS, 6s CS, 4s CS, 2s4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS and Total CS.

In some embodiments, in methods of screening for uterine cancer (preferably CST) the methods involve the determination (preferably in blood samples) of one or more (or all) of: Ns2s HS, 6s HS and Total HS.

In some embodiments, in methods of screening for uterine cancer (preferably CST) the methods involve the determination (preferably in blood samples) of Total HA.

In some embodiments, in methods of screening for uterine cancer (preferably CST) an increase (e.g. in blood samples) in one or more (or all) of: 6s CS, 4s CS, 2s4s CS, 4s6s CS, the relative level of 6s CS with respect to 0s CS (e.g. the ratio 6s CS/0s CS), the relative level of 4s CS with respect to 0s CS (e.g. the ratio 4s CS/0s CS), Charge CS and Total CS, for example in comparison to a control level, is indicative of uterine cancer (preferably CST) in said subject.

In some embodiments, in methods of screening for uterine cancer (preferably CST) a decrease (e.g. in blood samples) in one (or both) of: 0s CS and the relative level of 4s CS with respect to 6s CS (e.g. the ratio 4s CS/6s CS), for example in comparison to a control level, is indicative of uterine cancer (preferably CST) in said subject.

In some embodiments, in methods of screening for uterine cancer (preferably CST) an increase (e.g. in blood samples) in Total HA, for example in comparison to a control level, is indicative of uterine cancer (preferably CST) in said subject.

In some embodiments, in methods of screening for uterine cancer (preferably CST) an increase (e.g. in blood samples) in one or more (or all) of: Ns2s HS, 6s HS and Total HS, for example in comparison to a control level, is indicative of uterine cancer (preferably CST) in said subject.

Preferred GAG forms (or properties) that may be used in uterine cancer (preferably CST) screening (e.g. in blood samples) are those GAG forms identified as having a % in ROPE value of less than 5.00 in Table AB, e.g. less than 4.00. 3.00, 2.00, 1.00 or even a value of 0.00.

In some embodiments, in methods of screening for uterine cancer (preferably CST) an increase (e.g. in blood samples) in one or more (or all) of the GAG properties that are indicated in Table AB as having an increased value in uterine cancer (preferably CST) in comparison to healthy may be indicative of uterine cancer (preferably CST). In some embodiments, in methods of screening for uterine cancer (preferably CST) a decrease (e.g. in blood samples) in one or more (or all) of the GAG properties that are indicated in Table AB as having a decreased value in uterine cancer (preferably CST) in comparison to healthy may be indicative of uterine cancer (preferably CST).

In some embodiments, in methods of screening (e.g. in blood samples) for uterine cancer (preferably CST) the methods involve the determination of one or more (or all) of: 0s CS, Total CS and 6s HS.

In Example 11 herein, a scoring system (formula) has been designed using measurements of multiple GAG forms such that a high score (or elevated score) results in a positive diagnosis (i.e. the finding of the presence of uterine cancer, particularly CST), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table AB. Scoring systems are discussed elsewhere herein and that discussion may be applied, *mutatis mutandis,* to methods of screening for (e.g. diagnosing) uterine cancer (preferably CST).

For uterine cancer (preferably CST), a preferred scoring system giving rise to a score when a blood sample from a subject is analysed is:

$$CST\ GAG\ score = Charge\ CS + \frac{1}{6}[6s\ CS] + \frac{1}{50}(Tot\ CS + [Ns2s\ HS]) - \frac{1}{4}Tot\ HS$$

In the above scoring system the terms in square brackets represent the fraction (mass fraction) of the particular GAG form concerned (disaccharide for the corresponding GAG) (as described elsewhere herein), *Charge CS* is the weighted charge of CS, *Tot CS* is the total concentration of CS (in μg/ml) and *Tot HS* is the total concentration of HS (in μg/ml).

The performance of CST disease score given above was evaluated using the receiver operating characteristic (ROC) curves and the area under the curve (AUC) was found to be 1 (perfect classifier). This is described in Example 11. These findings demonstrate that alterations in GAG chemical composition (e.g. in blood samples) occurring in uterine cancer (particularly CST) can be summarised into scores. In turn, these scores accurately distinguished uterine cancer (particularly CST) individuals from healthy individuals and can thus be used for screening, diagnosis, etc.. The discussion elsewhere herein in connection with scoring systems, may be applied, *mutatis mutandis,* to embodiments of the invention in which uterine cancer (preferably CST) is screened for (e.g. diagnosed).

As described elsewhere herein, in the methods of the invention, appropriate threshold or cut-off scores or values can be calculated by methods known in the art, for example from the ROC curve, for use in the methods of the invention. For the CST marker (scoring) system discussed above, an optimal threshold (cut-off) score was determined/selected which would maximise accuracy in the classification of uterine cancer (particularly CST) subjects as opposed to healthy subjects, i.e. a sample whose marker score is below this threshold (cut-off) value has the maximum probability of not being uterine cancer (particularly CST), or, put another way, a sample whose marker score is above this cut-off value has the maximum probability of being uterine cancer (particularly CST). This optimal cut-off score was 0.71. Thus, in one embodiment, the cut-off score is 0.71, wherein a score above this cut-off score is indicative of uterine cancer (preferably CST) and a score below this cut-off score is indicative of not being uterine cancer (preferably CST) (e.g. indicative of a normal or healthy sample). In some embodiments, a score that is at least 5%, at least 10%, at least 20%, at least 30%, at least 40%, at least 50% or more higher than 0.71 is indicative of uterine cancer (preferably CST). In some embodiments, a score that is at least 5%, at least 10%, at least 20%, at least 30%, at least 40%, at least 50% or more lower than 0.71 is indicative of there being no uterine cancer (preferably CST) (e.g. indicative of a normal or healthy subject).

As indicated above, a preferred cancer in accordance with the invention is blood cancer (e.g. non-Hodgkin lymphoma, NHL).

In some embodiments, in methods of screening for blood cancer (preferably NHL) particularly preferred GAG forms to be measured or determined (preferably in blood samples) in the methods of the invention are one or more (or all) of: Charge CS, 6s CS, Tris CS, Charge HS and Total HS.

In some embodiments, in methods of screening for blood cancer (preferably NHL) the methods involve the determination (preferably in blood samples) of one or more (or all) of: 0s CS, 6s CS, 4s CS, 2s6s CS, 2s4s CS, 4s6s CS, Tris CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Total CS, Tris HS, Ns2s HS, Ns HS, 6s HS, 0s HS, Charge HS and Total HS. In some such embodiments, an alteration in the level of one or more (or all) of said GAG forms, e.g. in comparison to a control level, is indicative of blood cancer (preferably NHL).

In some embodiments, in methods of screening for blood cancer (preferably NHL) the methods involve the determination (preferably in blood samples) of one or more (or all) of: 0s CS, 6s CS, 4s CS, 2s6s CS, 2s4s CS, 4s6s CS, Tris CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS and Total CS.

In some embodiments, in methods of screening for blood cancer (preferably NHL) the methods involve the determination (preferably in blood samples) of one or more (or all) of: Tris HS, Ns2s HS, Ns HS, 6s HS, 0s HS, Charge HS and Total HS.

In some embodiments, in methods of screening for blood cancer (preferably NHL) an increase (e.g. in blood samples) in one or more (or all) of: 6s CS, 4s CS, 2s6s CS, 2s4s CS, 4s6s CS, Tris CS, the relative level of 6s CS with respect to 0s CS (e.g. the ratio 6s CS/0s CS), the relative level of 4s CS with respect to 0s CS (e.g. the ratio 4s CS/0s CS), Charge CS and Total CS, for example in comparison to a control level, is indicative of blood cancer (preferably NHL) in said subject.

In some embodiments, in methods of screening for blood cancer (preferably NHL) a decrease (e.g. in blood samples) in one or more (or all) of: 0s CS, the relative level of 4s CS with respect to 6s CS (e.g. the ratio 4s CS/6s CS), for example in comparison to a control level, is indicative of blood cancer (preferably NHL) in said subject.

In some embodiments, in methods of screening for blood cancer (preferably NHL) an increase (e.g. in blood samples) in one or more (or all) of: Tris HS, Ns2s HS, Ns HS, 6s HS, Charge HS and Total HS, for example in comparison to a control level, is indicative of blood cancer (preferably NHL) in said subject.

In some embodiments, in methods of screening for blood cancer (preferably NHL) a decrease (e.g. in blood samples) in 0s HS, for example in comparison to a control level, is indicative of blood cancer (preferably NHL) in said subject.

Preferred GAG forms (or properties) that may be used in blood cancer (preferably NHL) screening (e.g. in blood samples) are those GAG forms identified as having a % in ROPE value of less than 5.00 in Table AD, e.g. less than 4.00.

3.00, 2.00, 1.00 or even a value of 0.00.

In some embodiments, in methods of screening for blood cancer (preferably NHL) an increase (e.g. in blood samples) in one or more (or all) of the GAG properties that are indicated in Table AD as having an increased value in NHL in comparison to healthy may be indicative of blood cancer (preferably NHL). In some embodiments, in methods of screening for blood cancer (preferably NHL) a decrease (e.g. in blood samples) in one or more (or all) of the GAG properties that are indicated in Table AD as having a decreased value in NHL in comparison to healthy may be indicative of blood cancer (preferably NHL).

In some embodiments, in methods of screening (e.g. in blood samples) for blood cancer (preferably NHL) the methods involve the determination of one or more (or all) of: 0s CS, Tris CS, Total CS, Tris HS and 6s HS.

In Example 12 herein, a scoring system (formula) has been designed using measurements of multiple GAG forms such that a high score (or elevated score) results in a positive diagnosis (i.e. the finding of the presence of blood cancer, particularly NHL), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table AD. Scoring systems are discussed elsewhere herein and that discussion may be applied, *mutatis mutandis,* to methods of screening for (e.g. diagnosing) blood cancer (preferably NHL).

For blood cancer (preferably NHL), a preferred scoring system giving rise to a score when a blood sample from a subject is analysed is:

$$NHL\ GAG\ score = \frac{5}{6}Charge\ CS + \frac{1}{5}[6s\ CS] - \frac{1}{7}[Tris\ CS] + \frac{1}{3}Charge\ HS - \frac{1}{20}Tot\ HS$$

In the above scoring system the terms in square brackets represent the fraction (mass fraction) of the particular GAG form concerned (disaccharide for the corresponding GAG) (as described elsewhere herein), *Charge* CS is the weighted charge of CS, *Charge HS* is the weighted charge of HS, and *Tot HS* is the total concentration of HS (in μg/ml).

The performance of the NHL disease score given above was evaluated using the receiver operating characteristic (ROC) curves and the area under the curve (AUC) was found to be 1 (perfect classifier). This is described in Example 12. These findings demonstrate that alterations in GAG chemical composition (e.g. in blood samples) occurring in blood cancer (particularly NHL) can be summarised into scores. In turn, these scores accurately distinguished blood cancer (particularly NHL) individuals from healthy individuals and can thus be used for screening, diagnosis, etc.. The discussion elsewhere herein in connection with scoring systems, may be applied, *mutatis mutandis,* to embodiments of the invention in which blood cancer (preferably NHL) is screened for (e.g. diagnosed).

As described elsewhere herein, in the methods of the invention, appropriate threshold or cut-off scores or values can be calculated by methods known in the art, for example from the ROC curve, for use in the methods of the invention. For the NHL marker (scoring) system discussed above, an optimal threshold (cut-off) score was determined/selected which would maximise accuracy in the classification of blood cancer (particularly NHL) subjects as opposed to healthy subjects, i.e. a sample whose marker score is below this threshold (cut-off) value has the maximum probability of not being blood cancer (particularly NHL), or, put another way, a sample whose marker score is above this cut-off value has the maximum probability of being blood cancer (particularly NHL). This optimal cut-off score was 0.97. Thus, in one embodiment, the cut-off score is 0.97, wherein a score above this cut-off score is indicative of blood cancer (preferably NHL) and a score below this cut-off score is indicative of not being blood cancer (preferably NHL) (e.g. indicative of a normal or healthy sample). In some embodiments, a score that is at least 5%, at least 10%, at least 20%, at least 30%, at least 40%, at least 50% or more higher than 0.97 is indicative of blood cancer (preferably NHL). In some embodiments, a score that is at least 5%, at least 10%, at least 20%, at least 30%, at least 40%, at least 50% or more lower than 0.97 is indicative of there being no blood cancer (preferably NHL) (e.g. indicative of a normal or healthy subject).

As indicated above, a preferred cancer in accordance with the invention is brain cancer (e.g. diffuse glioma, DG).

In some embodiments, in methods of screening for brain cancer (preferably DG) particularly preferred GAG forms to be measured or determined (preferably in blood samples) in the methods of the invention are one or more (or all) of: Charge CS, 6s CS, Total CS, Total HA and 0s HS.

In some embodiments, in methods of screening for brain cancer (preferably DG) the methods involve the determination (preferably in blood samples) of one or more (or all) of: 0s CS, 6s CS, 4s CS, 2s4s CS, 4s6s CS, Tris CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Total CS, Ns HS, 6s HS, 0s HS, Charge HS, Total HS and Total HA. In some such embodiments, an alteration in the level of one or more (or all) of said GAG forms, e.g. in comparison to a control level, is indicative of brain cancer (preferably DG).

In some embodiments, in methods of screening for brain cancer (preferably DG) the methods involve the determination (preferably in blood samples) of one or more (or all) of: 0s CS, 6s CS, 4s CS, 2s4s CS, 4s6s CS, Tris CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS and Total CS.

In some embodiments, in methods of screening for brain cancer (preferably DG) the methods involve the determination (preferably in blood samples) of one or more (or all) of: Ns HS, 6s HS, 0s HS, Charge HS and Total HS.

In some embodiments, in methods of screening for brain cancer (preferably DG) the methods involve the determination (preferably in blood samples) of Total HA.

In some embodiments, in methods of screening for brain cancer (preferably DG) an increase (e.g. in blood samples) in one or more (or all) of: 6s CS, 4s CS, 2s4s CS, 4s6s CS, Tris CS, the relative level of 6s CS with respect to 0s CS (e.g. the ratio 6s CS/0s CS), the relative level of 4s CS with respect to 0s CS (e.g. the ratio 4s CS/0s CS), Charge CS, and Total CS, for example in comparison to a control level, is indicative of brain cancer (preferably DG) in said subject.

In some embodiments, in methods of screening for brain cancer (preferably DG) a decrease (e.g. in blood samples) in one (or both) of: 0s CS and the relative level of 4s CS with respect to 6s CS (e.g. the ratio 4s CS/6s CS), for example in comparison to a control level, is indicative of brain cancer (preferably DG) in said subject.

In some embodiments, in methods of screening for brain cancer (preferably DG) an increase (e.g. in blood samples) in Total HA, for example in comparison to a control level, is indicative of brain cancer (preferably DG) in said subject.

In some embodiments, in methods of screening for brain cancer (preferably DG) an increase (e.g. in blood samples) in one or more (or all) of: Ns HS, 6s HS, Charge HS and Total HS, for example in comparison to a control level, is indicative of brain cancer (preferably DG) in said subject.

In some embodiments, in methods of screening for brain cancer (preferably DG) a decrease (e.g. in blood samples) in 0s HS, for example in comparison to a control level, is indicative of brain cancer (preferably DG) in said subject.

Preferred GAG forms (or properties) that may be used in brain cancer (preferably DG) screening (e.g. in blood samples) are those GAG forms identified as having a % in ROPE value of less than 5.00 in Table AF, e.g. less than 4.00. 3.00, 2.00, 1.00 or even a value of 0.00.

In some embodiments, in methods of screening for brain cancer (preferably DG) an increase (e.g. in blood samples) in one or more (or all) of the GAG properties that are indicated in Table AF as having an increased value in brain cancer (preferably DG) in comparison to healthy may be indicative of brain cancer (preferably DG). In some embodiments, in methods of screening for brain cancer (preferably DG) a decrease (e.g. in blood samples) in one or more (or all) of the GAG properties that are indicated in Table AF as having a decreased value in brain cancer (preferably DG) in comparison to healthy may be indicative of brain cancer (preferably DG).

In some embodiments, in methods of screening (e.g. in blood samples) for brain cancer (preferably DG) the methods involve the determination of one or more (or all) of: 0s CS, Tris CS, Total CS and 6s HS.

In Example 13 herein, a scoring system (formula) has been designed using measurements of multiple GAG forms such that a high score (or elevated score) results in a positive diagnosis (i.e. the finding of the presence of brain cancer, particularly DG), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table AF. Scoring systems are discussed elsewhere herein and that discussion may be applied, *mutatis mutandis,* to methods of screening for (e.g. diagnosing) brain cancer (preferably DG).

For brain cancer (preferably DG), a preferred scoring system giving rise to a score when a blood sample from a subject is analysed is:

$$DG\ GAG\ score = \frac{3}{5}Charge\ CS + \frac{1}{7}[6s\ CS] + \frac{1}{50}Total\ CS + \frac{1}{2}Total\ HA + \frac{1}{10000}[0s\ HS]$$

In the above scoring system the terms in square brackets represent the fraction (mass fraction) of the particular GAG form concerned (disaccharide for the corresponding GAG) (as described elsewhere herein), *Charge* CS is the weighted charge of CS, *Total* CS is the total concentration of CS (in μg/ml), and *Total HA* is the total concentration of HA (in μg/ml).

The performance of the DG disease score given above was evaluated using the receiver operating characteristic (ROC) curves and the area under the curve (AUC) was found to be 1 (perfect classifier). This is described in Example 13. These findings demonstrate that alterations in GAG chemical composition (e.g. in blood samples) occurring in brain cancer (particularly DG) can be summarised into scores. In turn, these scores accurately distinguished brain cancer (particularly DG) individuals from healthy individuals and can thus be used for screening, diagnosis, etc.. The discussion elsewhere herein in connection with scoring systems, may be applied, *mutatis mutandis,* to embodiments of the invention in which brain cancer (preferably DG) is screened for (e.g. diagnosed).

As described elsewhere herein, in the methods of the invention, appropriate threshold or cut-off scores or values can be calculated by methods known in the art, for example from the ROC curve, for use in the methods of the invention. For the DG marker (scoring) system discussed above, an optimal threshold (cut-off) score was determined/selected which would maximise accuracy in the classification of brain cancer (particularly DG) subjects as opposed to healthy subjects, i.e. a sample whose marker score is below this threshold (cut-off) value has the maximum probability of not being brain cancer (particularly DG), or, put another way, a sample whose marker score is above this cut-off value has the maximum probability of being brain cancer (particularly DG). This optimal cut-off score was 0.95. Thus, in one embodiment, the cut-off score is 0.95, wherein a score above this cut-off score is indicative of brain cancer (preferably DG) and a score below this cut-off score is indicative of not being brain cancer (preferably DG) (e.g. indicative of a normal or healthy sample). In some embodiments, a score that is at least 5%, at least 10%, at least 20%, at least 30%, at least 40%, at least 50% or more higher than 0.95 is indicative of brain cancer (preferably DG). In some embodiments, a score that is at least 5%, at least 10%, at least 20%, at least 30%, at least 40%, at least 50% or more lower than 0.95 is indicative of there being no brain cancer (preferably DG) (e.g. indicative of a normal or healthy subject).

As indicated above, a preferred cancer in accordance with the invention is lung cancer (LC).

In some embodiments, in methods of screening for LC, particularly preferred GAG forms to be measured or determined (preferably in blood samples) in the methods of the invention are one or more (or all) of: Total CS, 4s CS, 6s CS, 0s HS and Charge CS (and optionally the relative level of 4s CS with respect to 6s CS (e.g. 4s CS/6s CS) is measured or determined). In such embodiments, particularly preferred GAG forms to be measured or determined are one (or both) of: Charge CS and Total CS.

In some embodiments, in methods of screening for LC the methods involve the determination (preferably in blood samples) of one or more (or all) of: 0s CS, 2s CS, 6s CS, 4s CS, 2s4s CS, Tris CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Total CS, Ns6s HS, Ns2s HS, 6s HS, 0s HS, Charge HS and Total HS. In some such embodiments, an alteration in the level of one or more (or all) of said GAG forms, e.g. in comparison to a control level, is indicative of lung cancer.

In some embodiments, in methods of screening for LC the methods involve the determination (preferably in blood samples) of one or more (or all) of: 0s CS, 2s CS, 6s CS, 4s CS, 2s4s CS, Tris CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS and Total CS.

In some embodiments, in methods of screening for LC the methods involve the determination (preferably in blood samples) of one or more (or all) of: Ns6s HS, Ns2s HS, 6s HS, 0s HS, Charge HS and Total HS.

In some embodiments, in methods of screening for LC an increase (e.g. in blood samples) in one or more (or all) of: 2s CS, 6s CS, 4s CS, 2s4s CS, Tris CS, the relative level of 6s CS with respect to 0s CS (e.g. the ratio 6s CS/0s CS), the relative level of 4s CS with respect to 0s CS (e.g. the ratio 4s CS/0s CS), Charge CS and Total CS, for example in comparison to a control level, is indicative of LC in said subject.

In some embodiments, in methods of screening for LC a decrease (e.g. in blood samples) in one (or both) of: 0s CS and the relative level of 4s CS with respect to 6s CS (e.g. the ratio 4s CS/6s CS), for example in comparison to a control level, is indicative of LC in said subject.

In some embodiments, in methods of screening for LC an increase (e.g. in blood samples) in one or more (or all) of: 6s HS, Charge HS and Total HS, for example in comparison to a control level, is indicative of LC in said subject.

In some embodiments, in methods of screening for LC a decrease (e.g. in blood samples) in one or more (or all) of: Ns6s HS, Ns2s HS and 0s HS, for example in comparison to a control level, is indicative of LC in said subject.

Preferred GAG forms (or properties) that may be used in LC screening (e.g. in blood samples) are those GAG forms identified as having a % in ROPE value of less than 5.00 in Table AH, e.g. less than 4.00. 3.00, 2.00, 1.00 or even a value of 0.00.

In some embodiments, in methods of screening for LC an increase (e.g. in blood samples) in one or more (or all) of the GAG properties that are indicated in Table AH as having an increased value in LC in comparison to healthy may be indicative of LC. In some embodiments, in methods of screening for LC a decrease (e.g. in blood samples) in one or more (or all) of the GAG properties that are indicated in Table AH as having a decreased value in LC in comparison to healthy may be indicative of LC.

In some embodiments, in methods of screening (e.g. in blood samples) for LC the methods involve the determination of one or more (or all) of: 0s CS, Tris CS, Charge CS, Total CS, Ns6s HS and 6s HS.

In Example 14 herein, scoring systems (formulae) have been designed using measurements of multiple GAG forms such that a high score (or elevated score) results in a positive diagnosis (i.e. the finding of the presence of LC), but equally a skilled person could readily design and choose the scoring method and parameters used in such scoring method such that a low (or decreased) score gives rise to a positive diagnosis. The relevant features to be analysed in such scoring system can also be chosen based on the sample type to be analysed, again for example using the data as presented in Table AH. Scoring systems are discussed elsewhere herein and that discussion may be applied, *mutatis mutandis,* to methods of screening for (e.g. diagnosing) LC.

For LC, a preferred scoring system giving rise to a score when a blood sample from a subject is analysed is:

## LC scoring formula #1

$$LC\ GAG\ score = \frac{1}{6} Total\ CS - \left( \frac{1}{30} \frac{[4s\ CS]}{[6s\ CS]} + \frac{1}{25} [0s\ HS] \right)$$

For LC, another preferred scoring system giving rise to a score when a blood sample from a subject is analysed is:

## LC scoring formula #2

$$LC\ GAG\ score = \frac{5}{4} Charge\ CS + \frac{1}{25} Total\ CS$$

In the above scoring systems the terms in square brackets represent the fraction (mass fraction) of the particular GAG form concerned (disaccharide for the corresponding GAG) (as described elsewhere herein), *Total* CS is the total concentration of CS (in $\mu$g/ml) and *Charge* CS is the weighted charge of CS.

The performance of the LC scoring formula #1 given above was evaluated using the receiver operating characteristic (ROC) curves and the area under the curve (AUC) was found to be 0.986. This is described in Example 14.

The performance of the LC scoring formula #2 given above was evaluated using the receiver operating characteristic (ROC) curves and the area under the curve (AUC) was found to be 0.997. This is described in Example 14.

These findings demonstrate that alterations in GAG chemical composition (e.g. in blood samples) occurring in LC can be summarised into scores. In turn, these scores accurately distinguished LC individuals from healthy individuals and can thus be used for screening, diagnosis, etc.. The discussion elsewhere herein in connection with scoring systems, may be applied, *mutatis mutandis,* to embodiments of the invention in which LC is screened for (e.g. diagnosed).

As described elsewhere herein, in the methods of the invention, appropriate threshold or cut-off scores or values can be calculated by methods known in the art, for example from the ROC curve, for use in the methods of the invention. For the LC marker (scoring) systems discussed above, optimal thresholds (cut-off) scores were determined/selected which would maximise accuracy in the classification of LC subjects as opposed to healthy subjects, i.e. a sample whose marker score is below this threshold (cut-off) value has the maximum probability of not being LC, or, put another way, a sample whose marker score is above this cut-off value has the maximum probability of being LC.

The optimal cut-off score was 0.83 for LC scoring formula #1. Thus, in one embodiment, LC scoring formula #1 is used and the cut-off score is 0.83, wherein a score above this cut-off score is indicative of LC and a score below this cut-off score is indicative of not being LC (e.g. indicative of a normal or healthy sample). In some embodiments, a score that is at least 5%, at least 10%, at least 20%, at least 30%, at least 40%, at least 50% or more higher than 0.83 is indicative of LC. In some embodiments, a score that is at least 5%, at least 10%, at least 20%, at least 30%, at least 40%, at least 50% or more lower than 0.83 is indicative of there being no LC (e.g. indicative of a normal or healthy subject).

The optimal cut-off score was 0.88 for LC scoring formula #2. Thus, in one embodiment, LC scoring formula #2 is used and the cut-off score is 0.88, wherein a score above this cut-off score is indicative of LC and a score below this cut-off score is indicative of not being LC (e.g. indicative of a normal or healthy sample). In some embodiments, a score that is at least 5%, at least 10%, at least 20%, at least 30%, at least 40%, at least 50% or more higher than 0.88 is indicative of LC. In some embodiments, a score that is at least 5%, at least 10%, at least 20%, at least 30%, at least 40%, at least 50% or more lower than 0.88 is indicative of there being no LC (e.g. indicative of a normal or healthy subject).

As indicated above, a preferred cancer in accordance with the invention is blood cancer (e.g. CLL or NHL).

In some embodiments, in methods of screening for blood cancer the methods involve the determination (preferably in blood samples) of one or more (or all) of: 0s CS, 6s CS, 4s CS, 2s6s CS, 2s4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Total CS, Ns HS, 6s HS, 0s HS, Charge HS and Total HS. In some such embodiments, an alteration in the level of one or more of said GAG forms, e.g. in comparison to a control level, is indicative of blood cancer.

In some embodiments, in methods of screening for blood cancer an increase (e.g. in blood samples) in one or more (or all) of: 6s CS, 4s CS, 2s6s CS, 2s4s CS, 4s6s CS, the ratio 6s CS/0s CS, the ratio 4s CS/0s CS, Charge CS, Total CS, Ns HS, 6s HS, Charge HS and Total HS, for example in comparison to a control level, is indicative of blood cancer in said subject.

In some embodiments, in methods of screening for blood cancer a decrease (e.g. in blood samples) in one or more (or all) of: 0s CS, the ratio 4s CS/6s CS and 0s HS, for example in comparison to a control level, is indicative of blood cancer in said subject.

In some such embodiments, in methods of screening for NHL, methods further comprise the determination (preferably in blood samples) of one or more (or all) of: Tris CS, Tris HS and Ns2s HS. In some such embodiments, an increase in one or more (or all) of these GAG properties, e.g. in comparison to a control level, is indicative of NHL.

In some such embodiments, in methods of screening for CLL, methods further comprise the determination (preferably in blood samples) of one or more (or all) of: Ns6s HS, 2s6s HS and 2s HS. In some such embodiments, an increase in 2s HS and/or a decrease in one or both of Ns6s HS and 2s6s HS, e.g. in comparison to a control level, is indicative of CLL.

As indicated above, a preferred cancer in accordance with the invention is uterine cancer (e.g. EC or CST).

In some embodiments, in methods of screening for uterine cancer the methods involve the determination (preferably in blood samples) of one or more (or all) of: 0s CS, 6s CS, 4s CS, 2s4s CS, 4s6s CS, the relative level of 6s CS with respect to 4s CS (e.g. the ratio 6s CS/4s CS or the inverse ratio 4s CS/6s CS), the relative level of 6s CS with respect to 0s CS (e.g. the ratio 6s CS/0s CS or the inverse ratio 0s CS/6s CS), the relative level of 4s CS with respect to 0s CS (e.g. the ratio 4s CS/0s CS or the inverse ratio 0s CS/4s CS), Charge CS, Total CS, Total HA, and Total HS. In some such embodiments, an alteration in the level of one or more of said GAG forms, e.g. in comparison to a control level, is indicative of uterine cancer.

In some embodiments, in methods of screening for uterine cancer an increase (e.g. in blood samples) in one or more (or all) of 6s CS, 4s CS, 2s4s CS, 4s6s CS, the ratio 6s CS/0s CS, the ratio 4s CS/0s CS, Charge CS, Total CS, Total HA, and Total HS, for example in comparison to a control level, is indicative of uterine cancer in said subject.

In some embodiments, in methods of screening for uterine cancer a decrease (e.g. in blood samples) in one or more (or both) of: 0s CS and the ratio 4s CS/6s CS, for example in comparison to a control level, is indicative of uterine cancer in said subject.

In some such embodiments, in methods of screening for CST, methods further comprise the determination (preferably in blood samples) of one (or both) of: Ns2s HS and 6s HS. In some such embodiments, an increase in one (or both) of these GAG properties, e.g. in comparison to a control level, is indicative of CST.

As described above, in some embodiments, GAG scores (or formulae or scoring formulae) may be used, e.g. in combination with described cut-off scores, in order to discriminate between cancer and healthy samples (and thus between cancer subjects and healthy subjects). In some other embodiments, these exact GAG scores are not used but other embodiments of the invention are used, which for example may employ alternative GAG scores or indeed which do not employ GAG scores (formulae) at all. In some such other embodiments, a method of screening for (e.g. diagnosis of) cancer is provided that is indicative of cancer if an indication of cancer would have been made if the relevant specific GAG score (and optionally the associated cut-off score) had been used (i.e. had been used instead).

In another aspect, the present invention provides a method of screening for cancer in a subject, said method comprising

determining the level in a sample of an expression product of one or more genes selected from the group consisting of:

UST, CHST14, CHST13, CHSY1, DSE, CHSY3, CHST11, CHST15, CHPF2, CSGALNACT2, CHST12, CSGALNACT1, CHST7, CHPF, CHST3, HPSE, HGSNAT, HYAL4, GALNS, GLB1, GNS, GUSB, HEXA, HEXB, HYAL1, IDS, IDUA, ARSB, NAGLU, HPSE2, SGSH, SPAM1, HYAL3, HYAL2, HS3ST3B1, HS3ST3A1, B4GALT7, B3GALT6, B3GAT2, B3GAT3, B3GAT1, XYLT1, XYLT2, EXT1, EXT2, EXTL1, EXTL2, EXTL3, HS3ST5, GLCE, HS6ST3, NDST1, NDST4, HS6ST2, NDST3, HS6ST1, HS2ST1, HS3ST2, HS3ST1 and NDST2;
wherein said sample has been obtained from said subject; and
wherein an altered level in said sample of the expression product of one or more of said genes in comparison to a control level is indicative of cancer in said subject.

In methods of the invention that involve determining the level in a sample of an expression product of one or more genes, references to "cancer" include, at their broadest, any cancer (i.e. at its broadest is not limited to specific cancers described elsewhere herein.

However, in some embodiments of methods of the invention that involve determining the level in a sample of an expression product of one or more genes, preferred cancers are selected from the group consisting of prostate cancer, thyroid cancer, colon cancer, rectum cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer, skin cancer and kidney cancer.

In some embodiments of methods of the invention that involve determining the level in a sample of an expression product of one or more genes, preferred cancers are selected from the group consisting of prostate cancer, thyroid cancer, colon cancer, rectum cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer and skin cancer.

In some embodiments of methods of the invention that involve determining the level in a sample of an expression product of one or more genes, the cancer is not kidney cancer (e.g. not renal cell carcinoma such as clear cell renal cell carcinoma (or metastatic clear cell renal cell carcinoma), papillary renal cell carcinoma or chromphobe renal cell carcinoma).

In some embodiments of methods of the invention that involve determining the level in a sample of an expression product of one or more genes, the cancer is selected from the group consisting of prostate cancer, thyroid cancer, rectum cancer, pancreatic cancer, bladder cancer, liver cancer, bile duct cancer, stomach cancer, oesophageal cancer, brain cancer, blood cancer, ovarian cancer and skin cancer in a subject. Thus, in some embodiments, the cancer is not breast cancer, colon cancer, head and neck cancer, lung cancer or uterine cancer.

In some embodiments of methods of the invention that involve determining the level in a sample of an expression product of one or more genes, the cancer is selected from the group consisting of prostate cancer, thyroid cancer, colon cancer, rectum cancer, uterine cancer, pancreatic cancer, bladder cancer, liver cancer, bile duct cancer, stomach cancer, oesophageal cancer, head and neck cancer, blood cancer and ovarian cancer. Thus, in some embodiments, the cancer is not skin cancer, lung cancer, brain cancer or breast cancer.

Particular types of cancers (or groups of cancers) described elsewhere herein in connection with other methods of the invention (e.g. methods of the invention that involve determining the level and/or chemical composition of one or more glycosaminoglycans) may also be preferred cancers in connection with methods of the invention that involve determining the level in a sample of an expression product of one or more genes.

In some preferred embodiments of methods of the invention that involve determining the level in a sample of an expression product of one or more genes, the cancer is prostate cancer.

In some embodiments of methods of the invention that involve determining the level in a sample of an expression product of one or more genes, said one or more genes is selected from the group consisting of HPSE, HGSNAT, HYAL4, GALNS, GLB1, GNS, GUSB, HEXA, HEXB, HYAL1, IDS, IDUA, ARSB, NAGLU, HPSE2, SGSH, SPAM1, HYAL3, HYAL2, HS3ST1 and NDST2. In some embodiments, the level of an expression product of one or more of EXT1, EXT2, EXTL1, EXTL2, EXTL3, XYLT1, XYLT2, B3GAT2, B3GAT3, B3GAT1, B4GALT7, B3GALT6, DSE, UST, HS3ST2, HS3ST3B1, GLCE, NDST1, NDST4, NDST3, HS2ST1, HS6ST1, HS6ST2, HS6ST3, CSGALNACT2, CSGALNACT1, CHPF, CHPF2, CHSY1, CHSY3, CHST14, CHST13, CHST11, CHST12, CHST15, CHST7, HS3ST3A1, HS3ST5, and CHST3 is not determined.

In some embodiments of methods of the invention that involve determining the level in a sample of an expression product of one or more genes, if the cancer being screened for is kidney cancer (e.g. clear cell renal cell carcinoma), said one or more genes is selected from the group consisting of HPSE, HGSNAT, HYAL4, GALNS, GLB1, GNS, GUSB, HEXA, HEXB, HYAL1, IDS, IDUA, ARSB, NAGLU, HPSE2, SGSH, SPAM1, HYAL3, HYAL2, HS3ST1 and NDST2. In some embodiments, if the cancer being screened for is kidney cancer, (e.g. clear cell renal cell carcinoma) the level of an expression product of one or more of EXT1, EXT2, EXTL1, EXTL2, EXTL3, XYLT1, XYLT2, B3GAT2, B3GAT3, B3GAT1, B4GALT7, B3GALT6, DSE, UST, HS3ST2, HS3ST3B1, GLCE, NDST1, NDST4, NDST3, HS2ST1, HS6ST1, HS6ST2, HS6ST3, CSGALNACT2, CSGALNACT1, CHPF, CHPF2, CHSY1, CHSY3, CHST14, CHST13, CHST11, CHST12, CHST15, CHST7, HS3ST3A1, HS3ST5, and CHST3 is not determined.

In some embodiments of methods of the invention that involve determining the level in a sample of an expression product of one or more genes, said one or more genes is selected from the group consisting of UST, DSE, HPSE, HGSNAT, HYAL4, GALNS, GLB1, GNS, GUSB, HEXA, HEXB, HYAL1, IDS, IDUA, ARSB, NAGLU, HPSE2, SGSH, SPAM1, HYAL3, HYAL2, HS3ST3B1, HS3ST3A1, B4GALT7, B3GALT6, B3GAT2, B3GAT3, B3GAT1, XYLT1, XYLT2, HS3ST5, HS3ST2, HS3ST1 and NDST2. In some embodiments, the level of an expression product of one or more of EXT1, EXT2, EXTL1, EXTL2, EXTL3, CHST11, CHSY3, CHST14, CHST13, CHST12, CHSY1, CSGALNACT2, CHST15, CHPF, CHPF2, CHST7, CHST3, CSGALNACT1, HS6ST2, HS2ST1, GLCE, NDST1, NDST4, NDST3, HS6ST3 and HS6ST1 is not determined.

In some embodiments of methods of the invention that involve determining the level in a sample of an expression product of one or more genes, if the cancer being screened for is kidney cancer, head and neck cancer, breast cancer, colon cancer, lung cancer or uterus cancer, said one or more genes is selected from the group consisting of UST, DSE, HPSE, HGSNAT, HYAL4, GALNS, GLB1, GNS, GUSB, HEXA, HEXB, HYAL1, IDS, IDUA, ARSB, NAGLU, HPSE2, SGSH, SPAM1, HYAL3, HYAL2, HS3ST3B1, HS3ST3A1, B4GALT7, B3GALT6, B3GAT2, B3GAT3, B3GAT1, XYLT1, XYLT2, HS3ST5, HS3ST2, HS3ST1 and NDST2. In some embodiments, if the cancer being screened for is kidney cancer, head and neck cancer, breast cancer, colon cancer, lung cancer or uterus cancer, the level of an expression product of one or more of EXT1, EXT2, EXTL1, EXTL2, EXTL3, CHST11, CHSY3, CHST14, CHST13, CHST12, CHSY1, CSGALNACT2, CHST15, CHPF, CHPF2, CHST7, CHST3, CSGALNACT1, HS6ST2, HS2ST1, GLCE, NDST1, NDST4, NDST3, HS6ST3 and HS6ST1 is not determined.

In some embodiments, one or more of CHPF2, HS6ST2, or EXTL1 are not determined (e.g. if the cancer being screened for is kidney cancer such as clear cell renal cell carcinoma).

In some embodiments, NDST2 is not determined.

In some embodiments of methods of the invention that involve determining the level in a sample of an expression product of one or more genes, said gene(s) is a gene(s) of the chondroitin sulphate biosynthesis pathway. In some embodiments of methods of the invention that involve determining the level in a sample of an expression product of one or more genes, said gene(s) is a gene(s) of the heparan sulphate biosynthesis pathway.

In some embodiments of methods of the invention that involve determining the level in a sample of an expression product of one or more genes, said one or more genes is selected from the group consisting of CHPF2, CHPF, B4GALT7, BGAT3, EXTL1 and HPSE1.

In some embodiments, in skin cancer, an alteration in the level of an expression product of one or more genes selected from the group consisting of B3GALT6, CHSY3, CHST14, CHSY1, CHST11, CHPF2, GNS, HEXA, ARSB is indicative of skin cancer in said subject.

In some embodiments, in skin cancer, an increase in the level of an expression product of one or more genes selected from the group consisting of B3GALT6, CHSY3, CHST14, CHSY1, CHST11, CHPF2, GNS, HEXA, ARSB is indicative of skin cancer in said subject.

In some embodiments, in ovarian cancer, an alteration in the level of an expression product of one or more genes selected from the group consisting of B3GALT6, CHST14, CHSY1, NDST1, HS3ST2 is indicative of ovarian cancer in said subject.

In some embodiments, in ovarian cancer, an increase in the level of an expression product of one or more genes selected from the group consisting of B3GALT6, CHST14, CHSY1, NDST1, HS3ST2 is indicative of ovarian cancer in said subject.

In some embodiments, in blood cancer, an alteration in the level of an expression product of one or more genes selected from the group consisting of CHSY3, CHST15, EXT1, HS3ST3A1.1 is indicative of blood cancer in said subject.

In some embodiments, in blood cancer, an increase in the level of an expression product of one or more genes selected from the group consisting of CHSY3, CHST15, EXT1, HS3ST3A1 is indicative of blood cancer in said subject.

In some embodiments, in bladder cancer, an alteration in the level of an expression product of one or more genes selected from the group consisting of B3GALT6, XYLT2, B4GALT7, B3GAT3, CHPF, CHST14, CHST11, CHPF2, CHST12, GLCE, HS2ST1, HS6ST2, HS6ST1, HPSE, GLB1, GUSB, HYAL3, GALNS, IDUA, SGSH, NAGLU, HEXA, B3GAT2, UST, CSGALNACT1, HS3ST5, EXTL1, HPSE2, HYAL1 is indicative of bladder cancer in said subject.

In some embodiments, in bladder cancer, an increase in the level of an expression product of one or more genes selected from the group consisting of B3GALT6, XYLT2, B4GALT7, B3GAT3, CHPF, CHST14, CHST11, CHPF2, CHST12, GLCE, HS2ST1, HS6ST2, HS6ST1, HPSE, GLB1, GUSB, HYAL3, GALNS, IDUA, SGSH, NAGLU, HEXA is indicative of bladder cancer in said subject.

In some embodiments, in bladder cancer, a decrease in the level of an expression product of one or more genes selected from the group consisting of B3GAT2, UST, CSGALNACT1, HS3ST5, EXTL1, HPSE2, HYAL1 is indicative of bladder cancer in said subject.

In some embodiments, in breast cancer, an alteration in the level of an expression product of one or more genes selected from the group consisting of B3GALT6, XYLT2, B4GALT7, B3GAT3, CHPF, CHST11, CHPF2, CHST15, HS6ST1, HS6ST3, HS3ST3A1, HPSE, GLB1, HYAL3, GALNS, XYLT1, B3GAT1, B3GAT2, DSE, CHST7, UST, CSGALNACT1, CHST3, NDST1, HS6ST2, HS3ST1, EXTL2, EXTL3, HPSE2, GNS, HYAL1, IDS is indicative of breast cancer in said subject.

In some embodiments, in breast cancer, an increase in the level of an expression product of one or more genes selected from the group consisting of B3GALT6, XYLT2, B4GALT7, B3GAT3, CHPF, CHST11, CHPF2, CHST15, HS6ST1, HS6ST3, HS3ST3A1, HPSE, GLB1, HYAL3, GALNS is indicative of breast cancer in said subject.

In some embodiments, in breast cancer, a decrease in the level of an expression product of one or more genes selected from the group consisting of XYLT1, B3GAT1, B3GAT2, DSE, CHST7, UST, CSGALNACT1, CHST3, NDST1, HS6ST2, HS3ST1, EXTL2, EXTL3, HPSE2, GNS, HYAL1, IDS is indicative of breast cancer in said subject.

In some embodiments, in colon cancer, an alteration in the level of an expression product of one or more genes selected from the group consisting of B3GALT6, XYLT2, B4GALT7, B3GAT3, CHPF, CHSY3, CHST14, CHSY1, CSGALNACT2, CSGALNACT1, GLCE, HS2ST1, HS6ST2, HS3ST1, EXTL2, EXTL3, HYAL3, HYAL2, XYLT1, B3GAT1, CHST15, CHST7, UST, CHST13, HS3ST5, EXT1, NDST1, HS6ST3, EXTL1, HS3ST3B1, HPSE, HPSE2, HS3ST3B1 is indicative of colon cancer in said subject.

In some embodiments, in colon cancer, an increase in the level of an expression product of one or more genes selected from the group consisting of B3GALT6, XYLT2, B4GALT7, B3GAT3, CHPF, CHSY3, CHST14, CHSY1, CSGALNACT2, CSGALNACT1, GLCE, HS2ST1, HS6ST2, HS3ST1, EXTL2, EXTL3, HYAL3, HYAL2 is indicative of colon cancer in said subject.

In some embodiments, in colon cancer, a decrease in the level of an expression product of one or more genes selected from the group consisting of XYLT1, B3GAT1, CHST15, CHST7, UST, CHST13, HS3ST5, EXT1, NDST1, HS6ST3, EXTL1, HPSE, HPSE2, HS3ST3B1 is indicative of colon cancer in said subject.

In some embodiments, in brain cancer (e.g. glioblastoma multiforme), an alteration in the level of an expression product of one or more genes selected from the group consisting of B4GALT7, DSE, CHPF, CHST14, CHSY1, CHST11, CHPF2, CHST7, UST, CHST3, CHST12, EXT2, EXT1, HS2ST1, HS3ST1, EXTL2, GLB1, GUSB, GNS, HYAL2, GALNS, IDUA, SGSH, HEXB, NAGLU, HEXA, ARSB, HS3ST5, NDST3, HS3ST2, HS6ST3, EXTL1, IDS is indicative of brain cancer (e.g. glioblastoma multiforme) in said subject.

In some embodiments, in brain cancer (e.g. glioblastoma multiforme), an increase in the level of an expression product of one or more genes selected from the group consisting of B4GALT7, DSE, CHPF, CHST14, CHSY1, CHST11, CHPF2, CHST7, UST, CHST3, CHST12, EXT2, EXT1, HS2ST1, HS3ST1, EXTL2, GLB1, GUSB, GNS, HYAL2, GALNS, IDUA, SGSH, HEXB, NAGLU, HEXA, ARSB is indicative of brain cancer (e.g. glioblastoma multiforme) in said subject.

In some embodiments, in brain cancer (e.g. glioblastoma multiforme), a decrease in the level of an expression product of one or more genes selected from the group consisting of HS3ST5, NDST3, HS3ST2, HS6ST3, EXTL1, IDS is indicative of brain cancer (e.g. glioblastoma multiforme) in said subject.

In some embodiments, in head and neck cancer, an alteration in the level of an expression product of one or more genes selected from the group consisting of B3GALT6, XYLT2, B4GALT7, B3GAT3, DSE, CHPF, CHSY3, CHST14, CHSY1, CHST11, CHPF2, CHST15, CHST7, UST, CSGALNACT2, CHST3, CHST12, EXT2, EXT1, HS2ST1, EXTL2, EXTL3, GLB1, GUSB, GNS, GALNS, HEXB, NAGLU, HEXA, ARSB, XYLT1, HS3ST5, HS3ST1, HS3ST2, EXTL1, HPSE2, HYAL4 is indicative of head and neck cancer in said subject.

In some embodiments, in head and neck cancer, an increase in the level of an expression product of one or more genes selected from the group consisting of B3GALT6, XYLT2, B4GALT7, B3GAT3, DSE, CHPF, CHSY3, CHST14, CHSY1, CHST11, CHPF2, CHST15, CHST7, UST, CSGALNACT2, CHST3, CHST12, EXT2, EXT1, HS2ST1, EXTL2, EXTL3, GLB1, GUSB, GNS, GALNS, HEXB, NAGLU, HEXA, ARSB is indicative of head and neck cancer in said subject.

In some embodiments, in head and neck cancer, a decrease in the level of an expression product of one or more genes selected from the group consisting of XYLT1, HS3ST5, HS3ST1, HS3ST2, EXTL1, HPSE2, HYAL4 is indicative of head and neck cancer in said subject.

In some embodiments, in lung squamous cell carcinoma, an alteration in the level of an expression product of one or more genes selected from the group consisting of XYLT2, B4GALT7, B3GAT3, CHPF, CHST14, CHPF2, CHST7, CHST3, EXT2, EXT1, HS6ST2, HS3ST1, HS6ST1, HS3ST3A1, HPSE, HYAL3, GALNS, B3GAT1, CHSY3, CHST11, CHST13, CSGALNACT2, CSGALNACT1, HS3ST5, NDST1, HS3ST2, EXTL1, HPSE2, GLB1, GNS, HYAL1, HYAL2, IDUA, HEXB, IDS, ARSB is indicative of lung squamous cell carcinoma in said subject.

In some embodiments, in lung squamous cell carcinoma, an increase in the level of an expression product of one or more genes selected from the group consisting of XYLT2, B4GALT7, B3GAT3, CHPF, CHST14, CHPF2, CHST7, CHST3, EXT2, EXT1, HS6ST2, HS3ST1, HS6ST1, HS3ST3A1, HPSE, HYAL3, GALNS is indicative of lung squamous cell carcinoma in said subject.

In some embodiments, in lung squamous cell carcinoma, a decrease in the level of an expression product of one or more genes selected from the group consisting of B3GAT1, CHSY3, CHST11, CHST13, CSGALNACT2, CSGALNACT1, HS3ST5, NDST1, HS3ST2, EXTL1, HPSE2, GLB1, GNS, HYAL1, HYAL2, IDUA, HEXB, IDS, ARSB is indicative of lung squamous cell carcinoma in said subject.

In some embodiments, in lung adenocarcinoma, an alteration in the level of an expression product of one or more genes selected from the group consisting of XYLT2, B3GAT1, B4GALT7, B3GAT3, CHPF, CHPF2, CHST15, HS6ST2, HS3ST1, HS3ST3A1, HPSE, HYAL3, CHSY1, CHST7, UST, CSGALNACT1, HS3ST5, NDST1, HPSE2, HYAL1, HYAL2 is indicative of lung adenocarcinoma in said subject.

In some embodiments, in lung adenocarcinoma, an increase in the level of an expression product of one or more genes selected from the group consisting of XYLT2, B3GAT1, B4GALT7, B3GAT3, CHPF, CHPF2, CHST15, HS6ST2, HS3ST1, HS3ST3A1, HPSE, HYAL3 is indicative of lung adenocarcinoma in said subject.

In some embodiments, in lung adenocarcinoma, a decrease in the level of an expression product of one or more genes selected from the group consisting of CHSY1, CHST7, UST, CSGALNACT1, HS3ST5, NDST1, HPSE2, HYAL1, HYAL2 is indicative of lung adenocarcinoma in said subject.

In some embodiments, in pancreatic cancer, an alteration in the level of an expression product of one or more genes selected from the group consisting of CHPF, HS3ST1 is indicative of pancreatic cancer in said subject.

In some embodiments, in pancreatic cancer, an increase in the level of an expression product of one or more genes selected from the group consisting of CHPF, HS3ST1 is indicative of pancreatic cancer in said subject.

In some embodiments, in rectal cancer, an alteration in the level of an expression product of one or more genes selected from the group consisting of B3GALT6, B4GALT7, B3GAT3, CHPF, CHSY3, CHSY1, CHPF2, HS2ST1, HS6ST2, HYAL3, HYAL2, IDUA, CHST15, UST, EXT1, HS3ST2, HPSE, HPSE2 is indicative of rectal cancer in said subject.

In some embodiments, in rectal cancer, an increase in the level of an expression product of one or more genes selected from the group consisting of B3GALT6, B4GALT7, B3GAT3, CHPF, CHSY3, CHSY1, CHPF2, HS2ST1, HS6ST2, HYAL3, HYAL2, IDUA is indicative of rectal cancer in said subject.

In some embodiments, in rectal cancer, a decrease in the level of an expression product of one or more genes selected from the group consisting of CHST15, UST, EXT1, HS3ST2, HPSE, HPSE2 is indicative of rectal cancer in said subject.

In some embodiments, in uterine cancer (e.g. endometrial cancer such as uterine corpus endometrial carcinoma), an alteration in the level of an expression product of one or more genes selected from the group consisting of B4GALT7, B3GAT3, CHPF, CHPF2, HS6ST1, GLB1, HYAL3, GALNS, XYLT1, DSE, CHSY3, CHST14, CHSY1, CHST7, UST, CSGALNACT2, CSGALNACT1, CHST3, HS6ST2, HS6ST3, EXTL1, EXTL2, HPSE2, HYAL1, HGSNAT, IDS is indicative of uterine cancer (e.g. endometrial cancer such as uterine corpus endometrial carcinoma) cancer in said subject.

In some embodiments, in uterine cancer (e.g. endometrial cancer such as uterine corpus endometrial carcinoma), an increase in the level of an expression product of one or more genes selected from the group consisting of B4GALT7, B3GAT3, CHPF, CHPF2, HS6ST1, GLB1, HYAL3, GALNS is indicative of uterine cancer (e.g. endometrial cancer such as uterine corpus endometrial carcinoma) cancer in said subject.

In some embodiments, in uterine cancer (e.g. endometrial cancer such as uterine corpus endometrial carcinoma), a decrease in the level of an expression product of one or more genes selected from the group consisting of XYLT1, DSE, CHSY3, CHST14, CHSY1, CHST7, UST, CSGALNACT2, CSGALNACT1, CHST3, HS6ST2, HS6ST3, EXTL1, EXTL2, HPSE2, HYAL1, HGSNAT, IDS is indicative of uterine cancer (e.g. endometrial cancer such as uterine corpus endometrial carcinoma) in said subject.

In some embodiments, in bile duct cancer (e.g. cholangiocarcinoma), an alteration in the level of an expression product of one or more genes selected from the group consisting of B3GALT6, XYLT2, B4GALT7, B3GAT3, DSE, CHPF, CHST14, CHSY1, CHPF2, CHST15, CSGALNACT2, CSGALNACT1, CHST3, CHST12, EXT2, EXT1, NDST1, GLCE, HS2ST1, HS3ST1, HS6ST1, NDST2, EXTL2, EXTL3, GLB1, GNS, HYAL2, HGSNAT, GALNS, IDUA, SGSH, HEXB, HEXA, IDS, ARSB CHST13, HS3ST3B1, HYAL1, HS3ST3B1 is indicative of bile duct cancer (e.g. cholangiocarcinoma) in said subject.

In some embodiments, in bile duct cancer (e.g. cholangiocarcinoma), an increase in the level of an expression product of one or more genes selected from the group consisting of B3GALT6, XYLT2, B4GALT7, B3GAT3, DSE, CHPF, CHST14, CHSY1, CHPF2, CHST15, CSGALNACT2, CSGALNACT1, CHST3, CHST12, EXT2, EXT1, NDST1, GLCE, HS2ST1, HS3ST1, HS6ST1, NDST2, EXTL2, EXTL3, GLB1, GNS, HYAL2, HGSNAT, GALNS, IDUA, SGSH, HEXB, HEXA, IDS, ARSB is indicative of bile duct cancer (e.g. cholangiocarcinoma) in said subject.

In some embodiments, in bile duct cancer (e.g. cholangiocarcinoma), a decrease in the level of an expression product of one or more genes selected from the group consisting of CHST13, HYAL1, HS3ST3B1 is indicative of bile duct cancer (e.g. cholangiocarcinoma) in said subject.

In some embodiments, in oesophagus cancer, an alteration in the level of an expression product of one or more genes selected from the group consisting of B3GALT6, B4GALT7, B3GAT3, DSE, CHPF, CHSY3, CHST14, CHSY1, CHST11, CHPF2, CHST15, CHST7, CSGALNACT2, CHST12, EXT2, EXT1, GLCE, HS2ST1, HS6ST2, HS3ST1, HS6ST1, EXTL3, HS3ST3A1, HPSE, GLB1, GUSB, HYAL3, GALNS, HEXB, HEXA, IDS, B3GAT1, NDST3, HS6ST3, HPSE2 is indicative of oesophagus cancer in said subject.

In some embodiments, in oesophagus cancer, an increase in the level of an expression product of one or more genes selected from the group consisting of B3GALT6, B4GALT7, B3GAT3, DSE, CHPF, CHSY3, CHST14, CHSY1, CHST11, CHPF2, CHST15, CHST7, CSGALNACT2, CHST12, EXT2, EXT1, GLCE, HS2ST1, HS6ST2, HS3ST1, HS6ST1, EXTL3, HS3ST3A1, HPSE, GLB1, GUSB, HYAL3, GALNS, HEXB, HEXA, IDS is indicative of oesophagus cancer in said subject.

In some embodiments, in oesophagus cancer, a decrease in the level of an expression product of one or more genes selected from the group consisting of B3GAT1, NDST3, HS6ST3, HPSE2 is indicative of oesophagus cancer in said subject.

In some embodiments, in chromophobe renal cell carcinoma, an alteration in the level of an expression product of one or more genes selected from the group consisting of B4GALT7, B3GAT3, CHPF2, UST, CSGALNACT1, NDST1, HS6ST3, GLB1, HYAL1, HEXA, IDS, XYLT1, B3GAT1, CHST15, CHST7, CHST13, CSGALNACT2, HS3ST5, NDST3, GLCE, HS2ST1, HS6ST2, HS6ST1, EXTL1, EXTL2, HS3ST3B1, HS3ST3A1, HYAL2, HS3ST3B1, ARSB is indicative of chromophobe renal cell carcinoma in said subject.

In some embodiments, in chromophobe renal cell carcinoma, an increase in the level of an expression product of one or more genes selected from the group consisting of B4GALT7, B3GAT3, CHPF2, UST, CSGALNACT1, NDST1, HS6ST3, GLB1, HYAL1, HEXA, IDS is indicative of chromophobe renal cell carcinoma in said subject.

In some embodiments, in chromophobe renal cell carcinoma, a decrease in the level of an expression product of one or more genes selected from the group consisting of XYLT1, B3GAT1, CHST15, CHST7, CHST13, CSGALNACT2, HS3ST5, NDST3, GLCE, HS2ST1, HS6ST2, HS6ST1, EXTL1, EXTL2, HS3ST3A1, HYAL2, ARSB is indicative of chromophobe renal cell carcinoma in said subject.

In some embodiments, in clear cell renal cell carcinoma, an alteration in the level of an expression product of one or more genes selected from the group consisting of B4GALT7, B3GAT2, CHPF, CHSY3, CHST14, CHSY1, CHST11, CHPF2, CHST15, CHST7, CHST13, CSGALNACT2, CHST12, HS3ST2, GUSB, GALNS, IDUA, B3GAT1, UST, CHST3, HS3ST5, NDST3, GLCE, HS6ST2, HS3ST1, HS6ST1, HS6ST3, EXTL1, EXTL2, HS3ST3B1, HS3ST3A1, HPSE2, GLB1, HYAL3, GNS, HYAL4, HYAL1 is indicative of clear cell renal cell carcinoma in said subject.

In some embodiments, in clear cell renal cell carcinoma , an increase in the level of an expression product of one or more genes selected from the group consisting of B4GALT7, B3GAT2, CHPF, CHSY3, CHST14, CHSY1, CHST11, CHPF2, CHST15, CHST7, CHST13, CSGALNACT2, CHST12, HS3ST2, GUSB, GALNS, IDUA is indicative of clear cell renal cell carcinoma in said subject.

In some embodiments, in clear cell renal cell carcinoma , a decrease in the level of an expression product of one or more genes selected from the group consisting of B3GAT1, UST, CHST3, HS3ST5, NDST3, GLCE, HS6ST2, HS3ST1, HS6ST1, HS6ST3, EXTL1, EXTL2, HS3ST3A1, HPSE2, GLB1, HYAL3, GNS, HYAL4, HYAL1, HS3ST3B1 is indicative of clear cell renal cell carcinoma in said subject.

In some embodiments, in papillary renal cell carcinoma, an alteration in the level of an expression product of one or more genes selected from the group consisting of XYLT2, B4GALT7, B3GAT3, CHPF, CHST14, CHST11, CHPF2, CHST13, CHST12, HS3ST1, GUSB, GALNS, IDUA, SGSH, HEXB, UST, CSGALNACT1, NDST3, NDST1, GLCE, HS6ST2, HS6ST1, HS6ST3, EXTL1, HS3ST3B1, HS3ST3A1, HPSE, HYAL4, HYAL1, HYAL2 is indicative of papillary renal cell carcinoma in said subject.

In some embodiments, in papillary renal cell carcinoma, an increase in the level of an expression product of one or more genes selected from the group consisting of XYLT2, B4GALT7, B3GAT3, CHPF, CHST14, CHST11, CHPF2, CHST13, CHST12, HS3ST1, GUSB, GALNS, IDUA, SGSH, HEXB is indicative of papillary renal cell carcinoma in said subject.

In some embodiments, in papillary renal cell carcinoma, a decrease in the level of an expression product of one or more genes selected from the group consisting of UST, CSGALNACT1, NDST3, NDST1, GLCE, HS6ST2, HS6ST1, HS6ST3, EXTL1, HS3ST3A1, HPSE, HYAL4, HYAL1, HYAL2, HS3ST3B1 is indicative of papillary renal cell carcinoma in said subject.

In some embodiments, in liver cancer, an alteration in the level of an expression product of one or more genes selected from the group consisting of B3GALT6, XYLT2, B4GALT7, B3GAT3, CHST14, CHST11, CHPF2, CHST15, CHST13, CSGALNACT1, CHST3, CHST12, EXT2, EXT1, NDST1, GLCE, HS2ST1, HS3ST2, HS6ST1, 8509 (Entrez ID), EXTL2, EXTL3, GLB1, HYAL3, GNS, HYAL2, IDUA, SGSH, HEXB, HEXA, ARSB, B3GAT1, DSE, CHST7, UST, NDST3, HS3ST3B1, HS3ST3A1 is indicative of liver cancer in said subject.

In some embodiments, in liver cancer, an increase in the level of an expression product of one or more genes selected from the group consisting of B3GALT6, XYLT2, B4GALT7, B3GAT3, CHST14, CHST11, CHPF2, CHST15, CHST13, CSGALNACT1, CHST3, CHST12, EXT2, EXT1, NDST1, GLCE, HS2ST1, HS3ST2, HS6ST1, 8509 (Entrez ID), EXTL2, EXTL3, GLB1, HYAL3, GNS, HYAL2, IDUA, SGSH, HEXB, HEXA, ARSB is indicative of liver cancer in said subject.

In some embodiments, in liver cancer, a decrease in the level of an expression product of one or more genes selected from the group consisting of B3GAT1, DSE, CHST7, UST, NDST3, HS3ST3A1, HS3ST3B1 is indicative of liver cancer in said subject.

In some embodiments, in stomach cancer, an alteration in the level of an expression product of one or more genes selected from the group consisting of B3GALT6, B4GALT7, B3GAT3, CHPF, CHSY3, CHSY1, CHST11, CHPF2, CHST13, CSGALNACT2, EXT2, EXT1, GLCE, HS2ST1, HS3ST1, HS3ST2, EXTL2, HPSE, GLB1, GUSB, GNS, GALNS, HEXB, HEXA, ARSB, B3GAT1, UST, HS6ST2, NDST4, HS6ST3, EXTL1, HPSE2 is indicative of stomach cancer in said subject.

In some embodiments, in stomach cancer, an increase in the level of an expression product of one or more genes selected from the group consisting of B3GALT6, B4GALT7, B3GAT3, CHPF, CHSY3, CHSY1, CHST11, CHPF2, CHST13, CSGALNACT2, EXT2, EXT1, GLCE, HS2ST1, HS3ST1, HS3ST2, EXTL2, HPSE, GLB1, GUSB, GNS, GALNS, HEXB, HEXA, ARSB is indicative of stomach cancer in said subject.

In some embodiments, in stomach cancer, a decrease in the level of an expression product of one or more genes selected from the group consisting of B3GAT1, UST, HS6ST2, NDST4, HS6ST3, EXTL1, HPSE2 is indicative of stomach cancer in said subject.

In some embodiments, in thyroid cancer, an alteration in the level of an expression product of one or more genes selected from the group consisting of B3GAT1, CHST11, CHPF2, CHST7, CSGALNACT2, HS6ST2, HPSE, HYAL2, XYLT1, B3GAT2, CHST14, UST, CSGALNACT1, EXT1, NDST3, HS6ST1, HS6ST3, HS3ST3B1, HS3ST3A1 is indicative of thyroid cancer in said subject.

In some embodiments, in thyroid cancer, an increase in the level of an expression product of one or more genes selected from the group consisting of B3GAT1, CHST11, CHPF2, CHST7, CSGALNACT2, HS6ST2, HPSE, HYAL2 is indicative of thyroid cancer in said subject.

In some embodiments, in thyroid cancer, a decrease in the level of an expression product of one or more genes selected from the group consisting of XYLT1, B3GAT2, CHST14, UST, CSGALNACT1, EXT1, NDST3, HS6ST1, HS6ST3, HS3ST3A1, HS3ST3B1 is indicative of thyroid cancer in said subject.

In some embodiments, in prostate cancer, an alteration in the level of an expression product of one or more genes selected from the group consisting of B3GALT6, B3GAT1, B3GAT3, CHPF, CSGALNACT1, HS3ST2, HS6ST3, GUSB, HYAL3, IDUA, SGSH, DSE, CHST11, CHST15, UST, CHST3, HS3ST5, EXT1, GLCE, EXTL1, HS3ST3B1, HS3ST3A1, HPSE, HPSE2, HYAL1, IDS is indicative of prostate cancer in said subject.

In some embodiments, in prostate cancer, an increase in the level of an expression product of one or more genes selected from the group consisting of B3GALT6, B3GAT1, B3GAT3, CHPF, CSGALNACT1, HS3ST2, HS6ST3, GUSB, HYAL3, IDUA, SGSH is indicative of prostate cancer in said subject.

In some embodiments, in prostate cancer, a decrease in the level of an expression product of one or more genes selected from the group consisting of DSE, CHST11, CHST15, UST, CHST3, HS3ST5, EXT1, GLCE, EXTL1, HS3ST3A1, HPSE, HPSE2, HYAL1, IDS, HS3ST3B1 is indicative of prostate cancer in said subject.

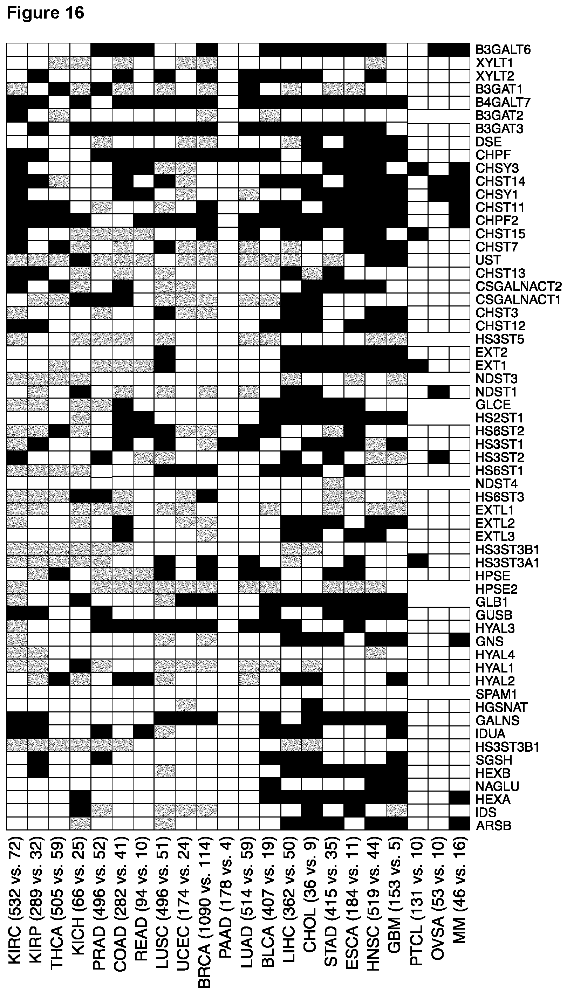

Other preferred genes (or groups of genes), expression products of which can be determined in accordance with the present invention, can be readily determined from Table K and/or Figure 16 herein (e.g. genes which show an alteration, increase, or decrease in particular cancer types discussed herein). Table K and Figure 16 use abbreviations for the cancers. Cancer types and subtypes corresponding to these abbreviations are given in Table G herein. For example, in some embodiments, genes whose expression level is altered in at least 5, at least 10 or at least 15 cancer types (e.g. 5-17, 10-17 or 15-17) may be preferred.

In some embodiments, the level of an expression product of a single gene is determined. In other embodiments, the level of an expression product of more than one gene is determined (e.g. the level of expression product of two or more, or three or more, or four or more is determined). By "more than one" is meant 2, 3, 4, 5, 6, 7, 8, 9, 10 etc.... 60 (including all integers between 2 and 60). In some embodiments, the level of at least 10, at least 20, at least 30, at least 40, at least 50 or all 60 genes may be determined. A determination of the expression product level for each and every possible combination of the genes described herein can be performed.

In methods of the present invention, it is not necessary that the level of each one of the expression products whose level is determined is altered in comparison to a control level in order for there to be an indication of cancer in the subject. Put another way, a sample in which the level of one or more expression products is unaltered (or not significantly altered) in comparison with a control level may still be a "cancer" sample (e.g. if the level of one or more of the other genes is altered in comparison to a control level).

As discussed herein, methods of the present invention may comprise determining or measuring one or more specific GAG forms (or groups of GAG forms) "selected from the group consisting of" certain specific GAG forms (or groups of GAG forms) set forth herein, or may comprise determining or measuring the level of an expression product of one or more specific genes (or groups of genes) "selected from the group consisting of" certain specific genes (or groups of genes) set forth herein. For the avoidance of doubt, in some embodiments in which one or more of the specific GAG forms (or groups of GAG forms) or one or more of the specific genes (or groups of genes) discussed herein is measured or determined, one or more other (or distinct) GAG forms or one or more other (or distinct) genes and/or one or more other biomarkers may additionally be measured or determined. Thus, "selected from the group consisting of" may be an "open" term. In some embodiments, only one or more of the specific GAG forms (or groups of GAG forms) discussed herein is measured or determined (e.g. other GAG forms or other biomarkers are not measured or determined). In some embodiments, only one or more of the specific genes (or groups of genes) discussed herein is measured or determined (e.g. other genes or other biomarkers are not measured or determined).

As discussed above, the present invention provides a method of screening for cancer (e.g. prostate cancer) in a subject. Alternatively viewed, the present invention provides a method of diagnosing cancer (e.g. prostate cancer) in a subject. Alternatively viewed, the present invention provides a method for the prognosis of cancer (e.g. prostate cancer) in a subject (prognosis of the future severity, course and/or outcome of cancer, e.g. prostate cancer). Alternatively viewed, the present invention provides a method of monitoring for the occurrence of cancer (e.g. prostate cancer) in a subject at risk. Alternatively viewed, the present invention provides a method for monitoring the progression of cancer (e.g. prostate cancer) in a subject. Alternatively viewed, the present invention provides a method of determining the clinical severity of cancer (e.g. prostate cancer) in a subject. Alternatively viewed, the present invention provides a method of determining the risk of progression of cancer (e.g. prostate cancer) in a subject. Alternatively viewed, the present invention provides a method of guiding treatment based on the risk assessment of cancer (e,g, prostate cancer) in a subject. Alternatively viewed, the present invention provides a method for predicting the response of a subject to therapy for cancer (e.g. prostate cancer). Alternatively viewed, the present invention provides a method of determining the efficacy of a therapeutic or surgical regime for cancer (e.g. prostate cancer) in a subject. Alternatively viewed, the present invention provides a method for detecting the recurrence or relapse of cancer (e.g. prostate cancer) (e.g. in patients with early stage cancer, e.g. prostate cancer). Alternatively viewed, the present invention provides a method of patient selection or treatment selection, for example as it provides a means of distinguishing patients with small cancerous masses (e.g prostatic masses) which are not cancer (e.g. prostate cancer), e.g. are non-malignant, benign or indolent masses (but which can display some problematic symptoms, e.g. prostatic symptoms, and may be suspected to be cancer, e.g. prostate cancer) from patients with cancer (e.g. prostate cancer). Thus, alternatively viewed, the present invention provides a method for distinguishing cancer (e.g. prostate cancer) from non-malignant diseases. In some embodiments, the present invention provides a method for determining whether a metastasis is due to prostate cancer. Alternatively viewed, the present invention provides a method for predicting whether metastasis is expected from a given cancer (e.g. prostate cancer). Alternatively viewed, the present invention provides a method of screening for cancer (e.g. prostate cancer) in the general population. Alternatively viewed, the present invention provides a method of screening for cancer (e.g. prostate cancer) in a population at risk of having or developing cancer (e.g. prostate cancer) (e.g. genetically predisposed individuals or individuals presenting risk factors or individuals presenting symptoms).

Thus, the method of screening for cancer (e.g. prostate cancer) in accordance with the present invention can be used, for example, for diagnosing cancer (e.g. prostate cancer), for the prognosis of cancer (e.g. prostate cancer), for monitoring for the occurrence of cancer (e.g. prostate cancer) in a subject at risk, for monitoring the progression of cancer (e.g. prostate cancer), for determining the clinical severity of cancer (e.g. prostate cancer), for predicting the response of a subject to therapy for cancer (e.g. prostate cancer), for determining the efficacy of a therapeutic or surgical regime being used to treat cancer (e.g. prostate cancer), for detecting the recurrence or relapse of cancer (e.g. prostate cancer), for patient selection or treatment selection, for distinguishing small cancerous masses (e.g. prostatic masses) suspicious of cancer (e.g. prostate cancer) from other non malignant diseases, for determining whether a metastasis is due to a given cancer (e.g. prostate cancer), for screening for cancer (e.g. prostate cancer) in the general population or for screening for cancer (e.g. prostate cancer) in a population at risk of having or developing prostate cancer (e.g. genetically predisposed individuals or individuals presenting risk factors or individuals presenting symptoms).

Thus, in one aspect the present invention provides a method for diagnosing cancer (e.g. prostate cancer) in a subject. In some embodiments, a positive diagnosis (i.e. the presence of cancer, e.g. prostate cancer) is made if the level of one or more of the GAG forms (or expression products) in the sample is altered (increased or decreased as the case may be) in comparison to a control level. GAG forms (or expression products) for which an increased level is indicative of (e.g. diagnostic of) cancer (e.g. prostate cancer) are described elsewhere herein. GAG forms (or expression products) for which a decreased level is indicative of (e.g. diagnostic of) cancer (e.g. prostate cancer) are described elsewhere herein. Alternatively, a number of different GAG forms or properties (or expression products) are analysed as described elsewhere herein to arrive at a diagnosis, e.g. using a scoring system or method. The methods of the invention may also be used to ascertain whether a metastasis is due to a given type of cancer (e.g. prostate cancer).

In another aspect, the present invention provides a method for the prognosis of cancer (e.g. prostate cancer) in a subject. In such methods the level of one or more of the GAG forms (or expression products) discussed above in the sample is indicative of the future severity, course and/or outcome of cancer (e.g. prostate cancer). For example, an alteration (increase or decrease as the case may be) in the level of one or more of the GAG forms (or expression products) in the sample in comparison to a control level may indicate a poor prognosis. A highly altered level (or score), e.g. compared to control levels (or scores), may indicate a particularly poor prognosis.

Thus, in some embodiments, an increased level of one or more of the GAG forms (or expression products) for which an increased level is indicative of cancer (e.g. prostate cancer) is suggestive of (i.e. indicative of) a poor prognosis. In some embodiments, a decreased level of one or more of the GAG forms (or expression products) for which a decreased level is indicative of cancer (e.g. prostate cancer) is suggestive of (i.e. indicative of) a poor prognosis. Conversely, if one or more GAG forms (or expression products) has an unaltered level (or an essentially unaltered level) that can be indicative of a good prognosis.

Serial (periodic) measuring of the level of one or more of the GAG forms (biomarkers) (or expression products) in accordance with the present invention may also be used for prognostic purposes looking for either increasing or decreasing levels (or scores) over time. In some embodiments, an altering level (increase or decrease, as appropriate) of one or more of the GAG forms (or expression products) over time (in comparison to a control level, e.g. a level moving further away from the control level) may indicate a worsening prognosis. In some embodiments, an altering level (increase or decrease, as appropriate) of one or more of the GAG forms (or expression products) over time (in comparison to a control level, e.g. a level moving closer to the control level) may indicate an improving prognosis.

In one aspect the present invention provides a method for monitoring for the occurrence of cancer (e.g. prostate cancer) in a subject at risk of developing cancer (e.g. prostate cancer). Such methods and the GAG forms (or expression products) which are measured are similar to the diagnostic methods as described herein, but are carried out on subjects that are at particular risk for developing cancer (e.g. prostate cancer) and thus may benefit from closer monitoring. Such "at risk" subjects would be readily identified by a person skilled in the art but would include for example subjects with a family history of cancer (e.g. prostate cancer) or a genetic predisposition to cancer (e.g. prostate cancer), or subjects in remission from cancer (e.g. prostate cancer), or subjects with recognized risk factors for cancer (e.g. prostate cancer). For example, a recognized risk factor for prostate cancer is age, for example males over 40 years old, or over 50 years old, or over 55 years old, or preferably over 65 years old, e.g. 40-65, or 50-65, or 55-65, or 60-65, or 70-75, or 40-75, or 50-75, or 55-75, or 60-75, or 65-75 (e.g. 66-71), or 70-75, or 40-85, or 50-85, or 55-85, or 60-85, or 65-85, or 70-85.

In this way, it can be seen that in some embodiments of the invention, the methods can be carried out on "healthy" patients (subjects) or at least patients (subjects) which are not manifesting any clinical symptoms of cancer (e.g. prostate cancer), for example, patients with very early or pre-clinical stage cancer (e.g. prostate cancer), e.g. patients where the primary tumor is so small that it cannot be assessed or detected or patients in which cells are undergoing pre-cancerous changes associated with cancer (e.g. prostate cancer) but have not yet become malignant.

Thus, the methods of the present invention can also be used to monitor disease progression. Such monitoring can take place before, during or after treatment of cancer (e.g. prostate cancer) by surgery or therapy, e.g. pharmaceutical therapy. Thus, in another aspect the present invention provides a method for monitoring the progression of cancer (e.g. prostate cancer) in a subject.

Methods of the present invention can be used in the active monitoring of patients which have not been subjected to surgery or therapy, e.g. to monitor the progress of cancer (e.g. prostate cancer) in untreated patients. Again, serial measurements can allow an assessment of whether or not, or the extent to which, the cancer (e.g. prostate cancer) is worsening, thus, for example, allowing a more reasoned decision to be made as to whether therapeutic or surgical intervention is necessary or advisable.

As discussed above, monitoring can also be carried out, for example, in an individual, e.g. a healthy individual, who is thought to be at risk of developing cancer (e.g. prostate cancer), in order to obtain an early, and ideally pre-clinical, indication of cancer (e.g. prostate cancer).

In another aspect, the present invention provides a method for determining the clinical severity of cancer (e.g. prostate cancer) in a subject. In such methods the level of one or more of the GAG forms (or expression products) in the sample shows an association with the severity of the cancer (e.g. prostate cancer). Thus, the level of one or more of the GAG forms (or expression products) is indicative of the severity of the cancer (e.g. prostate cancer). In some embodiments, the more altered (more increased or more decreased as the case may be) the level (or score) of one or more of the GAG forms (or expression products) in comparison to a control level, the greater the likelihood of a more severe form of cancer (e.g. prostate cancer). In some embodiments the methods of the invention can thus be used in the selection of patients for therapy.

Serial (periodical) measuring of the level (or score) of one or more of the GAG forms (biomarkers) (or expression products) may also be used to monitor the severity of cancer (e.g. prostate cancer) looking for either increasing or decreasing levels over time. Observation of altered levels (increase or decrease as the case may be) may also be used to guide and monitor therapy, both in the setting of subclinical disease, i.e. in the situation of "watchful waiting" before treatment or surgery, e.g. before initiation of pharmaceutical therapy or surgery, or during or after treatment to evaluate the effect of treatment and look for signs of therapy failure.

Thus, the present invention also provides a method for predicting the response of a subject to therapy or surgery. For example, a subject with a less severe form or an early stage of cancer (e.g. prostate cancer), as determined by the level of one or more of the GAG forms (or expression products) in a sample in accordance with the present invention, is generally more likely to be responsive to therapy or surgery, in particular surgery. In such methods the choice of therapy or surgery may be guided by knowledge of the level of one or more of the GAG forms (or expression products) in the sample. In some embodiments of methods for predicting the response of a subject to therapy or surgery, the level of HA is not measured or determined.

The present invention also provides a method of patient selection or treatment selection as it provides a means of distinguishing patients with high risk cancer (e.g. prostate cancer) from patients with low risk cancer (e.g. prostate cancer). Thus, alternatively viewed, the methods of the present invention provide a method for distinguishing high and low risk cancer (e.g. prostate cancer) and may guide appropriate treatment.

In some embodiments, the invention provides a method of distinguishing between high (or higher) risk cancer (e.g. prostate cancer e.g. with a Gleason score of 8 or more) and low (or lower) risk cancer (e.g. prostate cancer e.g. with a Gleason score of 6 or less) in subjects that have been diagnosed (e.g. recently diagnosed e.g. < 1 month or < 6 months or < 1 year since diagnosis) with cancer (e.g. prostate cancer). High risk and low risk are discussed elsewhere herein. High (or higher) risk may mean a subject has a poor (or worse) prognosis and low (or lower) risk may mean a subject has a good (or better) prognosis. Subjects of intermediate risk (e.g. prostate cancer subjects with a Gleason score of 7) may also be identified. In some embodiments, the invention provides a method of classifying prostate cancer subjects of intermediate risk (e.g. with a Gleason score of 7) as high (or higher) risk prostate cancer. In some embodiments, the invention provides a method of classifying prostate cancer subjects of intermediate risk (e.g. with a Gleason score of 7) as low (or lower) risk prostate cancer. Methods of the invention may be used to assess the severity, aggressiveness, metastatic potential or risk level in a subject diagnosed (e.g. recently diagnosed) with cancer (e.g. prostate cancer).

In some embodiments, the invention provides a method of monitoring (e.g. continuously monitoring or performing active surveillance of) a subject having cancer (e.g. prostate cancer) (e.g. a subject being treated for cancer, e.g. prostate cancer). Such monitoring may guide which treatment to use or whether no treatment should be given.

In some embodiments, the more altered (more increased or decreased as the case may be) the level (or score) of one or more of the GAG forms (or expression products) in comparison to a control level (or score), the greater the likelihood of high risk cancer (e.g. prostate cancer) (or the lesser the likelihood of low risk cancer, e.g. prostate cancer). Conversely, in some embodiments, the less altered (less increased or decreased as the case may be) the level (or score) of one or more of the GAG forms (or expression products) in comparison to a control level (or score), the lesser the likelihood of high risk cancer (e.g. prostate cancer) (or the greater the likelihood of low risk cancer, e.g. prostate cancer).

In some embodiments, low (or lower) risk patients may be put under watchful waiting or active surveillance and may not be given treatment (e.g. pharmaceutical therapy or surgery). In some embodiments, high (or higher) risk patients may be given treatment, e.g. resection (e.g. prostatectomy), radiation therapy, hormone therapy or other treatment (e.g. as detailed elsewhere herein).

The present invention also provides a method of determining (or monitoring) the efficacy of a therapeutic regime being used to treat cancer (e.g. prostate cancer), in other words following or monitoring a response to treatment. In such methods, an alteration (increase or decrease as the case may be) in the level (or scores) of one or more of the GAG forms (or expression products) in accordance with the present invention indicates the efficacy of the therapeutic regime being used. For example, if the level of one or more of the GAG forms (or expression products) for which an increased level (or score) is indicative of cancer (e.g. prostate cancer) is reduced during (or after) therapy, this is indicative of an effective therapeutic regime. Conversely, for example, if the level of one or more of the GAG forms (or expression products) for which a decreased level (or score) is indicative of cancer (e.g. prostate cancer) is increased during (or after) therapy, this is indicative of an effective therapeutic regime. In such methods, serial (periodical) measuring of the level of one or more of the GAG forms (biomarkers) (or expression products) over time can also be used to determine the efficacy of a therapeutic regime being used. Similar methods can be used to provide a method of determining (or monitoring) the efficacy of a surgical regime being used to treat cancer (e.g. prostate cancer).

The present invention also provides a method for detecting the recurrence (relapse) of cancer (e.g. prostate cancer), for example in a subject that has previously had cancer (e.g. prostate cancer) but been successfully treated, e.g. by surgery or therapy (e.g. pharmaceutical therapy) such that they are judged to be in remission or cured, or for example to predict metastatic relapse in patients during follow-up. Such subjects form an "at risk" category and may well benefit from regular monitoring for cancer (e.g. prostate cancer). Such methods for detecting the recurrence (or relapse) of cancer (e.g. prostate cancer) use the diagnostic methods as described herein in order to detect the presence or absence of cancer (e.g. prostate cancer).

The present invention also provides a method of patient selection or treatment selection as it provides a means of distinguishing patients with cancer (e.g. prostate cancer) from patients with non-malignant diseases, e.g. non-malignant prostate diseases. Thus, alternatively viewed, the methods of the present invention provide a method for distinguishing cancer (e.g. prostate cancer) from non-malignant diseases.

Such methods of patient selection or treatment selection or methods for distinguishing cancer (e.g. prostate cancer) from non-malignant diseases use the diagnostic methods as described herein in order to detect the presence or absence of cancer (e.g. prostate cancer).

The features and discussion herein in relation to the method of screening for cancer (e.g. prostate cancer) (e.g. in relation to preferred GAG forms or expression products or combinations thereof for measurement) apply, *mutatis mutandis,* to the other related methods of present invention (e.g. to a method of diagnosing cancer (e.g. prostate cancer), etc.).

In one embodiment, the invention provides the use of the methods of the invention (e.g. screening, diagnostic or prognostic methods, etc., as described herein) in conjunction with other known screening, diagnostic or prognostic methods for cancer (e.g. prostate cancer), such as radiological imaging (e.g. computed tomography, CT, scan) or magnetic resonance imaging (MRI scan), or histological assessment, e.g. using a tumor biopsy, or the PSA (prostate specific antigen) test or the DRE (digital rectal examination) test or the Prostate Core Mitomic Test[TM]. Thus, for example, the methods of the invention can be used to confirm a diagnosis of cancer (e.g. prostate cancer) in a subject. In some embodiments the methods of the present invention are used alone.

The level of the GAG form (or expression products) in question can be determined or measured by analyzing the sample which has been obtained from or removed from the subject by an appropriate means. The determination is typically carried out *in vitro.*

Levels of one or more of the GAG forms (or expression products) in the sample can be measured (determined) by any appropriate assay, a number of which are well known and documented in the art. As described elsewhere herein, electrophoresis, e.g. agarose gel electrophoresis or capillary electrophoresis (in particular capillary electrophoresis with fluorescence detection such as CE-LIF) or liquid chromatography, in particular HPLC (high-performance liquid chromatography) in combination with mass spectrometry (MS) are preferred techniques for measuring (determining) the levels of one or more of the GAG forms in accordance with the present invention.

Suitable electrophoresis, e.g. capillary electrophoresis, and liquid chromatography, e.g. HPLC techniques for GAG form analysis, together with appropriate mass spectrometry methods (and associated data processing techniques) are well known and documented in the art.

A particularly preferred method for determining the level of one or more of the GAG forms in the sample is described herein in the Examples. A preferred method used in the invention is capillary electrophoresis with laser-induced fluorescence detection, CE-LIF (e.g. as described in Galeotti et al., 2014, Electrophoresis 35: 811-818; and Kottler et al., 2013, Electrophoresis 34: 2323-2336). HPLC combined with post column derivatization and fluorimetric detection can also be used, e.g. as described in Volpi 2006, Curr Pharm Des 12:639-658, as can HPLC combined with ESI-MS (electrospray ionization-mass spectrometry), e.g. as described in Volpi and Linhardt, 2010, Nature protocols 5:993-1004, also with fluorimetric detection, e.g. as described in Galeotti and Volpi, 2011, Anal Chem 83:6770-6777, or Volpi et al., 2014, Nature Protocols 9:541-558. Agarose gel electrophoresis can also be used, e.g. FACE (fluorophore assisted carbohydrate electrophoresis) as described in Volpi and Maccari, 2006, Analyt Technol Biomed Life Sci, 834:1-13; and Volpi and Maccari, 2002, Electrophoresis 23:4060-4066.

Appropriate methods of sample preparation, e.g. GAG extraction and purification are also known and described in the art, for example Volpi and Maccari, 2005, Biomacromolecules 6:3174-3180 and Clin Chim Acta 356:125-133, Coppa et al., 2011 Glycobiology 21:295-303.

In some embodiments HPLC and mass spectrometry (and associated data processing techniques) is used to obtain a fraction of the level of one or more particular GAG forms (e.g. the sulfated or unsulfated disaccharide forms) in the sample in comparison to the total amount. For example, after sample preparation, GAGs can be digested using enzymes, separated in an HPLC column and characterized using MS. As described elsewhere herein, the quantities of one or more individual GAG forms (e.g. a particular sulfated or unsulfated disaccharide form) are conveniently normalised (i.e. divided) by the sum of all the quantities of individual GAG forms measured, to yield fractions.

In accordance with the present invention, a quantitative, semi-quantitative or qualitative assessment (determination) of the level of one or more of the GAG forms can be made.

Appropriate methods of doing this would be well known to a skilled person in the art and any of these could be used. However, a convenient method to achieve such quantification of disaccharide composition or the appropriate properties or forms of CS or HS (and separation of the disaccharide forms) is to use electrophoresis, in particular capillary electrophoresis, and preferably capillary electrophoresis with fluorescence detection, e.g. capillary electrophoresis with laser-induced fluorescence detection (CE-LIF) (e.g. as described in Galeotti 2014, supra, or Kottler 2013, supra). An alternative method is to use liquid chromatography, preferably HPLC (high-performance liquid chromatography), for example SAX HPLC or for example as described in Volpi 2006, supra, Galeotti and Volpi 2011, supra, Volpi et al., 2014, supra or Volpi and Linhardt, 2010, supra. Preferably mass spectrometry is also used (HPLC-MS), for example electrospray ionization mass spectrometry (ESI-MS), e.g. HPLC ESI-MS. Particularly preferred methods are outlined in the Examples. Thus, one example of a particularly preferred method is capillary electrophoresis (e.g. for example, capillary electophoresis with laser-induced fluorescence detection). Another example would be HPLC followed by MS (HPLC-MS), e.g. HPLC ESI-MS. Alternatively, mass spectrometry can be used without chromatography, e.g. liquid chromatography.

Generally, the determination of the GAG properties or forms in accordance with the present invention does not involve the measurement of GAG molecules in the exact same form as found in the body fluid of a subject (e.g. does not involve the measurement of a naturally occurring form of GAG). For example, such native or naturally occurring GAG molecules are often found in the form of long sugar chains attached to proteins, whereas for levels to be determined in accordance with the present invention generally such GAG molecules have to be at least separated or extracted from the proteins to which they are attached and often further processed. Thus, generally the methods of the invention are carried out on samples which have been processed in some way (e.g. are man-made rather than native samples).

Thus, in some embodiments, methods of the invention may include a step of processing a sample. In some embodiments, the methods of the invention may thus be performed on such processed samples or materials derived from such processed samples. Processing steps include, but are not limited to, extraction or purification of GAGs from the sample, steps of fragmentation or cleavage or digestion of proteins present in the sample, e.g. as a means of separating or extracting or removing GAGs from the protein to which they are attached, e.g. through the use of a protease such as proteinase K, purification of GAGs, e.g. using an anion-exchange resin, isolating cells from the sample, isolating cell components from the sample, extracting (e.g. isolating or purifying) proteins/peptides and/or nucleic acid molecules (DNA or RNA) from the sample. Said processing steps thus also include steps carried out on a body fluid sample to prepare it for analysis, e.g. in the case of a blood sample, such steps might include the steps to prepare an appropriate blood component for analysis, e.g. plasma or serum, or, in the case of a urine sample, the removal of cells or other impurities. A processing step may involve one or more of digestion, extraction, purification, boiling, filtration, lyophilization, fractionation, centrifugation, concentration, dilution, inactivation of interfering components, addition of reagents, derivatization, complexation and the like. Exemplary processing steps are described in the Examples.

In some methods of the invention where the levels of certain individual disaccharide forms are measured, the GAGs, e.g. the full length GAG molecules or the GAG molecules attached to proteins on serine residues, or polymerised polysaccharide chains of GAGs, or chains of repeating disaccharide units of GAGs, are subjected to a processing step, for example a step of fragmentation or cleavage or digestion, e.g. by chemical digestion or enzyme treatment, e.g. with chondroitinase ABC or chondroitinase B, in order to obtain the disaccharide units which are then analysed.

Other methods to determine levels or compositions of GAGs which might be used are known in the art. However, examples are analytical techniques involving the use of antibodies to various GAG forms, e.g. techniques such as Western blot, ELISA or FACS, or methods involving agarose gel electrophoresis (e.g. fluorophore-assisted carbohydrate electrophoresis (FACE)) or polyacrylamide gel electrophoresis (PAGE).

Thus, in some embodiments, the level of one or more GAG forms (e.g. specific sulfated or unsulfated forms of CS or HS disaccharides, which have for example been derived from the full length GAG molecule or a chain of repeating disaccharide units of a GAG molecule by fragmentation, cleavage or digestion) in association with (e.g. physical association with or in complex with or derivitized with) a reagent that is being used to detect the GAG form is determined. Thus, in some embodiments the level of a complex of a GAG form and the reagent used to detect the GAG form is determined. Reagents suitable for detecting particular GAG forms are discussed elsewhere herein, but include antibodies, or some kind of fluorophore (or other detectable label or dye) attached to (or used to derivitize) the GAG form in question, for example to make it detectable by a fluorimeter (or other detection device). Thus, purely by way of example, in some embodiments the level of a GAG form in association with (e.g. in complex with or derivitized with) an antibody or fluorophore or the like may be determined.

As described above, in certain methods of the present invention, the level in a sample of an expression product(s) of certain genes is determined.

As referred to herein, an "expression product" of a gene includes mRNA molecules transcribed from the gene or polypeptides (proteins) encoded by the gene. The level of the mRNA or polypeptide (protein) in question can be determined by analysing the sample which has been obtained from or removed from the subject by an appropriate means. The determination is typically carried out *in vitro.*

Nucleotide and amino acid sequences of genes to which the present invention relates are known in the art, for example such sequences are provided in the Uniprot database (http://www.uniprot.org/). Official gene symbols and official (approved) gene names (e.g. *as per* Hugo Gene Nomenclature Committee, HGNC) of genes to which methods of the present invention relate are set forth in Table H herein.

It will be appreciated that an mRNA molecule will comprise the same sequence as the DNA molecule from which it was transcribed, with the exception the mRNA molecule will comprise uracil whereas the DNA molecule from which it was transcribed would instead comprise thymine at the corresponding positions.

In one embodiment, the expression product detected by the methods of the invention is an mRNA molecule. As discussed elsewhere herein, it is not necessary to detect the presence of the entire mRNA molecule (i.e. the entire mRNA nucleotide sequence); detecting the presence of a fragment or portion of an mRNA molecule can be indicative of the presence of the entire mRNA molecule.

In another embodiment, the expression product detected by the methods of the invention is a polypeptide. As discussed elsewhere herein, it is not necessary to detect the presence of the entire polypeptide (i.e. the polypeptide's entire amino acid sequence); detecting the presence of a fragment or portion of a polypeptide may be indicative of the presence of the entire polypeptide.

A number of different methods for detecting nucleic acids (e.g. mRNA) are known and described in the literature and any of these may be used according to the present invention. At its simplest, the nucleic acid may be detected by hybridisation to a probe (e.g. an oligonucleotide probe) and many such hybridisation protocols have been described (see e.g. Sambrook et al., Molecular cloning: A Laboratory Manual, 3rd Ed., 2001, Cold Spring Harbor Press, Cold Spring Harbor, NY). Typically, the detection will involve a hybridisation step and/or an *in vitro* amplification step.

In one embodiment, the target nucleic acid in a sample may be detected by using an oligonucleotide with a label attached thereto, which can hybridise to the nucleic acid sequence of interest. Such a labelled oligonucleotide will allow detection by direct means or indirect means. In other words, such an oligonucleotide may be used simply as a conventional oligonucleotide probe. After contact of such a probe with the sample under conditions which allow hybridisation, and typically following a step (or steps) to remove unbound labelled oligonucleotide and/or non-specifically bound oligonucleotide, the signal from the label of the probe emanating from the sample may be detected. In preferred embodiments the label is selected such that it is detectable only when the probe is hybridised to its target.

In another embodiment, the target nucleic acid (e.g. mRNA) in a sample may be determined by using an oligonucleotide probe which is labelled only when hybridised to its target sequence, i.e. the probe may be selectively labelled. Conveniently, selective labelling may be achieved using labelled nucleotides, i.e. by incorporation into the oligonucleotide probe of a nucleotide carrying a label. In other words, selective labelling may occur by chain extension of the oligonucleotide probe using a polymerase enzyme which incorporates a labelled nucleotide, preferably a labelled dideoxynucleotide (e.g. ddATP, ddCTP, ddGTP, ddTTP, ddUTP). This approach to the detection of specific nucleotide sequences is sometimes referred to as primer extension analysis. Suitable primer extension analysis techniques are well known to the skilled man, e.g. those techniques disclosed in WO99/50448, the contents of which are incorporated herein by reference.

In one embodiment of the present invention, the presence and level of mRNA gene products, or fragments thereof, are detected by a primer-dependent nucleic acid amplification reaction. The amplification reaction is allowed to proceed for a duration (e.g. number of cycles) and under conditions that generate a sufficient amount of amplification product. Most conveniently the polymerase chain reaction (PCR) will be used, although the skilled man would be aware of other techniques. For instance LAR/LCR, SDA, Loop-mediated isothermal amplification and nucleic acid sequence based amplification (NASBA)/3SR (Self-Sustaining Sequence Replication) may be used. If an mRNA gene product is to be detected, it will generally first be converted into a cDNA molecule by reverse transcription using a reverse transcriptase enzyme to generate a cDNA molecule. Upon completion of the reverse transcription reaction, the cDNA can be used as the template for the primer-dependent nucleic acid amplification reaction. A person skilled in the art will be well aware of how to generate cDNA molecules from mRNA molecules.

Many variations of PCR have been developed, for instance Real Time PCR (also known as quantitative PCR, qPCR), hot-start PCR, competitive PCR, and so on, and these may all be employed where appropriate to the needs of the skilled man.

In one basic embodiment using a PCR based amplification, oligonucleotide primers are contacted with a reaction mixture containing or potentially containing the target sequence and free nucleotides in a suitable buffer. Thermal cycling of the resulting mixture in the presence of a DNA polymerase results in amplification of the sequence between the primers.

Optimal performance of the PCR process is influenced by choice of temperature, time at temperature, and length of time between temperatures for each step in the cycle. A person skilled in the art is readily able to do this.

Methods of the present invention may be performed with any of the standard mastermixes and enzymes available.

Modifications of the basic PCR method such as qPCR (Real Time PCR) have been developed that can provide quantitative information on the template being amplified. Numerous approaches have been taken although the two most common techniques use double-stranded DNA binding fluorescent dyes or selective fluorescent reporter probes.

Double-stranded DNA binding fluorescent dyes, for instance SYBR Green, associate with the amplification product as it is produced and when associated the dye fluoresces. Accordingly, by measuring fluorescence after every PCR cycle, the relative amount of amplification product can be monitored in real time. Through the use of internal standards and controls, this information can be translated into quantitative data on the amount of template at the start of the reaction.

Fluorescent reporter probes used in qPCR may be sequence specific oligonucleotides, typically RNA or DNA, that have a fluorescent reporter molecule at one end and a quencher molecule at the other (e.g. the reporter molecule is at the 5' end and a quencher molecule at the 3' end or vice versa). The probe is designed so that the reporter is quenched by the quencher. The probe is also designed to hybridise selectively to particular regions of complementary sequence which might be in the template. If these regions are between the annealed PCR primers the polymerase, if it has exonuclease activity, will degrade (depolymerise) the bound probe as it extends the nascent nucleic acid chain it is polymerising. This will relieve the quenching and fluorescence will rise. Accordingly, by measuring fluorescence after every PCR cycle, the relative amount of amplification product can be monitored in real time. Through the use of internal standard and controls, this information can be translated into quantitative data.

The amplification product may be detected, and amounts (levels) of amplification product can be determined by any convenient means. A vast number of techniques are routinely employed as standard laboratory techniques and the literature has descriptions of more specialised approaches. At its most simple the amplification product may be detected by visual inspection of the reaction mixture at the end of the reaction or at a desired time point. Typically the amplification product will be resolved with the aid of a label that may be preferentially bound to the amplification product. Typically a dye substance, e.g. a colorimetric, chromomeric fluorescent or luminescent dye (for instance ethidium bromide or SYBR green) is used. In other embodiments a labelled oligonucleotide probe that preferentially binds the amplification product is used.

In some embodiments, a microarray may be used to determine the level of nucleic acid expression products of one or more of the genes.

In some embodiments, RNA-seq by next generation sequencing may be used to determine the level of nucleic acid expression products of one or more of the genes. RNA-seq (RNA sequencing) is sometimes referred to as whole transcriptome shotgun sequencing (WTSS). RNA-seq uses the capabilities of next generation sequencing to reveal a snapshot of RNA presence and quantity from a genome at a given moment in time. In some cases RNA can be converted to cDNA (via reverse transcription) prior to sequencing. In other cases RNA can be directly sequenced without conversion to cDNA. In some cases, cDNA is followed by adapter ligation prior to sequencing. RNA or cDNA is subsequently amplified by PCR to generate sufficient quantities of fragments prior to sequencing. In some cases, dUTP is incorporated during second strand cDNA synthesis to prevent PCR amplification and reduce bias introduced by PCR in the level determination. In some cases, a different adapter of known orientation is incorporated during second strand cDNA synthesis.

Suitable microarray platforms or machines and suitable RNA-seq platforms or machines are known in the art and can be used in the present invention. Suitable platforms or machines include those from manufacturers including Affymetrix, Agilent, Applied Microarrays, Arrayit, Illumina, and Pacific Biosciences, for example platforms or machines such as Affymetrix GeneChip Systems, Illumina MiniSeq System, Illumina MiSeq Series, Illumina NextSeq System, Illumina HiSeq Series, Pacific Biosciences PacBio RS II, or Pacific Biosciences Sequel Systems.

In some preferred embodiments of the present invention measuring the level of one or more expression products is by a nucleic acid (DNA/RNA) based method and preferably involves nucleic acid amplification.

Levels of one or more of the polypeptides in the sample can be measured (determined) by any appropriate assay, a number of which are well known and documented in the art and some of which are commercially available. The level of one or more of the polypeptides (proteins/biomarkers) can be determined e.g. by an immunoassay such as a radioimmunoassay (RIA) or fluorescence immunoassay, immunoprecipitation and immunoblotting (e.g. Western blotting) or Enzyme-Linked ImmunoSorbent Assay (ELISA). Immunoassays are a preferred technique for determining the levels of one or more of the polypeptides in accordance with the present invention.

Preferred assays are ELISA-based assays, although RIA-based assays can also be used effectively. Both ELISA- and RIA-based methods can be carried out by methods which are standard in the art and would be well known to a skilled person. Such methods generally involve the use of an antibody to a relevant polypeptide under investigation, or fragment thereof, which is incubated with the sample to allow detection of said polypeptide (or fragment thereof) in the sample. Any appropriate antibodies can be used. For example, an appropriate antibody to a polypeptide under investigation, or an antibody which recognises particular epitopes of said polypeptide, can be prepared by standard techniques, e.g. by immunization of experimental animals, which are known to a person skilled in the art. The same antibody to a given polypeptide under investigation or fragments thereof can generally be used to detect said polypeptide in either a RIA-based assay or an ELISA-based assay, with the appropriate modifications made to the antibody in terms of labeling etc., e.g. in an ELISA assay the antibodies would generally be linked to an enzyme to enable detection. Any appropriate form of assay can be used, for example the assay may be a sandwich type assay or a competitive assay.

In simple terms, in ELISA an unknown amount of antigen is affixed to a surface, and then a specific antibody is washed over the surface so that it can bind to the antigen. This antibody is linked to an enzyme, and in the final step a substance is added that the enzyme can convert to some detectable signal. Thus in the case of fluorescence ELISA, when light of the appropriate wavelength is shone upon the sample, any antigen/antibody complexes will fluoresce so that the amount of antigen in the sample can be determined through the magnitude of the fluorescence. For RIA, a known quantity of an antigen is made radioactive, frequently by labeling it with gamma-radioactive isotopes of iodine attached to tyrosine. This radiolabeled antigen is then mixed with a known amount of antibody for that antigen, and as a result, the two chemically bind to one another. Then, a sample from a patient containing an unknown quantity of that same antigen is added. This causes the unlabeled (or "cold") antigen from the sample to compete with the radiolabeled antigen for antibody binding sites. As the concentration of "cold" antigen is increased, more of it binds to the antibody, displacing the radiolabeled variant, and reducing the ratio of antibody-bound radiolabeled antigen to free radiolabeled antigen. The bound antigens are then separated from the unbound ones, and the radioactivity of the free antigen remaining in the supernatant is measured. A binding curve can then be plotted, and the exact amount of antigen in the patient's sample can be determined. Measurements are usually also carried out on standard samples with known concentrations of marker (antigen) for comparison.

In some embodiments, immunohistochemistry with appropriate antibodies could be carried out.

The use of immunoblotting (e.g. Western blotting) can also be used for measuring the level of one or more of the polypeptides in accordance with the present invention.

Preferred agents for use in determining the level of one or more of the polypeptides in accordance with the present invention are antibodies (antibodies to the polypeptide whose level is to be determined).

In other preferred embodiments, the level of one or more of the polypeptides in the sample can be measured (determined) by mass spectrometry. Suitable mass spectrometry methods (and associated data processing techniques) are well known and documented in the art. In some embodiments mass spectrometry (and associated data processing techniques) is used to obtain a ratio of the level of a polypeptide in the sample in comparison to a control. In some embodiments, protein fragments may be quantified using chromatography coupled with mass spectrometry.

Reference herein to the "polypeptides" whose level is to be determined in accordance with the invention includes reference to all forms of said polypeptides (as appropriate) which might be present in a subject, including derivatives, mutants and analogs thereof, in particular fragments thereof or modified forms of the polypeptides or their fragments. Exemplary and preferred modified forms include forms of these molecules which have been subjected to post translational modifications such as glycosylation or phosphorylation. In some embodiments, the level of unmodified forms of the polypeptides (or their fragments) is determined.

It is well understood in the art that when detecting the presence of a polypeptide (protein) in a sample, it is not necessary to detect the presence of the full-length polypeptide (i.e. the entire polypeptide sequence); detecting the presence of a fragment (or portion) of a polypeptide can be indicative of the presence of the entire polypeptide (protein).

Thus, in certain embodiments of the methods of the invention described herein, any fragments (or portions) of the polypeptides, in particular naturally occurring fragments, can be analysed as an alternative to the polypeptides themselves (full length polypeptides). Suitable fragments for analysis should be characteristic of the full-length polypeptide (protein).

Suitable fragments can be at least 6 consecutive amino acids in length. For example, at least 7, at least 8, at least 9, at least 10, at least 15, at least 20, at least 25, at least 50, at least 75, at least 100, at least 150, at least 200 or at least 500 consecutive amino acids in length. Suitable fragments can represent at least 5%, at least 10%, at least 20%, at least 30%, at least 40%, at least 50%, at least 60%, at least 70%, at least 80%, or at least 90% of the length of the full-length polypeptide (protein).

In some embodiments the level of the full-length polypeptide is determined.

It is also well understood in the art that when detecting the presence of an mRNA in a sample it is not necessary to detect the presence of the entire mRNA molecule (i.e. the entire mRNA nucleotide sequence); detecting the presence of a fragment (or portion) of an mRNA molecule can be indicative of the presence of the entire mRNA molecule.

Thus, in certain embodiments of the methods of the invention described herein, any fragments (or portions) of the mRNAs can be analysed as an alternative to the full length mRNAs. Suitable fragments for analysis should be characteristic of the full-length mRNA. Suitable fragments can be at least 17 nucleotides in length. For example, at least 18, at least 19, at least 20, at least 25, at least 30, at least 35, at least 40, at least 50, at least 75, at least 100, at least 150, at least 200 or at least 500 consecutive nucleotides in length. Suitable fragments can represent at least 5%, at least 10%, at least 20%, at least 30%, at least 40%, at least 50%, at least 60%, at least 70%, at least 80%, or at least 90% of the length of the full-length mRNA molecule.

In some embodiments the level of the full-length mRNA molecule is determined.

In some embodiments, the level of an expression product in association with (e.g. physical association with or in complex with) the reagent that is being used to detect the expression product is determined. Thus, in some embodiments the level of a complex of an expression product and the reagent used to detect the expression product is determined. Reagents suitable for detecting expression products are discussed elsewhere herein. Purely by way of example, in some embodiments the level of a nucleic acid (DNA or RNA) expression product in association with (e.g. in complex with) a primer (or extended primer) or probe (e.g fluorecent reporter probe) or dye or the like may be determined. By way of another example, in some embodiments the level of a polypeptide expression product in association with (e.g. in complex with) an antibody may be determined.

In some embodiments, the level of one or more expression products is the level in cancer cells (e.g. metastatic cancer cells), for example as isolated from a sample such as a blood sample (or other type of sample described herein), or in some embodiments, the level of one or more expression products is the level in an exosome sample (e.g. cancer derived exosome sample). In some such embodiments, the level of one or more expression products (e.g. nucleic acid expression products) in cancer cells (e.g. metastatic cancer cells) or in exosomes (e.g. cancer derived exosomes) may be determined using an array (e.g. a microarray). A particularly preferred cancer is prostate cancer.

In one aspect, the present invention provides a solid support (e.g. a chip) comprising a group of one or more probes (e.g. nucleic acid probes) capable of detecting the presence or level of an expression product (e.g. nucleic acid expression product) of one or more of the genes or groups of genes described herein. In some embodiments, said group of one or more probes comprises or consists of at least 5, at least 10, at least 20, at least 30, at least 40, at least 50 or 60 probes (e.g. 5-60 or 10-60 or 20-60 or 30-60 or 40-60 or 50-60).

An altered level (or composition) of one or more of the GAG forms (GAG properties) or expression products as described herein includes any measurable alteration or change of the GAG form (biomarker) or expression product in question when the GAG form or expression product in question is compared with a control level. An altered level includes an increased or decreased level. Preferably, the level is significantly altered, compared to the level found in an appropriate control sample or subject. More preferably, the significantly altered levels are statistically significant, preferably with a p-value of <0.05 or a % in ROPE value of $\leq 5.00$.

In some embodiments, an alteration in level (e.g. of an expression product) of $\geq 2\%$, $\geq 3\%$, $\geq 5\%$, $\geq 10\%$, $\geq 25\%$, $\geq 50\%$, $\geq 75\%$, $\geq 100\%$, $\geq 200\%$, $\geq 300\%$, $\geq 400\%$, $\geq 500\%$, $\geq 600\%$, $\geq 700\%$, $\geq 800\%$, $\geq 900\%$, $\geq 1000\%$, $\geq 2000\%$, $\geq 5000\%$, or $\geq 10,000\%$ compared to the level found in an appropriate control sample or subject or population (i.e. when compared to a control level) may be indicative of the presence of cancer (e.g. prostate cancer).

The "increase" in the level or "increased" level of one or more of the GAG forms (GAG properties) or expression products as described herein includes any measurable increase or elevation of the GAG form (biomarker) or expression product in question when the GAG form or expression product in question is compared with a control level. Preferably, the level is significantly increased, compared to the level found in an appropriate control sample or subject. More preferably, the significantly increased levels are statistically significant, preferably with a p-value of <0.05 or a % in ROPE value of $\leq 5.00$.

In some embodiments, an increase in level (e.g. of an expression product) of $\geq 2\%$, $\geq 3\%$, $\geq 5\%$, $\geq 10\%$, $\geq 25\%$, $\geq 50\%$, $\geq 75\%$, $\geq 100\%$, $\geq 200\%$, $\geq 300\%$, $\geq 400\%$, $\geq 500\%$, $\geq 600\%$, $\geq 700\%$, $\geq 800\%$, $\geq 900\%$, $\geq 1000\%$, $\geq 2000\%$, $\geq 5000\%$, or $\geq 10,000\%$ compared to the level found in an appropriate control sample or subject or population (i.e. when compared to a control level) may be indicative of the presence of cancer (e.g. prostate cancer).

The "decrease" in the level or "decreased" level of one or more of the GAG forms (GAG properties) or expression products as described herein includes any measurable decrease or reduction of the GAG form (biomarker) or expression product in question when the GAG form or expression product in question is compared with a control level. Preferably, the level is significantly decreased, compared to the level found in an appropriate control sample or subject. More preferably, the significantly decreased levels are statistically significant, preferably with a p-value of <0.05 or a % in ROPE value of ≤5.00.

In some embodiments, a decrease in level (e.g. of an expression product) of ≥2%, ≥ 3%, ≥ 5%, ≥ 10%, ≥ 25%, ≥ 50%, ≥75%, ≥100%, ≥200%, ≥300%, ≥400%, ≥500%, ≥600%, ≥700%, ≥800%, ≥900%, ≥1000%, ≥2000%, ≥5000%, or ≥10,000% compared to the level found in an appropriate control sample or subject or population (i.e. when compared to a control level) is indicative of the presence of cancer (e.g. prostate cancer).

A "control level" is the level of a GAG form (GAG property) or expression product in a control subject or population (e.g. in a sample that has been obtained from a control subject or population). Appropriate control subjects or samples for use in the methods of the invention would be readily identified by a person skilled in the art, for example an appropriate control group is as described in the Examples. Such subjects might also be referred to as "normal" subjects or as a reference population. Examples of appropriate populations of control subjects would include healthy subjects, for example, individuals who have no history of any form of prostate disease (e.g. prostate cancer) and no other concurrent disease, or subjects who are not suffering from, and preferably have no history of suffering from, any form of prostate disease, in particular individuals who are not suffering from, and preferably have no history of suffering from, prostate cancer. Other preferred control subjects would include individuals who are not suffering from, and preferably have no history of, cancer (e.g. not suffering from, and preferably have no history of, any of the types of cancer referred to herein). Other examples of appropriate populations of control subjects would include healthy subjects, for example, individuals who have no history of any form of kidney disease (e.g. RCC) and no other concurrent disease, or subjects who are not suffering from, and preferably have no history of suffering from, any form of kidney disease, in particular individuals who are not suffering from, and preferably have no history of suffering from, renal cancer or RCC. Preferably such control subjects are also not suffering from, and more preferably have no history of suffering from, liver cancers. Preferably such control subjects are also not suffering from inflammatory pathologies. Preferably control subjects are not regular users of any medication. In a preferred embodiment control subjects are healthy subjects.

The control level may correspond to the level of the equivalent GAG form in appropriate control subjects or samples, e.g. may correspond to a cut-off level or range found in a control or reference population. Alternatively, said control level may correspond to the level of the marker (GAG form or expression product) in question in the same individual subject, or a sample from said subject, measured at an earlier time point (e.g. comparison with a "baseline" level in that subject). This type of control level (i.e. a control level from an individual subject) is particularly useful for embodiments of the invention where serial or periodic measurements of GAG form(s) (or expression product(s)) in individuals, either healthy or ill, are taken looking for changes in the levels of the GAG form(s) (or expression product(s)). In this regard, an appropriate control level will be the individual's own baseline, stable, nil, previous or dry value (as appropriate) as opposed to a control or cut-off level found in the general control population. Control levels may also be referred to as "normal" levels or "reference" levels. The control level may be a discrete figure or a range.

Although the control level for comparison could be derived by testing an appropriate set of control subjects, the methods of the invention would not necessarily involve carrying out active tests on control subjects as part of the methods of the present invention but would generally involve a comparison with a control level which had been determined previously from control subjects and was known to the person carrying out the methods of the invention.

For methods of the present invention which involve the determination of the level of one or more expression products, control levels and subjects may be, *mutatis mutandis,* as discussed elsewhere herein. Alternatively, for methods of the present invention which involve the determination of the level of one or more expression products, a control level may be the level of an expression product in a healthy tissue sample (control tissue sample) of a control subject or population. Thus, a control tissue sample (e.g. prostate tissue sample) may be from the same tissue as the test sample (i.e. the potentially cancerous sample e.g. potentially cancerous prostate sample) being screened. The control tissue sample may thus be a normal tissue sample that is matched to the test tissue sample (potentially cancerous sample).

The methods of screening, diagnosis etc., of the present invention are for cancer. Particular cancers to which the present invention relates are described elsewhere herein. In preferred embodiments, the prostate cancer subtype is prostate adenocarcinoma (also referred to herein as PRAD). In some embodiments, the thyroid cancer subtype is thyroid carcinoma. In some embodiments, the colon cancer subtype is colon adenocarcinoma. In some embodiments, the rectum cancer subtype is rectum adenocarcinoma. Thus, in some embodiments, the cancer is colorectal adenocarcinoma. Colon cancer and rectum cancer may be collectively referred to as colorectal cancer. In some embodiments, the colorectal cancer is of TNM stage I, II, III or IV. In some embodiments, the lung cancer subtype is lung squamous cell carcinoma or lung adenocarcima or non-small cell lung cancer. In some embodiments, the lung cancer is of TNM stage I, II, III or IV. In some embodiments, the uterine cancer subtype is uterine corpus endometrial carcinoma or cervical cancer or endometrial cancer. In some embodiments, the uterine cancer subtype is cervical cancer or endometrial cancer. In some embodiments, the uterine cancer (e.g. cervical cancer or endometrial cancer) is of FIGO stage I, II, III or IV. In some embodiments, the breast cancer subtype is breast invasive carcinoma. In some embodiments, the pancreatic cancer subtype is pancreatic adenocarcinoma. In some embodiments, the bladder cancer subtype is bladder carcinoma. In some embodiments, the liver cancer subtype is liver hepatocellular carcinoma. In some embodiments, the bile duct cancer subtype is cholangiocarcinoma. In some embodiments, the stomach cancer subtype is stomach adenocarcinoma. In some embodiments, the oesophageal cancer subtype is oesophageal carcinoma. In some embodiments, the head and neck cancer subtype is head and neck squamous cell carcinoma. In some embodiments, the brain cancer subtype is glioblastoma multiforme or diffuse glioma (e.g. astrocytoma, glioblastoma multiforme or oligoastrocytoma). In some embodiments, the blood cancer subtype is peripheral T-cell lymphoma or non-Hodgkins lymphoma or chronic lymphoid leukaemia. In some embodiments, the blood cancer subtype is non-Hodgkins lymphoma or chronic lymphoid leukaemia. In some embodiments, the chronic lymphoid leukaemia is of Binet stage A, B or C. In some embodiments, the blood cancer subtype of non-Hodgkins lymphoma is diffuse large B-cell lymphoma. In some embodiments, the non-Hodgkins lymphoma is of Ann Arbor stage I, II, III or IV. In some embodiments, the ovarian cancer subtype is serous ovarian adenocarcinoma. In some embodiments, the ovarian cancer is of FIGO stage I, II, III or IV. In some embodiments, the skin cancer subtype is skin melanoma (e.g. malignant melanoma). In some embodiments the melanoma is skin melanoma or uveal melanoma (e.g. malignant skin melanoma or uveal melanoma). In some embodiments, the neuroendocrine tumour is a gastrointestinal neuroendocrine tumour. In some embodiments the neuroendocrine tumour (e.g. gastrointestinal neuroendocrine tumour) is of ENETS grade G1 or G2. In some embodiments, the kidney cancer is renal cell carcinoma such as clear cell renal cell carcinoma, papillary renal cell carcinoma or chromophobe renal cell carcinoma. The methods of the present invention can be carried out on any stage of cancer (e.g. prostate cancer), for example can be used for early or initial stages of cancer (e.g. prostate cancer) or advanced or late stage cancer disease (e.g. prostate cancer).

The classification of cancer (e.g. prostate cancer) at a given stage can be carried out by any art recognised and accepted definition.

The classification of cancer (e.g. prostate cancer) at a given risk can be carried out by any art recognised and accepted definition. For example, for prostate cancer, risk may be assessed by a digital rectal exam or by assessing blood PSA level at diagnosis or by determining the Gleason score at clinical diagnosis or by determining the Gleason score at pathological diagnosis or by evaluating a prostate biopsy results (e.g. by evaluating tumor size, and/or tumor grade and/or tumor volume) or by determining stage according to the TNM system or by assessing the results of other tests (x-rays, CT and/or MRI scans, and bone scans) or any combination of the above. A person skilled in the art can readily determine risk based on one or more of the assessment above, for example by determining low risk if the Gleason score is 6 or less, intermediate risk if the Gleason score is 7, and high risk if the Gleason score is 8 or more.

In some embodiments, the cancer (e.g. prostate cancer) may be a non-metastatic form of cancer (e.g. prostate cancer). In some embodiments, the cancer (e.g. prostate cancer) may be a metastatic form of cancer (e.g. prostate cancer) (as opposed to localized or confined cancer, e.g. prostate cancer).

In some embodiments the cancer may be a low-stage/grade disease (cancer). A low-stage/grade disease (cancer) may be defined as TNM stage I to III for CRC and LC, FIGO stage I for CST, FIGO stage I to II for EC and OV, non-Grade IV glioma for DG, ENETS grade G1 for GNET, Binet stage A or B for LL, Ann Arbor stage I to II for NHL, and Gleason grade < 7 for PCa.

The methods of the present invention can be carried out on any appropriate body fluid sample. In this regard, although the present invention is exemplified with blood and urine, appropriate GAG forms (or expression products) to be measured in other types of body fluid sample could be determined by a skilled person following the teaching as provided herein. Typically the sample has been obtained from (removed from) a subject (e.g. as described elsewhere herein, preferably a human subject (typically a human male subject in the case of prostate cancer screening). In other aspects, the method further comprises a step of obtaining a sample from the subject.

Reference herein to "body fluid" includes reference to all fluids derived from the body of a subject. Exemplary fluids include blood (including all blood derived components, for example plasma, serum, etc.), urine, saliva, tears, bronchial secretions or mucus. Preferably, the body fluid is a circulatory fluid (especially blood or a blood component) or urine. Especially preferred body fluids are blood or urine. In some preferred embodiments the sample is a blood sample (e.g. a plasma or serum sample). In some preferred embodiments the sample is a plasma sample. In some preferred embodiments a plasma sample is a platelet-poor plasma sample. In some preferred embodiments the sample is a serum sample. In some preferred embodiments the sample is a urine sample. In some embodiments, the sample is not a urine sample. The body fluid or sample may be in the form of a liquid biopsy.

The term "sample" also encompasses any material derived by processing a body fluid sample (e.g. derived by processing a blood or urine sample). Processing of biological samples to obtain a test sample may involve one or more of: digestion, boiling, filtration, distillation, centrifugation, lyophilization, fractionation, extraction, concentration, dilution, purification, inactivation of interfering components, addition of reagents, derivatization, complexation and the like, e.g. as described elsewhere herein.

Any suitable method for isolating urine or blood (e.g.serum or plasma) samples may be employed.

Methods of the present invention which involve determining the level of an expression product of a gene can be carried out on any appropriate sample. Typically the sample has been obtained from (removed from) a subject, preferably a human subject (typically a male human subject in the case of screening for prostate cancer). In other aspects, the method further comprises a step of obtaining a sample from the subject. In some embodiments of methods of the present invention which involve determining the level of an expression product of a gene, the sample is a tissue sample from a subject (e.g. a tissue biopsy from tissue suspected of being cancerous). In some embodiments, body fluid samples may be used (e.g. as discussed elsewhere herein).

Any sample that is directly or indirectly affected by the suspected cancer (e.g. tumour) may be used. In some embodiments, the sample is blood or plasma. A blood (e.g. plasma or serum) sample may comprise DNA and/or RNA from circulating tumour cells and or proteins that have diffused from a tumour (e.g. a prostate tumour). In some embodiments, a sample may comprise circulating tumour cells (e.g. metastatic tumour cells). In some embodiments, a sample may comprise exosomes (e.g. purified or enriched exosomes). In some embodiments the sample is urine. Urine samples may comprise DNA and/or RNA and/or proteins that have diffused from a tumour.

The term "sample" also encompasses any material derived by processing (e.g. as described above) a biological sample. Derived materials include, but are not limited to, cells isolated from the sample (e.g. cancer cells or metastactic cancer cells), cell components, exosomes, proteins/peptides and nucleic acid molecules (DNA or RNA) extracted from the sample.

In some embodiments, methods of the invention may include a step of processing a sample. In some embodiments, methods of the invention may thus be performed on such processed samples or materials derived from such processed samples. Processing steps include, but are not limited to, isolating cells from the sample, isolating cell components from the sample, isolating or enriching exosomes from the sample, extracting (e.g. isolating or purifying) proteins/peptides and/or nucleic acid molecules (DNA or RNA) from the sample. A processing step may involve one or more of filtration, distillation, centrifugation, extraction, concentration, dilution, purification, inactivation of interfering components, addition of reagents, derivatization, amplification, adapter ligation, and the like.

Samples can be used immediately or can be stored for later use (e.g. at -80°C).

The methods of the invention as described herein can be carried out on any type of subject which is capable of suffering from cancer (e.g. prostate cancer). The methods are generally carried out on mammals (typically male mammals in the case of prostate cancer screening), for example humans, primates (e.g. monkeys), laboratory mammals (e.g. mice, rats, rabbits, guinea pigs), livestock mammals (e.g. horses, cattle, sheep, pigs) or domestic pets (e.g. cats, dogs). Preferably the subject is a human (typically a male human in the case of prostate cancer screening).

In one embodiment, the subject (e.g. a human) is a subject at risk of developing cancer (e.g. prostate cancer) or at risk of the occurrence of cancer (e.g. prostate cancer), e.g. a healthy subject or a subject not displaying any symptoms of disease (e.g. prostate disease) or any other appropriate "at risk" subject as described elsewhere herein. In another embodiment the subject is a subject having, or suspected of having (or developing), or potentially having (or developing) cancer (e.g. prostate cancer). In some embodiments, particularly for prostate cancer screening, the "at risk" subject may be a human male over 40 years old, or over 50 years old, or over 55 years old, or over 60 years old, or preferably over 65 years old. In some embodiments, the "at risk" subject may be a human male with a PSA (prostate specific antigen) level (e.g. in blood) that is ≥4ng/ml, or ≥5ng/ml, ≥6ng/ml, ≥7ng/ml, ≥8ng/ml, ≥9ng/ml (e.g. 9-19 ng/ml), ≥10ng/ml, or ≥11ng/ml, ≥12ng/ml, ≥13ng/ml, ≥14ng/ml, ≥15ng/ml, ≥16ng/ml, ≥17ng/ml, ≥18ng/ml, ≥19ng/ml, or ≥20ng/ml or ≥25ng/ml, or ≥50ng/ml (e.g. 4-20 or 4-50 ng/ml).

In some aspects, a method of the invention may further comprise an initial step of selecting a subject (e.g. a human subject) at risk of developing cancer (e.g. prostate cancer) or at risk of the occurrence of cancer (e.g. prostate cancer), or having or suspected of having (or developing) cancer (e.g. prostate cancer), or potentially having (or developing) cancer (e.g. prostate cancer). The subsequent method steps can be performed on a sample from such a selected subject.

In some aspects, methods of the invention are provided which further comprise a step of treating cancer (e.g. prostate cancer) by therapy (e.g. pharmaceutical therapy) or surgery. For example, if the result of a method of the invention is indicative of cancer (e.g. prostate cancer) in the subject (e.g. a positive diagnosis of cancer, e.g. prostate cancer, is made), then an additional step of treating the cancer (e.g. prostate cancer) by therapy or surgery can be performed. For example, if the result of a method of the invention is indicative of high risk cancer (e.g. prostate cancer) in the subject (e.g. a positive diagnosis of high risk cancer, e.g. prostate cancer, is made), then an additional step of treating the cancer (e.g. prostate cancer) by therapy or surgery can be performed. For example, if the result of a method of the invention is indicative of low risk cancer (e.g. prostate cancer) in the subject (e.g. a positive diagnosis of low risk cancer, e.g. prostate cancer, is made), then an additional step of watchful waiting or active surveillance can be performed. Methods of treating cancer (e.g. prostate cancer) by therapy or surgery are known in the art. For example, one surgical option for prostate cancer is prostatectomy, which is aimed at eradication of the tumour and can be either radical (total removal) or partial. Pharmaceutical treatment can include standard chemotherapy and immunotherapy and hormone therapy, in addition to therapies which are subject to ongoing clinical trials. Pharmaceutical treatment can include standard chemotherapy (e.g. gemcitabine, vinblastine, floxuridine, 5-fluorouracil, or capecitabine), targeted therapies (including tyrosine kinase inhibitors, mTOR pathway inhibitors, VEGF pathway inhibitors, or more specific examples such as sorafenib, sunitinib, temsirolimus, everolimus, bevacizumab, pazopanib, or axitinib), immunotherapy (such as interferon-gamma, interleukin-2, interferon-alpha, or PD-1 or PD-L1 blockers such as nivolumab) and hormone treatment (such as orchiectomy, luteinizing hormone-releasing hormone agonists or antagonists, anti-androgens, estrogen, or ketoconazole). Other forms of treatment include radiation therapy and cryotherapy and vaccine treatment (such as chimeric antigen receptors engineered (CAR) T cells).

Thus, in some embodiments, methods of the invention (e.g. screening or diagnosis methods) which further comprise a step of treating cancer (e.g. prostate cancer) may comprise administering to the subject a therapeutically effective amount of one or more agents selected from the group consisting of a chemotherapeutic agent, for example selected from gemcitabine, vinblastine, floxuridine, 5-fluorouracil, or capecitabine; an agent for targeted therapy, for example selected from tyrosine kinase inhibitors, mTOR pathway inhibitors, VEGF pathway inhibitors, or more specific examples such as sorafenib, sunitinib, temsirolimus, everolimus, bevacizumab, pazopanib, or axitinib; or an agent for immunotherapy, for example selected from interferon-gamma, interleukin-2, interferon-alpha, or PD-1 or PD-L1 blockers such as nivolumab, or an agent for hormone treatment, for example selected from luteinizing hormone-releasing hormone agonists (for example leuprolide, goserelin, triptorelin, histrelin) or antagonists (for example degarelix or CYP17 inhibitors), anti-androgens (for example flutamide, bicalutamide, nilutamide) or estrogen or ketoconazole.

Alternatively, methods of the invention are provided which further comprise a step of carrying out an additional diagnostic procedure, e.g. a CT scan or a PSA test.

Thus, in some embodiments, methods of the invention (e.g. screening or diagnosis methods) which further comprise a step of treating cancer (e.g. prostate cancer) may comprise administering to the subject a therapeutically effective amount of one or more agents selected from the group consisting of a chemotherapeutic agent, an agent for targeted therapy, or an agent for immunotherapy, or an agent for hormone therapy, or a dose of radiation therapy, or a dose of cryotherapy.

In some embodiments, if the level of one or more GAG properties (or expression products) in a sample, or a score based on these levels, is altered by a particular degree in comparison to a control level or score, then a further step of administering a therapeutically effective amount of a pharmaceutical agent (e.g. a chemotherapeutic agent etc., as described above) to the patient is performed and/or surgery is performed. Preferred degrees of alteration are discussed elsewhere herein.

In some embodiments, if a subject is already undergoing pharmaceutical therapy (e.g. chemotherapeutic therapy or other therapy as described above) and the level of one or more GAG properties (or expression products) in a sample, or a score based on these levels, is altered (or indeed not altered) by a particular degree in comparison to a control level (e.g. in comparison to a previously recorded level or score for the same subject), then this may be indicative that the current therapeutic agent is not being effective and that a therapeutic agent other than the previous therapeutic agent should be used. Thus, a step of administering a therapeutically effective amount of a therapeutic agent (e.g. a chemotherapeutic agent etc. as described above) other than the therapeutic agent previously administered to the subject may be performed.

In some embodiments, if a method of the invention reveals that a current treatment regimen is ineffective, e.g. if serial or periodic measurements of one or more GAG properties (or expression products) in a sample, or a score based on these levels reveal treatment is being ineffective, a step of altering (e.g. increasing) the dosage of the therapeutic agent may be performed.

More specifically for example, in some such aspects of the invention, methods are provided which comprise determining the level of one or more GAG properties (or expression products) in a sample and if one or more levels, or a score based on these levels, are determined to be greater than an appropriate cut-off level, e.g. a cut-off level pre-specified to maximise the accuracy for a positive diagnosis of cancer (e.g. prostate cancer), then said methods may comprise the further step of performing a surgery (e.g. prostatectomy), or performing an additional diagnostic procedure (e.g. a CT scan or a PSA test), or administering a therapeutically-effective amount of a recommended drug agent for the treatment of cancer (e.g. prostate cancer). Agents for example may comprise chemotherapies like gemcitabine, vinblastine, floxuridine, 5-fluorouracil, or capecitabine; targeted therapies, for example selected from tyrosine kinase inhibitors, mTOR pathway inhibitors, VEGF pathway inhibitors, or more specific examples such as sorafenib, sunitinib, temsirolimus, everolimus, bevacizumab, pazopanib, or axitinib; immunotherapies, for example selected from interferon-gamma, interleukin-2, interferon-alpha, or PD-1 or PD-L1 blockers such as nivolumab, or hormone therapies, for example luteinizing hormone-releasing hormone agonists (for example leuprolide, goserelin, triptorelin, histrelin) or antagonists (for example degarelix or CYP17 inhibitors), anti-androgens (for example flutamide, bicalutamide, nilutamide) or estrogen or ketoconazole.

Conversely, methods are provided which comprise determining the level of one or more GAG properties (or expression products) in a sample and if one or more levels, or a score based on these levels, are determined to be lower than an appropriate cut-off level, e.g. a cut-off level pre-specified to maximise the predictive value for a negative diagnosis of cancer (e.g. prostate cancer), then said methods may comprise the further step of not performing a surgery (e.g. prostatectomy), or performing an additional diagnostic procedure (e.g. a CT scan) or altering the current dosage of drug agent(s) or administering a therapeutically-effective amount of a distinct recommended drug agent for cancer (e.g. prostate cancer) to the one already being used. Agents for example may comprise chemotherapies like gemcitabine, vinblastine, floxuridine, 5-fluorouracil, or capecitabine; targeted therapies, for example selected from tyrosine kinase inhibitors, mTOR pathway inhibitors, VEGF pathway inhibitors, or more specific examples such as sorafenib, sunitinib, temsirolimus, everolimus, bevacizumab, pazopanib, or axitinib; immunotherapies, for example selected from interferon-gamma, interleukin-2, interferon-alpha, or PD-1 or PD-L1 blockers such as nivolumab, or hormone therapies, for example luteinizing hormone-releasing hormone agonists (for example leuprolide, goserelin, triptorelin, histrelin) or antagonists (for example degarelix or CYP17 inhibitors), anti-androgens (for example flutamide, bicalutamide, nilutamide) or estrogen or ketoconazole.

A yet further aspect provides a kit for screening for cancer (e.g. prostate cancer) (e.g. for diagnosing or for determining severity or prognosis of cancer, e.g. prostate cancer), which comprises one or more agents suitable for determining the level of one or more of the GAG properties (GAG forms) or expression products described herein, in a sample. Appropriate agents are discussed elsewhere herein and include, for example, antibodies. In some embodiments the kit is an ELISA kit. In preferred aspects said kits are for use in the methods of the invention as described herein. Preferably said kits comprise instructions for use of the kit components, for example in screening (e.g. diagnosis).

In one aspect, the present invention provides a method of detecting (or determining) the level and/or chemical composition of one or more of the glycosaminoglycans (GAGs) chondroitin sulfate (CS), heparan sulfate (HS) and hyaluronic acid (HA) in a body fluid sample, wherein said sample has been obtained from said subject.

In one aspect, the present invention provides a method of detecting the level and/or chemical composition of one or more of the glycosaminoglycans (GAGs) chondroitin sulfate (CS), heparan sulfate (HS) and hyaluronic acid (HA) in a patient, said method comprising:

(a) obtaining a body fluid sample from a human patient; and
(b) detecting the level and/or chemical composition of one or more of the glycosaminoglycans (GAGs) chondroitin sulfate (CS), heparan sulfate (HS) and hyaluronic acid (HA) in said sample.

The features and discussion herein in relation to the method of screening for cancer (e.g. prostate cancer) (e.g. method of diagnosing, method for prognosis etc.), for example in relation to preferred GAG forms or combinations thereof for measurement, can be applied, *mutatis mutandis,* to methods of detecting of the present invention.

In one aspect, the present invention provides a method of detecting (or determining) the level of an expression product of one or more genes selected from the group consisting of UST, CHST14, CHST13, CHSY1, DSE, CHSY3, CHST11, CHST15, CHPF2, CSGALNACT2, CHST12, CSGALNACT1, CHST7, CHPF, CHST3, HPSE, HGSNAT, HYAL4, GALNS, GLB1, GNS, GUSB, HEXA, HEXB, HYAL1, IDS, IDUA, ARSB, NAGLU, HPSE2, SGSH, SPAM1, HYAL3, HYAL2, HS3ST3B1, HS3ST3A1, B4GALT7, B3GALT6, B3GAT2, B3GAT3, B3GAT1, XYLT1, XYLT2, EXT1, EXT2, EXTL1, EXTL2, EXTL3, HS3ST5, GLCE, HS6ST3, NDST1, NDST4, HS6ST2, NDST3, HS6ST1, HS2ST1, HS3ST2, HS3ST1 and NDST2, wherein said sample has been obtained from said subject.

In one aspect, the present invention provides a method of detecting the level of an expression product of one or more genes selected from the group consisting of UST, CHST14, CHST13, CHSY1, DSE, CHSY3, CHST11, CHST15, CHPF2, CSGALNACT2, CHST12, CSGALNACT1, CHST7, CHPF, CHST3, HPSE, HGSNAT, HYAL4, GALNS, GLB1, GNS, GUSB, HEXA, HEXB, HYAL1, IDS, IDUA, ARSB, NAGLU, HPSE2, SGSH, SPAM1, HYAL3, HYAL2, HS3ST3B1, HS3ST3A1, B4GALT7, B3GALT6, B3GAT2, B3GAT3, B3GAT1, XYLT1, XYLT2, EXT1, EXT2, EXTL1, EXTL2, EXTL3, HS3ST5, GLCE, HS6ST3, NDST1, NDST4, HS6ST2, NDST3, HS6ST1, HS2ST1, HS3ST2, HS3ST1 and NDST2, said method comprising:

(a) obtaining a sample from a human patient; and
(b) detecting the level of an expression product of one or more of said genes in said sample.

The features and discussion herein in relation to the method of screening for cancer (e.g. method of diagnosing, method for prognosis etc.), for example in relation to preferred genes or combinations thereof for measurement, can be applied, *mutatis mutandis,* to methods of detecting of the present invention.

In another aspect, the present invention provides a method of screening for cancer in a subject, said method comprising
determining the level in a sample of an expression product of one or more genes selected from the group consisting of:

UST, CHST14, CHST13, CHSY1, DSE, CHSY3, CHST11, CHST15, CHPF2, CSGALNACT2, CHST12, CSGALNACT1, CHST7, CHPF, CHST3, HPSE, HGSNAT, HYAL4, GALNS, GLB1, GNS, GUSB, HEXA, HEXB, HYAL1, IDS, IDUA, ARSB, NAGLU, HPSE2, SGSH, SPAM1, HYAL3, HYAL2, HS3ST3B1, HS3ST3A1, B4GALT7, B3GALT6, B3GAT2, B3GAT3, B3GAT1, XYLT1, XYLT2, EXT1, EXT2, EXTL1, EXTL2, EXTL3, HS3ST5, GLCE, HS6ST3, NDST1, NDST4, HS6ST2, NDST3, HS6ST1, HS2ST1, HS3ST2, HS3ST1 and NDST2;

wherein said sample has been obtained from said subject. The features and discussion herein in relation to the method of screening for cancer (e.g. method of diagnosing, method for prognosis etc.), for example in relation to preferred genes or combinations thereof for measurement, can be applied, *mutatis mutandis,* to this aspect.

The invention will be further described with reference to the following nonlimiting Example with reference to the following drawings in which:

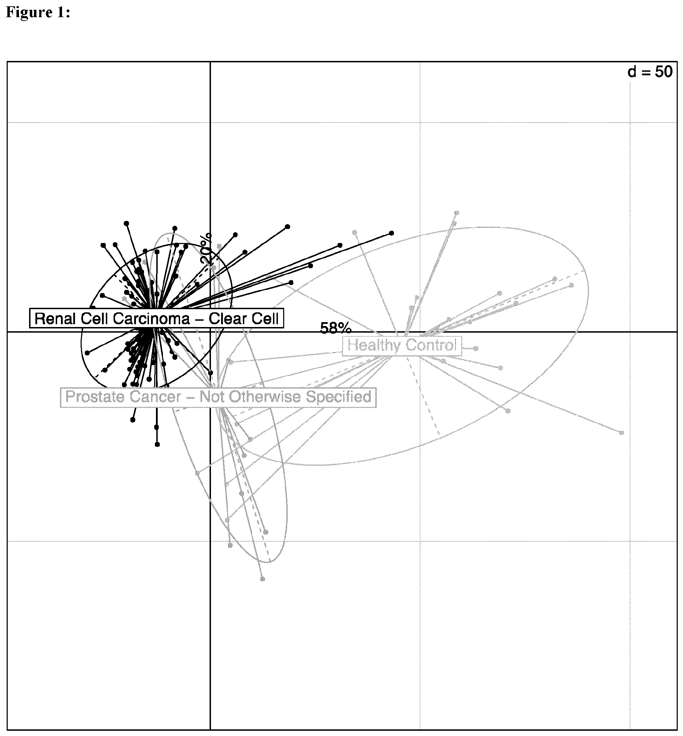

**Figure 1.** Principal component analysis of the GAG profiles in the blood of healthy (light grey), PRAD (dark grey), or clear cell RCC (black) subjects. Subjects belonging to a given group are represented as points connected to the geometrical center of the ellipsis representing that group. PRAD: Prostate Cancer. RCC: Renal Cell Carcinoma.

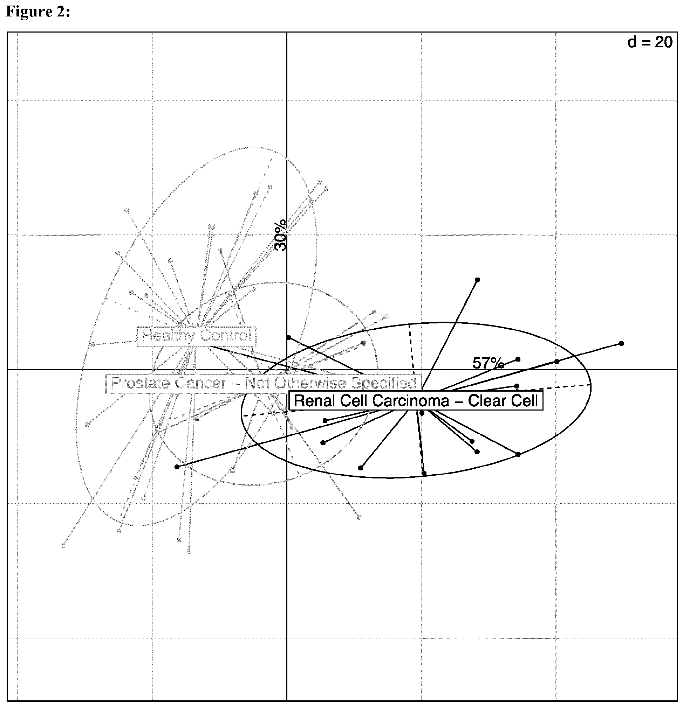

**Figure 2.** Principal component analysis of the GAG profiles in the urine of healthy (light grey), PRAD (dark grey), or clear cell RCC (black) subjects. Subjects belonging to a given group are represented as points connected to the geometrical center of the ellipsis representing that group. PRAD: Prostate Cancer. RCC: Renal Cell Carcinoma.

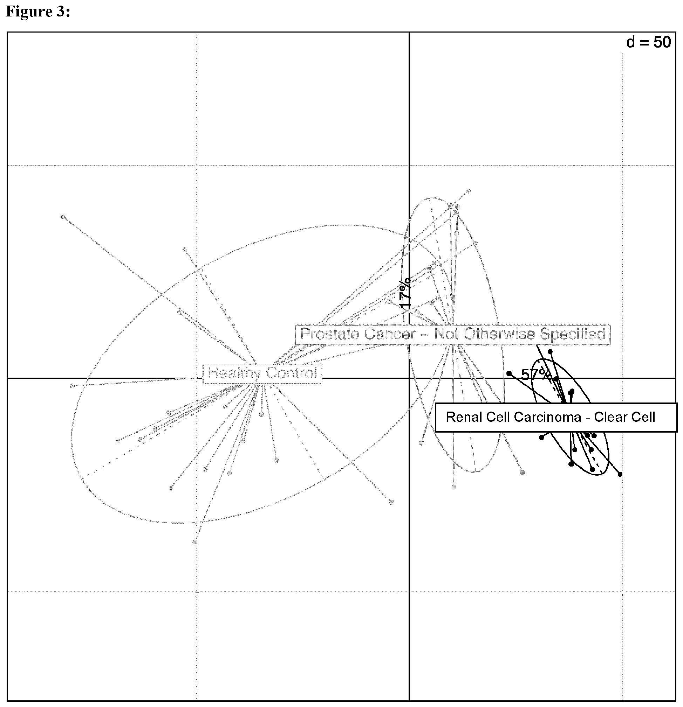

**Figure 3.** Principal component analysis of the GAG profiles in the blood and urine combined of healthy (light grey), PRAD (dark grey), or clear cell RCC (black) subjects. Subjects belonging to a given group are represented as points connected to the geometrical center of the ellipsis representing that group. PRAD: Prostate Cancer. RCC: Renal Cell Carcinoma.

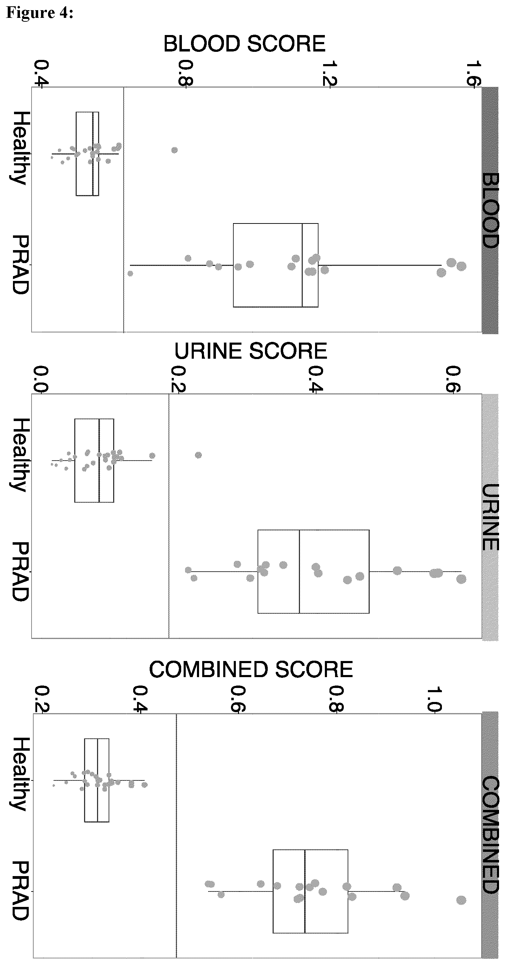

**Figure 4.** Boxplots of blood, urine, and combined scores in healthy vs. PRAD subjects. Individual subject scores are represented as dots. The horizontal lines identify the optimal cut-off score for which the highest accuracy in distinguishing PRAD from healthy individuals was achieved for each type of score. PRAD: Prostate Cancer.

**Figure 5.** ROC curves for the classification of subjects as PRAD versus healthy based on the blood (top), urine (middle), or combined (bottom) score. The points overlaid on each curve represent the optimal cut-off score at which the classification of subjects as PRAD versus healthy based on the different scores is most accurate (the two-dimensional coordinates of that point being the specificity and the sensitivity). PRAD: Prostate Cancer.

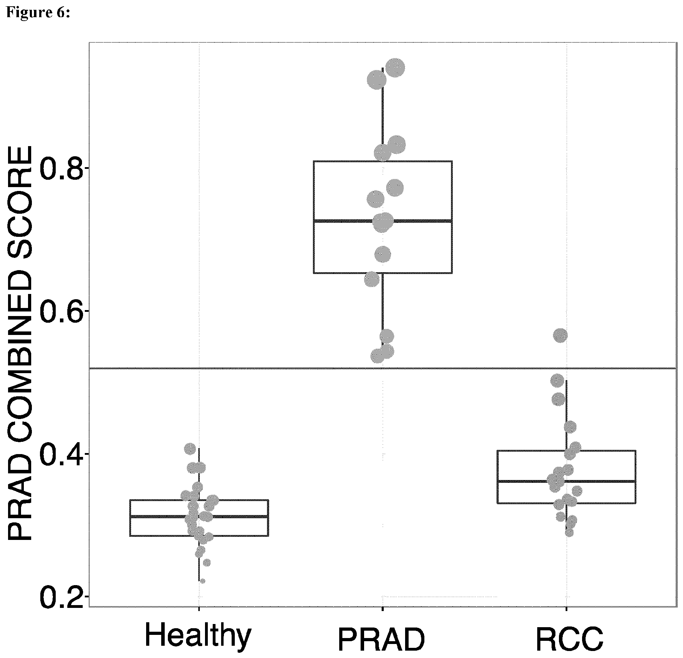

**Figure 6.** Boxplots of PRAD combined scores in healthy vs. PRAD vs. clear cell RCC subjects. The horizontal lines identify the optimal cut-off score previously detected to maximize the accuracy in distinguishing PRAD from healthy individuals. PRAD: Prostate Cancer. RCC: Renal Cell Carcinoma.

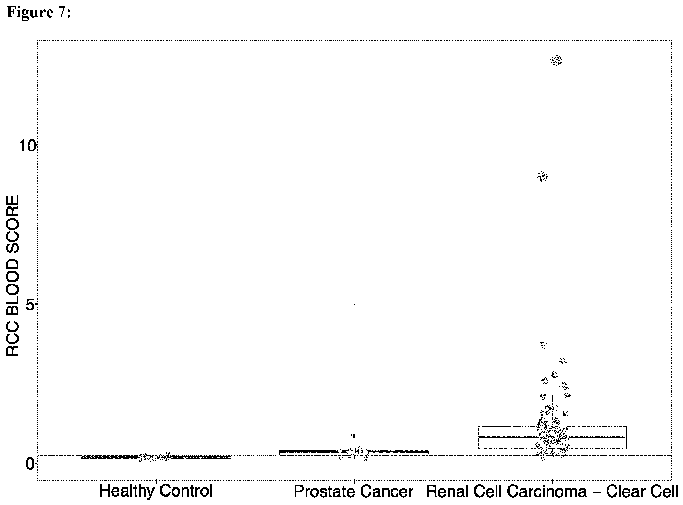

**Figure 7.** Boxplots of RCC blood scores in healthy vs. PRAD vs. clear cell RCC subjects. The horizontal lines identify the optimal cut-off score previously detected to maximize the accuracy in distinguishing RCC from healthy individuals. PRAD: Prostate Cancer. RCC: Renal Cell Carcinoma.

**Figure 8.** Ranking of the GAG properties in terms of decreased accuracy of a random forest classifier when the property is omitted (the decrease in accuracy is measured as the mean decrease in the Gini coefficient). The classifier was trained on GAG properties of matched blood and urine samples. Only the 30 best properties are shown.

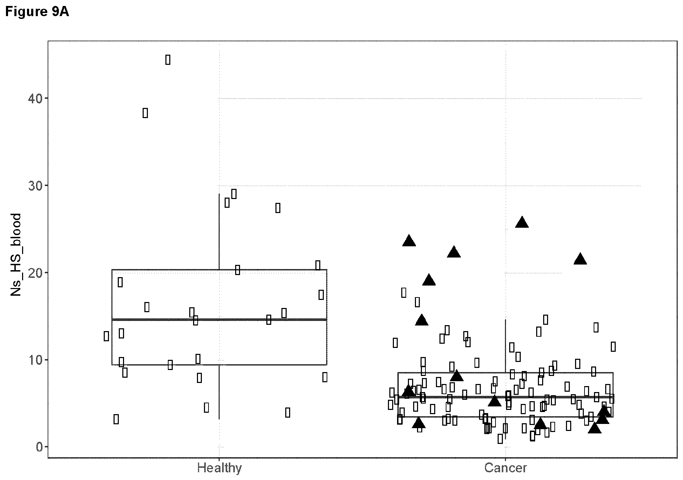

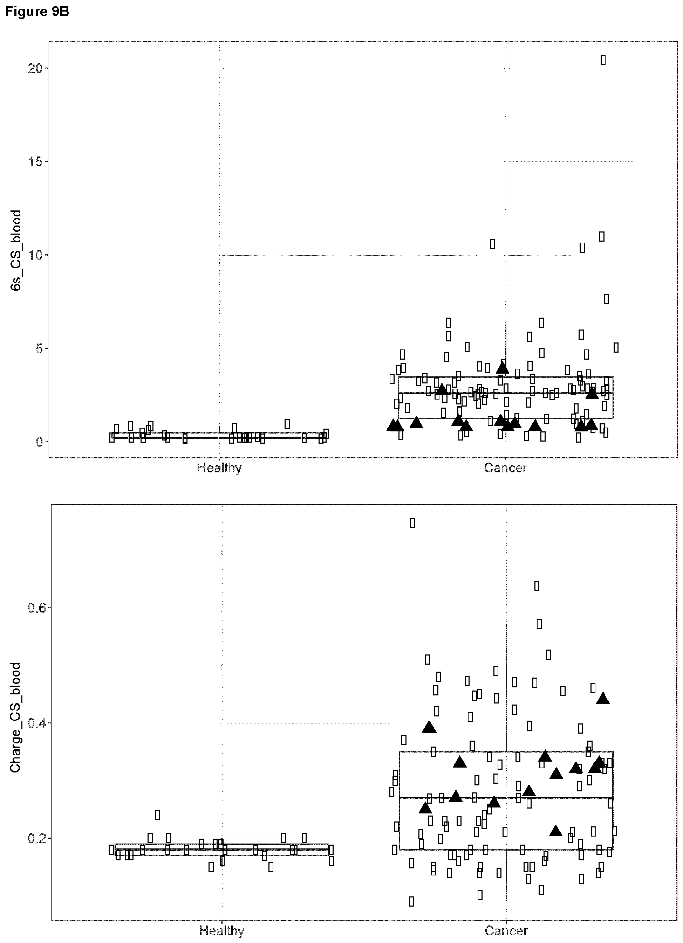

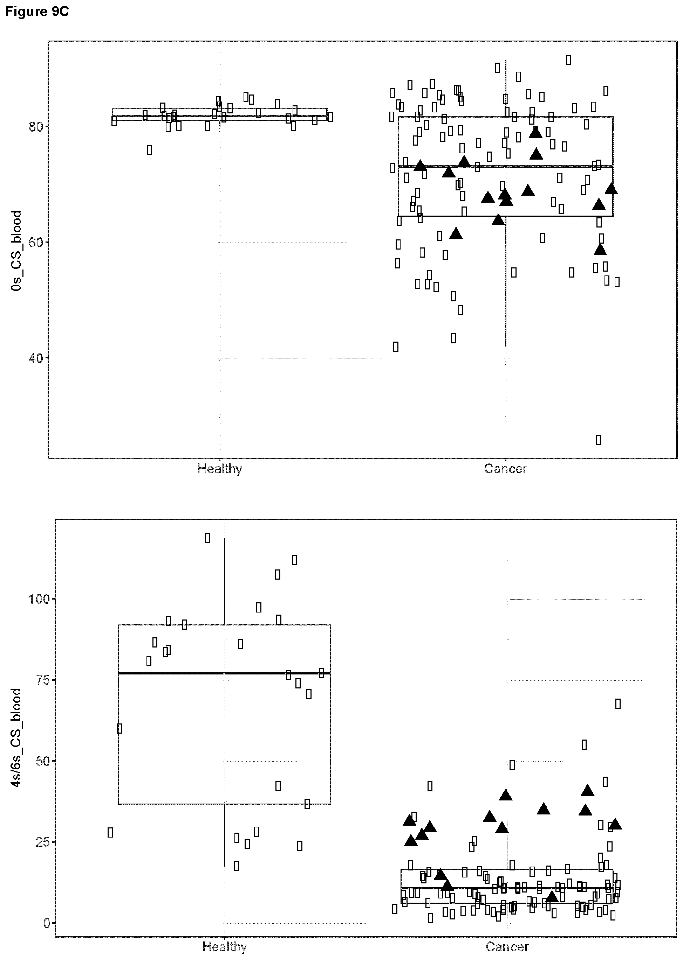

**Figure 9** - **(A/B/C)** GAG properties in the blood that were significantly altered in cancer as opposed to healthy volunteers but not due to cancer type specific effects. Triangle and circle samples in the boxplot representing the cancer population define two different types of cancer. (A: NsHS; B: 6sCS and Charge CS; C: 0sCS and 4s/6sCS).

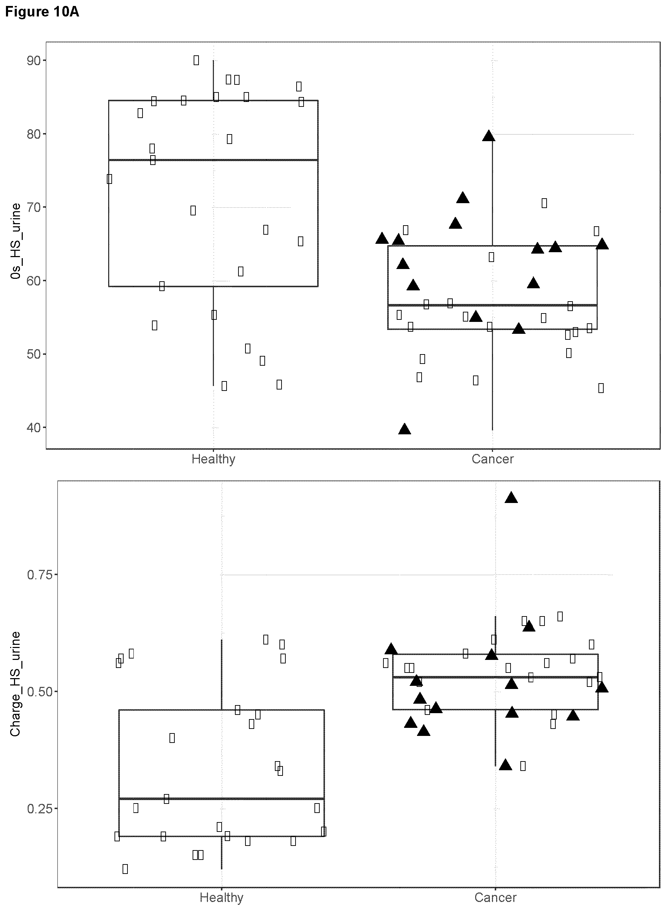

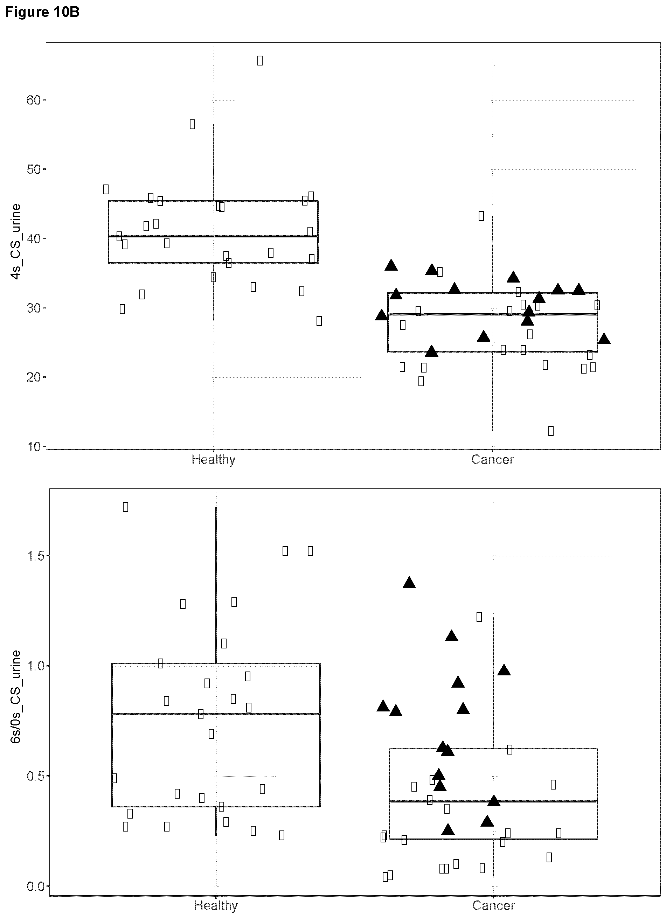

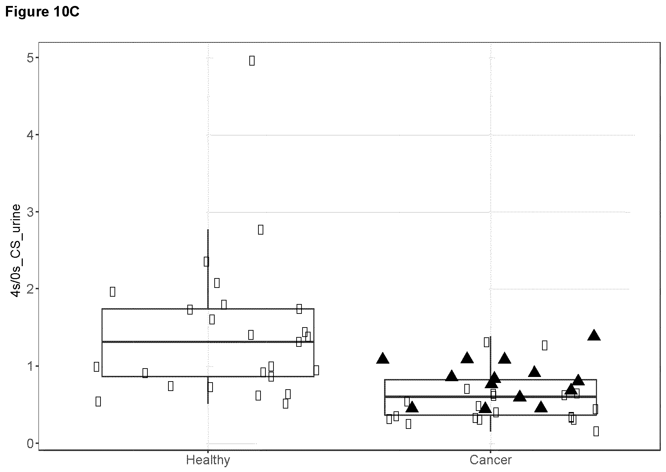

**Figure 10** - **(A/B/C)** GAG properties in the urine that were significantly altered in cancer as opposed to healthy volunteers but not due to cancer type specific effects. Triangle and circle samples in the boxplot representing the cancer population define two different types of cancer. (A: 0sHS and Charge HS; B: 4sCS and 6s/0sCS; C:4s/0sCS).

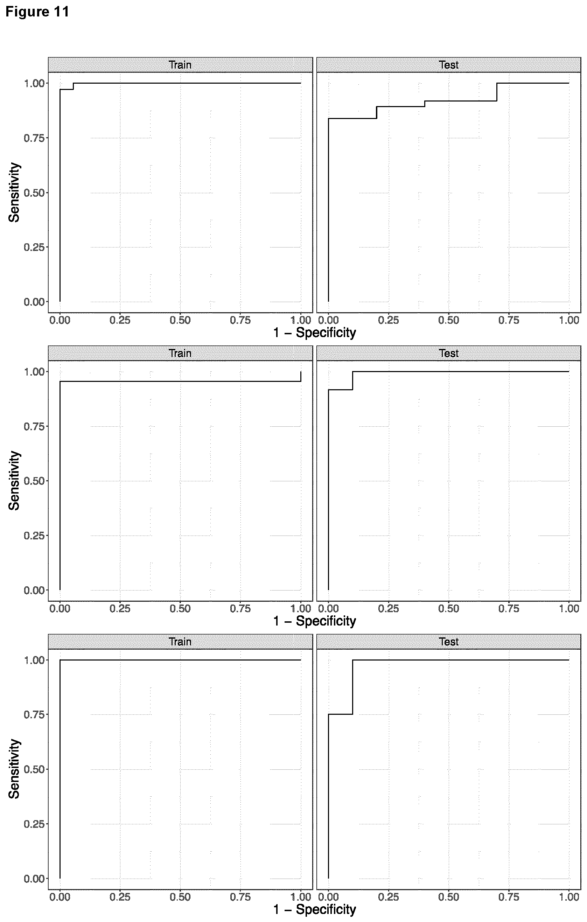

**Figure 11** - Receiver operating characteristic (ROC) curve for the classification of samples into "cases" - subjects with present diagnosis of cancer - vs. "controls" - healthy subject or with former diagnosis of cancer and currently with no evidence of disease - using a multilayer perceptron classifier trained on 66% of samples' GAG profiles (left) and tested on the remaining 33% of samples GAG profiles (right) as measured in the blood (top), urine (middle), or both blood and urine (bottom).

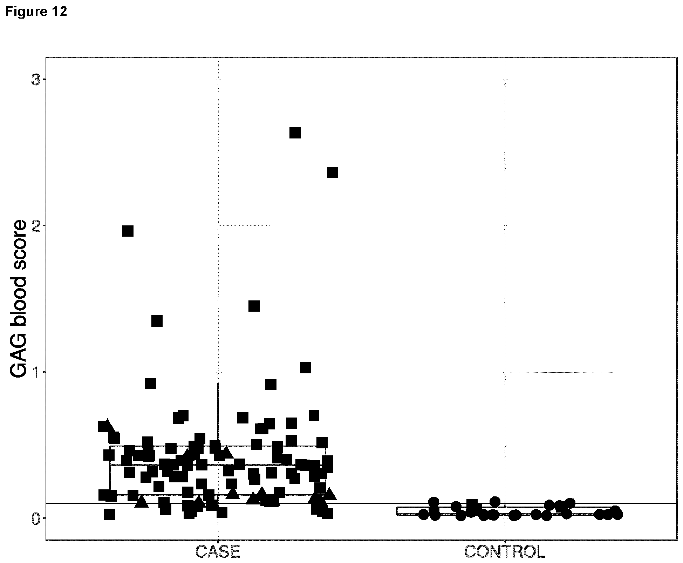

**Figure 12** - GAG scores based on blood GAG property measurements in "cases" - subjects with present diagnosis of cancer - vs. "controls" - healthy subject or with former diagnosis of cancer and currently with no evidence of disease.

The horizontal line identifies the optimal cut-off score for which the highest accuracy in distinguishing cases from controls was achieved. One "case" data point was omitted because the GAG scores was greater than 3. Key: squares - renal cell carcinoma; triangles - prostate cancer; circles - healthy subjects. Note that a "control" may be represented with a cancer symbol if the present status at the time of sampling was no evidence of disease.

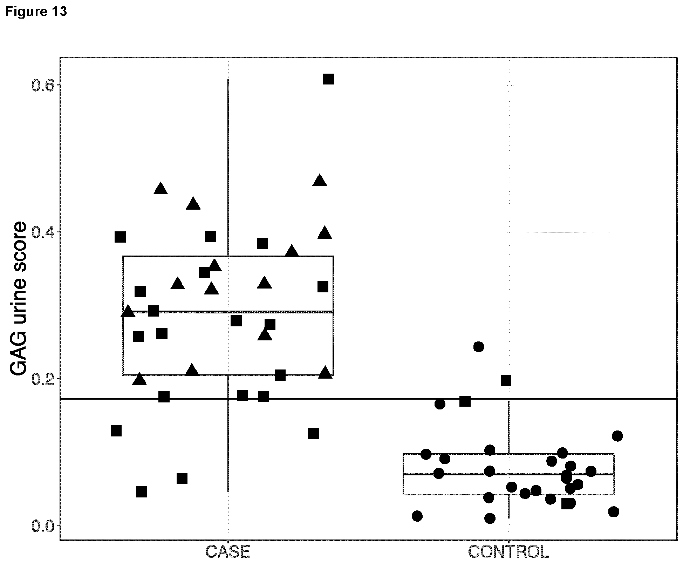

**Figure 13** - GAG scores based on urine GAG property measurements in "cases" - subjects with present diagnosis of cancer - vs. "controls" - healthy subject or with former diagnosis of cancer and currently with no evidence of disease. The horizontal line identifies the optimal cut-off score for which the highest accuracy in distinguishing cases from controls was achieved. Key: squares - renal cell carcinoma; triangles - prostate cancer; circles - healthy subjects. Note that a "control" may be represented with a cancer symbol if the present status at the time of sampling was no evidence of disease.

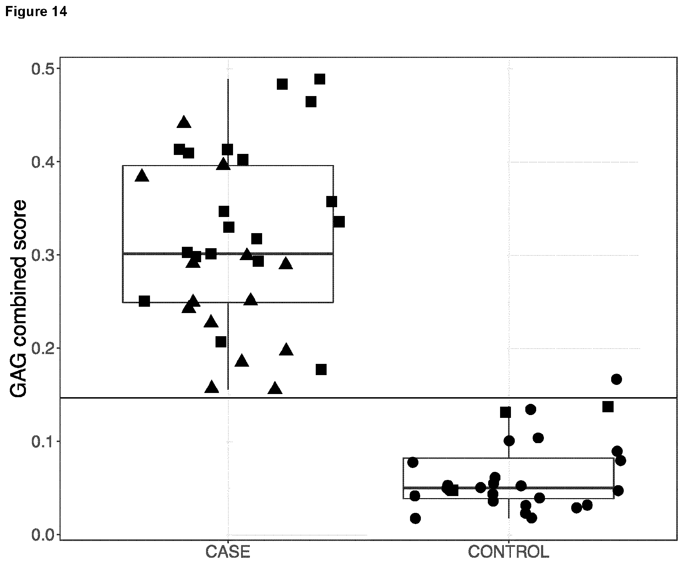

**Figure 14** - GAG scores based on blood and urine GAG property measurements in "cases" - subjects with present diagnosis of cancer - vs. "controls" - healthy subject or with former diagnosis of cancer and currently with no evidence of disease. The horizontal line identifies the optimal cut-off score for which the highest accuracy in distinguishing cases from controls was achieved. Key: squares - renal cell carcinoma; triangles - prostate cancer; circles - healthy subjects. Note that a "control" may be represented with a cancer symbol if the present status at the time of sampling was no evidence of disease.

**Figure 15.** ROC curves for the classification of subjects as cases versus controls based on the blood (top), urine (middle), or combined (bottom) score. The points overlaid on each curve represent the optimal cut-off score at which the classification of subjects as case versus control based on the different scores is most accurate (the two-dimensional coordinates of that point being the specificity and the sensitivity). The area-under-the-curve (AUC) of each ROC curve is also reported.

**Figure 16.** Regulation of glycosaminoglycan metabolism in 22 subtypes of cancer vs. matched normal tissue of origin. Heat-map of the direction of regulation of each gene associated with glycosaminoglycan metabolism (rows) in each cancer subtype (columns) compared to the matched normal tissue of origin. Grey entries indicate gene expression level down-regulation, black entries indicate gene expression level up-regulation, while white entries indicate no significant gene expression level difference with normal samples or no data available. Legend: MM - Malignant melanoma; PRAD - Prostate adenocarcinoma; THCA - Thyroid carcinoma; PAAD - Pancreatic adenocarcinoma; COAD - Colon adenocarcinoma; READ - Rectal adenocarcinoma; UCEC - Uterine corpus endometrial carcinoma; BRCA - Breast carcinoma; LUAD - Lung adenocarcinoma; BLAD - Urothelial bladder carcinoma; LUSC - Lung squamous cell carcinoma; LIHC - Liver hepatocellular carcinoma; CHOL - Cholangiocarcinoma; GBM - Glioblastoma multiforme; STAD - Stomach adenocarcinoma; HNSC - Head and neck squamous cell carcinoma; ESCA - Esophogael carcinoma; OVSA - Serous ovarian adenocarcinoma; PTCL - Peripheral T-cell Lymphoma; KIRC - Clear cell renal cell carcinoma; KICH - Chromophobe renal cell carcinoma; KIRP - Papillary renal cell carcinoma.

**Figure 17.** Boxplots of M GAG blood scores in healthy vs. melanoma. The horizontal line identifies the optimal cut-off score that maximizes the accuracy in distinguishing melanoma from healthy individuals for this score.

**Figure 18.** Boxplots of M GAG blood scores calculated using an alternative formula in healthy vs. melanoma. The horizontal line identifies the optimal cut-off score that maximizes the accuracy in distinguishing melanoma from healthy individuals for this alternative score.

**Figure 19.** Boxplots of CRC GAG blood scores in healthy vs. colorectal cancer (CRC). The horizontal line identifies the optimal cut-off score that maximizes the accuracy in distinguishing CRC from healthy individuals for this score.

**Figure 20.** Boxplots of GNET GAG blood scores in healthy vs. gastrointestinal neuroendocrine tumors (GNET). The horizontal line identifies the optimal cut-off score that maximizes the accuracy in distinguishing GNET from healthy individuals for this score.

**Figure 21.** Boxplots of CLL GAG blood scores in healthy vs. chronic lymphoid leukemia (CLL). The horizontal line identifies the optimal cut-off score that maximizes the accuracy in distinguishing CLL from healthy individuals for this score.

**Figure 22.** Boxplots of CLL GAG blood scores calculated using an alternative formula in healthy vs. chronic lymphoid leukemia (CLL). The horizontal line identifies the optimal cut-off score that maximizes the accuracy in distinguishing CLL from healthy individuals for this alternative score.

**Figure 23.** Boxplots of BCa GAG urine scores in healthy vs. bladder cancer (BCa). The horizontal line identifies the optimal cut-off score that maximizes the accuracy in distinguishing BCa from healthy individuals for this score.

**Figure 24.** Boxplots of BC GAG blood scores in healthy vs. breast cancer (BC). The horizontal line identifies the optimal cut-off score that maximizes the accuracy in distinguishing BC from healthy individuals for this score.

**Figure 25.** Boxplots of OV GAG blood scores in healthy vs. ovarian cancer (OV). The horizontal line identifies the optimal cut-off score that maximizes the accuracy in distinguishing OV from healthy individuals for this score.

**Figure 26.** Boxplots of EC GAG blood scores in healthy vs. endometrial cancer (EC). The horizontal line identifies the optimal cut-off score that maximizes the accuracy in distinguishing EC from healthy individuals for this score.

**Figure 27.** Boxplots of CST GAG blood scores in healthy vs. cervical cancer (CST). The horizontal line identifies the optimal cut-off score that maximizes the accuracy in distinguishing CST from healthy individuals for this score.

**Figure 28.** Boxplots of NHL GAG blood scores in healthy vs. non-Hodgkin lymphoma (NHL). The horizontal line identifies the optimal cut-off score that maximizes the accuracy in distinguishing NHL from healthy individuals for this score.

**Figure 29.** Boxplots of DG GAG blood scores in healthy vs. diffuse glioma (DG), grouped by subtype. The horizontal line identifies the optimal cut-off score that maximizes the accuracy in distinguishing DG from healthy individuals for this score.

**Figure 30.** Boxplots of LC GAG blood scores in healthy vs. lung cancer (LC). The horizontal line identifies the optimal cut-off score that maximizes the accuracy in distinguishing LC from healthy individuals for this score.

**Figure 31.** Boxplots of LC GAG blood scores calculated using an alternative formula in healthy vs. lung cancer (LC). The horizontal line identifies the optimal cut-off score that maximizes the accuracy in distinguishing LC from healthy individuals for this alternative score.

**Figure 32**. Boxplots of CS charge and total CS and HA concentration in each cancer type vs. healthy subjects. Key: breast cancer - BC, colorectal cancer - CRC, cervical cancer - CST, diffuse glioma - DG, endometrial cancer - EC, gastrointestinal neuroendocrine tumors - GNET, healthy - H, lymphoid leukemia - LL, Non-Hodgkin's lymphoma - NHL, lung cancer - NSCLC, ovarian cancer - OV, prostate cancer - PCa.

**Figure 33.** Boxplots of HS charge and total HS in each cancer type vs. healthy subjects. Key as in Figure 32.

**Figure 34**. Mean value of each property in CS composition in each cancer type group and in the healthy subjects' group. Mean values for each CS composition property belonging to the same group are connected by a distinct line. Key as in Figure 32.

**Figure 35.** Mean value of each property in HS composition in each cancer type group and in the healthy subjects' group. Mean values for each HS composition property belonging to the same group are connected by a distinct line. Key as in Figure 32.

**Figure 36**. Boxplots of cancer GAG blood scores calculated using an alternative formula in healthy vs. cancer grouped by type. Key as Figure 32. The horizontal line identifies the optimal cut-off score that maximizes the accuracy in distinguishing cancer from healthy individuals for this alternative score. On the right, the ROC corresponding to the cancer versus healthy classification using this alternative cancer GAG blood score.

## EXAMPLE 1

### *Introduction*

The number of prostate cancer cases is predicted to increase substantially in the near future. The rising prostate cancer population determines an urgent need to improve the current diagnostics landscape for prostate cancer. In particular, affordable and practical tools for prostate cancer diagnostics are needed to assist healthcare professionals in the early detection of prostate cancer, which typically correlates with more favorable clinical outcomes, or to guide treatment of current prostate cancer patients. Circulating biomarkers are molecules that can be measured in accessible body fluids of individuals, e.g. blood or urine, and whose levels are useful to assist in the diagnosis and/or prognosis and/or prediction of response to treatment. An example of a widely used biomarker is the prostate-specific antigen (PSA) for prostate cancer, but the clinical value of this biomarker for diagnosing prostate cancer is highly debated.

Emerging cancer biomarkers are circulating nucleic acids directly derived from cancer cells. These nucleic acids can be found for example in circulating free DNA, circulating small-RNA, circulating tumor cells, or extracellular micro-vesicles (including exosomes) containing small-RNA, mRNA and DNA. The minimally invasive technology for detection of such molecular biomarkers without the need for costly or invasive procedures is defined as liquid biopsy. Recently, the definition of liquid biopsy was extended to encompass other macromolecular classes of cancer biomarkers, for example proteins transported within circulating exosomes. It has previously been demonstrated that even circulating metabolites could serve as accurate molecular biomarkers for renal cell carcinoma (RCC), the most common form of kidney cancer (Gatto et al., 2016). In particular, quantitative profiling of glycosaminoglycans (GAGs) in a subjects' blood and/or urine could be scored computationally to derived GAG scores with demonstrated diagnostic value for RCC (Gatto et al., 2016).

In this study we measured circulating GAG levels in subjects presenting with prostate cancer.

## *Material and Methods*

### *Blood and urine sample collection*

28 matched urine and blood samples were collected from 14 patients with PRAD.

Whole blood tubes were centrifuged (2,500g for 15 minutes at 4 °C) and the serum extracted and collected in a separate tube. Blood GAG measurements in patients with PRAD were done on serum samples. Urine samples were collected in polypropilene tubes. The samples were stored at -80°C until they were shipped for analysis in dry ice.

Urine samples and blood (plasma) samples from healthy subjects and RCC subjects are as per Gatto *et al.,* 2016.

Serum and plasma GAGs correspond to blood GAGs. For the purpose of this study, serum and plasma are therefore referred to collectively as blood.

### *Glycosaminoglycan profile determination*

Sample preparation including extraction and purification steps were performed as previously described by Volpi and Maccari 2005[1 and 2] and Coppa et al., 2011), while sample GAGs separation and quantification were performed as described in Volpi et al., 2014; Volpi and Linhardt, 2010.

Briefly, to extract the GAGs, 500 $\mu$l of sample was lyophilized, reconstituted with 1 ml of a 20-mM TRIS-Cl buffer pH 7.4 and treated with protease (Proteinase K from Tritirachium album [E.C. 3.4.21.64], >500 units ml_1 from Sigma-Aldrich) at 60 °C for 12 h. After boiling for 10 min, centrifugation and filtration on 0.45-$\mu$m filters, the filtrate was lyophilized. The powder was dissolved in 1 ml of distilled water by prolonged mixing. After centrifugation at 5000 g for 15 min, 0.2 ml of 20% trichloro- acetic acid was added to the supernatant. After 2 h at 4 °C, the mixture was centrifuged at 5000 g for 15 min, and the supernatant was recovered and lyophilized. After solubilization in 0.4 ml of bidistilled water and centrifugation at 10 000 g for 10 min, the supernatant was collected and further analysed. To purify the GAGs, after reconstitution with 500 $\mu$l 10 mM NaCl, sample GAGs were further purified on anion-exchange resin (QAE Sephadex A-25). After centrifugation at 10,000 $\times$ g for 5 min, the supernatant was applied to a column (1 cm $\times$ 4 cm) packed with about 3 ml of resin previously equilibrated with 10 mM NaCl. After washing the resin with 20 ml of 10 mM NaCl, 10 ml of 2.5 M NaCl were added. Fifty milliliters of ethanol were added to the eluate (10 ml) and stored at -20 °C for 24 h. After centrifugation at 5000 $\times$ g for 15 min, the pellet was dried at 60 °C for 12 h. After reconstitution with 80 $\mu$l of 50 mM ammonium acetate pH 8.0, the material was treated with 20 $\mu$l of chondroitinase ABC at 37 °C for 12 h. To separate and quantify the GAGs, after boiling for 5 min, the samples were injected in HPLC with both post-column derivatization and fluorimetric detection and on-line electrospray ionization mass spectrometry (ESI-MS).

Nineteen independent GAG properties were measured in each sample (either blood or urine): CS concentration (in $\mu$g/mL), HS concentration (in $\mu$g/mL), HA concentration (in $\mu$g/mL), and mass fractions of disaccharide composition for both CS and HS. In addition, five dependent GAG properties were calculated from these measurements: the CS and HS charge; and the following CS ratio of mass fractions: 4s/6s, 6s/0s, and 4s/0s. The 24 GAG properties may be collectively referred to as GAG profile.

The overall similarity in the GAG profile between subjects belonging to the three groups (prostate cancer vs. renal cell carcinoma vs. healthy) was further examined using principal component analysis (PCA) by comparing GAG properties measured either solely in the blood or in the urine or in both fluids. Principal component analysis was implemented using R-package ade4 (centering was performed by the mean) (Dray and Dufour, 2007).

### Bio-marker design

The statistical significance of changes for each GAG property between the two groups (prostate cancer vs. healthy) was assessed by calculating the highest density interval (HDI) for the mean difference using Bayesian estimation under the following assumptions: GAG property values are sampled from a *t*-distribution of unknown and to be estimated normality (i.e. degrees of freedom); high uncertainty on the prior distributions; the marginal distribution is well approximated by a Markov chain Monte Carlo sampling with no thinning and chain length equal to 100'000. The estimation was performed using *BEST* R-package (the above assumptions are reflected by the default parameters). Bayesian estimation was preferred over the widely used *t*-test since it provides a robust and reliable estimation of mean difference even under uncertainty of the underlying score distribution for the two groups (that is the case when the number of samples is limited).

To verify whether changes in the GAG profile in PRAD patients could be condensed into GAG scores, that can be used to differentiate PRAD patients from healthy subjects, and therefore would be suitable as a cancer diagnostic, we utilized Lasso penalized logistic regression (Tibshirani, 1996) with leave-one-out cross-validation to select robust GAG properties that are most predictive of the clinical outcome (i.e., PRAD compared to healthy subject). The score was subsequently designed as a ratio, where the numerator is the sum of the properties associated with PRAD and the denominator is the sum of the properties associated with the healthy state. Each term was normalized using the regression coefficients.

For the purpose of this study and for reasons elsewhere stated, scores computed from blood GAGs regardless if the processed sample was serum or plasma are referred to as GAG blood scores.

### Accuracy metrics

For each marker (blood, urine, or combined), we evaluated its performance in the binary classification of a sample as either prostate cancer or healthy at varying threshold scores by deriving the receiver-operating-characteristic (ROC) curves. We measured the accuracy of each marker as the area under the curve (AUC) of its ROC curve (AUC is 1 for a perfect classifier and 0.5 for a random classifier). We selected as a potential cut-off value for a given marker the score for which the accuracy was maximum, i.e. a sample whose marker score is above this cut-off value has the maximum probability of being prostate cancer and below this cut-off value has the maximum probability of being healthy. The ROC curves were calculated using the pROC R-package, while the optimal cut-off using the OptimalCutpoints R-package.

### Results

We analyzed GAG profiles in prostate adenocarcinoma (PRAD). We collected a total of 28 matched urine and blood samples from 14 patients with PRAD. Clinical data for this cohort is reported in Table A. We quantified the profile of GAGs in the former samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile for both blood and/or urine samples (listed in Table B). We also compared the value of each property in the GAG profile of the PRAD group to previously measured values in the GAG profile of a group of 50 matched urine and blood samples from 25 healthy individuals. We assessed the statistical significance of changes for each GAG property between the two groups by calculating the highest density interval (HDI) for the mean difference using Bayesian estimation under the following assumptions: GAG property values are sampled from a t-distribution of unknown and to be estimated normality (i.e. degrees of freedom); high uncertainty on the prior distributions; the marginal distribution is well approximated by a Markov chain Monte Carlo sampling with no thinning and chain length equal to 100'000. The estimation was performed using *BEST* R-package (the above assumptions are reflected by the default parameters). Bayesian estimation was preferred over the widely used t-test since it provides a robust and reliable estimation of mean difference even under uncertainty of the underlying score distribution for the two groups (that is the case when the number of samples is limited). The results showed marked differences in several GAG properties between PRAD, compared to healthy subjects (Table B). We further examined overall similarity in the GAG profile between subjects belonging to the two groups using principal component analysis (PCA) by comparing GAG properties measured either solely in the blood (Figure 1) or in the urine (Figure 2) or in both fluids (Figure 3). Principal component analysis was implemented using R-package ade4 (centering was performed by the mean) (Dray and Dufour, 2007). This analysis demonstrated that subjects belonging to the two groups clustered separately in either fluid, suggesting that marked differences exist in the profile of GAGs for the two groups. To compare how similar the GAG profile in PRAD was to that of RCC, we included in each PCA the GAG profiles from the RCC group, analyzed in our previous study (Gatto et al., 2016) and the blood GAG profiles collected from an additional cohort of 40 clear cell RCC subjects. The RCC group from our previous study included specifically clear cell RCC patients, comprising 52 subjects with blood samples, 20 subjects with urine samples, and 19 subjects with both. Remarkably, a separation was observed both between PRAD and RCC subjects, and that also was separate from healthy subjects, indicating that unique changes in the GAG profile occur in the blood and urine of PRAD subjects.

To verify whether changes in the GAG profile in PRAD patients could be condensed into GAG scores, that can be used to differentiate PRAD patients from healthy subjects, and therefore would be suitable as a prostate cancer diagnostic, we utilized Lasso penalized logistic regression (Tibshirani, 1996) with leave-one-out cross-validation to select robust GAG properties that are most predictive of the clinical outcome (i.e., PRAD compared to healthy subject). The score was subsequently designed as a ratio, where the numerator is the sum of the properties associated with PRAD and the denominator is the sum of the properties associated with the healthy state. Each term was normalized using the regression coefficients. We derived three PRAD biomarker scores, based on either blood or urine or combined measurements:

$$Blood\ score = \frac{[4s\ CS]}{\frac{4}{10}[0s\ CS]}$$

$$Urine\ score = \frac{3[HA] + \frac{6}{10}[Ns2s\ HS]}{\frac{1}{3}[4s\ CS]}$$

$$Combined\ score = \frac{(Blood\ score\ +\ Urine\ score)}{2}$$

where terms in brackets represent the fraction (mass fraction) of the disaccharide for the corresponding GAG, and [*HA*] is the total concentration of HA (in μg/mL). We subsequently calculated the three scores for each sample and observed that PRAD samples have recurrently elevated scores with respect to healthy samples (Figure. 4). We computed significant non-null mean differences in all three scores between the two groups using robust Bayesian estimation. The mean difference was equal to 0.43 for the combined score (95% HDI 0.35 to 0.52), 0.54 for the blood score (95% HDI 0.39 to 0.69), and 0.29 for the urine score (95% HDI 0.21 to 0.37). The performance of the three scores was evaluated using the receiver-operating-characteristic (ROC) curves, and the area under the curve (AUC) was found to be 1 (perfect classifier) in the case of the combined score, 0.997 for the blood score, and 0.994 for the urine score (Figure. 5). We identified an optimal cut-off for each score to maximize the accuracy in the classification of PRAD subjects as opposed to healthy individuals using blood and/or urine GAG profile data. These cut-off scores were 0.63, 0.19, 0.47 for the blood, urine, and combined score respectively. Using these cut-offs, PRAD subjects could be distinguished from healthy individuals with 97.4% accuracy using the blood or urine score, and 100% accuracy using the combined score. Other metrics of accuracy were reported in Table C. Taken together, these results demonstrate that GAG profiling in accessible biofluids are suitable for diagnosing prostate cancer.

We also investigated whether scores designed to detect PRAD are not specific to RCC (renal cell carcinoma) and vice versa. Strikingly, the combined score designed to detect PRAD specifically correlated with the 14 subjects with PRAD, as opposed to 19 subjects with RCC and 25 healthy subjects (Figure 6). By optimizing the cut-off to maximize accuracy in the detection of PRAD, at a cut-off score equal to 0.52, PRAD can be distinguished from healthy subjects or subjects with RCC with 98.3% accuracy, conferring to the combined score an overall sensitivity and specificity for PRAD equal to 100% and 97.7% respectively. For comparison, a standard PSA test to detect PRAD in men over 50 years old at average risk assuming a cut-off value equal to 4 ng/ml has typical values for sensitivity and specificity equal to 21% (51% for high grade lesions with Gleason score greater or equal to 8) and 91% respectively (Wolf et al., 2010).

Conversely, the blood score designed to detect RCC specifically identified subjects with RCC, as opposed to subjects with PRAD and healthy subjects (Figure 7). By optimizing the cut-off to maximize accuracy in the detection of RCC as opposed to healthy subjects or subjects with PRAD, at a cut-off score equal to 0.23 for the previously derived blood score for RCC (Gatto et al., 2016), the overall accuracy also for the detection of subjects with RCC was 89.3%, achieving a sensitivity and specificity equal to 97.8% and 69.2%, respectively and an AUC = 0.941. These results demonstrate that different GAG scores (or GAG profiles) can be designed to specifically detect prostate or RCC, and that their significant AUC permit fine-tuned optimal cut-off scores, in order to maximize sensitivity and/or specificity, depending on the intended diagnostic use.

### *Conclusions*

Liquid biopsies hold promise to revolutionize prostate cancer diagnostics by providing an accurate, simple, minimally invasive, fast and/or cheaper alternative to tissue biopsies and medical imaging, currently the gold standard for prostate cancer diagnostics. In this study, we have demonstrated that an emerging liquid biopsy platform could leverage on blood and/or urine based measurements of GAG profiles. We demonstrated that there are measurable differences in circulating GAG levels and/or chemical composition between prostate cancer as opposed to healthy subjects. The GAG profiles can further be condensed into GAG scores that have great promise in terms of accuracy for prostate cancer detection or diagnosis. Furthermore, the prostate cancer GAG scores were shown to be specific to prostate cancer compared to RCC (renal cell carcinoma). The diagnostic GAG properties or scores disclosed herein could also be applied to change a number of clinical practices. For example, to monitor prostate cancer before and after surgery or drug treatment; to rule out the relapse of the disease during a longer period of time after which a patient is typically declared cured; to assess the occurrence of prostate cancer in a population at risk, such as genetically predisposed individuals or individuals presenting risk factors or individuals presenting symptoms; to ascertain whether a metastasis is due to prostate cancer; to predict recurrence or relapse in patients with early stage prostate cancer; to distinguish lesions suspicious of prostate cancer from non malignant diseases; and to screen for prostate cancer in the general population. This liquid biopsy has therefore the potential to become a crucial tool to enable secondary prevention of prostate cancer and reduce the burden caused by this disease in the general population.

## References

Coppa GV, Gabrielli O, Buzzega D, Zampini L, Galeazzi T, Maccari F, Bertino E, Volpi N (2011). Composition and structure elucidation of human milk glycosaminoglycans. Glycobiology 21, 295-303.

Dray, S., and Dufour, A.B. (2007). The ade4 package: Implementing the duality diagram for ecologists. Journal of Statistical Software 22, 1-20.

Gatto, F., Volpi, N., Nilsson, H., Nookaew, I., Maruzzo, M., Roma, A., Johansson, M.E., Stierner, U., Lundstam, S., Basso, U., et al. (2016). Glycosaminoglycan Profiling in Patients' Plasma and Urine Predicts the Occurrence of Metastatic Clear Cell Renal Cell Carcinoma. Cell Rep 15, 1822-1836.

Tibshirani, R. (1996). Regression shrinkage and selection via the Lasso. Journal of the Royal Statistical Society Series B-Methodological 58, 267-288.

Volpi N and Maccari F. (20051). Glycosaminoglycans composition of the largefreshwater mollusc bivalve Anodonta anodonta. Biomacromolecules 6, 3174-3180.

Volpi N and Maccari F (20052). Microdetermination of chondroitin sulfate in normal human plasma by fluorophore-assisted carbohydrate electrophore-sis (FACE). Clin Chim Acta 356, 125-133.

Volpi, N., Galeotti, F., Yang, B., and Linhardt, R.J. (2014). Analysis of glycosaminoglycan-derived, precolumn, 2-aminoacridone-labeled disaccharides with LC-fluorescence and LC-MS detection. Nature protocols 9, 541-558.

Volpi, N., and Linhardt, R.J. (2010). High-performance liquid chromatography-mass spectrometry for mapping and sequencing glycosaminoglycan-derived oligosaccharides. Nature protocols 5, 993-1004.

Wolf, A.M., Wender, R.C., Etzioni, R.B., Thompson, I.M., D'Amico, A.V., Volk, R.J., Brooks, D.D., Dash, C., Guessous, I., Andrews, K., et al. (2010). American Cancer Society guideline for the early detection of prostate cancer: update 2010. CA Cancer J Clin 60, 70-98.

**Table A:** Clinical data for the cohorts analysed in this study. All results are presented as medians (25th, 75th percentile) or percent. Missing values were omitted.

|  | PRAD (n = 14) | Healthy (n=25) | ccRCC (n = 99) |
|---|---|---|---|
| Cohort characteristics |  |  |  |
| Matched blood+urine samples | 14 | 25 | 19 |
| Blood samples | 0 | 0 | 92 |
| Urine samples | 0 | 0 | 20 |

(continued)

| Baseline characteristics | | | |
|---|---|---|---|
| Age [years] | 69 (66 -71) | 61 (55 - 64) | 63 (56 - 71) |
| Female | 0% | 62.5% | 19% |
| BMI [kg/m$^2$] | 22 (22 - 28) | 26 (23 - 29) | 25 (23 - 28) |
| PSA level [ng/ml] | 11 (9 - 19) | - | - |

**Table B:** A metric of statistical confidence that any individual GAG property (CS, HS or HA levels, individual disaccharide compositions, and charge HS or charge CS) displays a significant and practically appreciable alteration between PRAD and healthy samples was calculated. An alteration was defined as significant and practically appreciable if the difference in means between case and control falls within a so-called Region Of Practical Equivalence in less than 5% of all possible statistical distributions that can fit the data relative to a measurement. Table B shows a summary of the results and shows the mean value for GAG properties as measured in PRAD vs. healthy subjects blood or urine and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table B also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in Prostate Cancer | ROPE threshold | % in ROPE |
|---|---|---|---|---|
| 0s CS urine | 34.9150 | 40.3666 | 2.0183 | 16.42 |
| 2s CS urine | 0.0852 | 0.1049 | 0.0052 | 12.42 |
| 6s CS urine | 21.5534 | 25.9494 | 1.2975 | 8.60 |
| 4s CS urine | 40.5560 | 30.5398 | 1.5270 | 0.01 |
| 2s6s CS urine | 0.6060 | 0.8793 | 0.0440 | 0.71 |
| 2s4s CS urine | 0.4569 | 0.6285 | 0.0314 | 1.30 |
| 4s6s CS urine | 1.1849 | 1.3990 | 0.0700 | 14.51 |
| Tris CS urine | 0.0387 | 0.0974 | 0.0049 | 0.11 |
| 4s/6s CS urine | 2.0071 | 1.1994 | 0.0600 | 0.00 |
| 6s/0s CS urine | 0.7510 | 0.7021 | 0.0351 | 19.13 |
| 4s/0s CS urine | 1.3096 | 0.8078 | 0.0404 | 0.48 |
| Charge CS urine | 0.6532 | 0.6289 | 0.0314 | 47.35 |
| Total HA urine | 0.0703 | 0.2386 | 0.0119 | 0.11 |
| Total CS urine | 0.9124 | 2.8992 | 0.1450 | 0.29 |
| 0s CS blood | 81.9248 | 68.7842 | 3.4392 | 0.00 |
| 2s CS blood | 0.0163 | 0.0478 | 0.0024 | 1.38 |
| 6s CS blood | 0.2109 | 0.8672 | 0.0434 | 0.00 |
| 4s CS blood | 17.6142 | 29.3080 | 1.4654 | 0.00 |
| 2s6s CS blood | 0.0100 | 0.0335 | 0.0017 | 0.27 |
| 2s4s CS blood | 0.0045 | 0.0269 | 0.0013 | 0.63 |
| 4s6s CS blood | 0.0222 | 0.0949 | 0.0047 | 0.14 |
| Tris CS blood | 0.0051 | 0.0164 | 0.0008 | 0.97 |
| 4s/6s CS blood | 69.2937 | 27.8682 | 1.3934 | 0.00 |
| 6s/0s CS blood | 0.0025 | 0.0102 | 0.0005 | 0.00 |
| 4s/0s CS blood | 0.2154 | 0.4310 | 0.0215 | 0.00 |

(continued)

| GAG property | Mean in Healthy Individuals | Mean in Prostate Cancer | ROPE threshold | % in ROPE |
|---|---|---|---|---|
| Charge CS blood | 0.1805 | 0.3163 | 0.0158 | 0.00 |
| Total HA blood | 0.1161 | 0.1393 | 0.0070 | 12.59 |
| Total CS blood | 5.3857 | 4.0150 | 0.2007 | 4.23 |
| Tris HS urine | 0.3545 | 1.6758 | 0.0838 | 0.01 |
| Ns6s HS urine | 1.1990 | 3.2476 | 0.1624 | 0.00 |
| Ns2s HS urine | 1.3681 | 4.8767 | 0.2438 | 0.00 |
| Ns HS urine | 12.2820 | 13.6728 | 0.6836 | 20.50 |
| 2s6s HS urine | 1.0458 | 1.6221 | 0.0811 | 6.48 |
| 6s HS urine | 6.8895 | 9.0528 | 0.4526 | 9.57 |
| 2s HS urine | 3.5227 | 2.3676 | 0.1184 | 3.32 |
| 0s HS urine | 71.8320 | 62.4626 | 3.1231 | 6.53 |
| Charge HS urine | 0.3329 | 0.5107 | 0.0255 | 0.28 |
| Total HS urine | 0.1443 | 0.3534 | 0.0177 | 2.49 |
| Tris HS blood | 0.3189 | 1.0385 | 0.0519 | 0.23 |
| Ns6s HS blood | 1.5484 | 1.1908 | 0.0595 | 7.95 |
| Ns2s HS blood | 0.6587 | 1.9633 | 0.0982 | 0.46 |
| Ns HS blood | 15.9138 | 11.2063 | 0.5603 | 5.07 |
| 2s6s HS blood | 0.9313 | 0.3779 | 0.0189 | 0.40 |
| 6s HS blood | 6.8018 | 15.1316 | 0.7566 | 2.60 |
| 2s HS blood | 4.7854 | 6.0821 | 0.3041 | 12.35 |
| 0s HS blood | 65.0374 | 56.7356 | 2.8368 | 16.16 |
| Charge HS blood | 0.4166 | 0.5036 | 0.0252 | 13.88 |
| Total HS blood | 0.0433 | 0.1573 | 0.0079 | 0.03 |

**Table C:** Accuracy metrics at optimal cut-off for the blood, urine, and combined score in distinguishing 14 PRAD vs. 25 healthy subjects.

| Score | AUC | Accuracy | Sensitivity | Specificity |
|---|---|---|---|---|
| Blood | 0.997 | 97.4% | 100% | 96% |
| Urine | 0.994 | 97.4% | 100% | 96% |
| Combined | 1 | 100% | 100% | 100% |

## EXAMPLE 2

**Introduction**

The number of cancer cases is predicted to increase substantially in the near future. In the US alone, the number of new cancer patients is expected to rise from 1.36 million in 2000 to almost 3.0 million in 2050. The rising cancer population determines an urgent need to improve the current diagnostics landscape for cancer. In particular, affordable and practical tools for cancer diagnostics are needed to assist healthcare professionals in the early detection of cancer, which typically correlates with more favorable clinical outcomes, or to guide treatment of current cancer patients. Circulating biomarkers are molecules that can be measured in accessible body fluids of individuals, e.g. blood or urine, and whose levels are useful to assist in the diagnosis and/or prognosis and/or prediction of response to treatment. Unfortunately, clinically proven circulating biomarkers are or will be available for only a minority of cancer types, even in cases of advanced metastasis. Examples of widely used biomarkers are the prostate-specific antigen (PSA) for prostate cancer, the carbohydrate antigen 125 (CA125) for ovarian cancer, or the carcinoembryonic antigen (CEA) for colorectal cancer. Even in these cases, the clinical value of these biomarkers for diagnosing cancer is highly debated.

Emerging cancer biomarkers are circulating nucleic acids directly derived from cancer cells. These nucleic acids can be found for example in circulating free DNA, circulating small-RNA, circulating tumor cells, or extracellular micro-vesicles (including exosomes) containing small-RNA, mRNA and DNA. The minimally invasive technology for detection of such molecular biomarkers without the need for costly or invasive procedures is defined as liquid biopsy. Recently, the definition of liquid biopsy was extended to encompass other macromolecular classes of cancer biomarkers, for example proteins transported within circulating exosomes. It has previously been demonstrated that even circulating metabolites could serve as accurate molecular biomarkers for renal cell carcinoma (RCC), the most common form of kidney cancer (Gatto et al., 2016). In particular, quantitative profiling of glycosaminoglycans (GAGs) in a subjects' blood and/or urine could be scored computationally to derived GAG scores with demonstrated diagnostic valuable for RCC (Gatto et al., 2016).

In this study, we analyzed data regarding circulating GAG levels in subjects presenting two different cancer types as well as in healthy volunteers. The goal was to identify if alterations from healthy volunteers in the level or chemical composition of specific GAGs were attributable to cancer in general and not significantly affected specifically in a given cancer type, in other words if there exist common patterns of alterations in the GAG profile across different cancers. Furthermore, we sought to analyze transcriptional regulation of GAG metabolism, spanning genes associated with biosynthesis and degradation of GAGs, in a wide spectrum of cancer types by histology. The goal was to identify if common pattern of transcriptional regulation across many different types of cancer may underlie eventual common patterns of alterations in the GAG profile across different cancers.

## Materials and Methods

Unless otherwise stated, the materials and methods used in this Example correspond to the materials and methods used in Example 1.

## Results

We analyzed GAG profiles in blood and urine samples from 114 subjects, including 25 healthy volunteers, 100 patients with diagnosis of renal cell carcinoma, and 14 patients with diagnosis of prostate adenocarcinoma. We adopted an ordinary least squares (OLS) to estimate changes in each individual property part of the GAG profiles that were attributable to cancer as opposed to changes attributable specifically to a particular type of cancer (e.g. prostate adenocarcinoma as opposed to renal cell carcinoma). The resulting linear model therefore was:

$$GAG\ property_{i,j} = I_i + \ \alpha_i[Cancer]_j + \beta_i[Prostate\ adenocarcinoma]_j + \ \varepsilon_i$$

where $i$ indexes a given GAG property in the GAG profile in a certain fluid (blood or urine) and $j$ indexes a given subject, $I$ is the intercept (e.g. the expected value in a healthy subject), $\alpha$ is the change attributable to the fact that the subject has cancer and $\beta$ is the change attributable to the fact that the subject has a specific type of cancer, prostate adenocarcinoma, while $\varepsilon$ is the error that accounts for the discrepancy between the actually observed value for the GAG property and the value predicted by the linear model - which is minimized in OLS. A statistical test was performed on each estimate for the coefficients in the linear model ($I$, $\alpha$ and $\beta$) by computing their t-statistics and calculating the probability $p$ to observe at least a $t$-value as extreme under the null hypothesis that true coefficient is zero. The null hypothesis was rejected if $p$ was lower than 0.01, after adjusting for multiple testing using the Holm correction (false discovery rate, FDR). This analysis was conducted using base packages in R, version 3.3.1.

Table D and Table E list the estimates for $I$ (i.e. "mean in healthy"), $\alpha$ (i.e., "deviation from mean in cancer") and $\beta$ (i.e. "deviation from mean due to cancer-type specific effects") and their respective FDR for each GAG property in the blood or urine, respectively. We focused on those GAG properties that displayed in a particular fluid significant changes attributable to cancer (FDR < 0.01) but not to effect specific to a particular cancer types (FDR > 0.01).

In the blood, the following GAG properties were significantly altered in cancer regardless of the cancer type: 0s CS (mass weight fraction, in %); 6s CS (mass weight fraction, in %); 4s/6s CS (ratio of mass weight fractions); Charge CS; Ns HS (mass weight fraction, in %). We observed an increase of 6s CS and Charge CS in the blood of cancer subjects compared to healthy subjects, and a decrease of 0s CS, the 4s/6s CS ratio and Ns HS (Figure 9A/B/C).

In the urine, the following GAG properties were significantly altered in cancer regardless of the cancer type: 4s CS (mass weight fraction, in %).; 6s/0s CS (ratio of mass weight fractions); 4s/0s (ratio of mass weight fractions); 0s HS (mass weight fraction, in %); Charge HS. We observed an increase of Charge HS in the urine of cancer subjects compared to healthy subjects, and a decrease of 4s CS; 6s/0s CS; 4s/0s CS; 0s HS (Figure 10A/B/C).

These results indicate the alteration of specific properties of the GAG profile from normal values in healthy volunteers can be expected in the urine and blood of patients bearing cancer, regardless of the cancer type.

We sought to design a machine learning classifier to discriminate cancer vs. control subjects using blood and/or urine measurements of the GAG profile. The samples were classified as either "cases" if they were taken from subjects with a present diagnosis of cancer or "controls" if they were taken from healthy subjects or subjects with a former diagnosis of cancer but with present no evidence of disease. Therefore, we added 8 subjects to the cohort with a diagnosis of renal cell carcinoma but no evidence of disease at the time of sampling.

We trained a multilayer perceptron using the open source software weka 3.8.0 (supplied by the University of Waikato, Hamilton, New Zealand) and default parameters on 66% of the cohort samples and tested the accuracy of the model in the remaining 33% of the samples. The results on the ability of the classifier to distinguish cases vs. controls in the training vs. test set for each fluid are shown in Table F. We observed that the classifier had consistently high specificity in the detection of "cases" ranging 97.5% to 100% in the training set depending on which fluid was used, and this specificity was maintained in the test set with values ranging 81.1% to 95.4%. Importantly, the classifier probed robust to arbitrary choices of the classification threshold, as shown by the fact that classifier was virtually perfect in the training set according to the area under the receiver operating characteristic curve (AUC) with values ranging 0.975 to 1 - where AUC is a metric that spans an interval from 0 to 1, where 0.5 is a random classifier and 1 is a perfect classifier (Figure 11). This elevated discriminatory power was observed also in the test set, with AUC values ranging 0.922 to 0.992. These results rule out the possibility that the multilayer perceptron had been overfitted to the training data and suggest that its ability to detect "cases" could generalize to any independently collected sample.

We sought to leverage on common cancer-type independent alterations in the GAG profile of cases as opposed to control samples to design blood, urine, or combined GAG scores aimed at discriminating "case" vs. "control" subjects. For this purpose, all samples available in our cohort as described in Table F were used. We adopted Lasso penalized logistic regression (Tibshirani, 1996, *supra*) with leave-one-out cross-validation to select robust GAG properties that were most predictive of the clinical outcome (i.e., case compared to control). Next, we filtered out from this selection those GAG properties for which we observed that the difference in mean between case vs. control had an estimated highest density interval (HDI) which included the value 0. The HDI for each GAG property was calculated using Bayesian estimation under the following assumptions: for each property, the GAG property values were assumed to be sampled from a t-distribution of unknown and to be estimated normality (i.e. degrees of freedom); high uncertainty on the prior distributions; the marginal distribution was well approximated by a Markov chain Monte Carlo sampling with no thinning and chain length equal to 100'000. The estimation was performed using BEST R-package (the above assumptions were reflected by the default parameters). Each GAG score was then subsequently designed as a ratio, where the numerator was the sum of the filtered properties associated with case and the denominator was the sum of the filtered properties associated with control. Each term was normalized using the regression coefficients from the logistic regression. The following GAG scores were designed:

$$Blood\ score = \frac{10[6s\ CS] + 20\left[\frac{6s}{0s}CS\right]}{\frac{4}{100}\left[\frac{4s}{6s}CS\right] + [0s\ CS]}$$

$$Urine\ score = 2\frac{[Ns2s\ HS]}{[4s\ CS]}$$

$$Combined\ score = \frac{(Blood\ score + Urine\ score)}{2}$$

where terms in brackets represent the fraction (mass fraction) of the disaccharide for the corresponding GAG. We subsequently calculated the three scores for each sample and observed that case samples have recurrently elevated scores with respect to control samples (Figures 12, 13 and 14). The difference in GAG scores between case and controls probed able to discriminate subjects with very high accuracy in that ROC curves generated for each GAG score had an AUC equal to 0.961 for blood, 0.942 for urine, and 0.998 for combined (Figure 15). Overall, these results indicate that scores can be designed from blood and/or urine measurements of specific GAG properties to detect cancer regardless of the cancer type.

We also retrieved gene expression data for 19 cancer subtypes encompassing 17 cancer types by histology: bile duct cancer, bladder cancer, blood cancer, brain cancer, breast cancer, colorectal cancer, head and neck cancer, liver cancer, lung cancer, oesophageal cancer, ovarian cancer, pancreatic cancer, prostate cancer, skin cancer, stomach cancer, thyroid cancer, and uterine cancer. Gene expression data in the form of RNAseq-generated read count tables was retrieved for 16 of these subtypes from the Cancer Genome Atlas (TCGA) project (ttps://gdc-portal.nci.nih.gov/). Gene expression data in the form of microarray-generated signal intensity tables was retrieved for the remaining 3 subtypes from three public datasets deposited at the Gene Expression Omnibus (GEO) (Raskin et al., 2013, Mok et al., 2009, Iqbal et al., 2010). We also retrieved TCGA data for renal cell carcinoma (RCC) and three subtypes of RCC: chromophobe RCC, clear cell RCC, and papillary RCC. Both primary tumour tissue samples and matched normal tissues samples were retrieved for each cancer subtype. A summary of the data retrieved for this analysis in reported in Table G.

The gene expression data was pre-processed as previously described (Ritchie et al., 2015) using the limma R-package (Smyth, 2004). Differential gene expression analysis to assess changes in transcriptional regulation between tumor and normal samples was performed separately for each cancer subtype using the limma R-package in the case of microarray data and using the voom R-package (Law et al., 2014) in the case of RNA-seq data, as previously described (Ritchie et al., 2015). For a given gene, changes in transcriptional regulation in either direction, e.g. up-regulation or down-regulation, from normal tissues were considered statistically significant if we observed a false discovery rate $q < 0.001$ and a minimum absolute fold-change in expression level > 50%.

Next, we focused on 60 genes associated with GAG metabolism. A complete list of genes is shown in Table H. We observed a cancer subtype-specific profile of differential transcriptional regulation in comparison to normal tissues (Figure 16 and Table K). On average, 30 of 60 (50%) GAG genes were differentially regulated in each cancer type (12 down-regulated and 18 up-regulated). At least 2 GAG genes were differentially regulated in all cancer subtypes analyzed. The maximum number of differentially regulated GAG genes was 39, which was observed in cholangiocarcinoma. None of the analyzed cancer subtypes displayed a profile of transcriptional regulation of GAG genes identical to any of the other subtypes. These results suggest that each cancer subtype can affect the level and chemical composition of GAGs in a cancer subtype-specific fashion, which may reflect the cancer-type specific effects observed in GAG profile alterations noted above. Strikingly, we observed GAG genes that displayed the same pattern of transcriptional regulation virtually in all cancer types examined: CHPF2 was up-regulated in 17 of 22 cancer types, CHPF and B4GALT7 in 16 of 22 types and B3GAT3 in 15 of 22 types. None of these was found to be significantly down-regulated in the remaining types. EXTL1 and HPSE2 were significantly down-regulated in 11 and 12 of 22 types respectively. None of these was found to be significantly up-regulated in the remaining types. These patterns underlie regulatory programs vastly conserved across different cancer types.

B4GALT7 and B3GAT3 encode for enzymes required to synthesize the linkage region of any GAGs, while CHPF and CHPF2 encode for the primary enzymes responsible for the polymerization of CS by adding non-sulfated CS monomers to the growing chain. Their up-regulation in most cancers can be expected to alter the level and composition of the emerging GAGs by increasing the degree of non-sulfated CS, resulting in the common patterns here observed in the urine of two different cancer types, where the sulfated fraction was significantly reduced compared to the unsulfated fraction (Figure 10B/C). The opposite pattern in the blood can be ascribed as the physiological consequence of GAG filtration from the blood stream operated by the kidneys, which results in an enrichment of sulfated CS species in the blood (Figure 9B/C). On the contrary, repression of EXTL1, which participates in the polymerization of HS by adding non-sulfated HS monomers to the linkage region, may decrease the degree of non-sulfated HS, resulting the common pattern here observed in the urine of two different cancer types, where the sulfated fraction was significantly increased compared to the unsulfated fraction (Figure 10A). Once again, the physiological GAG filtration operated by the kidneys will result in the opposite pattern in the blood, where one sulfated form of HS was significantly decreased (Figure 9A). Repression of HPSE2, known to inhibit of HPSE, may elicit the heparanase activity of HPSE, which is aimed at degrading mature HS polymers thus exacerbating the reduction of nonsulfated HS normally observed in healthy individuals.

These observations suggest that common patterns in the regulation of GAG metabolism across cancers are linked to common alterations observed in the urine and blood GAG profile in different cancer types. This strengthens the conclusions that specific GAG properties are expected to be altered in body fluids compared to healthy conditions as a consequence of cancer, regardless of the cancer type.

**Table D** - Estimates for mean values of each GAG property in the blood of healthy volunteers and their change in presence of cancer and of cancer-type specific effects. FDR for each estimate are reported with FDR < 0.01 considered statistically significant (bold underline). GAG properties in the blood that displayed significant changes attributable to cancer but not to effect specific to a particular cancer types are highlighted in underline.

| GAG property | Mean in healthy | Deviation from mean in cancer | Deviation from mean due to cancer-type specific effects | FDR mean in health | FDR deviation from mean in cancer | FDR deviation from mean due to cancer-type specific effects |
|---|---|---|---|---|---|---|
| 0s CS blood | 81.87 | -9.88 | -3.24 | **9.61E-70** | **5.76E-03** | 1.00E+00 |
| 2s_CS_blood | 0.03 | 0.08 | -0.01 | 1.00E+00 | 9.02E-01 | 1.00E+00 |
| 6s CS blood | 0.36 | 2.79 | -1.81 | 1.00E+00 | **1.94E-05** | 2.87E-01 |
| 4s_CS_blood | 17.67 | 6.70 | 4.93 | **2.61E-14** | 9.62E-02 | 1.00E+00 |
| 2s6s_CS_blood | 0.03 | 0.07 | 0.04 | 1.00E+00 | 4.91E-01 | 1.00E+00 |
| 2s4s_CS_blood | 0.01 | 0.04 | 0.11 | 1.00E+00 | 1.00E+00 | 5.09E-02 |
| 4s6s_CS_blood | 0.03 | 0.11 | -0.01 | 1.00E+00 | 1.84E-01 | 1.00E+00 |
| Tris_CS_blood | 0.01 | 0.04 | 0.04 | 1.00E+00 | 1.00E+00 | 1.00E+00 |
| 4s/6s CS blood | 68.77 | -56.37 | 15.16 | **3.41E-41** | **1.51E-28** | 8.80E-02 |
| 6s/0s_CS_blood | 0.00 | 0.05 | -0.03 | 1.00E+00 | 1.64E-01 | 1.00E+00 |
| 4s/0s_CS_blood | 0.22 | 0.17 | 0.04 | **1.69E-03** | 1.32E-01 | 1.00E+00 |
| Charge CS blood | 0.18 | 0.10 | 0.04 | **2.84E-11** | **5.81E-03** | 1.00E+00 |
| Tot_HA_blood | 0.12 | -0.04 | 0.06 | **5.78E-10** | 8.45E-01 | 3.52E-01 |
| Tot_CS_blood | 5.37 | -0.18 | -1.20 | **1.21E-13** | 1.00E+00 | 1.00E+00 |
| Tris_HS_blood | 1.18 | 1.00 | -1.03 | 3.82E-01 | 1.00E+00 | 1.00E+00 |
| Ns6s_HS_blood | 1.69 | 0.79 | -1.28 | **1.11E-04** | 1.00E+00 | 3.52E-01 |
| Ns2s_HS_blood | 1.69 | 0.98 | -0.12 | **1.69E-03** | 9.02E-01 | 1.00E+00 |
| Ns HS blood | 16.44 | -10.18 | 5.14 | **7.87E-25** | **6.72E-10** | 1.62E-01 |
| 2s6s_HS_blood | 1.99 | 0.28 | -1.51 | **9.40E-05** | 1.00E+00 | 3.31E-01 |
| 6s_HS_blood | 6.87 | -0.24 | 9.10 | **1.68E-05** | 1.00E+00 | **9.76E-05** |
| 2s_HS_blood | 5.35 | -1.21 | 6.30 | **1.71E-05** | 1.00E+00 | **9.87E-04** |
| 0s_HS_blood | 64.79 | 8.55 | -16.63 | **1.98E-45** | 3.31E-01 | **4.15E-03** |
| Charge_HS_blood | 0.43 | -0.04 | 0.12 | **1.70E-17** | 1.00E+00 | 1.00E+00 |
| Tot_HS_blood | 0.05 | 0.08 | 0.06 | 3.17E-01 | 2.09E-02 | 9.02E-01 |

**Table E** - Estimates for mean values of each GAG property in the urine of healthy volunteers and their change in presence of cancer and of cancer-type specific effects. FDR for each estimate are reported with FDR < 0.01 considered statistically significant (bold underline). GAG properties in the urine that displayed significant changes attributable to cancer but not to effect specific to a particular cancer types are highlighted in underline.

| GAG property | Mean in healthy | Deviation from mean in cancer | Deviation from mean due to cancer-type specific effects | FDR mean in health | FDR deviation from mean in cancer | FDR deviation from mean due to cancer-type specific effects |
|---|---|---|---|---|---|---|
| 0s_CS_urine | 35.01 | 23.38 | -17.87 | **5.81E-18** | **4.16E-06** | **5.81E-03** |
| 2s_CS_urine | 0.10 | 0.00 | 0.02 | **7.32E-05** | 1.00E+00 | 1.00E+00 |

(continued)

| GAG property | Mean in healthy | Deviation from mean in cancer | Deviation from mean due to cancer-type specific effects | FDR mean in health | FDR deviation from mean in cancer | FDR deviation from mean due to cancer-type specific effects |
|---|---|---|---|---|---|---|
| 6s_CS_urine | 21.65 | -7.94 | 12.16 | **3.57E-20** | 1.29E-02 | **2.81E-04** |
| 4s CS urine | 40.91 | -14.72 | 4.28 | **4.69E-34** | **1.33E-07** | 1.00E+00 |
| 2s6s_CS_urine | 0.61 | -0.16 | 0.43 | **1.07E-13** | 1.00E+00 | **2.39E-03** |
| 2s4s_CS_urine | 0.46 | -0.13 | 0.30 | **4.27E-16** | 4.40E-01 | **5.91E-04** |
| 4s6s_CS_urine | 1.18 | -0.37 | 0.59 | **4.02E-16** | 3.01E-01 | 2.54E-02 |
| Tris_CS_urine | 0.08 | -0.05 | 0.09 | **1.10E-03** | 1.00E+00 | 1.60E-01 |
| 4s/6s_CS_urine | 2.02 | 0.43 | -1.24 | **8.83E-16** | 1.00E+00 | **2.41E-03** |
| 6s/0s CS urine | 0.76 | -0.47 | 0.41 | **1.06E-12** | **3.79E-03** | 7.46E-02 |
| 4s/0s CS urine | 1.43 | -0.92 | 0.30 | **1.19E-13** | **9.33E-04** | 1.00E+00 |
| Charge_CS_urine | 0.65 | -0.24 | 0.21 | **7.16E-31** | **3.75E-06** | **6.25E-04** |
| Tot_HA_urine | 0.07 | 0.13 | 0.05 | 7.90E-01 | 3.43E-01 | 1.00E+00 |
| Tot_CS_urine | 0.98 | 3.85 | -1.62 | 1.00E+00 | 5.63E-02 | 1.00E+00 |
| Tris_HS_urine | 0.44 | 0.69 | 1.02 | 9.50E-01 | 8.53E-01 | 2.81E-01 |
| Ns6s_HS_urine | 1.63 | 1.07 | 0.70 | **5.91E-04** | 8.53E-01 | 1.00E+00 |
| Ns2s_HS_urine | 1.49 | 1.71 | 1.74 | **1.06E-05** | **1.40E-03** | **7.97E-03** |
| Ns_HS_urine | 12.67 | 6.32 | -5.49 | **4.02E-12** | 5.95E-02 | 4.40E-01 |
| 2s6s_HS_urine | 1.18 | 0.37 | 0.24 | **2.39E-03** | 1.00E+00 | 1.00E+00 |
| 6s_HS_urine | 7.28 | 4.10 | -2.14 | **2.53E-08** | 1.93E-01 | 1.00E+00 |
| 2s_HS_urine | 3.81 | 1.88 | -3.14 | **2.78E-06** | 8.64E-01 | 1.37E-01 |
| 0s HS urine | 71.49 | -16.13 | 6.87 | **3.08E-35** | **8.32E-04** | 1.00E+00 |
| Charge HS urine | 0.34 | 0.21 | -0.02 | **8.72E-16** | **2.79E-04** | 1.00E+00 |
| Tot_HS_urine | 0.15 | 0.66 | -0.41 | 1.00E+00 | 4.45E-01 | 1.00E+00 |

**Table F** - Metrics of accuracy for a multilayer perceptron classifier trained on 66% of the samples and tested on the remaining 33% of the samples. Three classifier were trained based on either only the blood GAG profile, or only on the urine GAG profile, or on both blood and urine GAG profiles. Samples were classified as either "cases", if they were taken from subjects with a present diagnosis of cancer, or "controls", if they were taken from healthy subjects or subjects with a former diagnosis of cancer but with present no evidence of disease.

| Fluid | Samples in train st (*N* cases) | Samples in test set (*N* cases) | AUC in test set | Sensitivity in train set | Specificity in train set | AUC in test set | Sensitivity in train set | Specificity in train set |
|---|---|---|---|---|---|---|---|---|
| Plasma | 87 (69) | 47 (37) | 1 | 100% | 100% | 0.92 2 | 83% | 95.4% |
| Urine | 40 (22) | 22 (12) | 0.96 5 | 97.5% | 98% | 0.99 2 | 81.8% | 84.8% |
| Both plasma and urine | 39 (21) | 22 (12) | 1 | 100% | 100% | 0.97 5 | 77.3% | 81.1% |

**Table G: Number of samples analyzed for each cancer subtype and their source.**

| Cancer subtype | Abbreviation | Cancer type | *N* tumor samples | *N* normal samples | Source |
|---|---|---|---|---|---|
| Clear cell renal cell carcinoma | KIRC | Renal cell carcinoma | 532 | 72 | TCGA |
| Papillary renal cell carcinoma | KIRP | Renal cell carcinoma | 289 | 32 | TCGA |
| Chromophobe renal cell carcinoma | KICH | Renal cell carcinoma | 66 | 25 | TCGA |
| Thyroid carcinoma | THCA | Thyroid cancer | 505 | 59 | TCGA |
| Prostate adenocarcinoma | PRAD | Prostate cancer | 496 | 52 | TCGA |
| Colon adenocarcinoma | COAD | Colorectal cancer | 282 | 41 | TCGA |
| Rectum adenocarcinoma | READ | Colorectal cancer | 94 | 10 | TCGA |
| Lung squamous cell carcinoma | LUSC | Lung cancer | 496 | 51 | TCGA |
| Uterine corpus endometrial carcinoma | UCEC | Uterine cancer | 174 | 24 | TCGA |
| Breast invasive carcinoma | BRCA | Breast cancer | 1090 | 114 | TCGA |
| Pancreatic adenocarcinoma | PAAD | Pancreatic cancer | 178 | 4 | TCGA |
| Lung adenocarcinoma | LUAD | Lung cancer | 514 | 59 | TCGA |
| Bladder carcinoma | BLCA | Bladder cancer | 407 | 19 | TCGA |
| Liver hepatocellular carcinoma | LIHC | Liver cancer | 362 | 50 | TCGA |
| Cholangiocarcinoma | CHOL | Bile duct cancer | 36 | 9 | TCGA |
| Stomach adenocarcinoma | STAD | Stomach cancer | 415 | 35 | TCGA |
| Oesophageal carcinoma | ESCA | Oesophageal cancer | 184 | 11 | TCGA |
| Head and neck squamous cell carcinoma | HNSC | Head and neck cancer | 519 | 44 | TCGA |
| Glioblastoma multiforme | GBM | Brain cancer | 153 | 5 | TCGA |
| Peripheral T-cell lymphoma | PTCL | Blood cancer | 131 | 10 | GEO (GSE19 069) |
| Serous ovarian adenocarcinoma | OVSA | Ovarian cancer | 53 | 10 | GEO (GSE18 520) |
| Malignant melanoma | MM | Skin cancer | 46 | 16 | GEO (GSE15 605) |

**Table H: List of genes associated with GAG metabolism.**

| Gene symbol | GAG metabolic process | Gene description |
|---|---|---|
| UST | CS synthesis | uronyl-2-sulfotransferase [Source:HGNC Symbol;Acc:17223] |
| CHST14 | CS synthesis | carbohydrate (N-acetylgalactosamine 4-0) sulfotransferase 14 [Source:HGNC Symbol;Acc:24464] |
| CHST13 | CS synthesis | carbohydrate (chondroitin 4) sulfotransferase 13 [Source:HGNC Symbol;Acc:21755] |
| CHSY1 | CS synthesis | chondroitin sulfate synthase 1 [Source:HGNC Symbol;Acc:17198] |
| DSE | CS synthesis | dermatan sulfate epimerase [Source:HGNC Symbol;Acc:21144] |
| CHSY3 | CS synthesis | chondroitin sulfate synthase 3 [Source:HGNC Symbol;Acc:24293] |
| CHST11 | CS synthesis | carbohydrate (chondroitin 4) sulfotransferase 11 [Source:HGNC Symbol;Acc:17422] |
| CHST15 | CS synthesis | carbohydrate (N-acetylgalactosamine 4-sulfate 6-O) sulfotransferase 15 [Source:HGNC Symbol;Acc:18137] |
| CHPF2 | CS synthesis | chondroitin polymerizing factor 2 [Source:HGNC Symbol;Acc:29270] |
| CSGALNACT 2 | CS synthesis | chondroitin sulfate N-acetylgalactosaminyltransferase 2 [Source:HGNC Symbol;Acc:24292] |
| CHST12 | CS synthesis | carbohydrate (chondroitin 4) sulfotransferase 12 [Source:HGNC Symbol;Acc:17423] |
| CSGALNACT 1 | CS synthesis | chondroitin sulfate N-acetylgalactosaminyltransferase 1 [Source:HGNC Symbol;Acc:24290] |
| CHST7 | CS synthesis | carbohydrate (N-acetylglucosamine 6-O) sulfotransferase 7 [Source:HGNC Symbol;Acc:13817] |
| CHPF | CS synthesis | chondroitin polymerizing factor [Source:HGNC Symbol;Acc:24291] |
| CHST3 | CS synthesis | carbohydrate (chondroitin 6) sulfotransferase 3 [Source:HGNC Symbol;Acc:1971] |
| HPSE | GAG degradation | heparanase [Source:HGNC Symbol;Acc:5164] |
| HGSNAT | GAG degradation | heparan-alpha-glucosaminide N-acetyltransferase [Source:HGNC Symbol;Acc:26527] |
| HYAL4 | GAG degradation | hyaluronoglucosaminidase 4 [Source:HGNC Symbol;Acc:5323] |
| GALNS | GAG degradation | galactosamine (N-acetyl)-6-sulfate sulfatase [Source:HGNC Symbol;Acc:4122] |
| GLB1 | GAG degradation | galactosidase, beta 1 [Source:HGNC Symbol;Acc:4298] |
| GNS | GAG degradation | glucosamine (N-acetyl)-6-sulfatase [Source:HGNC Symbol;Acc:4422] |
| GUSB | GAG degradation | glucuronidase, beta [Source:HGNC Symbol;Acc:4696] |
| HEXA | GAG degradation | hexosaminidase A (alpha polypeptide) [Source:HGNC Symbol;Acc:4878] |
| HEXB | GAG degradation | hexosaminidase B (beta polypeptide) [Source:HGNC Symbol;Acc:4879] |

(continued)

| Gene symbol | GAG metabolic process | Gene description |
|---|---|---|
| HYAL1 | GAG degradation | hyaluronoglucosaminidase 1 [Source:HGNC Symbol;Acc:5320] |
| IDS | GAG degradation | iduronate 2-sulfatase [Source:HGNC Symbol;Acc:5389] |
| IDUA | GAG degradation | iduronidase, alpha-L- [Source:HGNC Symbol;Acc:5391] |
| ARSB | GAG degradation | arylsulfatase B [Source:HGNC Symbol;Acc:714] |
| NAGLU | GAG degradation | N-acetylglucosaminidase, alpha [Source:HGNC Symbol;Acc:7632] |
| HPSE2 | GAG degradation | heparanase 2 [Source:HGNC Symbol;Acc:18374] |
| SGSH | GAG degradation | N-sulfoglucosamine sulfohydrolase [Source:HGNC Symbol;Acc:10818] |
| SPAM1 | GAG degradation | sperm adhesion molecule 1 (PH-20 hyaluronidase, zona pellucida binding) [Source:HGNC Symbol;Acc:11217] |
| HYAL3 | GAG degradation | hyaluronoglucosaminidase 3 [Source:HGNC Symbol;Acc:5322] |
| HYAL2 | GAG degradation | hyaluronoglucosaminidase 2 [Source:HGNC Symbol;Acc:5321] |
| HS3ST3B1 | GAG degradation | heparan sulfate (glucosamine) 3-O-sulfotransferase 3B1 [Source:HGNC Symbol;Acc:5198] |
| HS3ST3B1 | GAG degradation | heparan sulfate (glucosamine) 3-O-sulfotransferase 3B1 [Source:HGNC Symbol;Acc:5198] |
| HS3ST3A1 | GAG degradation | heparan sulfate (glucosamine) 3-O-sulfotransferase 3A1 [Source:HGNC Symbol;Acc:5196] |
| HS3ST3A1 | GAG degradation | heparan sulfate (glucosamine) 3-O-sulfotransferase 3A1 [Source:HGNC Symbol;Acc:5196] |
| B4GALT7 | GAG synthesis | xylosylprotein beta 1,4-galactosyltransferase, polypeptide 7 [Source:HGNC Symbol;Acc:930] |
| B3GALT6 | GAG synthesis | UDP-Gal:betaGal beta 1,3-galactosyltransferase polypeptide 6 [Source:HGNC Symbol;Acc:17978] |
| B3GAT2 | GAG synthesis | beta-1,3-glucuronyltransferase 2 (glucuronosyltransferase S) [Source:HGNC Symbol;Acc:922] |
| B3GAT3 | GAG synthesis | beta-1,3-glucuronyltransferase 3 (glucuronosyltransferase I) [Source:HGNC Symbol;Acc:923] |
| B3GAT1 | GAG synthesis | beta-1,3-glucuronyltransferase 1 (glucuronosyltransferase P) [Source:HGNC Symbol;Acc:921] |
| XYLT1 | GAG synthesis | xylosyltransferase I [Source:HGNC Symbol;Acc:15516] |
| XYLT2 | GAG synthesis | xylosyltransferase II [Source:HGNC Symbol;Acc:15517] |
| EXT1 | HS synthesis | exostosin glycosyltransferase 1 [Source:HGNC Symbol;Acc:3512] |
| EXT2 | HS synthesis | exostosin glycosyltransferase 2 [Source:HGNC Symbol;Acc:3513] |
| EXTL1 | HS synthesis | exostosin-like glycosyltransferase 1 [Source:HGNC Symbol;Acc:3515] |

(continued)

| Gene symbol | GAG metabolic process | Gene description |
|---|---|---|
| EXTL2 | HS synthesis | exostosin-like glycosyltransferase 2 [Source:HGNC Symbol;Acc:3516] |
| EXTL3 | HS synthesis | exostosin-like glycosyltransferase 3 [Source:HGNC Symbol;Acc:3518] |
| HS3ST5 | HS synthesis | heparan sulfate (glucosamine) 3-O-sulfotransferase 5 [Source:HGNC Symbol;Acc:19419] |
| GLCE | HS synthesis | glucuronic acid epimerase [Source:HGNC Symbol;Acc:17855] |
| HS6ST3 | HS synthesis | heparan sulfate 6-O-sulfotransferase 3 [Source:HGNC Symbol;Acc:19134] |
| NDST1 | HS synthesis | N-deacetylase/N-sulfotransferase (heparan glucosaminyl) 1 [Source:HGNC Symbol;Acc:7680] |
| NDST4 | HS synthesis | N-deacetylase/N-sulfotransferase (heparan glucosaminyl) 4 [Source:HGNC Symbol;Acc:20779] |
| HS6ST2 | HS synthesis | heparan sulfate 6-O-sulfotransferase 2 [Source:HGNC Symbol;Acc:19133] |
| NDST3 | HS synthesis | N-deacetylase/N-sulfotransferase (heparan glucosaminyl) 3 [Source:HGNC Symbol;Acc:7682] |
| HS6ST1 | HS synthesis | heparan sulfate 6-O-sulfotransferase 1 [Source:HGNC Symbol;Acc:5201] |
| HS2ST1 | HS synthesis | heparan sulfate 2-O-sulfotransferase 1 [Source:HGNC Symbol;Acc:5193] |
| HS3ST2 | HS synthesis | heparan sulfate (glucosamine) 3-O-sulfotransferase 2 [Source:HGNC Symbol;Acc:5195] |
| HS3ST1 | HS synthesis | heparan sulfate (glucosamine) 3-O-sulfotransferase 1 [Source:HGNC Symbol;Acc:5194] |
| NDST2 (e.g. Entrez ID:8509) | HS Synthesis | N-deacetylase and N-sulfotransferase 2 [Source: HGNC] |

**Table K:** Regulation of GAG genes in different cancer types vs. matched normal tissue of origin. Entries with "1" indicate that the gene in that row was found to be up–regulated in the cancer type in that column; "-1" indicate that the gene in that row was found to be down–regulated in the cancer type in that column; "0" indicate that the gene in that row was not found to be regulated in the cancer type in that column; NA indicate unavailable information. Genes are identified by their official gene symbol or, in not available, by their entrez gene ID. Key as in Table G.

| Gene | BLCA | BRCA | COAD | GBM | HNSC | LUSC | LUAD | PAAD | READ | UCEC | CHOL | ESCA | KICH | KIRC | KIRP | LIHC | STAD | THCA | PRAD | MM | OVSA | PTCL |
|---|---|---|---|---|---|---|---|---|---|---|---|---|---|---|---|---|---|---|---|---|---|---|
| B3GALT6 | 1 | 1 | 1 | 0 | 1 | 0 | 0 | 0 | 1 | 0 | 1 | 1 | 0 | 0 | 0 | 1 | 1 | 0 | 1 | 1 | 1 | 0 |
| XYLT1 | 0 | -1 | -1 | 0 | -1 | 0 | 0 | 0 | 0 | -1 | NA | 0 | -1 | 0 | 0 | 0 | 0 | -1 | 0 | 0 | 0 | 0 |
| XYLT2 | 1 | 1 | 1 | 0 | 1 | 1 | 1 | 0 | 0 | 0 | 1 | 0 | 0 | 0 | 1 | 1 | 0 | 0 | 0 | 0 | 0 | 0 |
| B3GAT1 | 0 | -1 | -1 | 0 | 0 | -1 | 1 | 0 | NA | 0 | 0 | -1 | -1 | -1 | 0 | -1 | -1 | 1 | 1 | 0 | 0 | 0 |
| B4GALT7 | 1 | 1 | 1 | 1 | 1 | 1 | 1 | 0 | 1 | 1 | 1 | 1 | 1 | 1 | 1 | 1 | 1 | 0 | 0 | 0 | 0 | 0 |
| B3GAT2 | -1 | -1 | 0 | 0 | NA | 0 | 0 | NA | NA | NA | NA | 0 | NA | 1 | NA | NA | 0 | -1 | 0 | NA | NA | NA |
| B3GAT3 | 1 | 1 | 1 | 0 | 1 | 1 | 1 | 0 | 1 | 1 | 1 | 1 | 1 | 0 | 1 | 1 | 1 | 0 | 1 | 0 | 0 | 0 |
| DSE | 0 | -1 | 0 | 1 | 1 | 0 | 0 | 0 | 0 | -1 | 1 | 1 | 0 | 0 | 0 | -1 | 0 | 0 | -1 | 0 | 0 | 0 |
| CHPF | 1 | 1 | 1 | 1 | 1 | 1 | 1 | 1 | 1 | 1 | 1 | 1 | 0 | 1 | 1 | 0 | 1 | 0 | 1 | 0 | 0 | 0 |
| CHSY3 | 0 | 0 | 1 | 0 | 1 | -1 | 0 | 0 | 1 | -1 | 0 | 1 | 0 | 1 | 0 | 0 | 1 | 0 | 0 | 1 | 0 | 1 |
| CHST14 | 1 | 0 | 1 | 1 | 1 | 1 | 0 | 0 | 0 | -1 | 1 | 1 | 0 | 1 | 1 | 1 | 0 | -1 | 0 | 1 | 1 | 0 |
| CHSY1 | 0 | 0 | 1 | 1 | 1 | 0 | -1 | 0 | 1 | -1 | 1 | 1 | 0 | 1 | 0 | 0 | 1 | 0 | 0 | 1 | 1 | 0 |
| CHST11 | 1 | 1 | 0 | 1 | 1 | -1 | 0 | 0 | 0 | 0 | NA | 1 | 0 | 1 | 1 | 1 | 1 | 1 | -1 | 1 | 0 | 0 |
| CHPF2 | 1 | 1 | 0 | 1 | 1 | 1 | 1 | 0 | 1 | 1 | 1 | 1 | 1 | 1 | 1 | 1 | 1 | 0 | 1 | 0 | 0 | 0 |
| CHST15 | 0 | 1 | -1 | 0 | 1 | 0 | 1 | 0 | -1 | 0 | 1 | 1 | -1 | 1 | 0 | 1 | 0 | 0 | -1 | 0 | 0 | 1 |
| CHST7 | 0 | -1 | -1 | 1 | 1 | 1 | -1 | 0 | 0 | -1 | 0 | 1 | -1 | 1 | 0 | -1 | 0 | 1 | 0 | 0 | 0 | 0 |
| UST | -1 | -1 | -1 | 1 | 1 | 0 | -1 | 0 | -1 | -1 | 0 | 0 | 1 | -1 | -1 | -1 | -1 | -1 | -1 | 0 | 0 | 0 |
| CHST13 | NA | 0 | -1 | NA | NA | -1 | 0 | NA | 0 | NA | -1 | NA | -1 | 1 | 1 | 1 | 1 | NA | NA | 0 | 0 | 0 |
| CSGALNACT2 | 0 | 0 | 1 | 0 | 1 | -1 | 0 | 0 | 0 | -1 | 1 | 1 | -1 | 1 | 0 | 0 | 1 | 1 | 0 | 0 | 0 | 0 |
| CSGALNACT1 | -1 | -1 | 1 | 0 | 0 | -1 | -1 | 0 | 0 | -1 | 1 | 0 | 1 | 0 | -1 | 1 | 0 | -1 | 1 | 0 | 0 | 0 |
| CHST3 | 0 | -1 | 0 | 1 | 1 | 1 | 0 | 0 | 0 | -1 | 1 | 0 | 0 | -1 | 0 | 1 | 0 | 0 | -1 | 0 | 0 | 0 |
| CHST12 | 1 | 0 | 0 | 1 | 1 | 0 | 0 | 0 | 0 | 0 | 1 | 1 | 0 | 1 | 1 | 1 | 0 | 0 | 0 | 0 | 0 | 0 |
| HS3ST5 | -1 | 0 | -1 | -1 | -1 | -1 | -1 | NA | NA | NA | NA | NA | -1 | -1 | NA | NA | 0 | NA | -1 | NA | NA | NA |
| EXT2 | 0 | 0 | 0 | 1 | 1 | 1 | 0 | 0 | 0 | 0 | 1 | 1 | 0 | 0 | 0 | 1 | 1 | 0 | 0 | 0 | 0 | 0 |
| EXT1 | 0 | 0 | -1 | 1 | 1 | 1 | 0 | 0 | -1 | 0 | 1 | 1 | 0 | 0 | 0 | 1 | 1 | -1 | -1 | 0 | 0 | 1 |

| Gene | | | | | | | | | | | | | | | | | | | | | | |
|---|---|---|---|---|---|---|---|---|---|---|---|---|---|---|---|---|---|---|---|---|---|---|
| NDST3 | NA | 0 | 0 | -1 | NA | NA | NA | NA | NA | NA | NA | -1 | -1 | -1 | -1 | -1 | 0 | -1 | NA | NA | NA | NA |
| NDST1 | 0 | -1 | -1 | 0 | 0 | -1 | -1 | 0 | 0 | 0 | 1 | 0 | 1 | 0 | -1 | 1 | 0 | 0 | 0 | 0 | 1 | 0 |
| GLCE | 1 | 0 | 1 | 0 | 0 | 0 | 0 | 0 | 0 | 0 | 1 | 1 | -1 | -1 | -1 | 1 | 1 | 0 | -1 | 0 | 0 | 0 |
| HS2ST1 | 1 | 0 | 1 | 1 | 1 | 0 | 0 | 0 | 1 | 0 | 1 | 1 | -1 | 0 | 0 | 1 | 1 | 0 | 0 | 0 | 0 | 0 |
| HS6ST2 | 1 | -1 | 1 | 0 | 0 | 1 | 1 | NA | 1 | -1 | NA | 1 | -1 | -1 | -1 | NA | -1 | 1 | 0 | 0 | 0 | 0 |
| HS3ST1 | 0 | -1 | 1 | 1 | -1 | 1 | 1 | 1 | 0 | 0 | 1 | 1 | 0 | -1 | 1 | 0 | 1 | 0 | 0 | 0 | 0 | 0 |
| HS3ST2 | 0 | 0 | 0 | -1 | -1 | -1 | 0 | 0 | -1 | 0 | NA | 0 | NA | 1 | NA | 1 | 1 | 0 | 1 | 0 | 1 | 0 |
| HS6ST1 | 1 | 1 | 0 | 0 | 0 | 1 | 0 | 0 | 0 | 1 | 1 | 1 | -1 | -1 | -1 | 1 | 0 | -1 | 0 | 0 | 0 | 0 |
| 8509 | 0 | 0 | 0 | 0 | 0 | 0 | 0 | 0 | 0 | 0 | 1 | 0 | 0 | 0 | 0 | 1 | 0 | 0 | 0 | NA | NA | NA |
| NDST4 | NA | NA | NA | NA | NA | NA | NA | NA | NA | NA | NA | NA | NA | NA | NA | NA | -1 | NA | 0 | NA | NA | NA |
| HS6ST3 | 0 | 1 | -1 | -1 | NA | 0 | 0 | NA | NA | -1 | NA | -1 | 1 | -1 | -1 | NA | -1 | -1 | 1 | 0 | 0 | 0 |
| EXTL1 | -1 | 0 | -1 | -1 | -1 | -1 | 0 | NA | NA | -1 | NA | NA | -1 | -1 | -1 | NA | -1 | 0 | -1 | 0 | 0 | 0 |
| EXTL2 | 0 | -1 | 1 | 1 | 1 | 0 | 0 | 0 | 0 | -1 | 1 | 0 | -1 | -1 | 0 | 1 | 1 | 0 | 0 | 0 | 0 | 0 |
| EXTL3 | 0 | -1 | 1 | 0 | 1 | 0 | 0 | 0 | 0 | 0 | 1 | 1 | 0 | 0 | 0 | 1 | 0 | 0 | 0 | 0 | 0 | 0 |
| HS3ST3B1 | NA | 0 | -1 | NA | 0 | 0 | 0 | NA | NA | 0 | -1 | 0 | -1 | -1 | -1 | -1 | 0 | -1 | -1 | 0 | 0 | 0 |
| HS3ST3A1 | 0 | 1 | 0 | NA | 0 | 1 | 1 | 0 | 0 | 0 | 0 | 1 | -1 | -1 | -1 | -1 | 0 | -1 | -1 | 0 | 0 | 1 |
| HPSE | 1 | 1 | -1 | NA | 0 | 1 | 1 | 0 | -1 | 0 | NA | 1 | 0 | 0 | -1 | 0 | 1 | 1 | -1 | 0 | 0 | 0 |
| HPSE2 | -1 | -1 | -1 | NA | -1 | -1 | -1 | NA | -1 | -1 | NA | -1 | NA | -1 | NA | NA | -1 | NA | -1 | 0 | 0 | 0 |
| GLB1 | 1 | 1 | 0 | 1 | 1 | -1 | 0 | 0 | 0 | 1 | 1 | 1 | 1 | -1 | 0 | 1 | 1 | 0 | 0 | NA | NA | NA |
| GUSB | 1 | 0 | 0 | 1 | 1 | 0 | 0 | 0 | 0 | 0 | 0 | 1 | 0 | 1 | 1 | 0 | 1 | 0 | 1 | 0 | 0 | 0 |
| HYAL3 | 1 | 1 | 1 | 0 | 0 | 1 | 1 | 0 | 1 | 1 | 0 | 1 | 0 | -1 | 0 | 1 | 0 | 0 | 1 | 0 | 0 | 0 |
| GNS | 0 | -1 | 0 | 1 | 1 | -1 | 0 | 0 | 0 | 0 | 1 | 0 | 0 | -1 | 0 | 1 | 1 | 0 | 0 | 1 | 0 | 0 |
| HYAL4 | NA | NA | NA | NA | -1 | NA | NA | NA | NA | NA | NA | NA | 0 | -1 | -1 | NA | NA | NA | NA | 0 | 0 | 0 |
| HYAL1 | -1 | -1 | 0 | NA | 0 | -1 | -1 | 0 | 0 | -1 | -1 | 0 | 1 | -1 | -1 | 0 | 0 | 0 | -1 | 0 | 0 | 0 |
| HYAL2 | 0 | 0 | 1 | 1 | 0 | -1 | -1 | 0 | 1 | 0 | 1 | 0 | -1 | 0 | -1 | 1 | 0 | 1 | 0 | 0 | 0 | 0 |
| SPAM1 | NA | NA | NA | NA | NA | NA | NA | NA | NA | NA | NA | NA | NA | NA | NA | NA | NA | NA | NA | NA | NA | NA |
| HGSNAT | 0 | 0 | 0 | 0 | 0 | 0 | 0 | 0 | 0 | -1 | 1 | 0 | 0 | 0 | 0 | 0 | 0 | 0 | 0 | 0 | 0 | 0 |
| GALNS | 1 | 1 | 0 | 1 | 1 | 1 | 0 | 0 | 0 | 1 | 1 | 1 | 0 | 1 | 1 | 0 | 1 | 0 | 0 | 0 | 0 | 0 |
| IDUA | 1 | 0 | 0 | 1 | 0 | -1 | 0 | 0 | 1 | 0 | 1 | 0 | 0 | 1 | 1 | 1 | 0 | 0 | 1 | 0 | 0 | 0 |
| SGSH | 1 | 0 | 0 | 1 | 0 | 0 | 0 | 0 | 0 | 0 | 1 | 0 | 0 | 0 | 1 | 1 | 0 | 0 | 1 | 0 | 0 | 0 |
| HEXB | 0 | 0 | 0 | 1 | 1 | -1 | 0 | 0 | 0 | 0 | 1 | 1 | 0 | 0 | 1 | 1 | 1 | 0 | 0 | 0 | 0 | 0 |
| NAGLU | 1 | 0 | 0 | 1 | 1 | 0 | 0 | 0 | 0 | 0 | 0 | 0 | 0 | 0 | 0 | 0 | 0 | 0 | 0 | 0 | 0 | 0 |
| HEXA | 1 | 0 | 0 | 1 | 1 | 0 | 0 | 0 | 0 | 0 | 1 | 1 | 1 | 0 | 0 | 1 | 1 | 0 | 0 | 1 | 0 | 0 |
| IDS | 0 | -1 | 0 | -1 | 0 | -1 | 0 | 0 | 0 | -1 | 1 | 1 | 1 | 0 | 0 | 0 | 0 | 0 | -1 | 0 | 0 | 0 |
| ARSB | 0 | 0 | 0 | 1 | 1 | -1 | 0 | 0 | 0 | 0 | 1 | 0 | -1 | 0 | 0 | 1 | 1 | 0 | 0 | 1 | 0 | 0 |

**References**

1. F. Gatto et al., Glycosaminoglycan Profiling in Patients' Plasma and Urine Predicts the Occurrence of Metastatic Clear Cell Renal Cell Carcinoma. Cell Rep 15, 1822-1836 (2016).
2. L. Raskin et al., Transcriptome profiling identifies HMGA2 as a biomarker of melanoma progression and prognosis. J Invest Dermatol 133, 2585-2592 (2013).
3. S. C. Mok et al., A gene signature predictive for outcome in advanced ovarian cancer identifies a survival factor: microfibril-associated glycoprotein 2. Cancer Cell 16, 521-532 (2009).
4. J. Iqbal et al., Molecular signatures to improve diagnosis in peripheral T-cell lymphoma and prognostication in angioimmunoblastic T-cell lymphoma. Blood 115, 1026-1036 (2010).
5. M. E. Ritchie et al., limma powers differential expression analyses for RNA-sequencing and microarray studies. Nucleic Acids Res 43, e47 (2015).
6. G. K. Smyth, Linear models and empirical bayes methods for assessing differential expression in microarray experiments. Stat Appl Genet Mol Biol 3, Article3 (2004).
7. C. W. Law, Y. Chen, W. Shi, G. K. Smyth, Voom: precision weights unlock linear model analysis tools for RNA-seq read counts. Genome Biol 15, R29 (2014).

## EXAMPLE 3

In this study we measured circulating GAG levels in subjects presenting with melanoma (M).

### *Material and Methods*

### *Plasma sample collection*

Blood samples were obtained from 14 healthy volunteers (H) and 16 patients with uveal ($N$ = 12) or skin melanoma ($N$ = 4). All patients had evidence of disease at the time of sampling.

Whole blood was collected in EDTA tubes and centrifuged first at 1,000$g$ for 10 minutes at 4 °C within 30 minutes from collection and then the supernatant was centrifuged again at 10,000g for 10 minutes at 4 °C. The supernatant is platelet-poor plasma was frozen at -80°C until they were shipped for analysis in dry ice.

Platelet-poor plasma GAGs correspond to blood GAGs. For the purpose of this study, platelet-poor plasma is therefore also interchangeably referred to as blood.

### *Glycosaminoglycan profile determination*

Sample preparation including extraction and purification steps were performed as previously described by Volpi and Maccari 2005[1 and 2] and Coppa et al., 2011, while sample GAGs separation and quantification were performed as described in Volpi et al., 2014; Volpi and Linhardt, 2010.

Briefly, to extract the GAGs, 500 µl of sample was lyophilized, reconstituted with 1 ml of a 20-mM TRIS-Cl buffer pH 7.4 and treated with protease (Proteinase K from Tritirachium album [E.C. 3.4.21.64], >500 units ml_1 from Sigma-Aldrich) at 60 °C for 12 h. After boiling for 10 min, centrifugation and filtration on 0.45-µm filters, the filtrate was lyophilized. The powder was dissolved in 1 ml of distilled water by prolonged mixing. After centrifugation at 5000 g for 15 min, 0.2 ml of 20% trichloro- acetic acid was added to the supernatant. After 2 h at 4 °C, the mixture was centrifuged at 5000 g for 15 min, and the supernatant was recovered and lyophilized. After solubilization in 0.4 ml of bidistilled water and centrifugation at 10 000 g for 10 min, the supernatant was collected and further analysed. To purify the GAGs, after reconstitution with 500 µl 10 mM NaCl, sample GAGs were further purified on anion-exchange resin (QAE Sephadex A-25). After centrifugation at 10,000 × g for 5 min, the supernatant was applied to a column (1 cm × 4 cm) packed with about 3 ml of resin previously equilibrated with 10 mM NaCl. After washing the resin with 20 ml of 10 mM NaCl, 10 ml of 2.5 M NaCl were added. Fifty milliliters of ethanol were added to the eluate (10 ml) and stored at -20 °C for 24 h. After centrifugation at 5000 × g for 15 min, the pellet was dried at 60 °C for 12 h. After reconstitution with 80 µl of 50 mM ammonium acetate pH 8.0, the material was treated with 20 µl of chondroitinase ABC at 37 °C for 12 h. To separate and quantify the GAGs, after boiling for 5 min, the samples were injected in a capillary electrophoresis instrument equipped with laser-induced fluorescence detector (CE-LIF).

Nineteen independent GAG properties were measured in each sample (blood): CS concentration (in µg/mL), HS concentration (in µg/mL), HA concentration (in µg/mL), and mass fractions of disaccharide composition for both CS and HS. In addition, five dependent GAG properties were calculated from these measurements: the CS and HS charge; and the following CS ratio of mass fractions: 4s/6s, 6s/0s, and 4s/0s. The 24 GAG properties may be collectively referred to as GAG profile.

### Biomarker score design

The statistical significance of changes for each GAG property between the two groups (melanoma vs. healthy) was assessed by calculating the highest density interval (HDI) for the mean difference using Bayesian estimation under the following assumptions: GAG property values are sampled from a t-distribution of unknown and to be estimated normality (i.e. degrees of freedom); high uncertainty on the prior distributions; the marginal distribution is well approximated by a Markov chain Monte Carlo sampling with no thinning and chain length equal to 100'000. The estimation was performed using *BEST* R-package (the above assumptions are reflected by the default parameters). Bayesian estimation was preferred over the widely used t-test since it provides a robust and reliable estimation of mean difference even under uncertainty of the underlying score distribution for the two groups (that is the case when the number of samples is limited).

To verify whether changes in the GAG profile in melanoma patients could be condensed into GAG scores, that can be used to differentiate melanoma patients from healthy subjects, and therefore would be suitable as a cancer diagnostic, we utilized Lasso penalized logistic regression (Tibshirani, 1996) with leave-one-out cross-validation to select robust GAG properties that are most predictive of the clinical outcome (i.e., melanoma compared to healthy subject). The score was subsequently designed as a ratio, where the numerator is the sum of the properties associated with melanoma and the denominator is the sum of the properties associated with the healthy state. Each term was normalized using the regression coefficients.

For the purpose of this study and for reasons elsewhere stated, scores computed from blood GAGs regardless if the processed sample was platelet-poor plasma are referred to as GAG blood scores.

### Accuracy metrics

We evaluated the performance of the GAG score in the binary classification of a sample as either melanoma or healthy at varying threshold scores by deriving the receiver-operating-characteristic (ROC) curves. We measured the accuracy of the GAG score as the area under the curve (AUC) of its ROC curve (AUC is 1 for a perfect classifier and 0.5 for a random classifier). We selected as a potential cut-off value for the GAG score the score for which the accuracy was maximum, i.e. a sample whose GAG score is above this cut-off value has the maximum probability of being melanoma and below this cut-off value has the maximum probability of being healthy. The ROC curves were calculated using the pROC R-package, while the optimal cut-off using the OptimalCutpoints R-package.

### Results

We analyzed a total of 16 blood samples from patients with M, of which 12 had uveal melanoma and 4 had skin (cutaneous) melanoma (M). We further analyzed 14 blood samples from healthy volunteers (H). Clinical data for this cohort is reported in Table L. We quantified the profile of GAGs in the 30 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table M). We compared the value of each property in the GAG profile in the M versus H group. We assessed the statistical significance of changes for each GAG property between the two groups by calculating the highest density interval (HDI) for the mean difference using Bayesian estimation. The results showed marked differences in several GAG properties between M and H subjects (Table M).

We condensed changes in the GAG profile in M vs. H subjects GAG scores. We derived one M GAG score (Melanoma scoring formula #1), based on the following formula using blood measurements:

$$M\ GAG\ score = \frac{7}{10}\,[4s6s\ CS] + 5\left(\frac{[6s\ CS]}{[6s\ CS] + [4s\ CS]}\right) + \frac{1}{10}\,[Tot\ CS]\left(1 + \frac{1}{[Tot\ HA]}\right)$$

$$-\frac{[0s\ HS]}{[0s\ HS] + [Ns\ HS]} - \frac{7}{1000}\,([2s\ HS] + [0s\ HS])$$

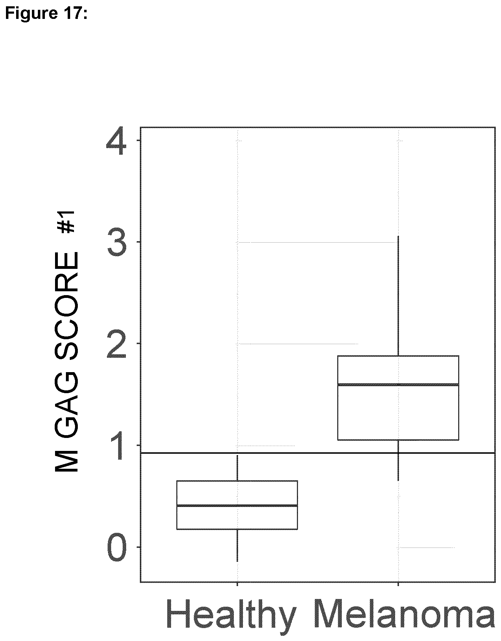

where terms in square brackets represent the fraction (mass fraction) of the disaccharide for the corresponding GAG, and [*Tot CS]* is the total concentration of CS (in μg/mL), and [*Tot HA*] is the total concentration of HA (in μg/mL). We subsequently calculated the score in each sample and observed that M samples have recurrently elevated scores with respect to H samples (Figure 17). The performance of the GAG score was evaluated using the receiver-operating-characteristic (ROC) curves, and the area under the curve (AUC) was found to be 0.973. We identified an optimal cut-off equal to 0.92 for this score. Using this cut-off, M subjects could be distinguished from healthy individuals with 93.3% accuracy, 87.3% sensitivity, and 100% specificity.

We also investigated whether variations of the above GAG score would also be effective in distinguishing M from H subjects. To this end, we derived an alternative M GAG score (melanoma scoring formula #2) based only on CS measurements:

$$M\ GAG\ score\ 2 = \frac{4[6s\ CS] + 5[\text{Tot CS}]}{100} + 5\left(\frac{[6s\ CS]}{[4s\ CS] + [6s\ CS]}\right)$$

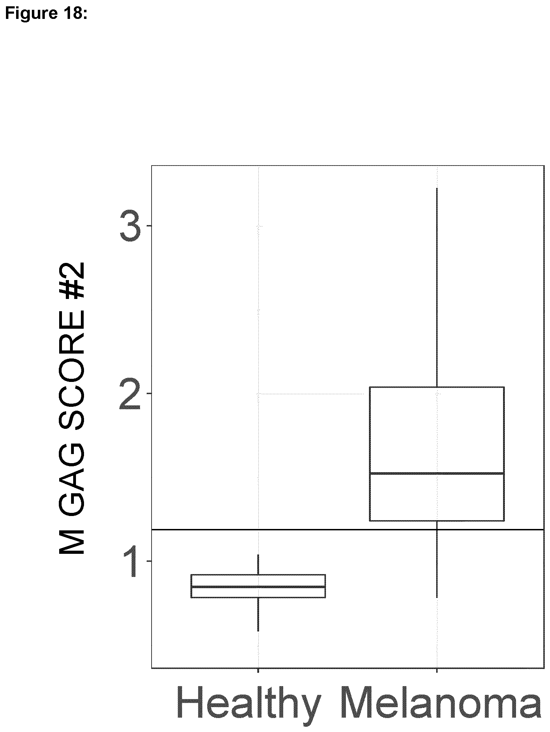

where terms in square brackets represent the fraction (mass fraction) of the disaccharide for the corresponding GAG, and [Tot CS] is the total concentration of CS (in $\mu$g/mL). Using this alternative scoring system, M samples had recurrently higher scores than H samples (Figure 18). The AUC was found to be 0.933. We identified an optimal cut-off equal to 1.19 for this score. Using this cut-off, M subjects could be distinguished from healthy individuals with 93.3% accuracy, 81.2% sensitivity, and 100% specificity.

These results indicate that there are measurable differences in circulating GAG levels and/or chemical composition between melanoma as opposed to healthy subjects, and thus that GAG properties may be useful in melanoma screening (e.g. diagnosis).

These results further demonstrate that different GAG scores can be designed to discriminate between melanoma and healthy subjects, and that their significant AUC permit fine-tuned optimal cut-off scores, in order to maximize sensitivity and/or specificity, depending on the intended diagnostic use.

**References**

Coppa GV, Gabrielli O, Buzzega D, Zampini L, Galeazzi T, Maccari F, Bertino E, Volpi N (2011). Composition and structure elucidation of human milk glycosaminoglycans. Glycobiology 21, 295-303.

Gatto, F., Volpi, N., Nilsson, H., Nookaew, I., Maruzzo, M., Roma, A., Johansson, M.E., Stierner, U., Lundstam, S., Basso, U., et al. (2016). Glycosaminoglycan Profiling in Patients' Plasma and Urine Predicts the Occurrence of Metastatic Clear Cell Renal Cell Carcinoma. Cell Rep 15, 1822-1836.

Tibshirani, R. (1996). Regression shrinkage and selection via the Lasso. Journal of the Royal Statistical Society Series B-Methodological 58, 267-288.

Volpi N and Maccari F. (20051). Glycosaminoglycans composition of the largefreshwater mollusc bivalve Anodonta anodonta. Biomacromolecules 6, 3174-3180.

Volpi N and Maccari F (20052). Microdetermination of chondroitin sulfate in normal human plasma by fluorophore-assisted carbohydrate electrophore-sis (FACE). Clin Chim Acta 356, 125-133.

Volpi, N., Galeotti, F., Yang, B., and Linhardt, R.J. (2014). Analysis of glycosaminoglycan-derived, precolumn, 2-aminoacridone-labeled disaccharides with LC-fluorescence and LC-MS detection. Nature protocols 9, 541-558.

Volpi, N., and Linhardt, R.J. (2010). High-performance liquid chromatography-mass spectrometry for mapping and sequencing glycosaminoglycan-derived oligosaccharides. Nature protocols 5, 993-1004.

**Table L**: Clinical data for the cohort analysed in this study. Results are presented as medians (25th, 75th percentile) or percent or counts. Missing values were omitted.

| Baseline characteristics | Melanoma | Healthy |
|---|---|---|
| Number of samples | 16 | 14 |
| Age [years] | 65 (53-71) | 40 (35 - 52) |
| Female | 62.5% | 78.6% |

(continued)

| Baseline characteristics | Melanoma | Healthy |
|---|---|---|
| Cancer subtype | | |
| Uveal melanoma | 12 | - |
| Skin melanoma | 4 | - |

**Table M**: A metric of statistical confidence that any individual GAG property (CS, HS or HA levels, individual disaccharide compositions, and charge HS or charge CS) displays a significant and practically appreciable alteration between M and healthy samples was calculated. An alteration was defined as significant and practically appreciable if the difference in means between case and control falls within a so-called Region Of Practical Equivalence in less than 5% of all possible statistical distributions that can fit the data relative to a measurement. Table M shows a summary of the results and shows the mean value for GAG properties as measured in M vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table M also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in Melanoma | ROPE threshold | % in ROPE |
|---|---|---|---|---|
| 0s CS blood | 77.699 | 75.170 | 3.81 | 70.45 |
| 2s CS blood | 0.025 | 0.026 | 0.00 | 17.49 |
| 6s CS blood | 1.201 | 3.291 | 0.12 | 0.14 |
| 4s CS blood | 20.775 | 20.531 | 1.04 | 41.82 |
| 2s6s CS blood | 0.074 | 0.178 | 0.02 | 8.63 |
| 2s4s CS blood | 0.035 | 0.047 | 0.00 | 8.18 |
| 4s6s CS blood | 0.092 | 0.150 | 0.01 | 2.13 |
| Tris CS blood | 0.008 | 0.010 | 0.00 | 18.63 |
| 4s/6s CS blood | 18.281 | 8.641 | 0.66 | 0.01 |
| 6s/0s CS blood | 0.015 | 0.042 | 0.00 | 0.30 |
| 4s/0s CS blood | 0.270 | 0.279 | 0.01 | 31.54 |
| Charge CS blood | 0.225 | 0.255 | 0.01 | 19.01 |
| Total HA blood | 0.142 | 0.151 | 0.02 | 56.68 |
| Total CS blood | 10.171 | 17.566 | 0.72 | 0.10 |
| Tris HS blood | 0.203 | 0.233 | 0.01 | 13.77 |
| Ns6s HS blood | 0.365 | 0.277 | 0.02 | 11.03 |
| Ns2s HS blood | 0.523 | 0.599 | 0.03 | 11.88 |
| Ns HS blood | 28.949 | 33.939 | 1.59 | 14.73 |
| 2s6s HS blood | 0.180 | 0.185 | 0.03 | 31.57 |
| 6s HS blood | 33.410 | 40.470 | 1.87 | 12.32 |
| 2s HS blood | 4.490 | 2.674 | 0.18 | 2.95 |
| 0s HS blood | 30.456 | 20.738 | 1.27 | 2.83 |
| Charge HS blood | 0.721 | 0.811 | 0.04 | 11.39 |
| Total HS blood | 1.872 | 2.882 | 0.12 | 3.13 |

### EXAMPLE 4

In this study we measured circulating GAG levels in subjects presenting with colorectal cancer (CRC).

### *Material and Methods*

### *Plasma sample collection*

Blood samples were obtained from 10 patients with colorectal cancers. All patients had evidence of disease and treatment-naive at the time of sampling.
Whole blood was collected in EDTA tubes and centrifuged to separate the plasma. Plasma was aliquoted into 220 uL vials and vials were frozen at -80°C until they were shipped for analysis in dry ice.
Plasma GAGs correspond to blood GAGs. For the purpose of this example, plasma is therefore also interchangeably referred to as blood.

### *Glycosaminoglycan profile determination*

Sample preparation and the measurement of GAG properties (GAG profile determination) was done as *per* Example 3.

### *Biomarker score design*

A control group was formed using historical GAG profiles obtained from blood samples of 58 healthy individuals. These samples belonged to four distinct historical cohorts. The GAG profile was measured as described in the previous section.
Biomarker score design was as described in Example 3, but concerned colorectal cancer versus healthy groups (instead of melamona versus healthy).
For the purpose of this study and for reasons elsewhere stated, scores computed from blood GAGs regardless if the processed sample was plasma are referred to as GAG blood scores.

### *Accuracy metrics*

We evaluated the performance of the CRC GAG score as in Example 3, but classifying a sample as colorectal cancer or healthy (instead of melanoma or healthy).

### *Results*

We analyzed a total of 10 blood samples from patients with CRC. We quantified the profile of GAGs in the 10 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table O). We further included as controls a group of 58 historical GAG profiles measured in blood samples of healthy individuals (H). Clinical data for this cohort is reported in Table N. We compared the value of each property in the GAG profile in the CRC versus H group. We assessed the statistical significance of changes for each GAG property between the two groups by calculating the highest density interval (HDI) for the mean difference using Bayesian estimation. The results showed marked differences in several GAG properties between CRC and H subjects (Table O).
We condensed changes in the GAG profile in CRC vs. H subjects into GAG scores. We derived one CRC GAG score, based on the following formula using blood measurements:

$$CRC\ GAG\ score = \frac{5}{2}\left(\frac{[6s\ CS]}{[4s\ CS] + [6s\ CS]}\right) + \frac{2}{15}\left(\frac{[2s6s\ CS]}{[2s6s\ CS] + [2s\ CS]}\right) + \frac{1}{300}[6s\ HS]$$

$$+ \frac{1}{30}([2s\ HS] + Tot\ HS)$$

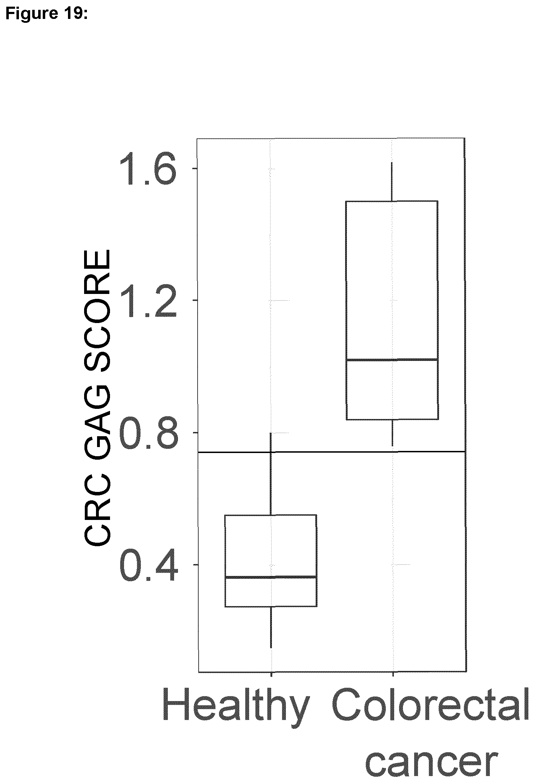

where terms in square brackets represent the fraction (mass fraction) of the disaccharide for the corresponding GAG, and [*Tot HS*] is the total concentration of HS (in μg/mL). We subsequently calculated the score in each sample and observed that CRC samples have recurrently elevated scores with respect to H samples (Figure 19). The performance of the GAG score was evaluated using the receiver-operating-characteristic (ROC) curves, and the area under the curve (AUC) was found to be 0.998. We identified an optimal cut-off equal to 0.74 for this score. Using this cut-off, CRC subjects could be distinguished from healthy individuals with 98.5% accuracy, 100% sensitivity, and 98.2% specificity.

These results indicate that there are measurable differences in circulating GAG levels and/or chemical composition between colorectal cancer as opposed to healthy subjects, and thus that GAG properties may be useful in colorectal cancer screening (e.g. diagnosis). These results further demonstrate that GAG scores can be designed to discriminate between colorectal cancer and healthy subjects, and that their significant AUC permit fine-tuned optimal cut-off scores, in order to maximize sensitivity and/or specificity, depending on the intended diagnostic use.

**Table N**: Clinical data for the cohort analysed in this study. Results are presented as medians (25th, 75th percentile) or percent or counts. Missing values were omitted.

| Baseline characteristics | Colorectal cancer | Healthy |
|---|---|---|
| Number of samples | 10 | 58 |
| Age [years] | 74 (70 - 78) | 56 (47 - 63) |
| Female | 50% | 65.5% |
| TNM pathologic stage T | | |
| T2 | 1 | - |
| T3 | 4 | - |
| T4 | 2 | - |
| Not Available | 3 | - |
| TNM pathologic stage N | | |
| N0 | 3 | - |
| N1 | 3 | - |
| N2 | 3 | - |
| Not Available | 1 | - |
| TNM pathologic stage M | | |
| M0 | 7 | - |
| M1 | 2 | - |
| Not Available | 1 | - |
| TNM stage | | |
| I-III | 7 | |
| IV | 3 | |

**Table O**: A metric of statistical confidence that any individual GAG property (CS, HS or HA levels, individual disaccharide compositions, and charge HS or charge CS) displays a significant and practically appreciable alteration between CRC and healthy samples was calculated. An alteration was defined as significant and practically appreciable if the difference in means between case and control falls within a so-called Region Of Practical Equivalence in less than 5% of all possible statistical distributions that can fit the data relative to a measurement. Table O shows a summary of the results and shows the mean value for GAG properties as measured in CRC vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table O also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in Colorectal cancer | ROPE threshold | % in ROPE |
|---|---|---|---|---|
| 0s CS blood | 79.184 | 74.490 | 3.7483 | 40.44 |

(continued)

| GAG property | Mean in Healthy Individuals | Mean in Colorectal cancer | ROPE threshold | % in ROPE |
|---|---|---|---|---|
| 2s CS blood | 0.022 | 0.072 | 0.0056 | **0.04** |
| 6s CS blood | 1.079 | 4.019 | 0.1137 | **1.35** |
| 4s CS blood | 19.577 | 21.535 | 1.1014 | 25.81 |
| 2s6s CS blood | 0.060 | 0.268 | 0.0152 | **0.15** |
| 2s4s CS blood | 0.020 | 0.068 | 0.0048 | **0.01** |
| 4s6s CS blood | 0.060 | 0.093 | 0.0068 | **4.36** |
| Tris CS blood | 0.008 | 0.025 | 0.0041 | **1.59** |
| 4s/6s CS blood | 36.456 | 9.332 | 1.3002 | **0.00** |
| 6s/0s CS blood | 0.013 | 0.033 | 0.0018 | **2.35** |
| 4s/0s CS blood | 0.237 | 0.283 | 0.0156 | 17.51 |
| Charge CS blood | 0.199 | 0.269 | 0.0123 | 5.22 |
| Total HA blood | 0.178 | 0.132 | 0.0081 | 6.13 |
| Total CS blood | 5.444 | 8.719 | 0.3199 | **2.35** |
| Tris HS blood | 0.314 | 0.187 | 0.0327 | 9.26 |
| Ns6s HS blood | 0.965 | 0.324 | 0.0533 | **0.06** |
| Ns2s HS blood | 0.751 | 0.284 | 0.0566 | **0.05** |
| Ns HS blood | 18.970 | 16.824 | 1.1047 | 17.89 |
| 2s6s HS blood | 0.486 | 0.215 | 0.0448 | **2.03** |
| 6s HS blood | 7.147 | 28.052 | 1.0077 | **0.37** |
| 2s HS blood | 4.444 | 16.120 | 0.3736 | **0.09** |
| 0s HS blood | 58.306 | 33.676 | 2.3275 | **0.43** |
| Charge HS blood | 0.478 | 0.682 | 0.0289 | **1.79** |
| Total HS blood | 0.225 | 1.204 | 0.0509 | **0.10** |

## EXAMPLE 5

In this study we measured circulating GAG levels in subjects presenting with gastrointestinal neuroendocrine tumors (GNET).

### Material and Methods

### Plasma sample collection

Blood samples were obtained from 10 patients with a gastrointestinal neuroendocrine tumor. All patients had evidence of disease and treatment-naive at the time of sampling.

Whole blood was collected in EDTA tubes and centrifuged to separate the plasma. Plasma was aliquoted into 220 uL vials and vials were frozen at -80°C until they were shipped for analysis in dry ice.

Plasma GAGs correspond to blood GAGs. For the purpose of this example, plasma is therefore also interchangeably referred to as blood.

### Glycosaminoglycan profile determination

Sample preparation and the measurement of GAG properties (GAG profile determination) was done as *per* Example 3.

*Biomarker score design*

A control group was formed using historical GAG profiles obtained from blood samples of 58 healthy individuals. These samples belonged to four distinct historical cohorts. The GAG profile was measured as described in the previous section.

Biomarker score design was as described in Example 3, but concerned gastrointestinal neuroendocrine tumor versus healthy groups (instead of melanoma versus healthy).

For the purpose of this study and for reasons elsewhere stated, scores computed from blood GAGs regardless if the processed sample was plasma are referred to as GAG blood scores.

*Accuracy metrics*

We evaluated the performance of the GNET GAG score as in Example 3, but classifying a sample as GNET or healthy (instead of melanoma or healthy).

*Results*

We analyzed a total of 10 blood samples from patients with GNET. We quantified the profile of GAGs in the 10 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table Q). We further included as controls a group of 58 historical GAG profiles measured in blood samples of healthy individuals (H). Clinical data for this cohort is reported in Table P. We compared the value of each property in the GAG profile in the GNET versus H group. We assessed the statistical significance of changes for each GAG property between the two groups by calculating the highest density interval (HDI) for the mean difference using Bayesian estimation. The results showed marked differences in several GAG properties between GNET and H subjects (Table Q).

We condensed changes in the GAG profile in GNET vs. H subjects into GAG scores. We derived one GNET GAG score, based on the following formula using blood measurements:

$$GNET\ GAG\ score = \frac{3}{4}[2s6s\ CS] - \frac{3}{20}[Tris\ CS] + 2\ Charge\ CS + \frac{1}{30}[2s\ HS] - \frac{1}{300}[0s\ HS]$$

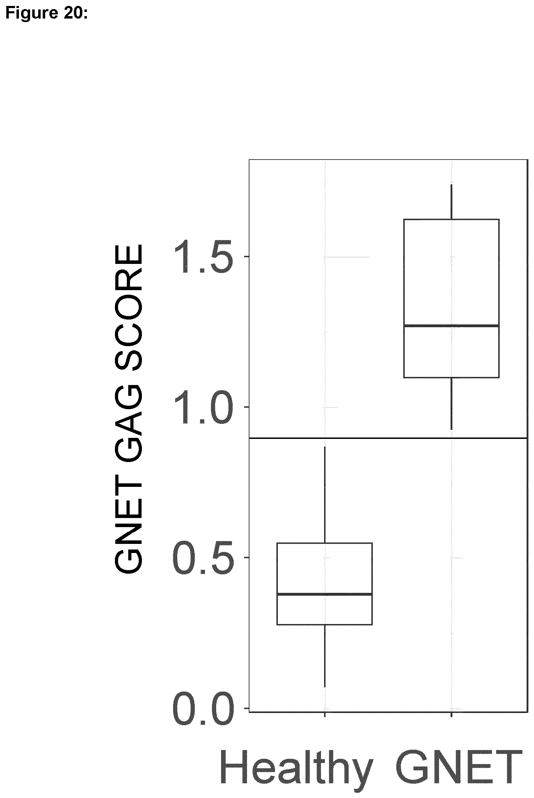

where terms in square brackets represent the fraction (mass fraction) of the disaccharide for the corresponding GAG, and *Charge CS* is the weighted charge of CS. We subsequently calculated the score in each sample and observed that GNET samples have recurrently elevated scores with respect to H samples (Figure 20). The performance of the GAG score was evaluated using the receiver-operating-characteristic (ROC) curves, and the area under the curve (AUC) was found to be 1 (perfect classifier). We identified an optimal cut-off equal to 0.90 for this score. Using this cut-off, GNET subjects could be distinguished from healthy individuals with 100% accuracy, 100% sensitivity, and 100% specificity.

These results indicate that there are measurable differences in circulating GAG levels and/or chemical composition between gastrointestinal neuroendocrine tumors as opposed to healthy subjects, and thus that GAG properties may be useful in gastrointestinal neuroendocrine tumour screening (e.g. diagnosis). These results further demonstrate that GAG scores can be designed to discriminate between gastrointestinal neuroendocrine tumors and healthy subjects, and that their significant AUC permit fine-tuned optimal cut-off scores, in order to maximize sensitivity and/or specificity, depending on the intended diagnostic use.

**Table P**: Clinical data for the cohort analysed in this study. Results are presented as medians (25th, 75th percentile) or percent or counts. Missing values were omitted.

| Baseline characteristics | GNET | Healthy |
|---|---|---|
| Number of samples | 10 | 58 |
| Age [years] | 58 (49 - 71) | 56 (47 - 63) |
| Female | 70% | 65.5% |
| ENETS Grade | | |
| G1 | 7 | |
| G2 | 3 | |

**Table Q**: A metric of statistical confidence that any individual GAG property (CS, HS or HA levels, individual disaccharide compositions, and charge HS or charge CS) displays a significant and practically appreciable alteration between GNET and healthy samples was calculated. An alteration was defined as significant and practically appreciable if the difference in means between case and control falls within a so-called Region Of Practical Equivalence in less than 5% of all possible statistical distributions that can fit the data relative to a measurement. Table Q shows a summary of the results and shows the mean value for GAG properties as measured in GNET vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table Q also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in GNET | ROPE threshold | % in ROPE |
|---|---|---|---|---|
| 0s CS blood | 79.184 | 66.570 | 3.7483 | **0.580** |
| 2s CS blood | 0.022 | 0.043 | 0.0056 | 5.740 |
| 6s CS blood | 1.079 | 3.577 | 0.1137 | **0.060** |
| 4s CS blood | 19.577 | 28.872 | 1.1014 | **0.453** |
| 2s6s CS blood | 0.060 | 0.541 | 0.0152 | **0.013** |
| 2s4s CS blood | 0.020 | 0.087 | 0.0048 | **0.007** |
| 4s6s CS blood | 0.060 | 0.202 | 0.0068 | **0.367** |
| Tris CS blood | 0.008 | 0.027 | 0.0041 | **0.907** |
| 4s/6s CS blood | 36.456 | 9.511 | 1.3002 | **0.000** |
| 6s/0s CS blood | 0.013 | 0.050 | 0.0018 | **0.060** |
| 4s/0s CS blood | 0.237 | 0.433 | 0.0156 | **0.387** |
| Charge CS blood | 0.199 | 0.345 | 0.0123 | **0.020** |
| Total HA blood | 0.178 | 0.146 | 0.0081 | 11.547 |
| Total CS blood | 5.444 | 6.139 | 0.3199 | 19.593 |
| Tris HS blood | 0.314 | 0.299 | 0.0327 | 17.340 |
| Ns6s HS blood | 0.965 | 0.412 | 0.0533 | **0.480** |
| Ns2s HS blood | 0.751 | 0.549 | 0.0566 | 12.900 |
| Ns HS blood | 18.970 | 27.823 | 1.1047 | **3.247** |
| 2s6s HS blood | 0.486 | 0.263 | 0.0448 | 5.327 |
| 6s HS blood | 7.147 | 33.651 | 1.0077 | **0.020** |
| 2s HS blood | 4.444 | 9.908 | 0.3736 | **0.940** |
| 0s HS blood | 58.306 | 26.747 | 2.3275 | **0.000** |
| Charge HS blood | 0.478 | 0.755 | 0.0289 | **0.053** |
| Total HS blood | 0.225 | 1.376 | 0.0509 | **0.033** |

## EXAMPLE 6

In this study we measured circulating GAG levels in subjects presenting a blood cancer, more specifically with chronic lymphoid leukemia (CLL).

## *Material and Methods*

*Plasma sample collection*

Blood samples were obtained from 10 patients with chronic lymphoid leukemia. All patients had evidence of disease and treatment-naive at the time of sampling.

Whole blood was collected in EDTA tubes and centrifuged to separate the plasma. Plasma was aliquoted into 220 uL vials and vials were frozen at -80°C until they were shipped for analysis in dry ice.

Plasma GAGs correspond to blood GAGs. For the purpose of this example, plasma is therefore also interchangeably referred to as blood.

*Glycosaminoglycan profile determination*

Sample preparation and the measurement of GAG properties (GAG profile determination) was done as *per* Example 3.

*Biomarker score design*

A control group was formed using historical GAG profiles obtained from blood samples of 58 healthy individuals. These samples belonged to four distinct historical cohorts. The GAG profile was measured as described in the previous section.

Biomarker score design was as described in Example 3, but concerned CLL versus healthy groups (instead of melanoma versus healthy).

For the purpose of this study and for reasons elsewhere stated, scores computed from blood GAGs regardless if the processed sample was plasma are referred to as GAG blood scores.

*Accuracy metrics*

We evaluated the performance of the CLL GAG scores as in Example 3, but classifying a sample as CLL or healthy (instead of melanoma or healthy).

*Results*

We analyzed a total of 10 blood samples from patients with CLL. We quantified the profile of GAGs in the 10 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table S). We further included as controls a group of 58 historical GAG profiles measured in blood samples of healthy individuals (H). Clinical data for this cohort is reported in Table R. We compared the value of each property in the GAG profile in the CLL versus H group. We assessed the statistical significance of changes for each GAG property between the two groups by calculating the highest density interval (HDI) for the mean difference using Bayesian estimation. The results showed marked differences in several GAG properties between CLL and H subjects (Table S).

We condensed changes in the GAG profile in CLL vs. H subjects into GAG scores. We derived one CLL GAG score (CLL GAG score #1), based on the following formula using blood measurements:

$$CLL\ GAG\ score = \frac{2}{15}[6s\ CS] + \frac{2}{3}[2s6s\ CS] - [4s6s\ CS] + \frac{1}{2}Charge\ CS - \frac{1}{150}[6s\ HS]$$
$$- \frac{1}{250}[0s\ HS] + \frac{2}{15}Tot\ HS$$

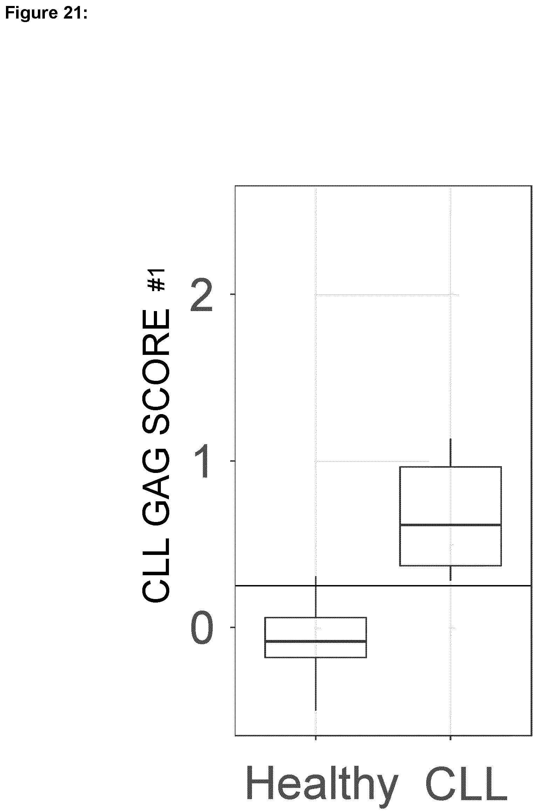

where terms in square brackets represent the fraction (mass fraction) of the disaccharide for the corresponding GAG, and *Tot HS* is the total concentration of HS (in μg/mL). We subsequently calculated the score in each sample and observed that CLL samples have recurrently elevated scores with respect to H samples (Figure 21). The performance of the GAG score was evaluated using the receiver-operating-characteristic (ROC) curves, and the area under the curve (AUC) was found to be 0.974. We identified an optimal cut-off equal to 0.25 for this score. Using this cut-off, CLL subjects could be distinguished from healthy individuals with 95.6% accuracy, 100% sensitivity, and 94.8% specificity.

We also investigated whether variations of the above GAG score would also be effective in distinguishing CLL from H subjects. To this end, we derived an alternative CLL GAG score (CLL GAG score #2) using only CS properties:

$$CLL\ GAG\ score = \frac{1}{12}[6s\ CS] + \frac{4}{7}[2s6s\ CS] - [4s6s\ CS] - \frac{1}{800}\left(\frac{[4s\ CS]}{[6s\ CS]}\right)$$

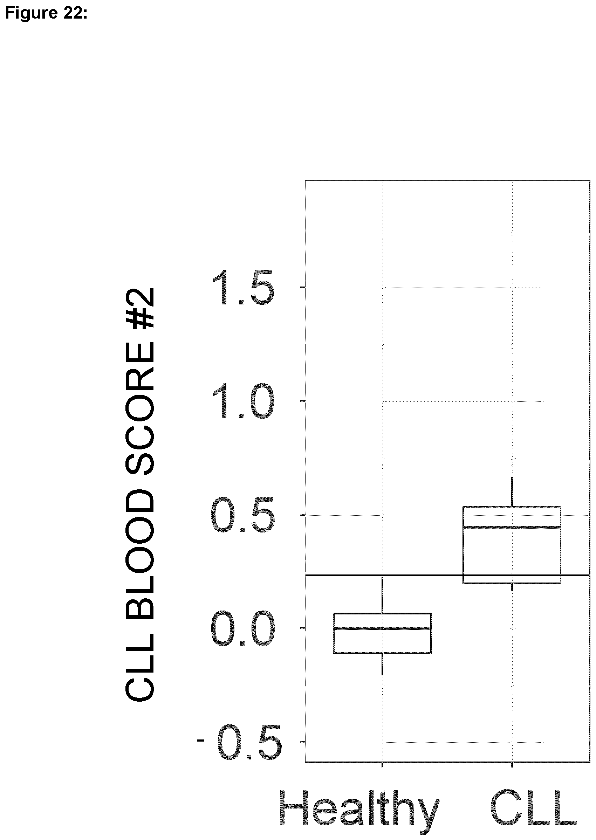

where terms in square brackets represent the fraction (mass fraction) of the disaccharide for the corresponding GAG. Using this alternative scoring system, CLL samples had recurrently higher scores than H samples (Figure 22). The AUC was found to be 0.974. We identified an optimal cut-off equal to 0.23 for this score. Using this cut-off, CLL subjects could be distinguished from healthy individuals with 95.6% accuracy, 70% sensitivity, and 100% specificity.

These results indicate that there are measurable differences in circulating GAG levels and/or chemical composition between CLL as opposed to healthy subjects, and thus that GAG properties may be useful in CLL screening (e.g. diagnosis). These results further demonstrate that GAG scores can be designed to discriminate between chronic lymphoid leukemia and healthy subjects, and that their significant AUC permit fine-tuned optimal cut-off scores, in order to maximize sensitivity and/or specificity, depending on the intended diagnostic use.

**Table R**: Clinical data for the cohort analysed in this study. Results are presented as medians (25th, 75th percentile) or percent or counts. Missing values were omitted.

| Baseline characteristics | | CLL | Healthy |
|---|---|---|---|
| Number of samples | | 10 | 58 |
| Age [years] | | 70 (65 - 73) | 56 (47 - 63) |
| Female | | 40% | 65.5% |
| Binet stage | | | |
| | A+B | 7 | |
| | C | 3 | |

**Table S**: A metric of statistical confidence that any individual GAG property (CS, HS or HA levels, individual disaccharide compositions, and charge HS or charge CS) displays a significant and practically appreciable alteration between CLL and healthy samples was calculated. An alteration was defined as significant and practically appreciable if the difference in means between case and control falls within a so-called Region Of Practical Equivalence in less than 5% of all possible statistical distributions that can fit the data relative to a measurement. Table S shows a summary of the results and shows the mean value for GAG properties as measured in CLL vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table S also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in CLL | ROPE threshold | % in ROPE |
|---|---|---|---|---|
| 0s CS blood | 79.184 | 69.171 | 3.7483 | **4.967** |
| 2s CS blood | 0.022 | 0.033 | 0.0056 | 23.153 |
| 6s CS blood | 1.079 | 4.101 | 0.1137 | **0.187** |
| 4s CS blood | 19.577 | 25.599 | 1.1014 | **3.207** |
| 2s6s CS blood | 0.060 | 0.520 | 0.0152 | **0.060** |
| 2s4s CS blood | 0.020 | 0.064 | 0.0048 | **0.013** |
| 4s6s CS blood | 0.060 | 0.206 | 0.0068 | **0.553** |
| Tris CS blood | 0.008 | 0.010 | 0.0041 | 80.113 |
| 4s/6s CS blood | 36.456 | 8.101 | 1.3002 | **0.000** |
| 6s/0s CS blood | 0.013 | 0.057 | 0.0018 | **0.147** |
| 4s/0s CS blood | 0.237 | 0.373 | 0.0156 | **1.960** |
| Charge CS blood | 0.199 | 0.319 | 0.0123 | **0.353** |

(continued)

| GAG property | Mean in Healthy Individuals | Mean in CLL | ROPE threshold | % in ROPE |
|---|---|---|---|---|
| Total HA blood | 0.178 | 0.139 | 0.0081 | 6.187 |
| Total CS blood | 5.444 | 7.733 | 0.3199 | **2.267** |
| Tris HS blood | 0.314 | 0.286 | 0.0327 | 24.720 |
| Ns6s HS blood | 0.965 | 0.461 | 0.0533 | **1.193** |
| Ns2s HS blood | 0.751 | 0.581 | 0.0566 | 15.807 |
| Ns HS blood | 18.970 | 31.397 | 1.1047 | **0.620** |
| 2s6s HS blood | 0.486 | 0.191 | 0.0448 | **0.933** |
| 6s HS blood | 7.147 | 25.152 | 1.0077 | **0.253** |
| 2s HS blood | 4.444 | 8.734 | 0.3736 | **2.033** |
| 0s HS blood | 58.306 | 28.838 | 2.3275 | **0.033** |
| Charge HS blood | 0.478 | 0.730 | 0.0289 | **0.087** |
| Total HS blood | 0.225 | 1.307 | 0.0509 | **0.007** |

## EXAMPLE 7

In this study we measured circulating GAG levels in subjects presenting with bladder cancer (BCa).

### *Material and Methods*

### *Urine sample collection*

Urine samples were obtained from 6 patients with bladder cancer. All patients had evidence of disease and treatment-naive at the time of sampling.

Urine was collected in polypropylene tubes and frozen at -80°C until they were shipped for analysis in dry ice.

### *Glycosaminoglycan profile determination*

Sample preparation and the measurement of GAG properties (GAG profile determination) was done as *per* Example 3, but on urine samples instead of blood samples.

### *Biomarker score design*

A control group was formed using historical GAG profiles obtained from urine samples of 25 healthy individuals. These samples belonged to two distinct historical cohorts. The GAG profile was measured as described in the previous section.

Biomarker design was as described in Example 3, but concerned bladder cancer versus healthy groups (instead of melanoma versus healthy).

### *Accuracy metrics*

We evaluated the performance of the BCa (bladder cancer) GAG score as in Example 3, but classifying a sample as bladder cancer or healthy (instead of melanoma or healthy).

### *Results*

We analyzed a total of 6 urine samples from patients with BCa. We quantified the profile of GAGs in the 6 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table T). We further included as controls a group of 25 historical GAG profiles measured in urine samples of healthy individuals (H). We compared the value of each property in the GAG profile in the BCa versus H group. We assessed the statistical significance of changes for each GAG property between the two groups by calculating the highest density interval (HDI) for the mean difference using Bayesian estimation. The results showed marked differences in several GAG properties between BCa and H subjects (Table T).

We condensed changes in the GAG profile in BCa vs. H subjects into GAG scores. We derived one BCa GAG score, based on the following formula using urine measurements:

$$BCa\ GAG\ score = \frac{3}{4}Charge\ CS + \frac{1}{3}[Tris\ HS] - \frac{1}{50}[Ns\ HS] + \frac{1}{250}[2s\ HS] + \frac{3}{4}Tot\ HS$$

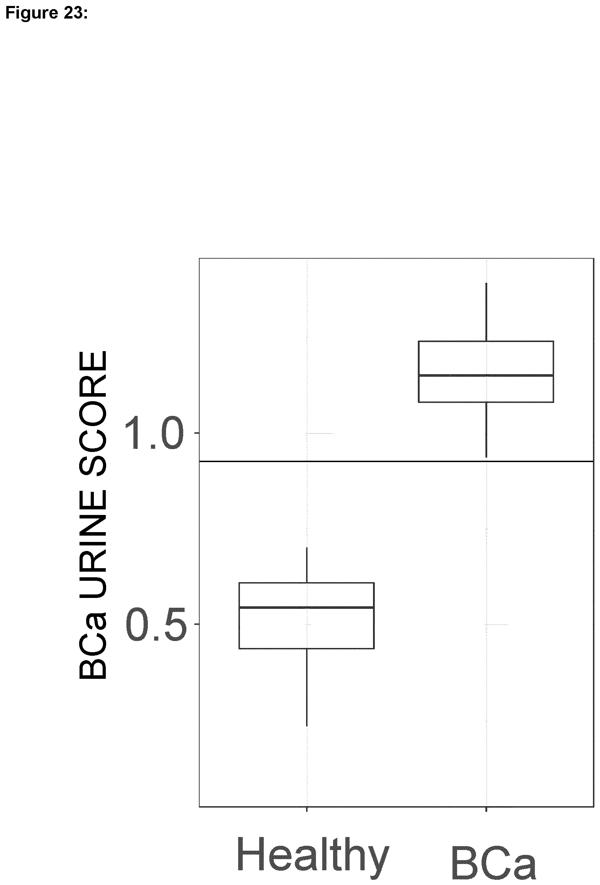

where terms in brackets represent the fraction (mass fraction) of the disaccharide for the corresponding GAG, *Charge CS* is the weighted charged of CS, and *Tot HS* is the total concentration of HS (in $\mu$g/mL). We subsequently calculated the score in each sample and observed that BCa samples have recurrently elevated scores with respect to H samples (Figure 23). The performance of the GAG score was evaluated using the receiver-operating-characteristic (ROC) curves, and the area under the curve (AUC) was found to be 1.0. We identified an optimal cut-off equal to 0.92 for this score. Using this cut-off, BCa subjects could be distinguished from healthy individuals with 100% accuracy, 100% sensitivity, and 100% specificity.

These results indicate that there are measurable differences in GAG levels and/or chemical composition between bladder cancer as opposed to healthy subjects, and thus that GAG properties may be useful in bladder cancer screening (e.g. diagnosis).

These results further demonstrate that GAG scores can be designed to discriminate between bladder cancer and healthy subjects, and that their significant AUC permit fine-tuned optimal cut-off scores, in order to maximize sensitivity and/or specificity, depending on the intended diagnostic use.

**Table T**: A metric of statistical confidence that any individual GAG property (CS, HS or HA levels, individual disaccharide compositions, and charge HS or charge CS) displays a significant and practically appreciable alteration between BCa and healthy samples was calculated. An alteration was defined as significant and practically appreciable if the difference in means between case and control falls within a so-called Region Of Practical Equivalence in less than 5% of all possible statistical distributions that can fit the data relative to a measurement. Table T shows a summary of the results and shows the mean value for GAG properties as measured in BCa vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table T also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in Bladder cancer | ROPE threshold | % in ROPE |
|---|---|---|---|---|
| 0s CS urine | 34.963 | 24.071 | 1.645 | **2.558** |
| 2s CS urine | 0.076 | 0.120 | 0.005 | **0.175** |
| 6s CS urine | 21.566 | 26.563 | 1.129 | **3.992** |
| 4s CS urine | 40.519 | 47.111 | 2.104 | 9.483 |
| 2s6s CS urine | 0.604 | 0.658 | 0.031 | 18.442 |
| 2s4s CS urine | 0.458 | 0.566 | 0.024 | 10.242 |
| 4s6s CS urine | 1.184 | 1.087 | 0.058 | 19.533 |
| Tris CS urine | 0.039 | 0.015 | 0.003 | **2.017** |
| 4s/6s CS urine | 2.006 | 1.806 | 0.099 | 23.075 |
| 6s/0s CS urine | 0.750 | 1.199 | 0.042 | **1.708** |
| 4s/0s CS urine | 1.307 | 2.160 | 0.079 | **2.417** |
| Charge CS urine | 0.653 | 0.782 | 0.034 | **2.992** |
| Total HA urine | 0.070 | 0.054 | 0.003 | 9.417 |
| Total CS urine | 0.927 | 0.690 | 0.047 | 8.283 |

(continued)

| GAG property | Mean in Healthy Individuals | Mean in Bladder cancer | ROPE threshold | % in ROPE |
|---|---|---|---|---|
| Tris HS urine | 0.421 | 0.908 | 0.026 | **0.083** |
| Ns6s HS urine | 1.195 | 0.995 | 0.077 | 8.325 |
| Ns2s HS urine | 1.395 | 2.416 | 0.083 | **2.917** |
| Ns HS urine | 12.366 | 8.003 | 0.588 | **4.692** |
| 2s6s HS urine | 1.041 | 0.831 | 0.056 | 12.725 |
| 6s HS urine | 6.880 | 6.158 | 0.354 | 13.250 |
| 2s HS urine | 3.557 | 9.911 | 0.269 | **2.000** |
| 0s HS urine | 71.768 | 66.827 | 3.528 | 31.158 |
| Charge HS urine | 0.334 | 0.377 | 0.017 | 16.833 |
| Total HS urine | 0.149 | 0.496 | 0.011 | **0.108** |

## EXAMPLE 8

In this study we measured circulating GAG levels in subjects presenting with breast cancer (BC).

### *Material and Methods*

### *Plasma sample collection*

Blood samples were obtained from 10 patients with breast cancer. All patients had evidence of disease and treatment-naive at the time of sampling.

Whole blood tubes were collected in EDTA tubes and centrifuged to separate the plasma. Plasma was aliquoted into 220 uL vials and vials were frozen at -80°C until they were shipped for analysis in dry ice.

Plasma GAGs correspond to blood GAGs. For the purpose of this example, plasma is therefore also interchangeably referred to as blood.

### *Glycosaminoglycan profile determination*

Sample preparation and the measurement of GAG properties (GAG profile determination) was done as *per* Example 3.

### *Biomarker score design*

A control group was formed using historical GAG profiles obtained from blood samples of 58 healthy individuals. These samples belonged to four distinct historical cohorts. The GAG profile was measured as described in the previous section.

Biomarker score design was as described in Example 3, but concerned breast cancer versus healthy groups (instead of melanoma versus healthy).

For the purpose of this study and for reasons elsewhere stated, scores computed from blood GAGs regardless if the processed sample was plasma are referred to as GAG blood scores.

### *Accuracy metrics*

We evaluated the performance of the BC GAG score as in Example 3, but classifying a sample as breast cancer or healthy (instead of melanoma or healthy).

### *Results*

We analyzed a total of 10 blood samples from patients with BC. We quantified the profile of GAGs in the 10 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table V). We further included as controls a group of 58 historical GAG profiles measured in blood samples of healthy individuals (H). Clinical data for this cohort is reported in Table U. We compared the value of each property in the GAG profile in the BC versus H group. We assessed the statistical significance of changes for each GAG property between the two groups by calculating the highest density interval (HDI) for the mean difference using Bayesian estimation. The results showed marked differences in several GAG properties between BC and H subjects (Table V).

We condensed changes in the GAG profile in BC vs. H subjects into GAG scores. We derived one BC GAG score, based on the following formula using blood measurements:

$$BC\ GAG\ score = 5\left(\frac{[6s\ CS]}{[0s\ CS]}\right) + \frac{5}{3} Charge\ CS + \frac{1}{50} Tot\ CS$$

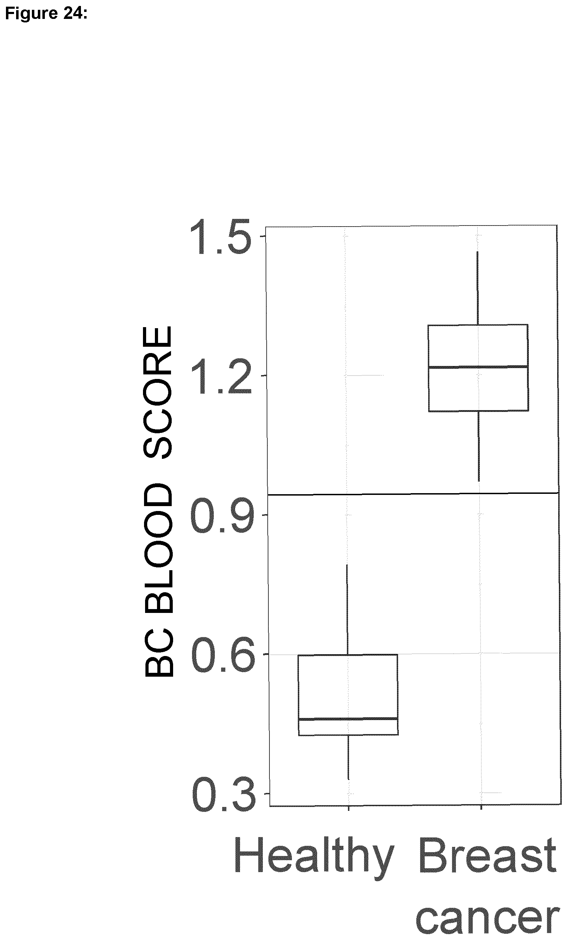

where terms in square brackets represent the fraction (mass fraction) of the disaccharide for the corresponding GAG, *Charge CS* is the weighted charge of CS, and *Tot CS* is the total concentration of CS (in $\mu$g/mL). We subsequently calculated the score in each sample and observed that BC samples have recurrently elevated scores with respect to H samples (Figure 24). The performance of the GAG score was evaluated using the receiver-operating-characteristic (ROC) curves, and the area under the curve (AUC) was found to be 1 (perfect classifier). We identified an optimal cut-off equal to 0.94 for this score. Using this cut-off, BC subjects could be distinguished from healthy individuals with 100% accuracy, 100% sensitivity, and 100% specificity.

These results indicate that there are measurable differences in circulating GAG levels and/or chemical composition between breast cancer as opposed to healthy subjects, and thus that GAG properties may be useful in breast cancer screening (e.g. diagnosis). These results further demonstrate that GAG scores can be designed to discriminate between breast cancer and healthy subjects, and that their significant AUC permit fine-tuned optimal cut-off scores, in order to maximize sensitivity and/or specificity, depending on the intended diagnostic use.

**Table U**: Clinical data for the cohort analysed in this study. Results are presented as medians (25th, 75th percentile) or percent or counts. Missing values were omitted.

| Baseline characteristics | BC | Healthy |
|---|---|---|
| Number of samples | 10 | 58 |
| Age [years] | 52 (47 - 63) | 56 (47 - 63) |
| Female | 100% | 65.5% |

**Table V**: A metric of statistical confidence that any individual GAG property (CS, HS or HA levels, individual disaccharide compositions, and charge HS or charge CS) displays a significant and practically appreciable alteration between BC and healthy samples was calculated. An alteration was defined as significant and practically appreciable if the difference in means between case and control falls within a so-called Region Of Practical Equivalence in less than 5% of all possible statistical distributions that can fit the data relative to a measurement. Table V shows a summary of the results and shows the mean value for GAG properties as measured in BC vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table V also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in BC | ROPE threshold | % in ROPE |
|---|---|---|---|---|
| 0s CS blood | 79.184 | 62.447 | 3.387 | **0.000** |
| 2s CS blood | 0.022 | 0.041 | 0.004 | 8.073 |
| 6s CS blood | 1.079 | 3.339 | 0.181 | **0.000** |
| 4s CS blood | 19.577 | 33.352 | 1.397 | **0.007** |
| 2s6s CS blood | 0.060 | 0.037 | 0.012 | 26.493 |
| 2s4s CS blood | 0.020 | 0.046 | 0.006 | 6.193 |
| 4s6s CS blood | 0.060 | 0.135 | 0.008 | **3.060** |

(continued)

| GAG property | Mean in Healthy Individuals | Mean in BC | ROPE threshold | % in ROPE |
|---|---|---|---|---|
| Tris CS blood | 0.008 | 0.018 | 0.004 | 8.760 |
| 4s/6s CS blood | 36.456 | 10.086 | 0.885 | 0.000 |
| 6s/0s CS blood | 0.013 | 0.054 | 0.003 | **0.000** |
| 4s/0s CS blood | 0.237 | 0.542 | 0.024 | **0.007** |
| Charge CS blood | 0.199 | 0.378 | 0.016 | **0.000** |
| Total HA blood | 0.178 | 0.131 | 0.010 | 7.280 |
| Total CS blood | 5.444 | 16.779 | 0.495 | **0.000** |
| Tris HS blood | 0.314 | 0.315 | 0.032 | 20.673 |
| Ns6s HS blood | 0.965 | 0.552 | 0.058 | 7.633 |
| Ns2s HS blood | 0.751 | 0.396 | 0.061 | **2.440** |
| Ns HS blood | 18.970 | 28.124 | 1.135 | **4.313** |
| 2s6s HS blood | 0.486 | 0.290 | 0.045 | 9.793 |
| 6s HS blood | 7.147 | 28.369 | 1.067 | **0.213** |
| 2s HS blood | 4.444 | 4.040 | 0.329 | 19.280 |
| 0s HS blood | 58.306 | 38.908 | 2.274 | **2.133** |
| Charge HS blood | 0.478 | 0.645 | 0.030 | 5.100 |
| Total HS blood | 0.225 | 1.491 | 0.061 | **0.053** |

### EXAMPLE 9

In this study we measured circulating GAG levels in subjects presenting with ovarian cancer (OV).

#### *Material and Methods*

#### *Plasma sample collection*

Blood samples were obtained from 9 patients with ovarian cancer. All patients had evidence of disease and treatment-naive at the time of sampling.

Whole blood tubes were collected in EDTA tubes and centrifuged to separate the plasma. Plasma was aliquoted into 220 uL vials and vials were frozen at -80°C until they were shipped for analysis in dry ice.

Plasma GAGs correspond to blood GAGs. For the purpose of this example, plasma is therefore also interchangeably referred to as blood.

#### *Glycosaminoglycan profile determination*

Sample preparation and the measurement of GAG properties (GAG profile determination) was done as *per* Example 3.

#### *Biomarker score design*

A control group was formed using historical GAG profiles obtained from blood samples of 58 healthy individuals. These samples belonged to four distinct historical cohorts. The GAG profile was measured as described in the previous section.

Biomarker score design was as described in Example 3, but concerned ovarian cancer versus healthy groups (instead of melanoma versus healthy).

For the purpose of this study and for reasons elsewhere stated, scores computed from blood GAGs regardless if the processed sample was plasma are referred to as GAG blood scores.

*Accuracy metrics*

We evaluated the performance of the OV GAG score as in Example 3, but classifying a sample as ovarian cancer or healthy (instead of melanoma or healthy).

*Results*

We analyzed a total of 9 blood samples from patients with OV. We quantified the profile of GAGs in the 9 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table X). We further included as controls a group of 58 historical GAG profiles measured in blood samples of healthy individuals (H). Clinical data for this cohort is reported in Table W. We compared the value of each property in the GAG profile in the OV versus H group. We assessed the statistical significance of changes for each GAG property between the two groups by calculating the highest density interval (HDI) for the mean difference using Bayesian estimation. The results showed marked differences in several GAG properties between OV and H subjects (Table X).

We condensed changes in the GAG profile in OV vs. H subjects into GAG scores. We derived one OV GAG score, based on the following formula using blood measurements:

$$OV\ GAG\ score = 2\ Charge\ CS + \frac{1}{50}(Tot\ CS - TotHS) + \frac{1}{2}Tot\ HA$$

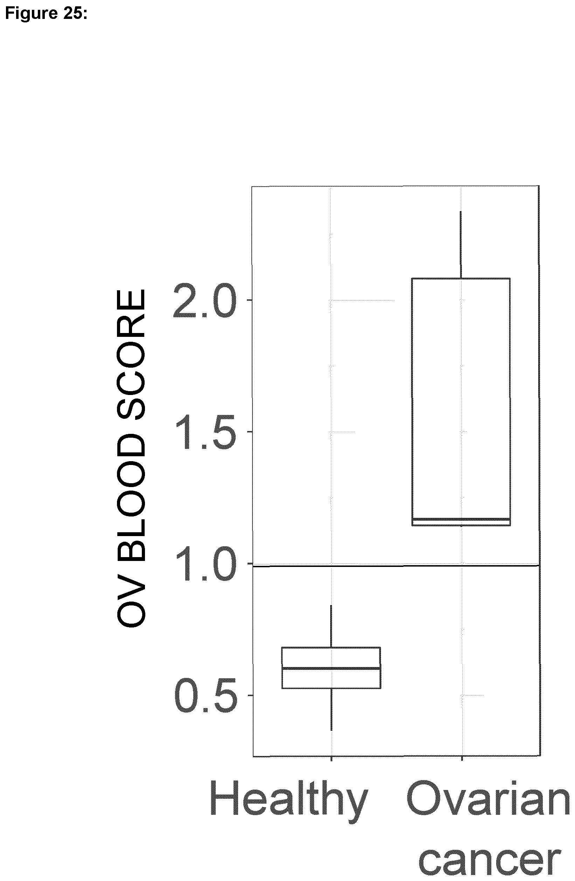

where *Charge CS* is the weighted charge of *CS*, *Tot* CS is the total concentration of CS (in $\mu$g/mL), *Tot HS* is the total concentration of HS (in $\mu$g/mL), *Tot HA* is the total concentration of HA (in $\mu$g/mL). We subsequently calculated the score in each sample and observed that OV samples have recurrently elevated scores with respect to H samples (Figure 25). The performance of the GAG score was evaluated using the receiver-operating-characteristic (ROC) curves, and the area under the curve (AUC) was found to be 1 (perfect classifier). We identified an optimal cut-off equal to 0.99 for this score. Using this cut-off, OV subjects could be distinguished from healthy individuals with 100% accuracy, 100% sensitivity, and 100% specificity.

These results indicate that there are measurable differences in circulating GAG levels and/or chemical composition between ovarian cancer as opposed to healthy subjects, and thus that GAG properties may be useful in ovarian cancer screening (e.g. diagnosis). These results further demonstrate that GAG scores can be designed to discriminate between ovarian cancer and healthy subjects, and that their significant AUC permit fine-tuned optimal cut-off scores, in order to maximize sensitivity and/or specificity, depending on the intended diagnostic use.

**Table W**: Clinical data for the cohort analysed in this study. Results are presented as medians (25th, 75th percentile) or percent or counts. Missing values were omitted.

| Baseline characteristics | OV | Healthy |
|---|---|---|
| Number of samples | 9 | 58 |
| Age [years] | 60 (54 - 65) | 56 (47 - 63) |
| Female | 100% | 65.5% |
| FIGO stage | | |
| I-II | 5 | |
| III-IV | 4 | |

**Table X**: A metric of statistical confidence that any individual GAG property (CS, HS or HA levels, individual disaccharide compositions, and charge HS or charge CS) displays a significant and practically appreciable alteration between OV and healthy samples was calculated. An alteration was defined as significant and practically appreciable if the difference in means between case and control falls within a so-called Region Of Practical Equivalence in less than 5% of all possible statistical distributions that can fit the data relative to a measurement. Table X shows a summary of the results and shows the mean value for GAG properties as measured in OV vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table X also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in OV | ROPE threshold | % in ROPE |
|---|---|---|---|---|
| 0s CS blood | 79.184 | 55.951 | 3.387 | **0.007** |
| 2s CS blood | 0.022 | 0.025 | 0.004 | 33.667 |
| 6s CS blood | 1.079 | 5.149 | 0.181 | **0.013** |
| 4s CS blood | 19.577 | 38.740 | 1.397 | **0.000** |
| 2s6s CS blood | 0.060 | 0.102 | 0.012 | **4.427** |
| 2s4s CS blood | 0.020 | 0.089 | 0.006 | **0.493** |
| 4s6s CS blood | 0.060 | 0.124 | 0.008 | **0.573** |
| Tris CS blood | 0.008 | 0.034 | 0.004 | 5.333 |
| 4s/6s CS blood | 36.456 | 6.880 | 0.885 | **0.000** |
| 6s/0s CS blood | 0.013 | 0.068 | 0.003 | **0.033** |
| 4s/0s CS blood | 0.237 | 0.591 | 0.024 | **0.067** |
| Charge CS blood | 0.199 | 0.451 | 0.016 | **0.000** |
| Total HA blood | 0.178 | 0.299 | 0.010 | **0.240** |
| Total CS blood | 5.444 | 18.854 | 0.495 | **0.000** |
| Tris HS blood | 0.314 | 0.396 | 0.032 | 18.673 |
| Ns6s HS blood | 0.965 | 0.655 | 0.058 | 10.493 |
| Ns2s HS blood | 0.751 | 0.756 | 0.061 | 18.793 |
| Ns HS blood | 18.970 | 12.247 | 1.135 | 6.920 |
| 2s6s HS blood | 0.486 | 0.445 | 0.045 | 15.600 |
| 6s HS blood | 7.147 | 7.856 | 1.067 | 25.573 |
| 2s HS blood | 4.444 | 5.371 | 0.329 | 12.693 |
| 0s HS blood | 58.306 | 65.639 | 2.274 | 14.513 |
| Charge HS blood | 0.478 | 0.425 | 0.030 | 24.547 |
| Total HS blood | 0.225 | 0.703 | 0.061 | **0.867** |

## EXAMPLE 10

In this study we measured circulating GAG levels in subjects presenting with a uterine cancer, more specifically endometrial cancer (EC).

### *Material and Methods*

*Plasma sample collection*

Blood samples were obtained from 9 patients with endometrial cancer. All patients had evidence of disease and treatment-naive at the time of sampling.

Whole blood tubes were collected in EDTA tubes and centrifuged to separate the plasma. Plasma was aliquoted into 220 uL vials and vials were frozen at -80°C until they were shipped for analysis in dry ice.

Plasma GAGs correspond to blood GAGs. For the purpose of this example, plasma is therefore also interchangeably referred to as blood.

*Glycosaminoglycan profile determination*

Sample preparation and the measurement of GAG properties (GAG profile determination) was done as *per* Example 3.

*Biomarker score design*

A control group was formed using historical GAG profiles obtained from blood samples of 58 healthy individuals. These samples belonged to four distinct historical cohorts. The GAG profile was measured as described in the previous section.

Biomarker score design was as described in Example 3, but concerned endometrial cancer versus healthy groups (instead of melanoma versus healthy).

For the purpose of this study and for reasons elsewhere stated, scores computed from blood GAGs regardless if the processed sample was plasma are referred to as GAG blood scores.

*Accuracy metrics*

We evaluated the performance of the EC GAG score as in Example 3, but classifying a sample as endometrial cancer or healthy (instead of melanoma or healthy).

*Results*

We analyzed a total of 9 blood samples from patients with EC. We quantified the profile of GAGs in the 9 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table Z). We further included as controls a group of 58 historical GAG profiles measured in blood samples of healthy individuals (H). Clinical data for this cohort is reported in Table Y. We compared the value of each property in the GAG profile in the EC versus H group. We assessed the statistical significance of changes for each GAG property between the two groups by calculating the highest density interval (HDI) for the mean difference using Bayesian estimation. The results showed marked differences in several GAG properties between EC and H subjects (Table Z).

We condensed changes in the GAG profile in EC vs. H subjects into GAG scores. We derived one EC GAG score, based on the following formula using blood measurements:

$$EC\ GAG\ score = \frac{8}{5}\ Charge\ CS + \frac{2}{15}[6s\ CS] - \frac{1}{500}[2s4s\ CS] + \frac{1}{65}Tot\ CS - \frac{1}{20}Tot\ HS$$

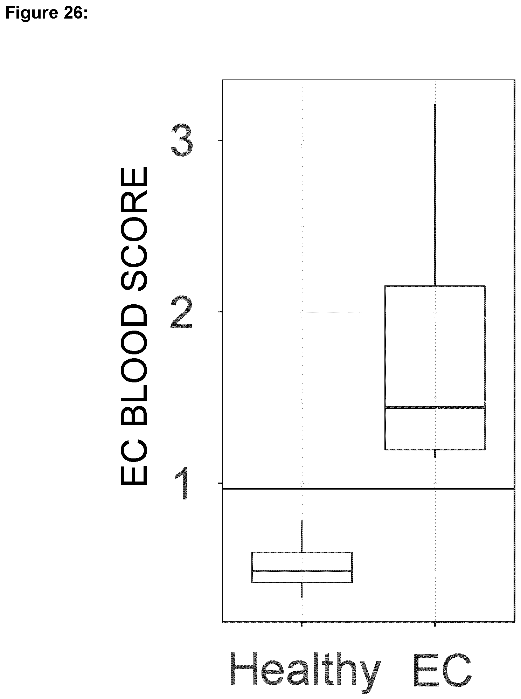

where terms in square brackets represent the fraction (mass fraction) of the disaccharide for the corresponding GAG, *Charge CS* is the weighted charge of CS, *Tot* CS is the total concentration of CS (in μg/mL), and *Tot HS* is the total concentration of HS (in μg/mL). We subsequently calculated the score in each sample and observed that EC samples have recurrently elevated scores with respect to H samples (Figure 26). The performance of the GAG score was evaluated using the receiver-operating-characteristic (ROC) curves, and the area under the curve (AUC) was found to be 1 (perfect classifier). We identified an optimal cut-off equal to 0.97 for this score. Using this cut-off, EC subjects could be distinguished from healthy individuals with 100% accuracy, 100% sensitivity, and 100% specificity.

These results indicate that there are measurable differences in circulating GAG levels and/or chemical composition between endometrial cancer as opposed to healthy subjects, and thus that GAG properties may be useful in endometrial cancer screening (e.g. diagnosis). These results further demonstrate that GAG scores can be designed to discriminate between endometrial cancer and healthy subjects, and that their significant AUC permit fine-tuned optimal cut-off scores, in order to maximize sensitivity and/or specificity, depending on the intended diagnostic use.

**Table Y**: Clinical data for the cohort analysed in this study. Results are presented as medians (25th, 75th percentile) or percent or counts. Missing values were omitted.

| Baseline characteristics | | EC | Healthy |
|---|---|---|---|
| Number of samples | | 9 | 58 |
| Age [years] | | 61 (59.5 - 67.5) | 56 (47 - 63) |
| Female | | 100% | 65.5% |
| FIGO stage | | | |
| | I-II | 5 | |
| | III-IV | 4 | |

**Table Z**: A metric of statistical confidence that any individual GAG property (CS, HS or HA levels, individual disaccharide compositions, and charge HS or charge CS) displays a significant and practically appreciable alteration between EC and healthy samples was calculated. An alteration was defined as significant and practically appreciable if the difference in means between case and control falls within a so-called Region Of Practical Equivalence in less than 5% of all possible statistical distributions that can fit the data relative to a measurement. Table Z shows a summary of the results and shows the mean value for GAG properties as measured in EC vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table Z also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in EC | ROPE threshold | % in ROPE |
|---|---|---|---|---|
| 0s CS blood | 79.184 | 57.236 | 3.387 | 0.000 |
| 2s CS blood | 0.022 | 0.043 | 0.004 | 7.780 |
| 6s CS blood | 1.079 | 5.060 | 0.181 | **0.000** |
| 4s CS blood | 19.577 | 37.511 | 1.397 | **0.000** |
| 2s6s CS blood | 0.060 | 0.112 | 0.012 | 7.433 |
| 2s4s CS blood | 0.020 | 0.070 | 0.006 | **0.267** |
| 4s6s CS blood | 0.060 | 0.138 | 0.008 | **0.647** |
| Tris CS blood | 0.008 | 0.015 | 0.004 | 25.200 |
| 4s/6s CS blood | 36.456 | 6.918 | 0.885 | **0.000** |
| 6s/0s CS blood | 0.013 | 0.073 | 0.003 | **0.000** |
| 4s/0s CS blood | 0.237 | 0.593 | 0.024 | **0.013** |
| Charge CS blood | 0.199 | 0.438 | 0.016 | **0.007** |
| Total HA blood | 0.178 | 0.278 | 0.010 | **3.327** |
| Total CS blood | 5.444 | 13.506 | 0.495 | **0.080** |
| Tris HS blood | 0.314 | 0.414 | 0.032 | 16.500 |
| Ns6s HS blood | 0.965 | 0.626 | 0.058 | 10.907 |
| Ns2s HS blood | 0.751 | 0.728 | 0.061 | 25.093 |
| Ns HS blood | 18.970 | 19.273 | 1.135 | 15.727 |
| 2s6s HS blood | 0.486 | 0.562 | 0.045 | 18.073 |
| 6s HS blood | 7.147 | 11.596 | 1.067 | 14.800 |
| 2s HS blood | 4.444 | 3.648 | 0.329 | 18.593 |
| 0s HS blood | 58.306 | 59.019 | 2.274 | 20.867 |

(continued)

| GAG property | Mean in Healthy Individuals | Mean in EC | ROPE threshold | % in ROPE |
|---|---|---|---|---|
| Charge HS blood | 0.478 | 0.445 | 0.030 | 23.753 |
| Total HS blood | 0.225 | 0.705 | 0.061 | **0.387** |

## EXAMPLE 11

In this study we measured circulating GAG levels in subjects presenting with a uterine cancer, more specifically cervical cancer (CST).

### Material and Methods

### Plasma sample collection

Blood samples were obtained from 9 patients with cervical cancer. All patients had evidence of disease and treatment-naive at the time of sampling.

Whole blood tubes were collected in EDTA tubes and centrifuged to separate the plasma. Plasma was aliquoted into 220 uL vials and vials were frozen at -80°C until they were shipped for analysis in dry ice.

Plasma GAGs correspond to blood GAGs. For the purpose of this example, plasma is therefore also interchangeably referred to as blood.

### Glycosaminoglycan profile determination

Sample preparation and the measurement of GAG properties (GAG profile determination) was done as *per* Example 3.

### Biomarker score design

A control group was formed using historical GAG profiles obtained from blood samples of 58 healthy individuals. These samples belonged to four distinct historical cohorts. The GAG profile was measured as described in the previous section.

Biomarker score design was as described in Example 3, but concerned cervical cancer versus healthy groups (instead of melanoma versus healthy).

For the purpose of this study and for reasons elsewhere stated, scores computed from blood GAGs regardless if the processed sample was plasma are referred to as GAG blood scores.

### Accuracy metrics

We evaluated the performance of the CST GAG score as in Example 3, but classifying a sample as cervical cancer or healthy (instead of melanoma or healthy).

### Results

We analyzed a total of 9 blood samples from patients with CST. We quantified the profile of GAGs in the 9 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table AB). We further included as controls a group of 58 historical GAG profiles measured in blood samples of healthy individuals (H). Clinical data for this cohort is reported in Table AA. We compared the value of each property in the GAG profile in the CST versus H group. We assessed the statistical significance of changes for each GAG property between the two groups by calculating the highest density interval (HDI) for the mean difference using Bayesian estimation. The results showed marked differences in several GAG properties between CST and H subjects (Table AB).

We condensed changes in the GAG profile in CST vs. H subjects into GAG scores. We derived one CST GAG score, based on the following formula using blood measurements:

$$CST\ GAG\ score = Charge\ CS + \frac{1}{6}[6s\ CS] + \frac{1}{50}(Tot\ CS + [Ns2s\ HS]) - \frac{1}{4}Tot\ HS$$

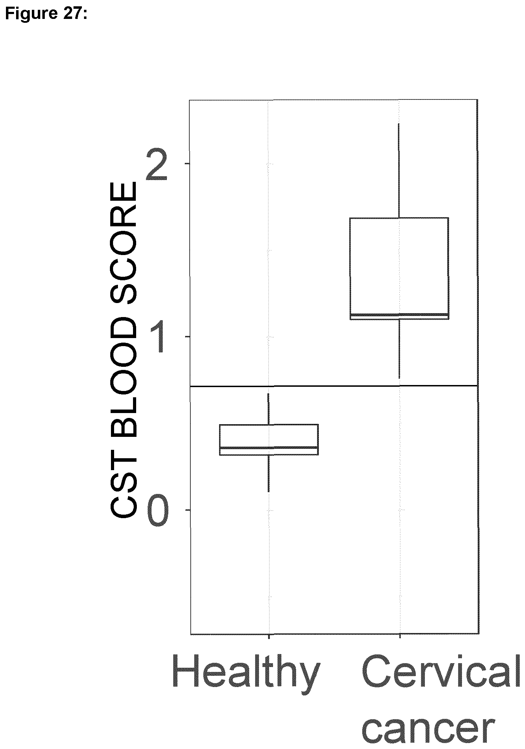

where terms in square brackets represent the fraction (mass fraction) of the disaccharide for the corresponding GAG, *Charge CS* is the weighted charge of CS, *Tot* CS is the total concentration of CS (in µg/mL), and *Tot HS* is the total concentration of HS (in µg/mL). We subsequently calculated the score in each sample and observed that CST samples have recurrently elevated scores with respect to H samples (Figure 27). The performance of the GAG score was evaluated using the receiver-operating-characteristic (ROC) curves, and the area under the curve (AUC) was found to be 1 (perfect classifier). We identified an optimal cut-off equal to 0.71 for this score. Using this cut-off, CST subjects could be distinguished from healthy individuals with 100% accuracy, 100% sensitivity, and 100% specificity.

These results indicate that there are measurable differences in circulating GAG levels and/or chemical composition between cervical cancer as opposed to healthy subjects, and thus that GAG properties may be useful in cervical cancer screening (e.g. diagnosis). These results further demonstrate that GAG scores can be designed to discriminate between cervical cancer and healthy subjects, and that their significant AUC permit fine-tuned optimal cut-off scores, in order to maximize sensitivity and/or specificity, depending on the intended diagnostic use.

**Table AA**: Clinical data for the cohort analysed in this study. Results are presented as medians (25th, 75th percentile) or percent or counts. Missing values were omitted.

| Baseline characteristics | | CST | Healthy |
|---|---|---|---|
| Number of samples | | 9 | 58 |
| Age [years] | | 41 (36 - 47) | 56 (47 - 63) |
| Female | | 100% | 65.5% |
| FIGO stage | | | |
| | I | 4 | |
| | II-IV | 5 | |

**Table AB**: A metric of statistical confidence that any individual GAG property (CS, HS or HA levels, individual disaccharide compositions, and charge HS or charge CS) displays a significant and practically appreciable alteration between CST and healthy samples was calculated. An alteration was defined as significant and practically appreciable if the difference in means between case and control falls within a so-called Region Of Practical Equivalence in less than 5% of all possible statistical distributions that can fit the data relative to a measurement. Table AB shows a summary of the results and shows the mean value for GAG properties as measured in CST vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table AB also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in CST | ROPE threshold | % in ROPE |
|---|---|---|---|---|
| 0s CS blood | 79.184 | 59.292 | 3.387 | **0.000** |
| 2s CS blood | 0.022 | 0.026 | 0.004 | 38.500 |
| 6s CS blood | 1.079 | 4.779 | 0.181 | **0.000** |
| 4s CS blood | 19.577 | 36.224 | 1.397 | **0.007** |
| 2s6s CS blood | 0.060 | 0.086 | 0.012 | 22.693 |
| 2s4s CS blood | 0.020 | 0.058 | 0.006 | **3.093** |
| 4s6s CS blood | 0.060 | 0.110 | 0.008 | **2.173** |
| Tris CS blood | 0.008 | 0.027 | 0.004 | 12.913 |
| 4s/6s CS blood | 36.456 | 7.374 | 0.885 | **0.000** |
| 6s/0s CS blood | 0.013 | 0.069 | 0.003 | **0.007** |

(continued)

| GAG property | Mean in Healthy Individuals | Mean in CST | ROPE threshold | % in ROPE |
|---|---|---|---|---|
| 4s/0s CS blood | 0.237 | 0.572 | 0.024 | **0.000** |
| Charge CS blood | 0.199 | 0.412 | 0.016 | **0.000** |
| Total HA blood | 0.178 | 0.264 | 0.010 | **4.607** |
| Total CS blood | 5.444 | 13.299 | 0.495 | **0.000** |
| Tris HS blood | 0.314 | 0.465 | 0.032 | 9.513 |
| Ns6s HS blood | 0.965 | 0.716 | 0.058 | 15.347 |
| Ns2s HS blood | 0.751 | 2.068 | 0.061 | **0.600** |
| Ns HS blood | 18.970 | 17.607 | 1.135 | 14.773 |
| 2s6s HS blood | 0.486 | 0.361 | 0.045 | 18.607 |
| 6s HS blood | 7.147 | 16.160 | 1.067 | **4.920** |
| 2s HS blood | 4.444 | 3.602 | 0.329 | 15.827 |
| 0s HS blood | 58.306 | 56.422 | 2.274 | 18.553 |
| Charge HS blood | 0.478 | 0.481 | 0.030 | 25.247 |
| Total HS blood | 0.225 | 0.803 | 0.061 | 0.833 |

## EXAMPLE 12

In this study we measured circulating GAG levels in subjects presenting with a blood cancer, more specifically Non-Hodgkin lymphoma (NHL).

### Material and Methods

### Plasma sample collection

Blood samples were obtained from 10 patients with Non-Hodgkin lymphoma, specifically diffuse large B-cell lymphoma. All patients had evidence of disease and treatment-naive at the time of sampling.

Whole blood tubes were collected in EDTA tubes and centrifuged to separate the plasma. Plasma was aliquoted into 220 uL vials and vials were frozen at -80°C until they were shipped for analysis in dry ice.

Plasma GAGs correspond to blood GAGs. For the purpose of this example, plasma is therefore also interchangeably referred to as blood.

### Glycosaminoglycan profile determination

Sample preparation and the measurement of GAG properties (GAG profile determination) was done as *per* Example 3.

### Biomarker score design

A control group was formed using historical GAG profiles obtained from blood samples of 58 healthy individuals. These samples belonged to four distinct historical cohorts. The GAG profile was measured as described in the previous section.

Biomarker score design was as described in Example 3, but concerned NHL versus healthy groups (instead of melanoma versus healthy).

For the purpose of this study and for reasons elsewhere stated, scores computed from blood GAGs regardless if the processed sample was plasma are referred to as GAG blood scores.

### Accuracy metrics

We evaluated the performance of the NHL GAG score as in Example 3, but classifying a sample as NHL or healthy (instead of melanoma or healthy).

*Results*

We analyzed a total of 10 blood samples from patients with NHL. We quantified the profile of GAGs in the 10 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table AD). We further included as controls a group of 58 historical GAG profiles measured in blood samples of healthy individuals (H). Clinical data for this cohort is reported in Table AC. We compared the value of each property in the GAG profile in the NHL versus H group. We assessed the statistical significance of changes for each GAG property between the two groups by calculating the highest density interval (HDI) for the mean difference using Bayesian estimation. The results showed marked differences in several GAG properties between NHL and H subjects (Table AD).
We condensed changes in the GAG profile in NHL vs. H subjects into GAG scores. We derived one NHL GAG score, based on the following formula using blood measurements:

$$NHL\ GAG\ score = \frac{5}{6}Charge\ CS + \frac{1}{5}[6s\ CS] - \frac{1}{7}[Tris\ CS] + \frac{1}{3}Charge\ HS - \frac{1}{20}Tot\ HS$$

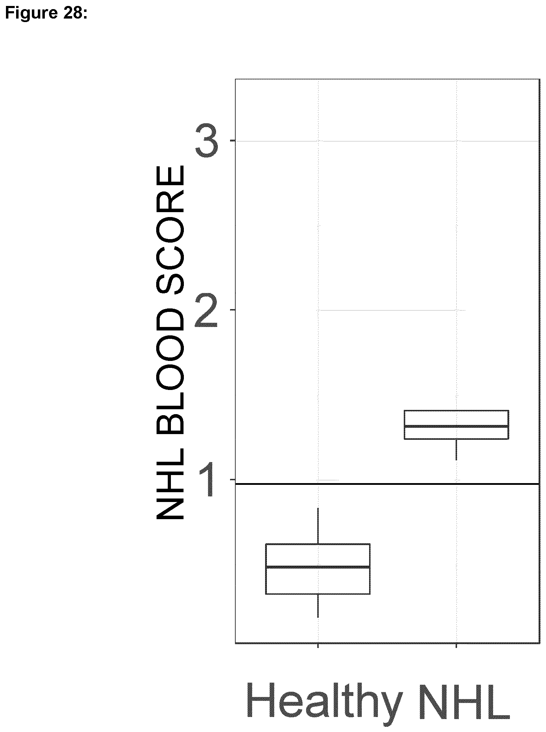

where terms in square brackets represent the fraction (mass fraction) of the disaccharide for the corresponding GAG, *Charge CS* is the weighted charge of CS, *Charge HS* is the weighted charge of HS, and *Tot HS* is the total concentration of HS (in μg/mL). We subsequently calculated the score in each sample and observed that NHL samples have recurrently elevated scores with respect to H samples (Figure 28). The performance of the GAG score was evaluated using the receiver-operating-characteristic (ROC) curves, and the area under the curve (AUC) was found to be 1 (perfect classifier). We identified an optimal cut-off equal to 0.97 for this score. Using this cut-off, NHL subjects could be distinguished from healthy individuals with 100% accuracy, 100% sensitivity, and 100% specificity.
These results indicate that there are measurable differences in circulating GAG levels and/or chemical composition between NHL as opposed to healthy subjects, and thus that GAG properties may be useful in NHL screening (e.g. diagnosis). These results demonstrate that GAG scores can be designed to discriminate between Non-Hodgkin lymphoma and healthy subjects, and that their significant AUC permit fine-tuned optimal cut-off scores, in order to maximize sensitivity and/or specificity, depending on the intended diagnostic use.

**Table AC**: Clinical data for the cohort analysed in this study. Results are presented as medians (25th, 75th percentile) or percent or counts. Missing values were omitted.

| Baseline characteristics | NHL | Healthy |
|---|---|---|
| Number of samples | 10 | 58 |
| Age [years] | 66 (61 - 70.5) | 56 (47 - 63) |
| Female | 60% | 65.5% |
| Ann Arbor Stage | | |
| I-II | 3 | |
| III-IV | 7 | |

**Table AD**: A metric of statistical confidence that any individual GAG property (CS, HS or HA levels, individual disaccharide compositions, and charge HS or charge CS) displays a significant and practically appreciable alteration between NHL and healthy samples was calculated. An alteration was defined as significant and practically appreciable if the difference in means between case and control falls within a so-called Region Of Practical Equivalence in less than 5% of all possible statistical distributions that can fit the data relative to a measurement. Table AD shows a summary of the results and shows the mean value for GAG properties as measured in NHL vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table AD also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in NHL | ROPE threshold | % in ROPE |
|---|---|---|---|---|
| 0s CS blood | 79.184 | 62.083 | 3.387 | **0.000** |
| 2s CS blood | 0.022 | 0.039 | 0.004 | 6.153 |
| 6s CS blood | 1.079 | 4.503 | 0.181 | **0.000** |
| 4s CS blood | 19.577 | 33.104 | 1.397 | **0.013** |
| 2s6s CS blood | 0.060 | 0.149 | 0.012 | **1.313** |
| 2s4s CS blood | 0.020 | 0.088 | 0.006 | **0.140** |
| 4s6s CS blood | 0.060 | 0.150 | 0.008 | **0.620** |
| Tris CS blood | 0.008 | 0.076 | 0.004 | **0.000** |
| 4s/6s CS blood | 36.456 | 6.834 | 0.885 | **0.000** |
| 6s/0s CS blood | 0.013 | 0.068 | 0.003 | **0.000** |
| 4s/0s CS blood | 0.237 | 0.502 | 0.024 | **0.007** |
| Charge CS blood | 0.199 | 0.388 | 0.016 | **0.000** |
| Total HA blood | 0.178 | 0.247 | 0.010 | 5.253 |
| Total CS blood | 5.444 | 8.753 | 0.495 | **1.273** |
| Tris HS blood | 0.314 | 0.745 | 0.032 | **0.600** |
| Ns6s HS blood | 0.965 | 0.728 | 0.058 | 17.687 |
| Ns2s HS blood | 0.751 | 1.324 | 0.061 | **1.420** |
| Ns HS blood | 18.970 | 31.578 | 1.135 | **0.440** |
| 2s6s HS blood | 0.486 | 0.324 | 0.045 | 18.307 |
| 6s HS blood | 7.147 | 17.917 | 1.067 | **1.700** |
| 2s HS blood | 4.444 | 4.217 | 0.329 | 19.887 |
| 0s HS blood | 58.306 | 39.880 | 2.274 | **0.280** |
| Charge HS blood | 0.478 | 0.656 | 0.030 | **0.773** |
| Total HS blood | 0.225 | 1.251 | 0.061 | **0.007** |

## EXAMPLE 13

In this study we measured circulating GAG levels in subjects presenting with brain cancer, more specifically diffuse glioma (DG).

### *Material and Methods*

*Plasma sample collection*

Blood samples were obtained from 11 patients with diffuse glioma, specifically 7 patients with astrocytoma, 3 with glioblastoma multiforme and 1 with oligoastrocytoma. All patients had evidence of disease and treatment-naive at the time of sampling.

Whole blood tubes were collected in EDTA tubes and centrifuged to separate the plasma. Plasma was aliquoted into 220 uL vials and vials were frozen at -80°C until they were shipped for analysis in dry ice.

Plasma GAGs correspond to blood GAGs. For the purpose of this example, plasma is therefore also interchangeably referred to as blood.

*Glycosaminoglycan profile determination*

Sample preparation and the measurement of GAG properties (GAG profile determination) was done as *per* Example 3.

*Biomarker score design*

A control group was formed using historical GAG profiles obtained from blood samples of 58 healthy individuals. These samples belonged to four distinct historical cohorts. The GAG profile was measured as described in the previous section.

Biomarker score design was as described in Example 3, but concerned diffuse glioma versus healthy groups (instead of melanoma versus healthy).

For the purpose of this study and for reasons elsewhere stated, scores computed from blood GAGs regardless if the processed sample was plasma are referred to as GAG blood scores.

*Accuracy metrics*

We evaluated the performance of the DG GAG score as in Example 3, but classifying a sample as DG or healthy (instead of melanoma or healthy).

*Results*

We analyzed a total of 11 blood samples from patients with DG. We quantified the profile of GAGs in the 11 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table AF). We further included as controls a group of 58 historical GAG profiles measured in blood samples of healthy individuals (H). Clinical data for this cohort is reported in Table AE. We compared the value of each property in the GAG profile in the DG versus H group. We assessed the statistical significance of changes for each GAG property between the two groups by calculating the highest density interval (HDI) for the mean difference using Bayesian estimation. The results showed marked differences in several GAG properties between DG and H subjects (Table AF).

We condensed changes in the GAG profile in DG vs. H subjects into GAG scores. We derived one DG GAG score, based on the following formula using blood measurements:

$$DG\ GAG\ score = \frac{3}{5} Charge\ CS + \frac{1}{7}[6s\ CS] + \frac{1}{50} Total\ CS + \frac{1}{2} Total\ HA + \frac{1}{10000}[0s\ HS]$$

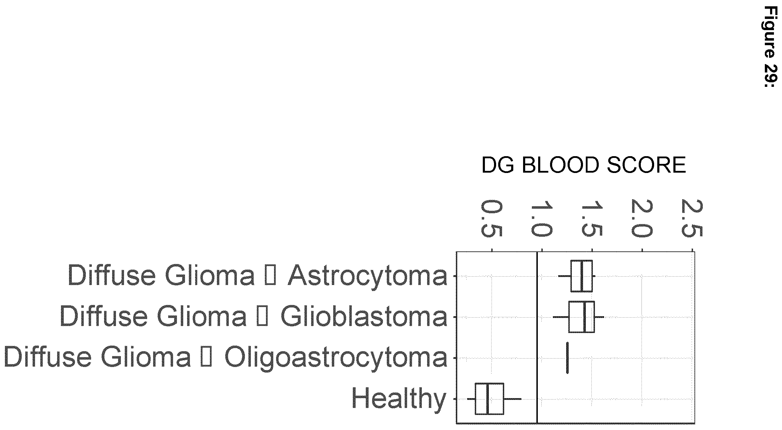

where terms in square brackets represent the fraction (mass fraction) of the disaccharide for the corresponding GAG, *Charge CS* is the weighted charge of CS, and *Total CS* is the total concentration of CS (in μg/mL), and *Total HA* is the total concentration of HA (in μg/mL). We subsequently calculated the score in each sample and observed that DG samples have recurrently elevated scores with respect to H samples (Figure 29). The performance of the GAG score was evaluated using the receiver-operating-characteristic (ROC) curves, and the area under the curve (AUC) was found to be 1 (perfect classifier). We identified an optimal cut-off equal to 0.95 for this score. Using this cut-off, DG subjects could be distinguished from healthy individuals with 100% accuracy, 100% sensitivity, and 100% specificity.

These results indicate that there are measurable differences in circulating GAG levels and/or chemical composition between DG as opposed to healthy subjects, and thus that GAG properties may be useful in DG screening (e.g. diagnosis). These results further demonstrate that GAG scores (or GAG profiles) can be designed to discriminate between diffuse glioma and healthy subjects, and that their significant AUC permit fine-tuned optimal cut-off scores, in order to maximize sensitivity and/or specificity, depending on the intended diagnostic use.

**Table AE**: Clinical data for the cohort analysed in this study. Results are presented as medians (25th, 75th percentile) or percent or counts. Missing values were omitted.

| Baseline characteristics | DG | Healthy |
|---|---|---|
| Number of samples | 11 | 58 |
| Age [years] | 66 (50.5 - 70.5) | 56 (47 - 63) |
| Female | 54.5% | 65.5% |
| Glioma subtype | | |
| Astrocytoma | 7 | - |
| Glioblastoma multiforme | 3 | - |
| Oligoastrocytoma | 1 | - |

**Table AF**: A metric of statistical confidence that any individual GAG property (CS, HS or HA levels, individual disaccharide compositions, and charge HS or charge CS) displays a significant and practically appreciable alteration between DG and healthy samples was calculated. An alteration was defined as significant and practically appreciable if the difference in means between case and control falls within a so-called Region Of Practical Equivalence in less than 5% of all possible statistical distributions that can fit the data relative to a measurement. Table AF shows a summary of the results and shows the mean value for GAG properties as measured in DG vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table AF also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in DG | ROPE threshold | % in ROPE |
|---|---|---|---|---|
| 0s CS blood | 79.184 | 57.146 | 3.387 | **0.000** |
| 2s CS blood | 0.022 | 0.037 | 0.004 | 13.253 |
| 6s CS blood | 1.079 | 5.269 | 0.181 | **0.000** |
| 4s CS blood | 19.577 | 36.417 | 1.397 | **0.000** |
| 2s6s CS blood | 0.060 | 0.129 | 0.012 | 5.447 |
| 2s4s CS blood | 0.020 | 0.077 | 0.006 | **0.053** |
| 4s6s CS blood | 0.060 | 0.159 | 0.008 | **0.553** |
| Tris CS blood | 0.008 | 0.083 | 0.004 | **0.007** |
| 4s/6s CS blood | 36.456 | 7.056 | 0.885 | **0.000** |
| 6s/0s CS blood | 0.013 | 0.096 | 0.003 | **0.000** |
| 4s/0s CS blood | 0.237 | 0.603 | 0.024 | **0.000** |
| Charge CS blood | 0.199 | 0.443 | 0.016 | **0.000** |
| Total HA blood | 0.178 | 0.307 | 0.010 | **0.860** |
| Total CS blood | 5.444 | 10.785 | 0.495 | **0.000** |
| Tris HS blood | 0.314 | 0.414 | 0.032 | 15.860 |
| Ns6s HS blood | 0.965 | 0.614 | 0.058 | 10.020 |
| Ns2s HS blood | 0.751 | 0.562 | 0.061 | 14.620 |
| Ns HS blood | 18.970 | 31.537 | 1.135 | **1.393** |
| 2s6s HS blood | 0.486 | 0.289 | 0.045 | 13.933 |

(continued)

| GAG property | Mean in Healthy Individuals | Mean in DG | ROPE threshold | % in ROPE |
|---|---|---|---|---|
| 6s HS blood | 7.147 | 18.620 | 1.067 | **1.820** |
| 2s HS blood | 4.444 | 5.401 | 0.329 | 11.213 |
| 0s HS blood | 58.306 | 32.310 | 2.274 | **0.413** |
| Charge HS blood | 0.478 | 0.747 | 0.030 | **0.507** |
| Total HS blood | 0.225 | 1.420 | 0.061 | **0.000** |

### EXAMPLE 14

In this study we measured circulating GAG levels in subjects presenting with lung cancer (LC).

### *Material and Methods*

### *Plasma sample collection*

Blood samples were obtained from 10 patients with lung cancer, specifically non-small cell lung cancer. All patients had evidence of disease and treatment-naive at the time of sampling.
Whole blood tubes were collected in EDTA tubes and centrifuged to separate the plasma. Plasma was aliquoted into 220 uL vials and vials were frozen at -80°C until they were shipped for analysis in dry ice.
Plasma GAGs correspond to blood GAGs. For the purpose of this example, plasma is therefore also interchangeably referred to as blood.

### *Glycosaminoglycan profile determination*

Sample preparation and the measurement of GAG properties (GAG profile determination) was done as *per* Example 3.

### *Biomarker score design*

A control group was formed using historical GAG profiles obtained from blood samples of 58 healthy individuals. These samples belonged to four distinct historical cohorts. The GAG profile was measured as described in the previous section.
Biomarker design was as described in Example 3, but concerned lung cancer versus healthy groups (instead of melanoma versus healthy).
For the purpose of this study and for reasons elsewhere stated, scores computed from blood GAGs regardless if the processed sample was plasma are referred to as GAG blood scores.

### *Accuracy metrics*

We evaluated the performance of the LC GAG score as in Example 3, but classifying a sample as lung cancer or healthy (instead of melanoma or healthy).

### *Results*

We analyzed a total of 10 blood samples from patients with LC. We quantified the profile of GAGs in the 10 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table AH). We further included as controls a group of 58 historical GAG profiles measured in blood samples of healthy individuals (H). Clinical data for this cohort is reported in Table AG. We compared the value of each property in the GAG profile in the LC versus H group. We assessed the statistical significance of changes for each GAG property between the two groups by calculating the highest density interval (HDI) for the mean difference using Bayesian estimation. The results showed marked differences in several GAG properties between LC and H subjects (Table AH).
We condensed changes in the GAG profile in LC vs. H subjects into GAG scores. We derived one LC GAG score (LC GAG score #1), based on the following formula using blood measurements:

$$LC\ GAG\ score = \frac{1}{6} Total\ CS - \left( \frac{1}{30} \frac{[4s\ CS]}{[6s\ CS]} + \frac{1}{25} [0s\ HS] \right)$$

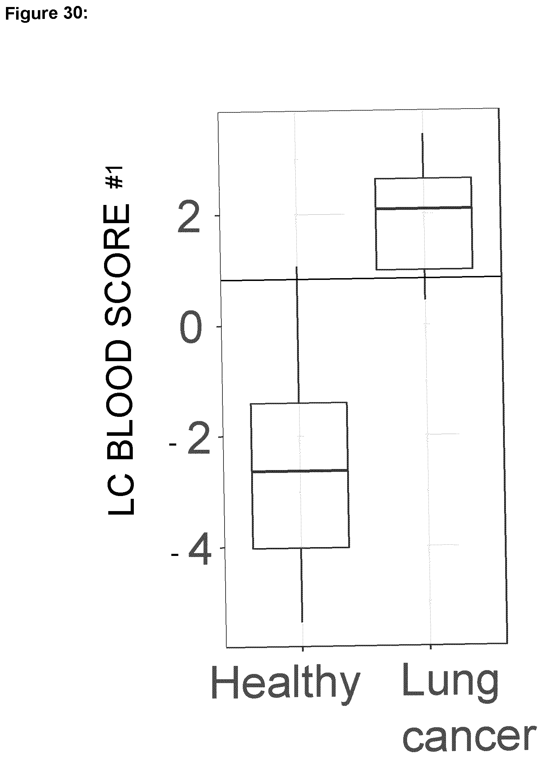

where terms in square brackets represent the fraction (mass fraction) of the disaccharide for the corresponding GAG, and *Total* CS is the total concentration of CS (in µg/mL). We subsequently calculated the score in each sample and observed that LC samples have recurrently elevated scores with respect to H samples (Figure 30). The performance of the GAG score was evaluated using the receiver-operating-characteristic (ROC) curves, and the area under the curve (AUC) was found to be 0.986. We identified an optimal cut-off equal to 0.83 for this score. Using this cut-off, LC subjects could be distinguished from healthy individuals with 97% accuracy, 90% sensitivity, and 98% specificity.

We also investigated whether variations of the above GAG score would also be effective in distinguishing LC from H subjects. To this end, we derived an alternative LC GAG score (LC GAG score #2) using only CS properties:

$$LC\ GAG\ score = \frac{5}{4} Charge\ CS + \frac{1}{25} Total\ CS$$

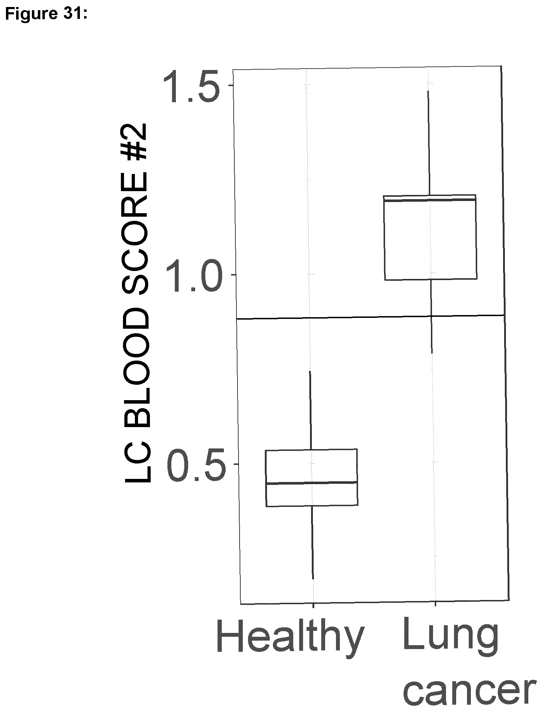

where *Total* CS is the total concentration of CS (in µg/mL), and *Charge* CS is the weighted charge of CS. Using this alternative scoring system, LC samples had recurrently higher scores than H samples (Figure 31). The AUC was found to be 0.997. We identified an optimal cut-off equal to 0.88 for this score. Using this cut-off, LC patients could be distinguished from healthy individuals with 98.5% accuracy, 90% sensitivity, and 100% specificity.

These results indicate that there are measurable differences in circulating GAG levels and/or chemical composition between lung cancer as opposed to healthy subjects, and thus that GAG properties may be useful in lung cancer screening (e.g. diagnosis). These results further demonstrate that GAG scores can be designed to discriminate between lung cancer and healthy subjects, and that their significant AUC permit fine-tuned optimal cut-off scores, in order to maximize sensitivity and/or specificity, depending on the intended diagnostic use.

**Table AG**: Clinical data for the cohort analysed in this study. Results are presented as medians (25th, 75th percentile) or percent or counts. Missing values were omitted.

| Baseline characteristics | | LC | Healthy |
|---|---|---|---|
| Number of samples | | 10 | 58 |
| Age [years] | | 62.5 (58 - 64) | 56 (47 - 63) |
| Female | | 50% | 65.5% |
| TNM stage | | | |
| | I-III | 4 | |
| | IV | 6 | |

**Table AH**: A metric of statistical confidence that any individual GAG property (CS, HS or HA levels, individual disaccharide compositions, and charge HS or charge CS) displays a significant and practically appreciable alteration between LC and healthy samples was calculated. An alteration was defined as significant and practically appreciable if the difference in means between case and control falls within a so-called Region Of Practical Equivalence in less than 5% of all possible statistical distributions that can fit the data relative to a measurement. Table AH shows a summary of the results and shows the mean value for GAG properties as measured in LC vs. H subjects blood and Bayesian estimation for the statistical significance of the mean difference (% in ROPE, Region Of Practical Equivalence, where a % in ROPE > 5 indicates that random distributions of values for the two groups have a practically equivalent mean in more than 5% of the cases, and it is therefore not significant). Table AH also shows results where certain ratios of individual types of disaccharide composition have been calculated. 4s/6s CS is the ratio of 4s CS to 6s CS. 6s/0s CS is the ratio of 6s CS to 0s CS (unsulfated CS). 4s/0s CS is the ratio of 4s CS to 0s CS (unsulfated CS).

| GAG property | Mean in Healthy Individuals | Mean in LC | ROPE threshold | % in ROPE |
|---|---|---|---|---|
| 0s CS blood | 79.184 | 67.533 | 3.387 | **0.600** |
| 2s CS blood | 0.022 | 0.043 | 0.004 | **4.407** |

(continued)

| GAG property | Mean in Healthy Individuals | Mean in LC | ROPE threshold | % in ROPE |
|---|---|---|---|---|
| 6s CS blood | 1.079 | 2.570 | 0.181 | **0.113** |
| 4s CS blood | 19.577 | 30.433 | 1.397 | **0.247** |
| 2s6s CS blood | 0.060 | 0.065 | 0.012 | 46.873 |
| 2s4s CS blood | 0.020 | 0.043 | 0.006 | **1.773** |
| 4s6s CS blood | 0.060 | 0.093 | 0.008 | 8.800 |
| Tris CS blood | 0.008 | 0.020 | 0.004 | **0.300** |
| 4s/6s CS blood | 36.456 | 10.924 | 0.885 | **0.000** |
| 6s/0s CS blood | 0.013 | 0.038 | 0.003 | **0.007** |
| 4s/0s CS blood | 0.237 | 0.418 | 0.024 | **0.253** |
| Charge CS blood | 0.199 | 0.332 | 0.016 | **0.140** |
| Total HA blood | 0.178 | 0.158 | 0.010 | 25.273 |
| Total CS blood | 5.444 | 17.306 | 0.495 | **0.000** |
| Tris HS blood | 0.314 | 0.221 | 0.032 | 13.747 |
| Ns6s HS blood | 0.965 | 0.326 | 0.058 | **0.753** |
| Ns2s HS blood | 0.751 | 0.386 | 0.061 | **2.087** |
| Ns HS blood | 18.970 | 26.430 | 1.135 | 7.213 |
| 2s6s HS blood | 0.486 | 0.370 | 0.045 | 23.787 |
| 6s HS blood | 7.147 | 40.953 | 1.067 | **0.013** |
| 2s HS blood | 4.444 | 4.067 | 0.329 | 17.167 |
| 0s HS blood | 58.306 | 17.681 | 2.274 | **0.000** |
| Charge HS blood | 0.478 | 0.842 | 0.030 | **0.000** |
| Total HS blood | 0.225 | 2.304 | 0.061 | **0.000** |

### EXAMPLE 15

In this study we measured circulating GAG levels in subjects presenting with different types of cancers and compared them with GAG levels in healthy subjects.

#### *Material and Methods*

#### *Plasma sample collection*

Blood samples were obtained from 105 patients with different types of cancer. All patients had evidence of disease and treatment-naive at the time of sampling. The following cancer types were examined: breast cancer (BC), colorectal cancer (CRC), cervical cancer (CST), diffuse glioma (DG), endometrial cancer (EC), gastrointestinal neuroendocrine tumors (GNET), lymphoid leukemia (LL), Non-Hodgkin's lymphoma (NHL), lung cancer (LC), ovarian cancer (OV), prostate cancer (PCa).

Whole blood was collected in EDTA tubes and centrifuged to separate the plasma. Plasma was aliquoted into 220 uL vials and vials were frozen at -80°C until they were shipped for analysis in dry ice.

Plasma GAGs correspond to blood GAGs. For the purpose of this example, plasma is therefore also interchangeably referred to as blood.

#### *Glycosaminoglycan profile determination*

Sample preparation and the measurement of GAG properties (GAG profile determination) was done as *per* Example 3.

*Biomarker score design*

A control group was formed using historical GAG profiles obtained from blood samples of 58 healthy individuals. These samples belonged to four distinct historical cohorts. The GAG profile was measured as described in the previous section.

The difference for each GAG property between each cancer type vs. the control group was described in terms of mean and standard deviation. The statistical significance in the change of each GAG property between groups was reported in previous Examples.

To verify whether changes in the GAG profile in cancer patients could be condensed into GAG scores, that can be used to differentiate cancer patients from healthy subjects, and therefore would be suitable as a cancer diagnostic, we utilized Lasso penalized logistic regression (Tibshirani, 1996, *supra*) with leave-one-out cross-validation to select robust GAG properties that are most predictive of the clinical outcome (i.e., cancer compared to healthy subject). The score was subsequently designed as a ratio, where the numerator is the sum of the properties associated with cancer and the denominator is the sum of the properties associated with the healthy state. Each term was normalized using the regression coefficients.

For the purpose of this study and for reasons elsewhere stated, scores computed from blood GAGs regardless if the processed sample was plasma are referred to as GAG blood scores.

*Accuracy metrics*

We evaluated the performance of the GAG score in the binary classification of a sample as either cancer or healthy at varying threshold scores by deriving the receiver-operating-characteristic (ROC) curves. We measured the accuracy of the GAG score as the area under the curve (AUC) of its ROC curve (AUC is 1 for a perfect classifier and 0.5 for a random classifier). We selected as a potential cut-off value for the GAG score the score for which the accuracy was maximum, i.e. a sample whose GAG score is above this cut-off value has the maximum probability of being cancer and below this cut-off value has the maximum probability of being healthy. The ROC curves were calculated using the pROC R-package, while the optimal cut-off using the OptimalCutpoints R-package.

*Results*

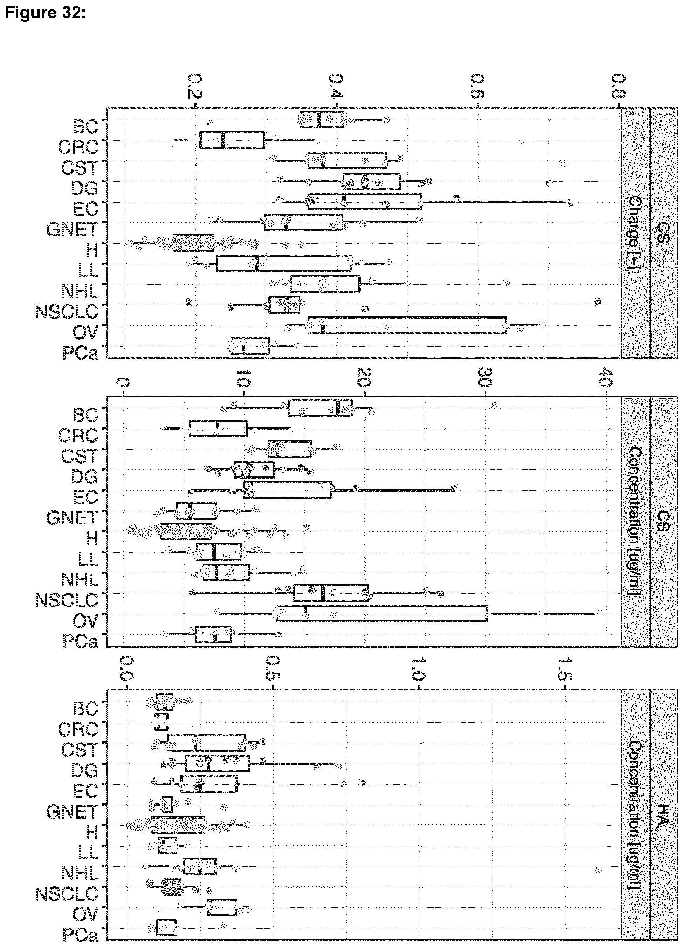

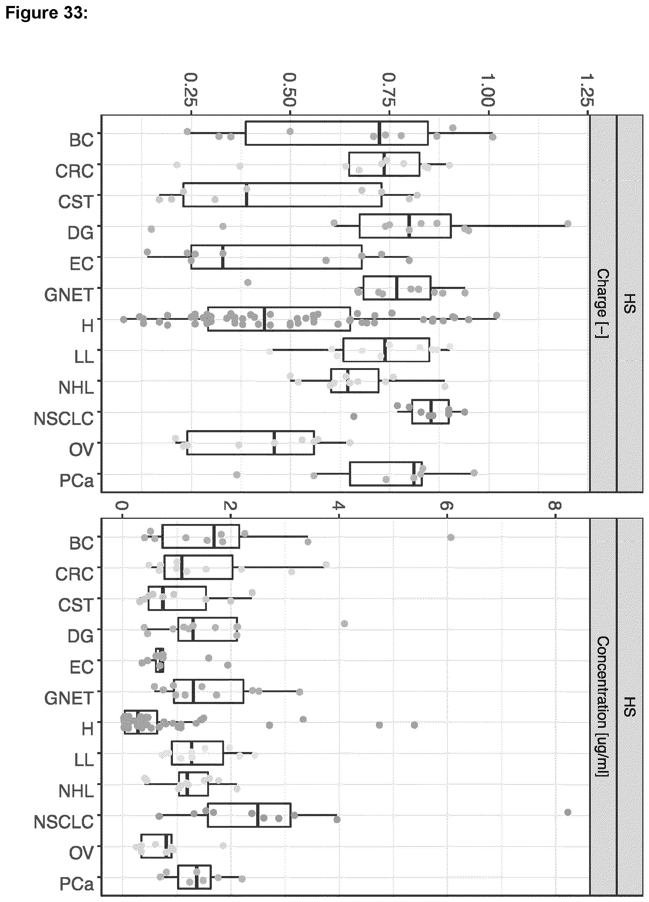

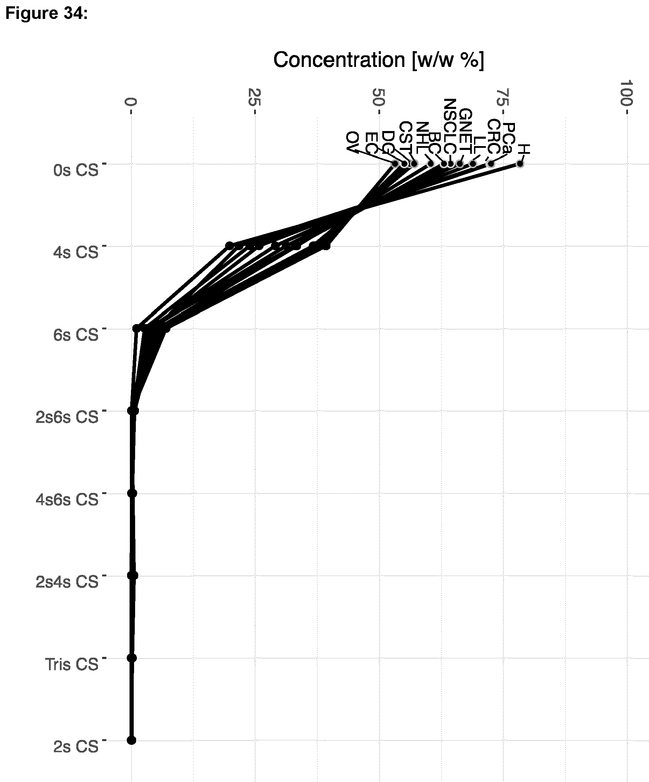

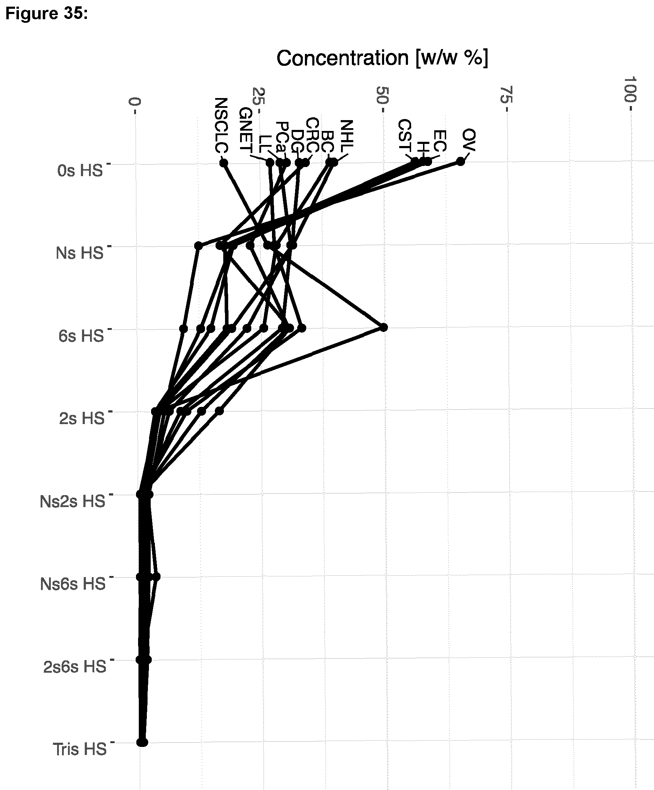

We analyzed a total of 105 blood samples from patients with cancer. We quantified the profile of GAGs in the 105 samples, comprising the level and disaccharide chemical composition of (i) chondroitin sulfate (CS), (ii) heparan sulfate (HS), and (iii) hyaluronic acid (HA), as previously described (Gatto et al., 2016 and above). In total, 24 different properties (including 19 independent properties) were measured as part of the GAG profile of the blood samples (listed in Table AJ). We further included as controls a group of 58 historical GAG profiles measured in blood samples of healthy individuals (H). Clinical data for this cohort is reported in Table AI. We reported the value of each property in the GAG profile in each cancer type versus H group in Table AJ. The results showed marked differences in several GAG properties between cancer and H subjects, as shown in Figure 32-35. The statistical significance of each difference with the healthy group was assessed separately for each cancer type in previous Examples.

We condensed changes in the GAG profile in cancer vs. H subjects into GAG scores. We derived an alternative Cancer GAG score (Cancer GAG Score#2/ Cancer Blood score #2) to the blood score designed in Example 2, based on the following formula using blood measurements:

$$Cancer\ GAG\ score = \frac{10}{3}\text{Charge CS} + \text{Total CS}\left(\frac{[6s\ CS]}{[4s\ CS] + [6s\ CS]}\right) + \log_2\left(1 + \frac{[2s\ HS]}{[0s\ HS]}\right)$$

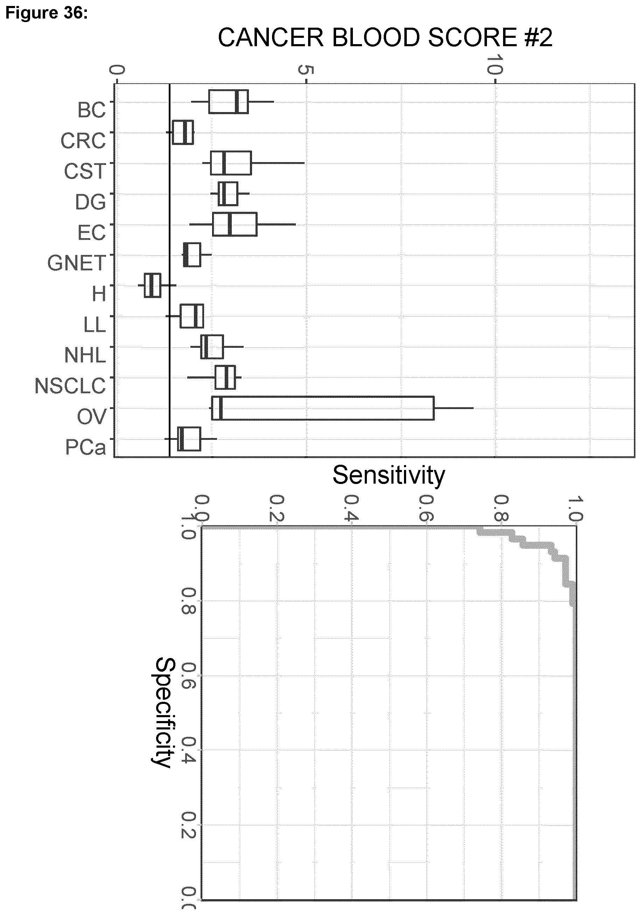

where terms in square brackets represent the fraction (mass fraction) of the disaccharide for the corresponding GAG, *Charge CS* is the weighted charge of CS and *Total CS* is the total concentration of CS (in μg/mL). We subsequently calculated the score in each sample and observed that cancer samples have recurrently elevated scores with respect to H samples (Figure 36). The performance of the GAG score was evaluated using the receiver-operating-characteristic (ROC) curves, and the area under the curve (AUC) was found to be 0.986. We identified an optimal cut-off equal to 1.39 for this score. Using this cut-off, cancer subjects could be distinguished from healthy individuals with 95.1% accuracy, 97.1% sensitivity, and 91.4% specificity.

These results demonstrate that GAG scores (or GAG profiles) can be designed to discriminate between cancer and healthy subjects, and that their significant AUC permit fine-tuned optimal cut-off scores, in order to maximize sensitivity and/or specificity, depending on the intended diagnostic use.

**Table AI**: Clinical data for the cohort analysed in this study. Results are presented as medians (25th, 75th percentile) or percent or counts. Missing values were omitted. * Low-stage/grade disease is defined as TNM stage I to III for CRC and LC, FIGO stage I for CST, FIGO stage I to II for EC and OV, non-Grade IV glioma for DG, ENETS grade G1 for GNET, Binet stage A or B for LL, Ann Arbor stage I to II for NHL, and Gleason grade < 7 for PCa (N.A. if not available).

| Baseline characteristics | | Cancer | Healthy |
|---|---|---|---|
| Number of samples | | 105 | 58 |
| Age [years] | | 64 (52 - 71) | 56 (47 - 63) |
| Female | | 66.7% | 65.5% |
| Cancer type | Total | of which low-stage/grade* | |
| Breast cancer (BC) | 10 | N.A. | - |
| Colorectal cancer (CRC) | 10 | 7 | - |
| Cervical cancer (CST) | 9 | 4 | - |
| Diffuse glioma (DG) | 11 | 8 | - |
| Endometrial cancer (EC) | 9 | 5 | - |
| Gastrointestinal neuroendocrine tumors (GNET) | 10 | 7 | - |
| Lymphoid leukemia (LL) | 10 | 7 | - |
| Non-Hodgkin's lymphoma (NHL) | 10 | 3 | - |
| Lung cancer (LC) | 10 | 4 | - |
| Ovarian cancer (OV) | 9 | 5 | - |
| Prostate cancer (PCa) | 7 | 5 | - |

**Table AJ**: Mean value (+/- standard deviation) for each GAG property in the healthy group and in each cancer type group. GAG properties referring to composition are expressed as mass fractions (weight/weight %).

| | H | BC | CR C | CS T | DG | EC | GN ET | LL | NH L | LC | OV | PCa |
|---|---|---|---|---|---|---|---|---|---|---|---|---|
| **CS Charge** | 0.20 +-0.05 | 0.37 +-0.07 | 0.29 +-0.14 | 0.43 +-0.12 | 0.46 +-0.10 | 0.46 +-0.13 | 0.35 +-0.09 | 0.32 +-0.11 | 0.40 +-0.10 | 0.36 +-0.16 | 0.47 +-0.15 | 0.28 +-0.04 |
| **CS Concentr ation** | 5.71 +-3.39 | 17.0 2 +-6.38 | 9.50 +-6.65 | 13.3 8 +-2.42 | 10.8 6 +-2.70 | 14.0 0 +-6.71 | 6.19 +-2.67 | 7.73 +-2.50 | 9.01 +-3.29 | 17.2 6 +-6.07 | 20.3 2 +-11.3 0 | 7.67 +-3.00 |
| **HA Concentr ation** | 0.18 +-0.10 | 0.13 +-0.04 | 0.14 +-0.07 | 0.27 +-0.15 | 0.34 +-0.20 | 0.34 +-0.26 | 0.15 +-0.07 | 0.14 +-0.05 | 0.37 +-0.45 | 0.16 +-0.06 | 0.29 +-0.10 | 0.16 +-0.08 |
| **HS Charge** | 0.49 +-0.24 | 0.64 +-0.27 | 0.67 +-0.22 | 0.48 +-0.27 | 0.74 +-0.29 | 0.45 +-0.25 | 0.75 +-0.16 | 0.73 +-0.14 | 0.66 +-0.12 | 0.84 +-0.08 | 0.42 +-0.17 | 0.73 +-0.20 |
| **HS Concentr ation** | 0.65 +-1.06 | 1.97 +-1.71 | 1.57 +-1.11 | 1.04 +-0.75 | 1.60 +-1.03 | 0.87 +-0.53 | 1.58 +-0.89 | 1.41 +-0.60 | 1.24 +-0.54 | 2.85 +-2.13 | 0.77 +-0.49 | 1.37 +-0.53 |
| **0s CS** | 78.4 1 +-6.30 | 63.0 5 +-6.55 | 72.3 0 +-13.3 3 | 57.0 5 +-12.0 7 | 56.1 7 +-8.66 | 55.0 6 +-13.0 9 | 66.2 6 +-9.15 | 68.9 0 +-10.1 3 | 60.4 0 +-9.85 | 64.3 9 +-15.5 6 | 53.1 9 +-15.2 4 | 72.5 6 +-3.39 |
| **2s CS** | 0.15 +-0.25 | 0.07 +-0.07 | 0.08 +-0.05 | 0.04 +-0.03 | 0.04 +-0.02 | 0.04 +-0.02 | 0.04 +-0.02 | 0.04 +-0.02 | 0.04 +-0.02 | 0.05 +-0.03 | 0.03 +-0.02 | 0.04 +-0.02 |
| **6s CS** | 1.10 +-0.82 | 3.39 +-0.81 | 4.96 +-7.07 | 5.53 +-2.63 | 5.58 +-1.89 | 6.18 +-3.29 | 3.64 +-1.77 | 4.27 +-2.96 | 5.43 +-2.89 | 3.88 +-4.38 | 7.03 +-4.57 | 2.76 +-1.37 |
| **4s CS** | 19.8 8 +-5.28 | 33.1 3 +-6.25 | 21.7 5 +-6.10 | 37.0 1 +-9.75 | 36.7 4 +-6.87 | 38.1 8 +-10.0 8 | 29.1 1 +-8.52 | 25.8 3 +-7.59 | 33.4 3 +-7.04 | 31.3 2 +-11.4 9 | 39.2 8 +-11.0 0 | 23.9 3 +-2.03 |
| **2s6s CS** | 0.10 +-0.12 | 0.07 +-0.09 | 0.69 +-1.11 | 0.11 +-0.08 | 0.29 +-0.34 | 0.15 +-0.11 | 0.57 +-0.31 | 0.67 +-0.66 | 0.22 +-0.20 | 0.11 +-0.11 | 0.14 +-0.11 | 0.45 +-0.25 |
| **2s4s CS** | 0.11 +-0.27 | 0.10 +-0.15 | 0.08 +-0.05 | 0.09 +-0.07 | 0.60 +-1.55 | 0.08 +-0.06 | 0.09 +-0.05 | 0.07 +-0.02 | 0.14 +-0.10 | 0.07 +-0.05 | 0.14 +-0.14 | 0.05 +-0.02 |
| **4s6s CS** | 0.12 +-0.18 | 0.14 +-0.10 | 0.10 +-0.04 | 0.12 +-0.05 | 0.33 +-0.45 | 0.17 +-0.11 | 0.23 +-0.16 | 0.21 +-0.15 | 0.24 +-0.21 | 0.13 +-0.10 | 0.15 +-0.08 | 0.19 +-0.10 |
| **Tris CS** | 0.12 +-0.23 | 0.05 +-0.08 | 0.03 +-0.03 | 0.05 +-0.04 | 0.24 +-0.28 | 0.13 +-0.21 | 0.06 +-0.09 | 0.01 +-0.01 | 0.09 +-0.08 | 0.06 +-0.09 | 0.04 +-0.04 | 0.03 +-0.02 |
| **4s/6s CS** | 37.9 0 +-34.1 2 | 10.1 5 +-2.72 | 9.34 +-5.56 | 7.38 +-2.05 | 7.13 +-2.43 | 6.95 +-1.98 | 9.70 +-5.42 | 8.11 +-3.71 | 6.82 +-1.78 | 10.8 7 +-3.82 | 6.86 +-2.35 | 10.3 3 +-4.47 |
| **6s/0s CS** | 0.01 +-0.01 | 0.05 +-0.02 | 0.10 +-0.19 | 0.12 +-0.10 | 0.10 +-0.04 | 0.14 +-0.13 | 0.06 +-0.03 | 0.07 +-0.05 | 0.10 +-0.09 | 0.10 +-0.20 | 0.17 +-0.16 | 0.04 +-0.02 |

| | H | BC | CR C | CS T | DG | EC | GN ET | LL | NH L | LC | OV | PCa |
|---|---|---|---|---|---|---|---|---|---|---|---|---|
| **4s/0s CS** | 0.26 +-0.10 | 0.54 +-0.14 | 0.34 +-0.21 | 0.74 +-0.51 | 0.69 +-0.28 | 0.80 +-0.54 | 0.46 +-0.20 | 0.40 +-0.18 | 0.59 +-0.27 | 0.62 +-0.65 | 0.88 +-0.55 | 0.33 +-0.04 |
| **Charge CS** | 0.20 +-0.05 | 0.37 +-0.07 | 0.29 +-0.14 | 0.43 +-0.12 | 0.46 +-0.10 | 0.46 +-0.13 | 0.35 +-0.09 | 0.32 +-0.11 | 0.40 +-0.10 | 0.36 +-0.16 | 0.47 +-0.15 | 0.28 +-0.04 |
| **Tris HS** | 0.87 +-1.30 | 0.47 +-0.41 | 0.26 +-0.28 | 0.54 +-0.37 | 0.43 +-0.24 | 0.54 +-0.49 | 0.43 +-0.36 | 0.32 +-0.21 | 0.79 +-0.51 | 0.31 +-0.27 | 0.87 +-0.81 | 0.38 +-0.22 |
| **Ns6s HS** | 1.51 +-1.67 | 0.72 +-0.64 | 0.40 +-0.32 | 0.85 +-0.51 | 3.61 +-8.35 | 0.66 +-0.32 | 0.47 +-0.36 | 0.47 +-0.34 | 0.90 +-0.57 | 0.47 +-0.45 | 2.10 +-2.79 | 0.92 +-0.71 |
| **Ns2s HS** | 1.54 +-1.85 | 0.72 +-0.77 | 0.55 +-0.59 | 2.04 +-1.32 | 1.95 +-3.63 | 0.82 +-0.41 | 0.59 +-0.36 | 0.61 +-0.39 | 1.69 +-1.18 | 0.46 +-0.32 | 2.23 +-2.74 | 0.69 +-0.38 |
| **Ns HS** | 19.2 7 +-12.3 8 | 28.2 5 +-15.9 6 | 16.8 7 +-10.6 1 | 17.8 6 +-16.6 6 | 31.5 0 +-16.4 7 | 19.5 4 +-16.0 0 | 27.8 4 +-12.3 5 | 31.1 5 +-11.4 5 | 31.5 7 +-8.71 | 26.4 8 +-22.1 6 | 12.5 6 +-12.7 7 | 22.9 7 +-15.6 5 |
| **2s6s HS** | 1.35 +-1.87 | 0.79 +-0.79 | 0.25 +-0.20 | 0.42 +-0.33 | 0.76 +-1.34 | 0.89 +-0.89 | 0.25 +-0.14 | 0.21 +-0.15 | 1.54 +-3.68 | 0.39 +-0.25 | 1.70 +-2.37 | 0.99 +-2.12 |
| **6s HS** | 12.9 2 +-15.4 9 | 25.6 4 +-16.2 2 | 30.8 6 +-21.8 2 | 18.2 4 +-16.1 9 | 22.2 7 +-18.5 1 | 14.9 8 +-13.6 6 | 33.3 1 +-16.4 9 | 29.4 1 +-20.9 0 | 19.2 2 +-12.7 2 | 49.8 1 +-30.1 5 | 9.48 +-8.57 | 30.6 7 +-24.4 6 |
| **2s HS** | 4.55 +-3.48 | 4.29 +-3.55 | 16.5 6 +-11.6 5 | 3.72 +-3.27 | 6.47 +-9.07 | 3.65 +-2.28 | 10.0 3 +-6.16 | 8.77 +-6.07 | 4.31 +-3.19 | 4.19 +-3.91 | 5.52 +-4.70 | 12.9 6 +-6.62 |
| **0s HS** | 58.0 0 +-21.9 3 | 39.1 6 +-28.6 4 | 34.2 6 +-22.1 1 | 56.3 6 +-28.6 5 | 33.0 0 +-26.2 5 | 58.9 3 +-24.5 2 | 27.1 1 +-16.2 6 | 29.1 0 +-15.0 7 | 40.0 2 +-11.1 8 | 17.7 9 +-8.86 | 65.5 7 +-15.9 0 | 30.4 6 +-21.7 8 |
| **Charge HS** | 0.48 +-0.24 | 0.64 +-0.27 | 0.67 +-0.22 | 0.48 +-0.27 | 0.74 +-0.29 | 0.45 +-0.25 | 0.75 +-0.16 | 0.73 +-0.14 | 0.66 +-0.12 | 0.84 +-0.08 | 0.42 +-0.17 | 0.73 +-0.20 |

EP 4 614 153 A2

111

Embodiments of the invention are also described in the claims of the International application as filed:

1. A method of screening for a cancer selected from the group consisting of prostate cancer, thyroid cancer, colon cancer, rectum cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer, skin cancer, melanoma and a neuroendocrine tumour in a subject, said method comprising determining the level and/or chemical composition of one or more of the glycosaminoglycans (GAGs) chondroitin sulfate (CS), heparan sulfate (HS), and hyaluronic acid (HA) in a body fluid sample, wherein said sample has been obtained from said subject.

2. The method of claim 1, wherein an altered level and/or chemical composition of chondroitin sulfate (CS) and/or heparan sulfate (HS) and/or hyaluronic acid (HA) in said sample in comparison to a control level and/or chemical composition is indicative of said cancer in said subject.

3. The method of claim 1 or claim 2, wherein said determination of the chemical composition comprises determining the level in the sample of one or more GAG properties selected from the group consisting of: one or more of the specific sulfated or unsulfated forms of CS or HS disaccharides, the fraction of sulfated disaccharides of HS out of the total HS disaccharides present (charge HS), the fraction of sulfated disaccharides of CS out of the total CS disaccharides present (charge CS), the total concentration of CS, the total concentration of HS and the total concentration of HA.

4. The method of any one of claims 1 to 3, wherein said determination of the chemical composition comprises determining the level in the sample of one or more of the GAG properties selected from the group consisting of: the specific sulfated or unsulfated forms of CS or HS disaccharides selected from the group consisting of: 0s CS, 2s CS, 6s CS, 4s CS, 2s6s CS, 2s4s CS, 4s6s CS, Tris CS, 0s HS, 2s HS, 6s HS, 2s6s HS, Ns HS, Ns2s HS, Ns6s HS, Tris HS, the relative level of 4s CS with respect to 6s CS, the relative level of 6s CS with respect to 0s CS and the relative level of 4s CS with respect to 0s CS; the fraction of sulfated disaccharides of HS out of the total HS disaccharides present (charge HS); the fraction of sulfated disaccharides of CS out of the total CS disaccharides present (charge CS); the total concentration of CS (CS tot); the total concentration of HS (HS tot); and the total concentration of HA (HA tot).

5. The method of claim 4, wherein said method comprises determining the level of up to 8, or all 8, of the sulfated and unsulfated CS forms: 0s CS, 2s CS, 6s CS, 4s CS, 2s6s CS, 2s4s CS, 4s6s CS and Tris CS, optionally together with total CS and/or charge CS; and/or

wherein said method comprises determining the level of up to 8, or all 8, of the sulfated and unsulfated HS forms: 0s HS, 2s HS, 6s HS, 2s6s HS, Ns HS, Ns2s HS, Ns6s HS and Tris HS, optionally together with total HS and/or charge HS; and/or
wherein said method comprises determining the total concentration of HA.

6. The method of any one of claims 1 to 5, wherein said determination of the chemical composition comprises determining the level in the sample of one or more of the GAG properties selected from the group consisting of: 6s CS, the ratio 6s CS/0s CS, the ratio 4s CS/6s CS, 0s CS, Ns2s HS, 4s CS.

7. The method of any one of claims 1 to 6, wherein said body fluid is a blood sample and wherein said determination of the chemical composition comprises determining the level in the sample of one or more of the GAG properties 6s CS, the ratio 6s CS/0s CS, the ratio 4s CS/6s CS and 0s CS.

8. The method of any one of claims 1 to 6, wherein said body fluid is a urine sample and wherein said determination of the chemical composition comprises determining the level in the sample of one or both of the GAG properties Ns2s Hs and 4s CS.

9. The method of any one claims 1 to 8, wherein said determination of the chemical composition comprises determining the level in the sample of one or more of the GAG properties 0s CS, 6s CS, the ratio 4s CS/6s CS, Charge CS, Ns HS, 4s CS, the ratio 6s CS/0s CS, the ratio 4s CS/0s CS, 0s HS, Charge HS.

10. The method of any one of claims 1 to 7 or 9, wherein said body fluid is a blood sample and wherein said determination of the chemical composition comprises determining the level in the sample of one or more of the GAG properties 0s CS, 6s CS, the ratio 4s CS/6s CS, Charge CS, Ns HS.

11. The method of any one of claims 1 to 6, 8 or 9 wherein said body fluid is a urine sample and wherein said determination of the chemical composition comprises determining the level in the sample of one or more of the GAG properties 4s CS, the ratio 6s CS/0s CS, the ratio 4s CS/0s CS, 0s HS, Charge HS.

12. The method of any one of claims 1 to 7, 9 or 10, wherein said body fluid sample is a blood sample and wherein an increase in the level of one or more or all of the GAG properties selected from the group consisting of 6s CS and Charge CS, in comparison to a control level, is indicative of cancer in said subject.

13. The method of any one of claims 1 to 7, 9, 10 or 12, wherein said body fluid sample is a blood sample and wherein a decrease in the level of one or more or all of the GAG properties selected from the group consisting of 0s CS, the ratio 4s CS/6s CS or Ns HS, in comparison to a control level, is indicative of cancer in said subject.

14. The method of any one of claims 1 to 6, 8, 9 or 11, wherein said body fluid sample is a urine sample and wherein an increase in the level of Charge HS, in comparison to a control level, is indicative of cancer in said subject.

15. The method of any one of claims 1 to 6, 8, 9, 11 or 14, wherein said body fluid sample is a urine sample and wherein a decrease in the level of one or more or all of the GAG properties selected from the group consisting of 4s CS, the ratio 6s CS/0s CS, the ratio 4s CS/0s CS or 0s HS, is indicative of cancer in said subject.

16. The method of any one of claims 1 to 5, wherein said body fluid sample is blood and wherein said method comprises determining the level in the sample of one or more of the GAG properties Charge CS, Total CS, 6s CS, 4s CS, 2s HS, 0s HS.

17. The method of any one of claims 1 to 5, wherein said body fluid sample is blood and wherein said method comprises determining the level in the sample of one or more of the GAG properties Charge CS, Total CS, Total HS, 0s CS, 2s CS, 6s CS, 4s CS, the relative level of 4s CS with respect to 6s CS (e.g. the ratio 4s CS/6s CS or the inverse ratio 6s CS/4s CS), the relative level of 6s CS with respect to 0s CS (e.g. the ratio 6s CS/0s CS or the inverse ratio 0s CS/6s CS) or the relative level of 4s CS with respect to 0s CS (e.g. the ratio 4s CS/0s CS or the inverse ratio 0s CS/4s CS).

18. The method of any one of claims 1 to 5 or 17, wherein said body fluid sample is blood and wherein an alteration in the level of one or more of the GAG properties Charge CS, Total CS, Total HS, 0s CS, 2s CS, 6s CS, 4s CS, the relative level of 4s CS with respect to 6s CS (e.g. the ratio 4s CS/6s CS or the inverse ratio 6s CS/4s CS), the relative level of 6s CS with respect to 0s CS (e.g. the ratio 6s CS/0s CS or the inverse ratio 0s CS/6s CS) or the relative level of 4s CS with respect to 0s CS (e.g. the ratio 4s CS/0s CS or the inverse ratio 0s CS/4s CS), in comparison to a control level, is indicative of cancer in said subject.

19. The method of any one of claims 1 to 5, 17 or 18, wherein said body fluid sample is blood and wherein an increase in the level of one or more of the GAG properties Charge CS, Total CS, Total HS, 6s CS, 4s CS, the ratio 6s CS/0s CS or the ratio 4s CS/0s CS, in comparison to a control level, is indicative of cancer in said subject.

20. The method of any one of claims 1 to 5, 17 or 18, wherein said body fluid sample is blood and wherein a decrease in the level of one or more of the GAG properties 0s CS, 2s CS or the ratio 4s CS/6s CS, in comparison to a control level, is indicative of cancer in said subject.

21. The method of any one of claims 1 to 15, wherein said cancer is prostate cancer.

22. The method of any one of claims 1 to 5 or 16, wherein said determination of the chemical composition comprises determining the level in the sample of one or more of the GAG properties selected from the group consisting of: 4s CS, 0s CS, Total HA and Ns2s HS.

23. The method of any one of claims 1 to 5 or 16 or 17, wherein said body fluid sample is a blood sample and wherein said determination of the chemical composition comprises determining the level in the sample of one or both of the GAG properties 4s CS and 0s CS.

24. The method of any one of claims 1 to 5 or 16 or 17, wherein said body fluid sample is a urine sample and wherein said determination of the chemical composition comprises determining the level in the sample of one or more or all of the GAG properties 4s CS, the total concentration of HA and Ns2s HS.

25. The method of any one of claims 1 to 5 or 16 or 17, wherein said body fluid sample is a blood sample and wherein said determination of the chemical composition comprises determining the level in the sample of one or more or all of the GAG properties selected from the group consisting of 0s CS, 2s CS, 6s CS, 4s CS, 2s6s CS, 2s4s CS, 4s6s CS, Tris CS, charge CS, Total CS, the relative level of 4s CS with respect to 6s CS, the relative level of 6s CS with respect to 0s CS, the relative level of 4s CS with respect to 0s CS, Tris HS, Ns2s HS, 2s6s HS, 6s HS and Total HS.

26. The method of any one of claims 1 to 5 or 16, 17, 18 or 20, wherein said body fluid sample is a blood sample and wherein an increase in the level of one or more or all of the GAG properties selected from the group consisting of 2s CS, 6s CS, 4s CS, 2s6s CS, 2s4s CS, 4s6s CS, Tris CS, charge CS, the ratio 6s CS/4s CS, the ratio 6s CS/0s CS, the the ratio 4s CS/0s CS, Tris HS, Ns2s HS, 6s HS and Total HS, in comparison to a control level, is indicative of prostate cancer in said subject.

27. The method of any one of claims 1 to 5 or 16, 17, 18 or 20, wherein said body fluid sample is a blood sample and wherein a decrease in the level of one or more or all of the GAG properties selected from the group consisting of 0s CS, the ratio 4s CS/6s, Total CS and 2s6s HS, in comparison to a control level, is indicative of prostate cancer in said subject.

28. The method of any one of claims 1 to 5 or 16, 17 or 19, wherein said body fluid sample is a urine sample and wherein said determination of the chemical composition comprises determining the level in the sample of one or more or all of the GAG properties selected from the group consisting of 4s CS, 2s6s CS, 2s4s CS, Tris CS, Total CS, the relative level of 4s CS with respect to 6s CS, the relative level of 4s CS with respect to 0s CS, charge HS, HS tot, 2s HS, Ns2s HS, Ns6s HS, Tris HS and Total HA.

29. The method of any one of claims 1 to 5 or 16, 17, 19 or 23, wherein said body fluid sample is a urine sample and wherein an increase in the level of one or more or all of the GAG properties selected from the group consisting of 2s6s CS, 2s4s CS, Tris CS, Total CS, the ratio 6s CS/4s CS, the ratio 0s CS/4s CS, charge HS, Total HS, Ns2s HS, Ns6s HS, Tris HS and Total HA, in comparison to a control level, is indicative of prostate cancer in said subject.

30. The method of any one of claims 1 to 5 or 16, 17, 19, 23 or 24, wherein said body fluid sample is a urine sample and wherein a decrease in the level of one or more or all of the GAG properties selected from the group consisting of 4s CS, the ratio 4s CS/6s CS, the ratio 4s CS/0s CS and 2s HS, in comparison to a control level, is indicative of prostate cancer in said subject.

31. The method of any one of claims 1 to 5 or 16,

wherein said body fluid is blood and said determination of the chemical composition comprises determining the level in the sample of one or more or all of the GAG properties selected from the group consisting of 0s CS, 2s CS, 6s CS, 4s CS, 2s6s CS, 2s4s CS, 4s6s CS, Tris CS, charge CS, Total CS, the relative level of 4s CS with respect to 6s CS, the relative level of 6s CS with respect to 0s CS, the relative level of 4s CS with respect to 0s CS, Tris HS, Ns2s HS, 2s6s HS, 6s HS and Total HS; or
wherein said body fluid is urine and said determination of the chemical composition comprises determining the level in the sample of one or more or all of the GAG properties selected from the group consisting of 2s6s CS, 2s4s CS, Tris CS, charge HS, 2s HS, Ns2s HS, Ns6s HS and Tris HS.

32. The method of any one of claims 1 to 5 or 16,

wherein said body fluid is blood and said determination of the chemical composition comprises determining the level in the sample of one or more or all of the GAG properties selected from the group consisting of 2s CS, 2s6s CS, 2s4s CS, 4s6s CS, Tris CS, charge CS, Tris HS, Ns2s HS, 2s6s HS or 6s HS; or
wherein said body fluid is urine and said determination of the chemical composition comprises determining the level in the sample of one or more or all of the GAG properties selected from the group consisting of 2s6s CS, 2s4s CS, Tris CS, charge HS, 2s HS, Ns2s HS, Ns6s HS and Tris HS.

33. The method of any one of claims 1 to 5, wherein said cancer is melanoma and said method comprises determining the level in the sample of one or more of the GAG properties 6s CS, 4s6s CS, the relative level of 6s CS with respect to 4s CS, the relative level of 6s CS with respect to 0s CS, Total CS, 2s HS, 0s HS and Total HS.

34. The method of any one of claims 1 to 5 or 33, wherein said cancer is melanoma and wherein an alteration in the level of one or more of the GAG properties 6s CS, 4s6s CS, the relative level of 6s CS with respect to 4s CS, the relative level of 6s CS with respect to 0s CS, Total CS, 2s HS, 0s HS and Total HS, in comparison to a control level, is indicative of melanoma in said subject.

35. The method of any one of claims 1 to 5, 33 or 34, wherein said cancer is melanoma and wherein

an increase in the level of one or more of the GAG properties: 6s CS, 4s6s CS, the ratio 6s CS/0s CS, Total CS and Total HS; and/or
a decrease in the level of one or more of the GAG properties: the ratio 4s CS/6s CS, 2s HS and 0s HS,
in comparison to a control level, is indicative of melanoma in said subject.

36. The method of any one of claims 1 to 5, wherein said cancer is colon cancer or rectum cancer and said method comprises determining the level in the sample of one or more of the GAG properties 2s CS, 6s CS, 2s6s CS, 2s4s CS, 4s6s CS, Tris CS, the relative level of 6s CS with respect to 4s CS, the relative level of 6s CS with respect to 0s CS, Total CS, Ns6s HS, Ns2s HS, 2s6s HS, 6s HS, 2s HS, 0s HS, Charge HS and Total HS.

37. The method of any one of claims 1 to 5 or 36, wherein said cancer is colon cancer or rectum cancer and wherein an alteration in the level of one or more of the GAG properties 2s CS, 6s CS, 2s6s CS, 2s4s CS, 4s6s CS, Tris CS, the relative level of 6s CS with respect to 4s CS, the relative level of 6s CS with respect to 0s CS, Total CS, Ns6s HS, Ns2s HS, 2s6s HS, 6s HS, 2s HS, 0s HS, Charge HS and Total HS, in comparison to a control level, is indicative of colon cancer or rectum cancer in said subject.

38. The method of any one of claims 1 to 5, 36 or 37, wherein said cancer is colon cancer or rectum cancer and wherein

an increase in the level of one or more of the GAG properties: 2s CS, 6s CS, 2s6s CS, 2s4s CS, 4s6s CS, Tris CS, the ratio 6s CS/0s CS, Total CS, 6s HS, 2s HS, Charge HS and Total HS; and/or
a decrease in the level of one or more of the GAG properties: the ratio 4s CS/6s CS, Ns6s HS, Ns2s HS, 2s6s HS and 0s HS,
in comparison to a control level, is indicative of colon cancer or rectum cancer in said subject.

39. The method of any one of claims 1 to 5, wherein said cancer is a neuroendocrine tumour, preferably a gastrointestinal neuroendocrine tumour, and said method comprises determining the level in the sample of one or more of the GAG properties 0s CS, 6s CS, 4s CS, 2s6s CS, 2s4s CS, 4s6s CS, Tris CS, the relative level of 6s CS with respect to 4s CS, the relative level of 6s CS with respect to 0s CS, the relative level of 4s CS with respect to 0s CS, Charge CS, Ns6s HS, Ns HS, 6s HS, 2s HS, 0s HS, Charge HS and Total HS.

40. The method of any one of claims 1 to 5 or 39, wherein said cancer is a neuroendocrine tumour, preferably a gastrointestinal neuroendocrine tumour, and wherein an alteration in the level of one or more of the GAG properties 0s CS, 6s CS, 4s CS, 2s6s CS, 2s4s CS, 4s6s CS, Tris CS, the relative level of 6s CS with respect to 4s CS, the relative level of 6s CS with respect to 0s CS, the relative level of 4s CS with respect to 0s CS, Charge CS, Ns6s HS, Ns HS, 6s HS, 2s HS, 0s HS, Charge HS and Total HS, in comparison to a control level, is indicative of a neuroendocrine tumour, preferably a gastrointestinal neuroendocrine tumour, in said subject.

41. The method of any one of claims 1 to 5, 39 or 40, wherein said cancer is a neuroendocrine tumour, preferably a gastrointestinal neuroendocrine tumour, and wherein

an increase in the level of one or more of the GAG properties: 6s CS, 4s CS, 2s6s CS, 2s4s CS, 4s6s CS, Tris CS, the ratio 6s CS/0s CS, the ratio 4s CS/0s CS, Charge CS, Ns HS, 6s HS, 2s HS, Charge HS and Total HS; and/or
a decrease in the level of one or more of the GAG properties: 0s CS, the ratio 4s CS/6s CS, Ns6s HS and 0s HS,
in comparison to a control level, is indicative of a neuroendocrine tumour, preferably a gastrointestinal neuroendocrine tumour, in said subject.

42. The method of any one of claims 1 to 5, wherein said cancer is blood cancer, preferably chronic lymphoid leukaemia, and said method comprises determining the level in the sample of one or more of the GAG properties 0s CS, 6s CS, 4s CS, 2s6s CS, 2s4s CS, 4s6s CS, the relative level of 6s CS with respect to 4s CS, the relative level of 6s CS with respect to 0s CS, the relative level of 4s CS with respect to 0s CS, Charge CS, Total CS, Ns6s HS, Ns HS, 2s6s HS, 6s HS, 2s HS, 0s HS, Charge HS and Total HS.

43. The method of any one of claims 1 to 5 or 42, wherein said cancer is blood cancer, preferably chronic lymphoid leukaemia, and wherein an alteration in the level of one or more of the GAG properties 0s CS, 6s CS, 4s CS, 2s6s CS, 2s4s CS, 4s6s CS, the relative level of 6s CS with respect to 4s CS, the relative level of 6s CS with respect to 0s CS, the relative level of 4s CS with respect to 0s CS, Charge CS, Total CS, Ns6s HS, Ns HS, 2s6s HS, 6s HS, 2s HS, 0s HS, Charge HS and Total HS, in comparison to a control level, is indicative of blood cancer, preferably chronic lymphoid leukaemia, in said subject.

44. The method of any one of claims 1 to 5, 42 or 43, wherein said cancer is blood cancer, preferably chronic lymphoid leukaemia, and wherein

an increase in the level of one or more of the GAG properties: 6s CS, 4s CS, 2s6s CS, 2s4s CS, 4s6s CS, the ratio 6s CS/0s CS, the ratio 4s CS/0s CS, Charge CS, Total CS, Ns HS, 6s HS, 2s HS, Charge HS and Total HS; and/or a decrease in the level of one or more of the GAG properties: 0s CS, the ratio 4s CS/6s CS, Ns6s HS, 2s6s HS and 0s HS, in comparison to a control level, is indicative of blood cancer, preferably chronic lymphoid leukaemia, in said subject.

45. The method of any one of claims 1 to 5, wherein said cancer is bladder cancer and said method comprises determining the level in the sample of one or more of the GAG properties 0s CS, 2s CS, 6s CS, Tris CS, the relative level of 6s CS with respect to 0s CS, the relative level of 4s CS with respect to 0s CS, Charge CS, Tris HS, Ns2s HS, Ns HS, 2s HS and Total HS.

46. The method of any one of claims 1 to 5 or 45, wherein said cancer is bladder cancer and wherein an alteration in the level of one or more of the GAG properties 0s CS, 2s CS, 6s CS, Tris CS, the relative level of 6s CS with respect to 0s CS, the relative level of 4s CS with respect to 0s CS, Charge CS, Tris HS, Ns2s HS, Ns HS, 2s HS and Total HS, in comparison to a control level, is indicative of bladder cancer in said subject.

47. The method of any one of claims 1 to 5, 45 or 46, wherein said cancer is bladder cancer and wherein

an increase in the level of one or more of the GAG properties: 2s CS, 6s CS, the ratio 6s CS/0s CS, the ratio 4s CS/0s CS, Charge CS, Tris HS, Ns2s HS, 2s HS and Total HS; and/or a decrease in the level of one or more of the GAG properties: 0s CS, Tris CS and Ns HS, in comparison to a control level, is indicative of bladder cancer in said subject.

48. The method of any one of claims 1 to 5, wherein said cancer is breast cancer and said method comprises determining the level in the sample of one or more of the GAG properties 0s CS, 6s CS, 4s CS, 4s6s CS, the relative level of 6s CS with respect to 4s CS, the relative level of 6s CS with respect to 0s CS, the relative level of 4s CS with respect to 0s CS, Charge CS, Total CS, Ns2s HS, Ns HS, 6s HS, 0s HS and Total HS.

49. The method of any one of claims 1 to 5 or 48, wherein said cancer is breast cancer and wherein an alteration in the level of one or more of the GAG properties 0s CS, 6s CS, 4s CS, 4s6s CS, the relative level of 6s CS with respect to 4s CS, the relative level of 6s CS with respect to 0s CS, the relative level of 4s CS with respect to 0s CS, Charge CS, Total CS, Ns2s HS, Ns HS, 6s HS, 0s HS and Total HS, in comparison to a control level, is indicative of breast cancer in said subject.

50. The method of any one of claims 1 to 5, 48 or 49, wherein said cancer is breast cancer and wherein

an increase in the level of one or more of the GAG properties: 6s CS, 4s CS, 4s6s CS, the ratio 6s CS/0s CS, the ratio 4s CS/0s CS, Charge CS, Total CS, Ns HS, 6s HS and Total HS; and/or a decrease in the level of one or more of the GAG properties: 0s CS, the ratio 4s CS/6s CS, Ns2s HS and 0s HS, in comparison to a control level, is indicative of breast cancer in said subject.

51. The method of any one of claims 1 to 5, wherein said cancer is ovarian cancer and said method comprises determining the level in the sample of one or more of the GAG properties 0s CS, 6s CS, 4s CS, 2s6s CS, 2s4s CS, 4s6s CS, the relative level of 6s CS with respect to 4s CS, the relative level of 6s CS with respect to 0s CS, the relative level of 4s CS with respect to 0s CS, Charge CS, Total CS, Total HS and Total HA.

52. The method of any one of claims 1 to 5 or 51, wherein said cancer is ovarian cancer and wherein an alteration in the level of one or more of the GAG properties 0s CS, 6s CS, 4s CS, 2s6s CS, 2s4s CS, 4s6s CS, the relative level of 6s CS with respect to 4s CS, the relative level of 6s CS with respect to 0s CS, the relative level of 4s CS with respect to 0s CS, Charge CS, Total CS, Total HS and Total HA, in comparison to a control level, is indicative of ovarian cancer in said subject.

53. The method of any one of claims 1 to 5, 51 or 52, wherein said cancer is ovarian cancer and wherein

an increase in the level of one or more of the GAG properties: 6s CS, 4s CS, 2s6s CS, 2s4s CS, 4s6s CS, the ratio 6s CS/0s CS, the ratio 4s CS/0s CS, Charge CS, Total CS, Total HA, and Total HS ; and/or
a decrease in the level of one or both of the GAG properties: 0s CS and the ratio 4s CS/6s CS,
in comparison to a control level, is indicative of ovarian cancer in said subject.

54. The method of any one of claims 1 to 5, wherein said cancer is uterine cancer, preferably endometrial cancer, and said method comprises determining the level in the sample of one or more of the GAG properties 0s CS, 6s CS, 4s CS, 2s4s CS, 4s6s CS, the relative level of 6s CS with respect to 4s CS, the relative level of 6s CS with respect to 0s CS, the relative level of 4s CS with respect to 0s CS, Charge CS, Total CS, Total HS and Total HA.

55. The method of any one of claims 1 to 5 or 54, wherein said cancer is uterine cancer, preferably endometrial cancer, and wherein an alteration in the level of one or more of the GAG properties 0s CS, 6s CS, 4s CS, 2s4s CS, 4s6s CS, the relative level of 6s CS with respect to 4s CS, the relative level of 6s CS with respect to 0s CS, the relative level of 4s CS with respect to 0s CS, Charge CS, Total CS, Total HS and Total HA, in comparison to a control level, is indicative of uterine cancer, preferably endometrial cancer, in said subject.

56. The method of any one of claims 1 to 5, 54 or 55, wherein said cancer is uterine cancer, preferably endometrial cancer, and wherein

an increase in the level of one or more of the GAG properties: 6s CS, 4s CS, 2s4s CS, 4s6s CS, the ratio 6s CS/0s CS, the ratio 4s CS/0s CS, Charge CS, Total CS, Total HA and Total HS; and/or
a decrease in the level of one or both of the GAG properties: 0s CS and the ratio 4s CS/6s CS,
in comparison to a control level, is indicative of uterine cancer, preferably endometrial cancer, in said subject.

57. The method of any one of claims 1 to 5, wherein said cancer is uterine cancer, preferably cervical cancer, and said method comprises determining the level in the sample of one or more of the GAG properties 0s CS, 6s CS, 4s CS, 2s4s CS, 4s6s CS, the relative level of 6s CS with respect to 4s CS, the relative level of 6s CS with respect to 0s CS, the relative level of 4s CS with respect to 0s CS, Charge CS, Total CS, Ns2s HS, 6s HS, Total HS and Total HA.

58. The method of any one of claims 1 to 5 or 57, wherein said cancer is uterine cancer, preferably cervical cancer, and wherein an alteration in the level of one or more of the GAG properties 0s CS, 6s CS, 4s CS, 2s4s CS, 4s6s CS, the relative level of 6s CS with respect to 4s CS, the relative level of 6s CS with respect to 0s CS, the relative level of 4s CS with respect to 0s CS, Charge CS, Total CS, Ns2s HS, 6s HS, Total HS and Total HA, in comparison to a control level, is indicative of uterine cancer, preferably cervical cancer, in said subject.

59. The method of any one of claims 1 to 5, 57 or 58, wherein said cancer is uterine cancer, preferably cervical cancer, and wherein

an increase in the level of one or more of the GAG properties: 6s CS, 4s CS, 2s4s CS, 4s6s CS, the ratio 6s CS/0s CS, the ratio 4s CS/0s CS, Charge CS, Total CS, Total HA, Ns2s HS, 6s HS and Total HS; and/or
a decrease in the level of one or both of the GAG properties: 0s CS and the ratio 4s CS/6s CS,
in comparison to a control level, is indicative of uterine cancer, preferably cervical cancer, in said subject.

60. The method of any one of claims 1 to 5, wherein said cancer is blood cancer, preferably non-Hodgkin's lymphoma, and said method comprises determining the level in the sample of one or more of the GAG properties 0s CS, 6s CS, 4s CS, 2s6s CS, 2s4s CS, 4s6s CS, Tris CS, the relative level of 6s CS with respect to 4s CS, the relative level of 6s CS with respect to 0s CS, the relative level of 4s CS with respect to 0s CS, Charge CS, Total CS, Tris HS, Ns2s HS, Ns HS, 6s HS, 0s HS, Charge HS and Total HS.

61. The method of any one of claims 1 to 5 or 61, wherein said cancer is blood cancer, preferably non-Hodgkin's lymphoma, and wherein an alteration in the level of one or more of the GAG properties 0s CS, 6s CS, 4s CS, 2s6s CS, 2s4s CS, 4s6s CS, Tris CS, the relative level of 6s CS with respect to 4s CS, the relative level of 6s CS with respect to 0s CS, the relative level of 4s CS with respect to 0s CS, Charge CS, Total CS, Tris HS, Ns2s HS, Ns HS, 6s HS, 0s HS, Charge HS and Total HS, in comparison to a control level, is indicative of blood cancer, preferably non-Hodgkin's lymphoma, in said subject.

62. The method of any one of claims 1 to 5, 60 or 61, wherein said cancer is blood cancer, preferably non-Hodgkin's lymphoma, and wherein

an increase in the level of one or more of the GAG properties: 6s CS, 4s CS, 2s6s CS, 2s4s CS, 4s6s CS, Tris CS, the ratio 6s CS/0s CS, the ratio 4s CS/0s CS, Charge CS, Total CS, Tris HS, Ns2s HS, Ns HS, 6s HS, Charge HS and Total HS; and/or a decrease in the level of one or more of the GAG properties: 0s CS, the ratio 4s CS/6s CS and 0s HS,
in comparison to a control level, is indicative of blood cancer, preferably non-Hodgkin's lymphoma, in said subject.

63. The method of any one of claims 1 to 5, wherein said cancer is brain cancer, preferably diffuse glioma, and said method comprises determining the level in the sample of one or more of the GAG properties 0s CS, 6s CS, 4s CS, 2s4s CS, 4s6s CS, Tris CS, the relative level of 6s CS with respect to 4s CS, the relative level of 6s CS with respect to 0s CS, the relative level of 4s CS with respect to 0s CS, Charge CS, Total CS, Ns HS, 6s HS, 0s HS, Charge HS, Total HS and Total HA.

64. The method of any one of claims 1 to 5 or 63, wherein said cancer is brain cancer, preferably diffuse glioma, and wherein an alteration in the level of one or more of the GAG properties 0s CS, 6s CS, 4s CS, 2s4s CS, 4s6s CS, Tris CS, the relative level of 6s CS with respect to 4s CS, the relative level of 6s CS with respect to 0s CS, the relative level of 4s CS with respect to 0s CS, Charge CS, Total CS, Ns HS, 6s HS, 0s HS, Charge HS, Total HS and Total HA, in comparison to a control level, is indicative of brain cancer, preferably diffuse glioma, in said subject.

65. The method of any one of claims 1 to 5, 63 or 64, wherein said cancer is brain cancer, preferably diffuse glioma, and wherein

an increase in the level of one or more of the GAG properties: 6s CS, 4s CS, 2s4s CS, 4s6s CS, Tris CS, the ratio 6s CS/0s CS, the ratio 4s CS/0s CS, Charge CS, Total CS, Total HA, Ns HS, 6s HS, Charge HS and Total HS; and/or a decrease in the level of one or more of the GAG properties: 0s CS, the ratio 4s CS/6s CS and 0s HS,
in comparison to a control level, is indicative of brain cancer, preferably diffuse glioma, in said subject.

66. The method of any one of claims 1 to 5, wherein said cancer is lung cancer and said method comprises determining the level in the sample of one or more of the GAG properties 0s CS, 2s CS, 6s CS, 4s CS, 2s4s CS, Tris CS, the relative level of 6s CS with respect to 4s CS, the relative level of 6s CS with respect to 0s CS, the relative level of 4s CS with respect to 0s CS, Charge CS, Total CS, Ns6s HS, Ns2s HS, 6s HS, 0s HS, Charge HS and Total HS.

67. The method of any one of claims 1 to 5 or 66, wherein said cancer is lung cancer and wherein an alteration in the level of one or more of the GAG properties: 0s CS, 2s CS, 6s CS, 4s CS, 2s4s CS, Tris CS, the relative level of 6s CS with respect to 4s CS, the relative level of 6s CS with respect to 0s CS, the relative level of 4s CS with respect to 0s CS, Charge CS, Total CS, Ns6s HS, Ns2s HS, 6s HS, 0s HS, Charge HS and Total HS, in comparison to a control level, is indicative of lung cancer in said subject.

68. The method of any one of claims 1 to 5, 66 or 67, wherein said cancer is lung cancer and wherein

an increase in the level of one or more of the GAG properties: 2s CS, 6s CS, 4s CS, 2s4s CS, Tris CS, the ratio 6s CS/0s CS, ratio 4s CS/0s CS, Charge CS, Total CS, 6s HS, Charge HS and Total HS; and/or a decrease in the level of one or more of the GAG properties: 0s CS, the ratio 4s CS/6s CS, Ns6s HS, Ns2s HS and 0s HS,
in comparison to a control level, is indicative of lung cancer in said subject.

69. The method of any one of claims 1 to 68, wherein said method comprises determining the level of more than one of said GAG properties.

70. The method of any one of claims 1 to 69, wherein said method comprises determining the level of two or more, three or more, four or more, or all, of said GAG properties.

71. The method of any one of claims 1 to 70, wherein said method is used for diagnosing said cancer.

72. The method of any one of claims 1 to 70, wherein said method is used for the prognosis of said cancer, for monitoring subjects at risk of the occurrence of said cancer, for monitoring the progression of said cancer in a subject, for determining the clinical severity of said cancer, for predicting the response of a subject to therapy or surgery for said cancer, for determining the efficacy of a therapeutic or surgical regime being used to treat said cancer, for detecting the recurrence or relapse of said cancer, for patient selection or treatment selection or for distinguishing small masses suspicious of said cancer from other non-malignant diseases.

73. The method of any one of claims 1 to 32 or 69 to 72, wherein said prostate cancer is prostate adenocarcinoma.

74. The method of any one of claims 1 to 73, wherein said subject is a subject at risk of developing said cancer, or at risk of the occurrence of said cancer, or is a subject having or suspected of having said cancer.

75. The method of any one of claims 1 to 6, 8, 9, 11, 14, 15, 21, 22, 24 or 28 to 74, wherein said body fluid sample is urine.

76. The method of any one of claims 1 to 7, 9, 10, 12, 13, 16 to 23. 25 to 27 or 31 to 74, wherein said body fluid sample is blood.

77. The method of any one of claims 1 to 76, wherein said level or chemical composition of said GAG or GAG property is determined by electrophoresis or by HPLC and mass spectrometry.

78. The method of claim 77, wherein said electrophoresis is capillary electrophoresis, preferably capillary electophoresis with laser-induced fluorescence detection, and/or wherein HPLC is combined with electrospray ionization-mass spectrometry.

79. The method of any one of claims 3 to 78, wherein the levels of one or more of the specific sulfated or unsulfated forms of CS or HS disaccharides are determined, and wherein the GAGs are subjected to a processing step to obtain the disaccharide units for analysis.

80. The method of any one of claims 1 to 79, wherein said method further comprises a step of treating said cancer by therapy or surgery.

81. A method of screening for cancer in a subject, said method comprising
determining the level in a sample of an expression product of one or more genes selected from the group consisting of:

UST, CHST14, CHST13, CHSY1, DSE, CHSY3, CHST11, CHST15, CHPF2, CSGALNACT2, CHST12, CSGALNACT1, CHST7, CHPF, CHST3, HPSE, HGSNAT, HYAL4, GALNS, GLB1, GNS, GUSB, HEXA, HEXB, HYAL1, IDS, IDUA, ARSB, NAGLU, HPSE2, SGSH, SPAM1, HYAL3, HYAL2, HS3ST3B1, HS3ST3A1, B4GALT7, B3GALT6, B3GAT2, B3GAT3, B3GAT1, XYLT1, XYLT2, EXT1, EXT2, EXTL1, EXTL2, EXTL3, HS3ST5, GLCE, HS6ST3, NDST1, NDST4, HS6ST2, NDST3, HS6ST1, HS2ST1, HS3ST2, HS3ST1 and NDST2;
wherein said sample has been obtained from said subject; and
wherein an altered level in said sample of the expression product of one or more of said genes in comparison to a control level is indicative of cancer in said subject.

82. The method of claim 81, wherein said cancer is selected from the group consisting of prostate cancer, thyroid cancer, colon cancer, rectum cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer, skin cancer and kidney cancer.

83. The method of claim 81 or claim 82, wherein said cancer is selected from the group consisting of prostate cancer, thyroid cancer, colon cancer, rectum cancer, lung cancer, uterine cancer, breast cancer, pancreatic cancer, bladder cancer, liver cancer, bile duct cancer, stomach cancer, oesophageal cancer, head and neck cancer, brain cancer, blood cancer, ovarian cancer and skin cancer.

**Claims**

1. A method of screening for a cancer selected from the group consisting of prostate cancer and bladder cancer in a subject, said method comprising determining the chemical composition of one or more of the glycosaminoglycans (GAGs) chondroitin sulfate (CS) and heparan sulfate (HS) in a urine sample, wherein said urine sample has been obtained from said subject,
   wherein said determination of the chemical composition comprises determining the level in the sample of one or more of the GAG properties selected from the group consisting of: Tris CS, the relative level of 4s CS with respect to 0s CS, Tris HS, 2s HS and Ns2s HS.

2. The method of claim 1, wherein an altered level one or more of said GAG properties in said sample in comparison to a control level is indicative of said cancer in said subject.

3. The method of claim 1 or claim 2, wherein the levels of one or more of the specific sulfated or unsulfated forms of CS or HS disaccharides are determined, and wherein the GAGs are subjected to a processing step to obtain the disaccharide units for analysis.

4. The method of any one of claims 1 to 3, wherein said method comprises determining the level of more than one of said GAG properties.

5. The method of any one of claims 1 to 4, wherein said method comprises determining the level of two or more, or all, of said GAG properties.

6. The method of any one of claims 1 to 5, wherein said method is used for diagnosing said cancer.

7. The method of any one of claims 1 to 5, wherein said method is used for the prognosis of said cancer, for monitoring subjects at risk of the occurrence of said cancer, for monitoring the progression of said cancer in a subject, for determining the clinical severity of said cancer, for predicting the response of a subject to therapy or surgery for said cancer, for determining the efficacy of a therapeutic or surgical regime being used to treat said cancer, for detecting the recurrence or relapse of said cancer, for patient selection or treatment selection or for distinguishing small masses suspicious of said cancer from other non-malignant diseases.

8. The method of any one of claims 1 to 7, wherein said prostate cancer is prostate adenocarcinoma.

9. The method of any one of claims 1 to 8, wherein said subject is a subject at risk of developing said cancer, or at risk of the occurrence of said cancer, or is a subject having or suspected of having said cancer.

10. The method of any one of claims 1 to 9, wherein said level or chemical composition of said GAG or GAG property is determined by electrophoresis or by HPLC and mass spectrometry.

11. The method of claim 10, wherein said electrophoresis is capillary electrophoresis, preferably capillary electophoresis with laser-induced fluorescence detection, and/or wherein said HPLC is combined with electrospray ionization-mass spectrometry.

**Figure 1:**

**Figure 2:**

**Figure 3:**

Figure 4:

**Figure 5:**

BLOOD SCORE
0.628 (0.960, 1.000)
AUC = 0.997

URINE SCORE
0.186 (0.960, 1.000)
AUC = 0.994

COMBINED SCORE
0.472 (1.000, 1.000)
AUC = 1

**Figure 6:**

**Figure 7:**

**Figure 8:**

**Figure 9A**

**Figure 9B**

**Figure 9C**

**Figure 10A**

**Figure 10B**

**Figure 10C**

**Figure 11**

**Figure 12**

**Figure 13**

**Figure 14**

Figure 15.

## Figure 16

**Figure 17:**

**Figure 18:**

**Figure 19:**

**Figure 20:**

**Figure 21:**

**Figure 22:**

**Figure 23:**

**Figure 24:**

**Figure 25:**

**Figure 26:**

**Figure 27:**

**Figure 28:**

Figure 29:

**Figure 30:**

**Figure 31:**

**Figure 32:**

**Figure 33:**

**Figure 34:**

**Figure 35:**

**Figure 36:**

CANCER BLOOD SCORE #2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9950448 A

**Non-patent literature cited in the description**

- **GALEOTTI et al.** *Electrophoresis*, 2014, vol. 35, 811-818
- **KOTTLER et al.** *Electrophoresis*, 2013, vol. 34, 2323-2336
- **VOLPI**. *Curr Pharm Des*, 2006, vol. 12, 639-658
- **VOLPI** ; **LINHARDT**. *Nature protocols*, 2010, vol. 5, 993-1004
- **GALEOTTI** ; **VOLPI**. *Anal Chem*, 2011, vol. 83, 6770-6777
- **VOLPI et al.** *Nature Protocols*, 2014, vol. 9, 541-558
- **VOLPI** ; **MACCARI**. *Analyt Technol Biomed Life Sci*, 2006, vol. 834, 1-13
- **VOLPI** ; **MACCARI**. *Electrophoresis*, 2002, vol. 23, 4060-4066
- **VOLPI** ; **MACCARI**. *Biomacromolecules*, 2005, vol. 6, 3174-3180
- *Clin Chim Acta*, vol. 356, 125-133
- **COPPA et al.** *Glycobiology*, 2011, vol. 21, 295-303
- **SAMBROOK et al.** Molecular cloning: A Laboratory Manual. Cold Spring Harbor Press, 2001
- **COPPA GV** ; **GABRIELLI O** ; **BUZZEGA D** ; **ZAMPINI L** ; **GALEAZZI T** ; **MACCARI F** ; **BERTINO E** ; **VOLPI N**. Composition and structure elucidation of human milk glycosaminoglycans. *Glycobiology*, 2011, vol. 21, 295-303
- **DRAY, S.** ; **DUFOUR, A.B.** The ade4 package: Implementing the duality diagram for ecologists. *Journal of Statistical Software*, 2007, vol. 22, 1-20
- **GATTO, F.** ; **VOLPI, N.** ; **NILSSON, H.** ; **NOOKAEW, I.** ; **MARUZZO, M.** ; **ROMA, A.** ; **JOHANSSON, M.E.** ; **STIERNER, U.** ; **LUNDSTAM, S.** ; **BASSO, U. et al.** Glycosaminoglycan Profiling in Patients' Plasma and Urine Predicts the Occurrence of Metastatic Clear Cell Renal Cell Carcinoma. *Cell Rep*, 2016, vol. 15, 1822-1836
- **TIBSHIRANI, R**. Regression shrinkage and selection via the Lasso. *Journal of the Royal Statistical Society Series B-Methodological*, 1996, vol. 58, 267-288

- **VOLPI N** ; **MACCARI F**. Glycosaminoglycans composition of the largefreshwater mollusc bivalve Anodonta anodonta. *Biomacromolecules*, 2005, vol. 6, 3174-3180
- **VOLPI N** ; **MACCARI F**. Microdetermination of chondroitin sulfate in normal human plasma by fluorophore-assisted carbohydrate electrophore-sis (FACE). *Clin Chim Acta*, 2005, vol. 356, 125-133
- **VOLPI, N.** ; **GALEOTTI, F.** ; **YANG, B.** ; **LINHARDT, R.J.** Analysis of glycosaminoglycan-derived, precolumn, 2-aminoacridone-labeled disaccharides with LC-fluorescence and LC-MS detection. *Nature protocols*, 2014, vol. 9, 541-558
- **VOLPI, N.** ; **LINHARDT, R.J.** High-performance liquid chromatography-mass spectrometry for mapping and sequencing glycosaminoglycan-derived oligosaccharides. *Nature protocols*, 2010, vol. 5, 993-1004
- **WOLF, A.M.** ; **WENDER, R.C.** ; **ETZIONI, R.B.** ; **THOMPSON, I.M.** ; **D'AMICO, A.V.** ; **VOLK, R.J.** ; **BROOKS, D.D.** ; **DASH, C.** ; **GUESSOUS, I.** ; **ANDREWS, K. et al.** American Cancer Society guideline for the early detection of prostate cancer: update 2010. *CA Cancer J Clin*, 2010, vol. 60, 70-98
- **F. GATTO et al.** Glycosaminoglycan Profiling in Patients' Plasma and Urine Predicts the Occurrence of Metastatic Clear Cell Renal Cell Carcinoma. *Cell Rep*, 2016, vol. 15, 1822-1836
- **L. RASKIN et al.** Transcriptome profiling identifies HMGA2 as a biomarker of melanoma progression and prognosis. *J Invest Dermatol*, 2013, vol. 133, 2585-2592
- **S. C. MOK et al.** A gene signature predictive for outcome in advanced ovarian cancer identifies a survival factor: microfibril-associated glycoprotein 2. *Cancer Cell*, 2009, vol. 16, 521-532
- **J. IQBAL et al.** Molecular signatures to improve diagnosis in peripheral T-cell lymphoma and prognostication in angioimmunoblastic T-cell lymphoma. *Blood*, 2010, vol. 115, 1026-1036

- **M. E. RITCHIE et al.** limma powers differential expression analyses for RNA-sequencing and microarray studies. *Nucleic Acids Res*, 2015, vol. 43, e47
- **G. K. SMYTH**. Linear models and empirical bayes methods for assessing differential expression in microarray experiments. *Stat Appl Genet Mol Biol*, 2004, vol. 3 (3)
- **C. W. LAW** ; **Y. CHEN** ; **W. SHI** ; **G. K. SMYTH**. Voom: precision weights unlock linear model analysis tools for RNA-seq read counts. *Genome Biol*, 2014, vol. 15, R29
- **TIBSHIRANI, R.** Regression shrinkage and selection via the Lasso. *Journal of the Royal Statistical Society Series B-Methodological*, 1996, vol. 58, 267-288